(19) Europäisches Patentamt
European Patent Office
Office européen des brevets

(11) EP 1 919 869 B1

(12) FASCICULE DE BREVET EUROPEEN

(45) Date de publication et mention de la délivrance du brevet:
**14.04.2010 Bulletin 2010/15**

(21) Numéro de dépôt: **06808258.5**

(22) Date de dépôt: **29.08.2006**

(51) Int Cl.:
***C07D 209/18*** (2006.01) ***C07D 401/12*** (2006.01)
***C07D 403/12*** (2006.01) ***C07D 405/12*** (2006.01)
***A61K 31/405*** (2006.01) ***A61K 31/4155*** (2006.01)
***A61K 31/4178*** (2006.01) ***A61P 3/00*** (2006.01)
***A61P 25/00*** (2006.01) ***C07D 413/12*** (2006.01)
***C07D 413/14*** (2006.01) ***C07D 417/12*** (2006.01)
***A61K 31/422*** (2006.01) ***A61K 31/427*** (2006.01)
***A61K 31/433*** (2006.01)

(86) Numéro de dépôt international:
**PCT/FR2006/050818**

(87) Numéro de publication internationale:
**WO 2007/026097 (08.03.2007 Gazette 2007/10)**

# (54) DÉRIVÉS DE L'INDOLE ACTIVATEURS DES PPAR

INDOLDERIVATE ALS PPAR-AKTIVATOREN

INDOLE DERIVATIVES AS PPAR ACTIVATORS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **30.08.2005 FR 0508858**

(43) Date de publication de la demande:
**14.05.2008 Bulletin 2008/20**

(73) Titulaire: **LABORATOIRES FOURNIER SA**
**21000 Dijon (FR)**

(72) Inventeurs:
- **BINET, Jean**
**F-21121 Fontaine Les Dijon (FR)**
- **BOUBIA, Benaïssa**
**F-21850 Saint Apollinaire (FR)**
- **DODEY, Pierre**
**F-21121 Fontaine Les Dijon (FR)**
- **LEGENDRE, Christiane**
**F-21370 Velard Sur Ouche (FR)**
- **BARTH, Martine**
**F-21380 Asnieres Les Dijon (FR)**
- **POUPARDIN-OLIVIER, Olivia**
**F-21490 Varois et Chaignot (FR)**

(74) Mandataire: **Hubert, Philippe et al**
**Cabinet Beau de Loménie**
**158, rue de l'Université**
**75340 Paris Cédex 07 (FR)**

(56) Documents cités:
**WO-A-2005/009958 US-B1- 6 288 103**

EP 1 919 869 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne des composés indoliques, leur procédé de fabrication ainsi que leur utilisation en thérapeutique pour la prévention ou le traitement de pathologies impliquant un dysfonctionnement des récepteurs nucléaires de type PPAR.

## Art antérieur

En thérapeutique, il est connu que les maladies du système cardiovasculaire sont un facteur de risque important pour la santé. Ces maladies sont fréquemment la conséquence de taux élevé de cholestérol et/ou de triglycéride et il est donc important de maintenir ces taux inférieurs à des valeurs couramment admises par le corps médical.

Dans le cas du cholestérol, il est en particulier nécessaire d'évaluer les quantités de cholestérol liées aux différentes lipoprotéines afin d'adapter les traitements pour éliminer le cholestérol lié aux LDL. Parmi les familles de composés utilisés pour réguler ces paramètres on connaît les statines, qui sont des inhibiteurs de l'HMG CoA réductase et qui permettent essentiellement de traiter des taux trop élevés en LDL-cholestérol, et les composés de la famille des fibrates, qui agissent en activant les récepteurs nucléaires PPARα (peroxisome proliferator activated receptor alpha) et qui permettent de baisser les taux de triglycérides et de cholestérol.

L'étude des récepteurs nucléaires PPAR a conduit à l'identification de 3 sous-types appelés PPARα, PPARγ et PPARδ. Ces différents récepteurs, en se liant à certains fragments précis de l'ADN, régulent l'expression de gènes cibles qui codent pour des protéines intervenant dans les mécanismes de régulation du métabolisme lipidique.

Ainsi :

- le PPARα est exprimé essentiellement dans le foie et est impliqué dans le catabolisme des acides gras en régulant la β- et la ω-oxydation;
- le PPARδ est exprimé de façon ubiquitaire, mais est présent principalement au niveau des reins, des muscles squelettiques, du coeur et de l'intestin.

Comme les autres récepteurs de type PPAR, le PPARδ forme un hétérodimère avec le RXR (retinoïd X receptor) et est alors capable de se lier à certains éléments des gènes cibles du noyau et contrôler les facteurs de transcription. Parmi les différentes études consacrées à ce récepteur nucléaire, il a été par exemple démontré que l'activation du PPARδ permet d'augmenter le taux de HDL-cholesterol chez la souris db/db (FEBS letters (2000), 473, 333-336) et le singe rhésus obèse insulino-dépendant et favorise l'efflux de cholestérol via l'Apo A1 dans les cellules THP-1-humaines, (Proc. Nat. Ac. Sci. USA (2001), 98, 5306-5311).

Suite à l'étude de ces différents récepteurs nucléaires, il apparaît que des composés qui sont capables d'activer soit les récepteurs PPAR α, soit les récepteurs PPAR δ, soit encore simultanément ces deux récepteurs, pourraient présenter un profil pharmacologique extrêmement intéressant pour traiter des pathologies telles que les hyperlipidémies, les hypercholestérolémies ainsi que les différentes maladies du système cardiovasculaire qui sont la conséquence d'un syndrome métabolique.

Parmi les documents de l'art antérieur citant de tels composés, on connaît par exemple le document WO 97/28149 qui décrit des agonistes des récepteurs PPAR δ, le document WO 01/60807 qui décrit des agonistes des récepteurs PPAR α ou encore les documents WO 05/009958 et WO06/060535 qui proposent des composés de l'indole actifs sur les récepteurs PPAR..

On citera encore les documents WO 02/071827 et Bioorg. Med. Chem. Lett., 14 (11) p.2759-2763 (06/2004), qui décrivent des dérivés modulateurs des récepteurs RXR et leur utilisation en thérapeutique pour traiter les pathologies impliquées dans le syndrome métabolique.

Par ailleurs, divers composés indoliques ont été décrits dans l'art antérieur. Ainsi :

- les documents WO 00/46196 et WO 99/07678 divulguent des composés dérivés de l'acide indole-2-carboxylique pour leur activité anti-inflammatoire ;
- le document WO 98/41092 décrit des dérivés d'indole-2-carboxamide actifs contre la douleur.

## Objet de l'invention

La présente invention concerne des composés dérivés de l'indole qui sont des activateurs des PPAR, et sont choisis parmi

i) les composés de formule :

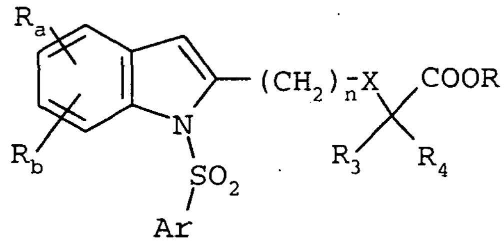

(I)

dans laquelle :

$R_a$ et $R_b$ représentent chacun indépendamment un atome d'hydrogène, un atome d'halogène, un groupe alkyle en $C_1$-$C_6$, $CF_3$, CN, CO-$R_2$, $OR_2$ ou un groupe phényle éventuellement substitué par un groupe alkyle en $C_1$-$C_4$ ou $CF_3$ ;
$R_2$ représente un groupe alkyle en $C_1$-$C_4$, $CF_3$ ou phényle éventuellement substitué par un groupe alkyle en $C_1$-$C_4$ ou $CF_3$ ;
$R_3$ et $R_4$ représentent chacun indépendamment un atome d'hydrogène ou un groupe alkyle en $C_1$-$C_4$
R représente un atome d'hydrogène ou un groupe alkyle en $C_1$-$C_3$ ;
n= 1, 2, ou 3 ;
X représente une liaison simple, un atome d'oxygène ou un atome de soufre.
Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les groupes alkyle en $C_1$-$C_6$, phényle, $CF_3$, CN, CO-$R_2$, $OR_2$, $SR_2$, NH-$COR_2$, morpholinyle, amino ou 4-morpholinosulfonyle,

ii) leurs sels pharmaceutiquement acceptables.

Des composés préférés selon l'invention sont les composés de formule (I) précitée dans laquelle l'une au moins des conditions suivantes est réalisée :

- l'un au moins de $R_a$ et $R_b$ est différent d'un atome d'hydrogène.
- Ar représente un groupe phényle ou hétéroaromatique azoté choisi parmi les groupes quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 1,2,3,4-tétrahydroquinolinyle, 1,2,3,4-tétrahydroisoquinolinyle, 1,2,3,4-tétrahydro-2-oxoquinolinyle, indolyle, 2,3-dihydroindolyle ou benzoxazolyle, éventuellement substitué par un ou plusieurs des atomes ou groupe d'atomes choisis parmi les atomes d'halogène, les groupes alkyle en $C_1$-$C_6$, $CF_3$, CN, CO-$R_2$, $OR_2$, $SR_2$, NH-$COR_2$, morpholinyle, amino ou 4-morpholinosulfonyle ;
  n est égal à 1 ou 2.

Une première famille particulière de composés selon l'invention est constituée des composés de formule (I) dans laquelle X représente un atome d'oxygène ainsi que leurs sels pharmaceutiquement acceptables.

Une seconde famille particulière de composés selon l'invention est constituée des composés de formule I dans laquelle X représente une liaison simple et l'un au moins de $R_3$ et $R_4$ représente un groupe alkyle en $C_1$-$C_4$, ainsi que leurs sels pharmaceutiquement acceptables.

Une troisième famille particulière de composés selon l'invention est constituée des composés de formule I dans laquelle X représente une liaison simple, $R_3$ et $R_4$ représentent un atome d'hydrogène ; ainsi que leurs sels pharmaceutiquement acceptables.

Selon un deuxième aspect, l'invention concerne les composés précités pour leur utilisation en tant que substances pharmacologiquement actives, ainsi que les compositions pharmaceutiques les contenant.

En outre, l'invention concerne l'utilisation d'au moins un composé de formule (I) ou l'un de ses sels pharmaceutiquement acceptable en tant que principe actif pour la préparation d'un médicament destiné à une utilisation en

thérapeutique, notamment pour lutter contre les hypercholestérolémies, les hyperlipidémies, les hypertriglycéridémies, les dyslipidémies, l'insulinorésistance, le diabète ou l'obésité ainsi que les maladies cardiovasculaires qui sont la conséquence d'un déséquilibre des lipoprotéines sériques. Les composés selon l'invention sont également utiles comme principes actifs de médicaments destinés à prévenir ou traiter les maladies liées à un dysfonctionnement endothélial, l'athérosclérose, l'infarctus du myocarde, l'hypertension, les problèmes cérébrovasculaires, certaines maladies inflammatoires comme par exemple l'arthrite rhumatoïde, et les neuro-dégénérescences comme notamment la maladie d'Alzheimer ou la maladie de Parkinson.

**Description détaillée**

Dans la présente description, on entend par groupe alkyle en $C_1$-$C_n$ (n étant un nombre entier) une chaîne hydrocarbonée ayant de 1 à n atomes de carbone linéaire, ramifiée ou cyclique. Par exemple et sans limitation, un groupe alkyle en $C_1$-$C_6$ peut être un groupe linéaire ou ramifié de formule générale $C_nH_{2n+1}$ tel que méthyle, éthyle, propyle, butyle, pentyle, hexyle, 1-méthyléthyle, 1-méthylpropyle, 2-méthylpropyle, 1,1-diméthyléthyle, 1-méthylbutyle, 1,1-diméthylpropyle, 1-méthylpentyle, 1,1-diméthylbutyle ou un groupe comportant un cycle, de formule générale $C_nH_{2n-1}$ tel que cyclopropyle, cyclobutyle, cyclopentyle, cyclohexyle ou cyclopentylméthyle. Par halogène, on entend un atome de fluor, chlore, brome ou iode, les atomes de fluor et de chlore étant préférés.

Les composés de formule (I) dans laquelle R représente un atome d'hydrogène sont des acides carboxyliques qui peuvent être utilisés sous la forme d'acides libres ou sous la forme de sels, lesdits sels étant obtenus par combinaison de l'acide avec une base minérale ou organique non toxique pharmaceutiquement acceptable. Parmi les bases minérales, on peut utiliser par exemple les hydroxydes de sodium, de potassium, de magnésium ou de calcium. Parmi les bases organiques, on peut utiliser par exemple les amines, les aminoalcools, des acides aminés basiques tels que la lysine ou l'arginine ou encore des composés porteurs d'une fonction ammonium quaternaire tels que par exemple la bétaïne ou la choline.

Les composés de formule (I) dans laquelle les substituants $R_3$ et $R_4$ sont différents présentent un centre d'asymétrie. Pour ces composés, l'invention couvre aussi bien le composé racémique que chacun des isomères optiques considérés séparement.

Parmi les composés selon l'invention, on préfère ceux dans lesquels Ar représente un groupe phényle ou un hétérocycle azoté. On préfère également les composés dans lesquels $R_a$ représente un atome d'halogène ou un groupe trifluorométhyle, ainsi que ceux dans lesquels n est égal à 1 ou 2.

Les composés selon l'invention peuvent être préparés selon un premier procédé consistant à :

a) faire réagir selon la réaction dite de SONOGASHIRA (voir par exemple : Tet. Lett., 1975, 4467), un composé de formule

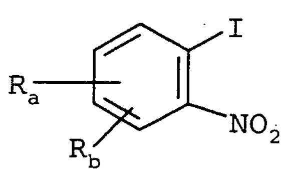

(II)

dans laquelle :

$R_a$ et $R_b$ représentent chacun indépendamment un atome d'hydrogène, de fluor, de chlore ou de brome, un groupe alkyle en $C_1$-$C_6$, $CF_3$, CN, CO-$R_2$ ou $OR_2$ ;

$R_2$ représente un groupe alkyle en $C_1$-$C_4$, $CF_3$ ou phényle éventuellement substitué par un groupe alkyle en $C_1$-$C_4$ ou $CF_3$ ;

avec un dérivé acétylénique de formule

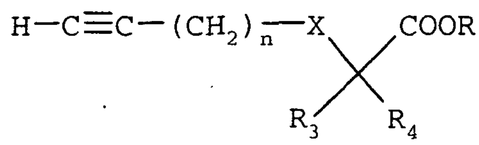

(III)

dans laquelle :

n = 1, 2, ou 3 ;
$R_3$ et $R_4$ représentent chacun indépendamment un atome d'hydrogène ou un groupe alkyle en $C_1$-$C_4$ ;
R représente un groupe alkyle en $C_1$-$C_3$ ;
X représente une liaison simple, un atome d'oxygène ou un atome de soufre ; en présence d'iodure cuivreux, d'un catalyseur à base de palladium tel que par exemple le tetrakis(triphénylphosphine) palladium et d'une base organique comme par exemple la triéthylamine, dans un solvant comme par exemple le diméthylformamide (DMF) à une température comprise entre 0 et 60 °C pendant 2 à 24 heures, pour obtenir le composé de formule dans laquelle :

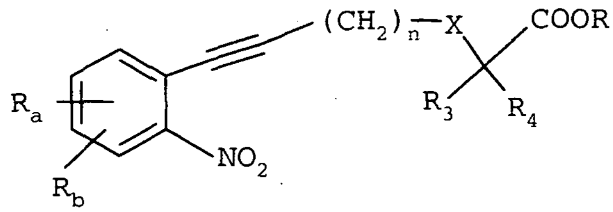

(IV)

$R_a$, $R_b$, n, X, $R_3$, $R_4$ et R conservent la même signification que dans les composés de départ ;

b) effectuer une réduction du groupe « nitro » porté par le composé de formule IV ci-dessus, par exemple par action de chlorure stanneux en présence d'éthanol et dans un solvant tel que par exemple l'acétate d'éthyle, à une température proche de la température ambiante et pendant 1 à 24 heures, pour obtenir l'aniline de formule

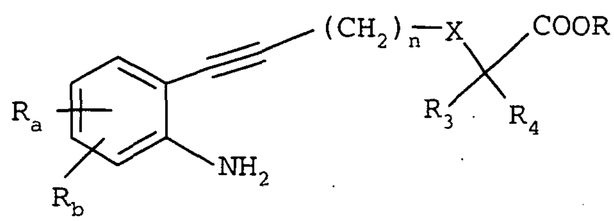

(V)

dans laquelle :

$R_a$, $R_b$, n, X, $R_3$, $R_4$ et R conservent la même signification que dans le composé de départ ;

c) faire réagir le composé de formule V avec un chlorure d'arylsulfonyle de formule

$$Ar-SO_2-Cl \quad (VI)$$

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les groupes alkyle en $C_1$-$C_6$, phényle, $CF_3$, CN, CO-$R_2$, $SR_2$, $OR_2$, NH-$COR_2$, morpholinyle, amino ou 4-morpholinosulfonyle
en présence de pyridine, à température ambiante, pendant 10 à 120 mn, pour obtenir le composé de formule

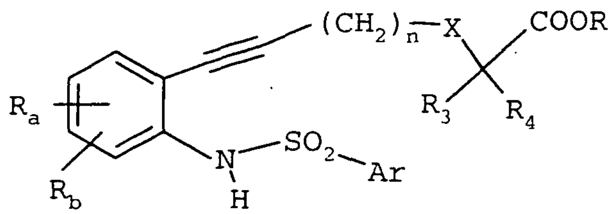

(VII)

dans laquelle :

$R_a$, $R_b$, n, X, $R_3$, $R_4$, R et Ar conservent la même signification que dans les composés de départ ;

d) effectuer une cyclisation du composé de formule VII, par exemple par action de l'acétate de cuivre II (voir par exemple J. Org. Chem., 2004, 69 (4), 1126-1136), dans un solvant tel que le 1,2-dichloroéthane à une température proche de la température de reflux du solvant, pendant 4 à 24 heures, pour obtenir le composé de formule

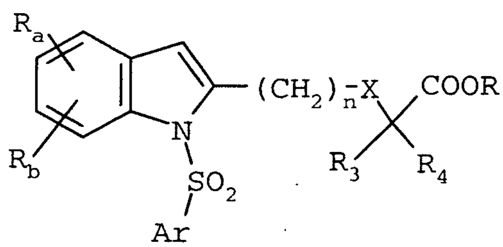

(Ia)

dans laquelle :

$R_a$, $R_b$, n, X, $R_3$, $R_4$, R et Ar conservent la même signification que dans les composés de départ ;

e) si nécessaire, hydrolyser la fonction ester du composé de formule Ia, par exemple par action d'une base minérale telle que 1a soude ou la lithine selon des modes opératoires bien connus de l'homme du métier, pour obtenir, après traitement acide, le composé de formule I sous sa forme d'acide libre :

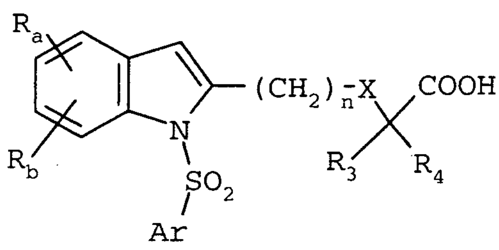

(Ib)

Selon une première variante du procédé de préparation, les composés de formule I peuvent être obtenus par une série de réactions consistant à :

a) effectuer une réaction d'halogénation, préférentiellement une iodation, d'une aniline de formule

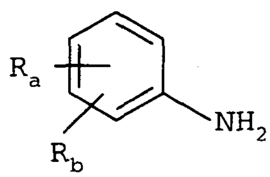

(VIII)

dans laquelle :

$R_a$ et $R_b$ représentent chacun indépendamment un atome d'hydrogène, un atome d'halogène, un groupe alkyle en $C_1$-$C_6$, $CF_3$, CN, CO-$R_2$ ou $OR_2$,
$R_2$ représente un groupe alkyle en $C_1$-$C_4$, $CF_3$, phényle éventuellement substitué par un groupe alkyle en $C_1$-$C_4$ ou $CF_3$,
à l'aide d'un agent halogénant tel que par exemple le dichloroiodate de benzyltriméthylammonium, dans un solvant tel que le dichlorométhane ou le méthanol, à température ambiante, pendant 5 à 24 heures pour obtenir le composé de formule

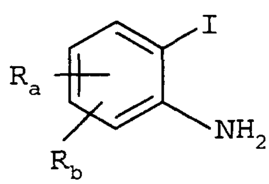

(IX)

dans laquelle :

$R_a$ et $R_b$ conservent la même signification que dans les composés de départ ;

b) faire réagir le composé de formule IX avec un dérivé acétylénique de formule

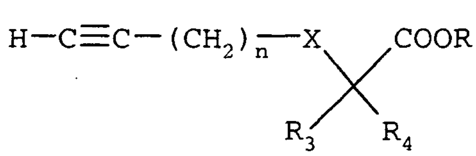

(III)

dans laquelle :

n= 1, 2, ou 3 ;
$R_3$ et $R_4$ représentent chacun indépendamment un atome d'hydrogène ou un groupe alkyle en $C_1$-$C_4$ ;
R représente un groupe alkyle en $C_1$-$C_3$ ;
X représente une liaison simple, un atome d'oxygène ou un atome de soufre, dans des conditions analogues à celles décrites pour l'étape a) du procédé général précédent,
pour obtenir le composé de formule

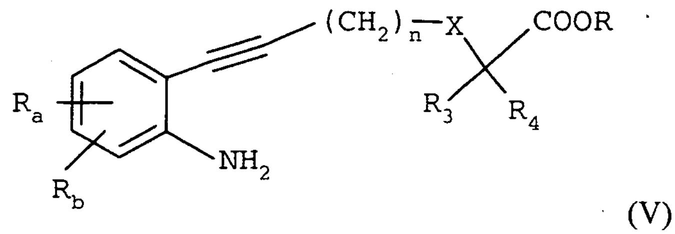
(V)

dans laquelle :

$R_a$, $R_b$, n, X, $R_3$, $R_4$ et R conservent la même signification que dans le composé de départ ;

c) cycliser le composé de formule V ci-dessus, dans des conditions analogues à celles décrites pour réaliser l'étape (d) du procédé général précédent, pour obtenir le composé indolique de formule

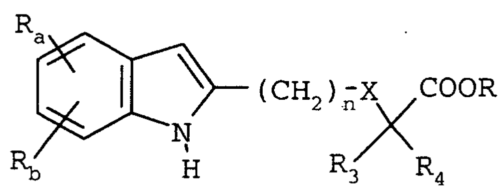
(X)

dans laquelle :

$R_a$, $R_b$, n, X, $R_3$, $R_4$ et R conservent la même signification que dans le composé de départ;

d) faire réagir le composé de formule (X) ci-dessus avec un chlorure de arylsulfonyle de formule

$$Ar—SO_2—Cl \quad (VI)$$

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les groupes alkyle en $C_1$-$C_6$, phényle, $CF_3$, CN, CO-$R_2$, $SR_2$, $OR_2$, NH-

$COR_2$, morpholinyle, amino ou 4-morpholinosulfonyle,
dans un solvant tel que par exemple le diméthylformamide, à température ambiante et pendant 1 à 12 heures, généralement après activation des composés indolique de formule (X) par l'hydrure de sodium, pour obtenir le composé de formule (Ia)

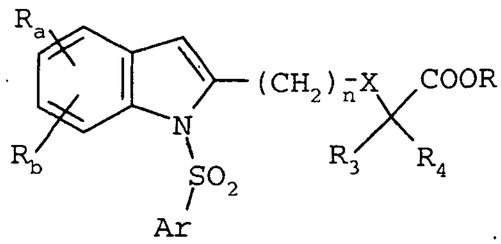
(Ia)

dans laquelle :

$R_a$, $R_b$, n, X, $R_3$, $R_4$, R et Ar conservent la même signification que dans les composés de départ ;

e) si nécessaire, hydrolyser la fonction ester du composé de formule Ia, par exemple par action d'une base minérale telle que la soude ou la lithine selon des modes opératoires bien connus de l'homme du métier, pour obtenir, après traitement acide, le composé de formule I sous sa forme d'acide libre :

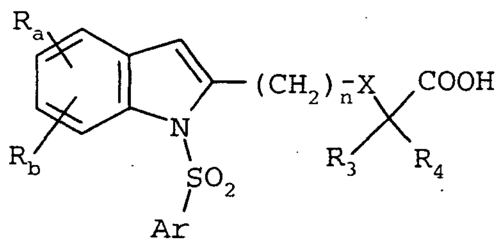
(Ib)

Selon une seconde variante du procédé de préparation, les composés de formule I peuvent être obtenus par une série de réactions consistant à :

a) faire réagir le composé de formule IX

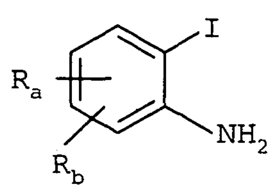
(IX)

dans laquelle :

$R_a$ et $R_b$ représentent chacun indépendamment un atome d'hydrogène, de fluor, de chlore ou de brome, un groupe alkyle en $C_1$-$C_6$, $CF_3$, CN, CO-$R_2$ ou $OR_2$ ;
$R_2$ représente un groupe alkyle en $C_1$-$C_4$, $CF_3$ ou phényle éventuellement substitué par un groupe alkyle en $C_1$-$C_4$ ou $CF_3$,
avec un chlorure de arylsulfonyle de formule

$$Ar-SO_2-Cl \quad (VI)$$

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les groupes alkyle en $C_1$-$C_6$, phényle, $CF_3$, CN, CO-$R_2$, $OR_2$, $SR_2$, NH-$COR_2$, morpholinyle, amino ou 4-morpholinosulfonyle,
dans un solvant tel que par exemple le diméthylformamide, à température ambiante et pendant 1 à 12 heures, , pour obtenir le composé de formule

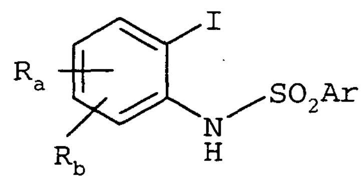

(XI)

dans laquelle :

$R_a$ , $R_b$ et Ar conservent la même signification que dans les composés de départ,

b) faire réagir le composé de formule XI avec un dérivé acétylénique de formule

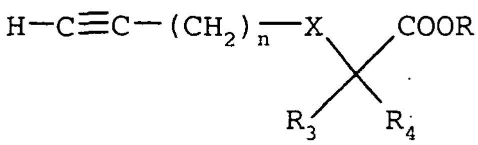

(III)

dans laquelle :

n= 1, 2, ou 3 ;
$R_3$ et $R_4$ représentent chacun indépendamment un atome d'hydrogène ou un groupe alkyle en $C_1$-$C_4$ ;
R représente un groupe alkyle en $C_1$-$C_3$ ,
X représente une liaison simple, un atome d'oxygène ou un atome de soufre, dans des conditions analogues à celles décrites pour l'étape a) du procédé général précédent,
pour obtenir le composé de formule

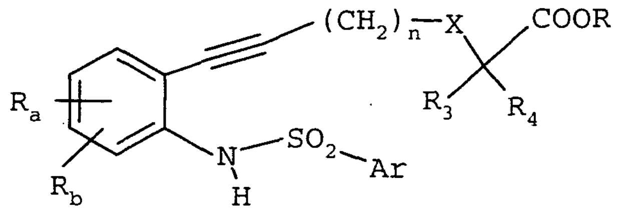

(VII)

dans laquelle $R_a$, $R_b$, n, X, $R_3$, $R_4$, R et Ar conservent la même signification que dans les composés de départ,
c) cycliser le composé de formule VII ci-dessus, dans des conditions analogues à celles décrites pour réaliser l'étape (d) du procédé général précédent, pour obtenir le composé indolique de formule

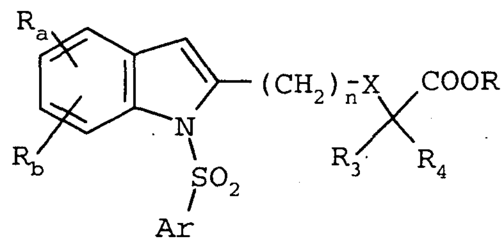

(Ia)

dans laquelle :

$R_a$, $R_b$, n, X, $R_3$, $R_4$, R et Ar conservent la même signification que dans les composés de départ,

d) si nécessaire, hydrolyser la fonction ester du composé de formule Ia, par exemple par action d'une base minérale telle que la soude ou la lithine selon des modes opératoires bien connus de l'homme du métier, pour obtenir, après traitement acide, le composé de formule I sous sa forme d'acide libre :

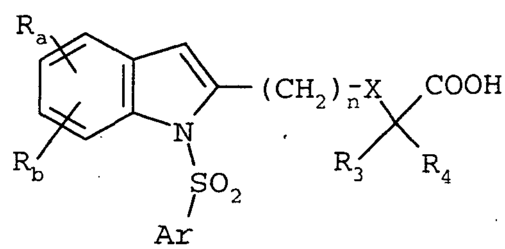

(Ib)

Au cours de ce dernier procédé, il est possible de réaliser les deux étapes b) et c) en une seule opération.
Les composés de formule I selon l'invention dans laquelle Ra (ce procédé convient également pour $R_b$) représente un noyau phényle éventuellement substitué peuvent être obtenus au départ du composé halogéné de formule

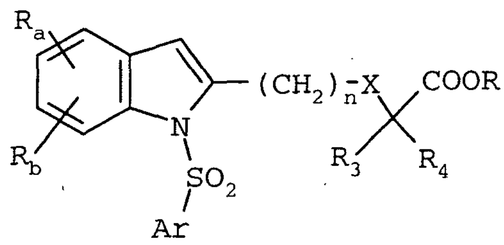

(Ic)

dans laquelle :

$R_a$ représente un atome d'halogène, préférentiellement l'atome de brome, Rb représente un atome d'hydrogène, un atome de fluor ou de chlore, un groupe alkyle en $C_1$-$C_6$, $CF_3$, CN, CO-$R_2$ ou $OR_2$ ;
$R_2$ représente un groupe alkyle en $C_1$-$C_4$, $CF_3$ ou phényle éventuellement substitué par un groupe alkyle en $C_1$-$C_4$ ou $CF_3$ ;
$R_3$ et $R_4$ représentent chacun indépendamment un atome d'hydrogène ou un groupe alkyle en $C_1$-$C_4$ ;
R représente un groupe alkyle en $C_1$-$C_3$ ;
n = 1, 2, ou 3 ;
X représente une liaison simple, un atome d'oxygène ou un atome de soufre,
Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes de chlore ou de fluor, les groupes alkyle en $C_1$-$C_6$, phényle, $CF_3$, CN, CO-$R_2$, $OR_2$, $SR_2$, NH-$COR_2$, morpholinyle, amino ou 4-morpholinosulfonyle ;
avec un acide phénylboronique de formule :

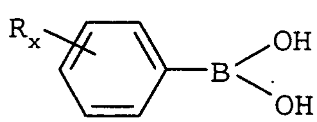

(XII)

dans laquelle :

$R_x$ représente un atome d'hydrogène ou un groupe alkyle en $C_1$-$C_4$ ou $CF_3$ ; selon une réaction dite de SUZUKI (voir par exemple Chem. Rev.,1995, 95, 2457) en présence de tétrakis(triphénylphosphine)palladium et d'une base comme par exemple le carbonate de sodium, dans un solvant tel que par exemple un mélange de tétrahydrofurane, méthanol et eau, à une température comprise entre 30 °C et la température de reflux du solvant, pendant 5 à 24 heures pour obtenir le composé de formule Id

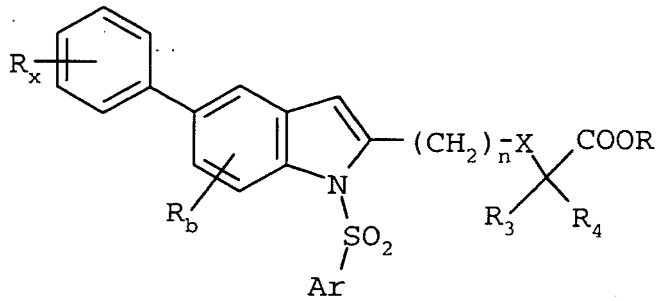

(Id)

dans laquelle :

R, $R_b$, $R_x$, X, $R_3$, $R_4$, n et Ar conservent la même signification que dans les composés de départ.

Les composés de l'invention sous forme de sels d'un acide de formule Ib avec une base minérale ou organique, peuvent être obtenus de façon classique, en utilisant les méthodes bien connues de l'homme de métier, par exemple en mélangeant des quantités stoechiométriques de l'acide et de la base dans un solvant, tel que par exemple l'eau ou un mélange hydroalcoolique, et en lyophilisant ensuite la solution obtenue.

Dans certaines des étapes réactionnelles décrites ci-dessus, il est possible de remplacer avantageusement les méthodes de chauffage traditionnelles bien connues de l'homme de métier, par un chauffage au moyen de micro-ondes en utilisant des réacteurs adaptés à ce mode de réaction. Dans ce cas, l'homme du métier comprendra que les durées de "chauffage" seront considérablement réduites, par comparaison aux durées nécessaires avec un chauffage classique.

Les exemples suivants de préparation de composés selon la formule (I) permettront de mieux comprendre l'invention.

Dans ces exemples, qui ne sont pas limitatifs de la portée de l'invention, on désigne par « préparation » les exemples décrivant la synthèse de composés intermédiaires et par « exemples » ceux décrivant la synthèse de composés de formule (I) selon l'invention. Parmi les abréviations, « mM » signifie millimole. Les points de fusion sont mesurés au banc Kofler ou à l'aide d'un appareil Mettler et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, dd pour doublet dédoublé, t pour triplet, q pour quadruplet, quin pour quintuplet, m pour multiplet). La fréquence de travail et le solvant utilisé sont indiqués pour chaque composé. La température ambiante est de 20°C ± 5°C. Dans certains cas, la structure des composés a été confirmée par spectroscopie de masse après chromatographie en phase liquide (couplage LC/MS) ; les mesures ont été faites sur colonne UPTISPHERE HDO avec phase HDO (colonne 50 x 2 mm x 3µm), débit 0,6 ml/mn (split 1/3), phase mobile : A = $H_2O$ + 0,5 % TFA (acide trifluoroacétique), B = Acétonitrile + 0,5 % TFA (programmation du gradient : B = 10 à 90 % en 7 mn, puis palier à 90 % pendant 2 mn, puis retour à 10 % en 1 mn et stabilisation à 10 % pendant 3 mn ; température de travail = 45 °C ; détection par UV de 210 à 260 nm. Le spectre de masse est obtenu par ionisation ESI+, spray à 3500 V, température du bloc source à 130 °C, désolvatation à 230 °C et gaz à 600 1/h, gaz de cône à 100 1/h et tension à 10V/30V/60V. Le résultat est exprimé par la masse (m/z) et par le temps de rétention (Tr).

## PREPARATION 1

### Acide 5-(5-chloro-2-nitrophényl)-4-pentynoïque, méthyl ester

On mélange 35,5 g (125 mM) de 4-chloro-2-iodo-1-nitrobenzène, 510 ml de triéthylamine, 2,88 g (2,5 mM) de tétrakis(triphénylphosphine)palladium, 0,72 g d'iodure cuivreux et 50 ml de diméthylformamide (DMF). On ajoute ensuite sous agitation, à température ambiante, 14 g (125 mM) d'ester méthylique de l'acide 4-pentynoïque et le mélange réactionnel est agité pendant 24 heures à température ambiante. On ajoute 100 ml de toluène et chasse les solvants sous pression réduite. Le résidu d'évaporation est repris par 150 ml d'acétate d'éthyle et 80 ml d'acide chlorhydrique N. La phase organique est séparée et lavée à l'eau puis séchée sur sulfate de magnésium et concentrée sous pression réduite. L'huile brune obtenue est purifiée par chromatographie sur gel de silice en éluant à l'aide d'un mélange cyclohexane/acétate d'éthyle (9/1 ; v/v). On obtient ainsi 21,5 g du produit attendu sous forme d'un solide jaune (rendement : 65 %).
F = 75-78 °C.

**PREPARATION 2**

**Acide 5-(2-amino-5-chlorophényl)-4-pentynoïque, méthyl ester**

On charge dans un ballon 90,6 g (400 mM) de chlorure stanneux, 70 ml d'acétate d'éthyle et 22 ml d'éthanol. Ce mélange est agité pendant 15 mn à température ambiante puis on ajoute lentement une solution de 21,5 g (80 mM) du composé obtenu selon la préparation 1. Le mélange réactionnel est agité pendant 24 heures à température ambiante puis versé sur un mélange de 200 g de glace et 200 ml de soude N. Le mélange obtenu est extrait 2 fois avec 200 ml d'acétate d'éthyle ; les phases organiques rassemblées sont lavées à l'eau, séchées sur sulfate de magnésium et concentrées sous pression réduite. L'huile obtenue est purifiée par chromatographie sur gel de silice en éluant à l'aide d'un mélange cyclohexane/acétate d'éthyle (80/20 ; v/v). On obtient ainsi 9,1 g du composé attendu sous forme d'un solide jaune orangé (rendement = 30 %).
F = 67 °C.

**PREPARATION 3**

**Acide [5-chloro-2-(phénylsulfonylamino)-phényl]-4-pentynoïque, méthyl** ester

On prépare une solution de 1,2 g (5 mM) du composé obtenu selon la préparation 2 dans 15 ml de pyridine et on ajoute 0,77 ml (6 mM) de chlorure de benzènesulfonyle. Le mélange est agité pendant 1 heure à température ambiante puis concentré sous pression réduite. L'huile résiduelle est purifié par chromatographie sur gel de silice en éluant à l'aide d'un mélange cyclohexane/acétate d'éthyle (8/2 ; v/v). On obtient ainsi 1,8 g du composé attendu sous forme d'un solide beige (rendement = 95 %).
$^1$H RMN (DMSO$d_6$, 300 MHz) $\delta$ = 2,56 (s, 4H), 3,65 (s, 3H), 7,28-7,36 ( m, 3H), 7,54-7,72 (m, 5H), 9,69 (s, 1H).

**Exemple 1**

**Acide 5-chloro-1-(phénylsulfonyl)-1*H*-indole-2-propanoïque, méthyl ester**

On prépare une solution de 300 mg (0,79 mM) d'ester obtenu selon la préparation 3 dans 35 ml de 1,2-dichloroéthane et on ajoute 15 mg (0,08 mM) d'acétate de cuivre (cuivrique) et on porte le mélange à reflux sous agitation pendant 24 heures. On élimine ensuite le solvant sous pression réduite et purifie le solide visqueux résiduel par chromatographie sur gel de silice en éluant avec un mélange toluène/acétate d'éthyle (9/1 ; v/v). On obtient 230 mg du composé obtenu sous forme d'un solide jaune (rendement = 77 %). F = 93-96 °C.

**Exemple 2**

**Acide 5-chloro-1-(phénylsulfonyl)-1*H*-indole-2-propanoïque**

On mélange 180 mg (0,46 mM) d'ester obtenu selon l'exemple 1 avec 16 ml de THF et 4 ml d'eau, et on ajoute 20 mg (0,48 mM) d'hydroxyde de lithium (LiOH, 1 $H_2O$) Le mélange est agité pendant 3 heures à température ambiante, puis concentré sous pression réduite. Le résidu d'évaporation est repris dans 10 ml d'eau et la solution est acidifiée par une solution d'acide chlorhydrique 1N. Le précipité blanc est extrait par l'acétate d'éthyle et la phase organique séparée est séchée sur sulfate de magnésium et concentrée sous pression réduite. On obtient ainsi 160 mg du produit attendu sous forme d'un solide jaune (rendement = 93 %).
F = 165-168 °C.

**Exemple 2a**

**Acide 5-chloro-1-(phénylsulfonyl)-1*H*-indole-2-propanoïque, sel de sodium**

On mélange 114 mg (0,313 mM) d'acide obtenu selon l'exemple 2 avec 10 ml d'eau et 0,313 ml d'une solution de soude N. On ajoute sous agitation quelques gouttes de méthanol afin d'obtenir une solution. Le mélange est agité 15 mn à température ambiante puis concentré partiellement sous pression réduite. La solution résiduelle est ensuite filtrée et lyophilisée. On obtient ainsi 115 mg du sel attendu sous forme d'une poudre fine blanche (rendement = 95%).
F ≥ 250 °C.

**PREPARATION 4**

**2-iodo-4-(trifluorométhyl)aniline .**

On prépare une solution de 5 g (31 mM) de 4-(trifluorométhyl)aniline dans 90 ml de méthanol et 30 ml de dichlorométhane et on ajoute 3,56 g (35,6 mM) de carbonate de calcium. On ajoute ensuite par fraction, sous agitation et à température ambiante, 14,9 g (42,7 mM) de dichloroiodure de triméthylbenzyl-ammonium. Le milieu réactionnel est agité pendant 24 heures à température ambiante, puis filtré afin d'éliminer les sels minéraux. Le filtrat est concentré sous pression réduite et le produit brut est purifié par chromatographie sur gel de silice en éluant à l'aide d'un mélange cyclohexane/acétate d'éthyle (8/2 ; v/v). On obtient ainsi 6,65 g du composé attendu sous forme d'une huile orange (rendement = 75 %).
$^1$H RMN ($CDCl_3$, 300 MHz) $\delta$ = 5,0 (s, 2H), 6,82 (d, *J* = 5,5 Hz, 1H), 7,38 (dd, *J* = 5,5 Hz, 1,3 Hz, 1H), 7,79 (d, J = 1,3 Hz, 1H).

**PREPARATION 5**

**Acide 5-[2-amino-5-(trifluorométhyl)phényl]-4-pentynoïque, méthyl ester**

On prépare une solution de 1,5 g (5,23 mM) du composé obtenu selon la préparation 4, 0,644 g (5,75 mM) de 4-pentynoate de méthyle et 90 mg (0,13 mM) de dichloro-bis(triphénylphosphine) palladium dans 1 ml de diméthylformamide et 2 ml de diéthylamine, et on ajoute 50 mg (0,26 mM) d'iodure cuivreux. Le mélange réactionnel est irradié au four micro-ondes à 120 °C pendant 10 minutes. Les solvants sont ensuite chassés sous pression réduite et le résidu d'évaporation est purifié par chromatographie sur gel de silice en éluant à l'aide d'un mélange cyclohexane/acétate d'éthyle (8/2 ; v/v). On obtient ainsi 1,16 g du composé attendu sous forme d'une huile orange (rendement = 82 %).
$^1$H RMN ($DMSOd_6$, 300 MHz) $\delta$ = 2,65-2,75 (m, 4H), 3,64 (s, 3H), 5,99 (s, 2H), 6,78 (d, *J* = 8,3Hz, 1H), 7,31 (d, *J* = 8,3 Hz, 1H), 7,33 (s, 1H).

**PREPARATION 6**

**Acide 5-(trifluorométhyl)-1*H*-indole-2-propanoïque, méthyl ester**

On prépare une solution de 1,16 g (4,28 mM) d'ester obtenu selon la préparation 5 dans 5 ml de 1,2-dichloréthane et on ajoute 1,3 g (6,4 mM) d'acétate cuivrique. Le mélange réactionnel est irradié au four micro-ondes à 150 °C pendant 30 minutes, puis refroidi et filtré. Le filtrat est concentré sous pression réduite. On obtient ainsi 1 g du composé attendu sous forme d'un solide brun (rendement = 86 %).
F = 106-108 °C.

**Exemple 3**

**Acide 1-(phénylsulfonyl)-5-(trifluorométhyl)-1*H*-indole-2-propa-noïque, méthyl ester**

On ajoute 0,18 g (4,6 mM) d'hydrure de sodium (à 60 % dans l'huile) à une solution de 1g (3,69 mM) d'ester obtenu selon la préparation 6, à 0 °C. Ce mélange est agité pendant 15 mn et on ajoute, toujours à 0 °C, 0,98 g (5,5 mM) de chlorure de benzènesulfonyle. Le mélange est agité pendant 30 mn à température ambiante, puis on ajoute 100 ml d'une solution de chlorure d'ammonium à 15 % dans l'eau. Le mélange est extrait 3 fois par 50 ml de dichlorométhane. Les phases organiques rassemblées sont séchées sur sulfate de magnésium et concentrées sous pression réduite. L'huile résiduelle est purifiée par chromatographie sur gel de silice en éluant à l'aide d'un mélange cyclohexane/acétate d'éthyle (8/2 ; v/v). On obtient ainsi 0,95 g du produit attendu sous forme d'une huile qui cristallise en étoiles orangées (rendement = 62 %). F = 81-83 °C.

**Exemple 4**

**Acide 1-(phénylsulfonyl)-5-(trifluorométhyl)-1*H*-indole-2-propanoïque**

En opérant de façon analogue à l'exemple 2, au départ du composé obtenu selon l'exemple 3, on obtient le produit attendu sous forme d'un solide beige (rendement = 85 %).
F = 170-172 °C.

**PREPARATION 7**

**Acide 5-(2-amino-5-bromophényl)-4-pentynoïque, méthyl ester**

En opérant de façon analogue à la préparation 5, au départ de 4-bromo-2-iodoaniline, on obtient le composé attendu sous forme d'une huile jaune (rendement = 23 %).
$^1$H RMN ($DMSOd_6$, 250 MHz) $\delta$ = 2,61-2,74 (m, 4H), 3,63 (s, 3H), 5,46 (s, 2H), 6,63 (dd, *J* = 8,3 Hz, 0,7 Hz, 1H), 7,1-7,2 (m, 2H).

**PREPARATION 8**

**Acide 5-bromo-1*H*-indole-2-propanoïque, méthyl ester**

En opérant de façon analogue à la préparation 6, au départ du composé obtenu selon la préparation 7, on obtient le composé attendu sous forme d'un solide brun (rendement = 98 %).
$^1$H RMN ($DMSOd_6$, 250 MHz) $\delta$ = 2,75 (t, *J* = 7,3 Hz, 2H), 2,98 (t, *J* = 7,2 Hz, 2H) , 3,60 (s, 3H), 6,14 (s, 1H), 7,1 (dd, *J* = 8,5 Hz, 1,9 Hz,1H), 7,2 (d, *J* = 8,5 Hz, 1H), 7,58 (d, *J* = 1,9 Hz, 1H), 11,1 (s, 1H).

**Exemple 5**

**Acide 5-bromo-1-(phénylsulfonyl)-1*H*-indole-2-propanoïque, méthyl ester**

En opérant de façon analogue à l'exemple 3, au départ du composé obtenu selon la préparation 8, on obtient le composé attendu sous forme d'un solide marron clair (rendement = 25 %).
F = 109-113 ˚C.

**Exemple 6**

**Acide 5-bromo-1-(phénylsulfonyl)-1*H*-indole-2-propanoïque**

En opérant de façon analogue à l'exemple 2, au départ du composé obtenu selon l'exemple 5, on obtient le composé attendu sous forme d'un solide beige (rendement = 81 %).
F = 188-190 ˚C

**Exemple 7**

**Acide 1-(phénylsulfonyl)-5-[4-(trifluorométhyl)phényl]-1*H*-indole2-propanoïque, méthyl ester**

On prépare une solution de 0,5 g (1,18 mM) d'ester obtenu selon l'exemple 5 et 0,68 g (0,59 mM) de tétrakis-(triphénylphosphine)palladium dans 5 ml de THF et on ajoute 0,84 g (4,4 mM) d'acide 4-(trifluorométhyl)phénylboronique en solution dans 2,5 ml de méthanol, puis 282 mg (2,6 mM) de carbonate de sodium en solution dans 1 ml d'eau. Le mélange est ensuite maintenu sous agitation à température de reflux du solvant pendant 24 heures. Après retour à température ambiante, le mélange est dilué avec 20 ml de dichlorométhane et séché sur sulfate de magnésium. La solution obtenue est concentrée sous pression réduite et le résidu d'évaporation est repris en solution dans 50 ml d'éther éthylique. La solution obtenue est lavée 3 fois par 15 ml d'une solution de soude 1N, puis à l'eau jusqu'à neutralité, séchée sur sulfate de magnésium et concentrée sous pression réduite. L'huile résiduelle est purifiée par chromatographie sur gel de silice en éluant à l'aide d'un mélange cyclohexane/acétate d'éthyle (85/15 ; v/v). On obtient ainsi 57 mg du composé attendu sous forme d'une huile beige (rendement = 10 %).
$^1$H RMN ($DMSOd_6$, 250 MHz) $\delta$ = 2,84 (t, *J* = 7,1 Hz, 2H), 3,32 (d, *J* = 7,2 Hz, 2H), 3,62 (s, 3H), 6,68 (s, 1H), 7,56-7,71 (m, 5H), 7,78-7,91 (m, 6H), 8,13 (d, 1H).

**Exemple 8**

**Acide 1-(phénylsulfonyl)-5-[4-(trifluorométhyl)phényl]-1*H*-indole2-propanoïque**

En opérant de façon analogue à l'exemple 2, au départ du composé obtenu selon l'exemple 7, on obtient le produit attendu sous forme d'un solide jaune (rendement = 76 %).
F = 162 ˚C.

**PREPARATION 9**

**Acide 5-(2-nitrophényl)-4-pentynoïque, méthyl ester.**

En opérant de façon analogue à la préparation 1, au départ de 1-iodo-2-nitrobenzène, on obtient le produit attendu sous forme d'un solide jaune (rendement = 53 %).
F = 44-46 °C.

**PREPARATION 10**

**Acide 6-(5-chloro-2-nitrophényl)-5-hexynoïque, méthyl ester.**

En opérant de façon analogue à la préparation 1, au départ d'ester méthylique de l'acide 5-hexynoïque, on obtient le produit attendu sous forme d'une huile brune (rendement = 73 %).
$^1$H RMN (DMSO$d_6$, 250 MHz) δ = 1,81 (m, 2H), 2,54 (m, 4H), 3,60 (s, 3H), 7,69 (dd, 1H), 7,81 (d, 1H), 8,09 (d, 1H).

**PREPARATION 11**

**Acide 7-(5-chloro-2-nitrophényl)-6-heptynoïque, méthyl ester.**

En opérant de façon analogue à la préparation 1, au départ d'ester méthylique de l'acide 6-heptynoïque, on obtient le produit attendu sous forme d'une huile brune (rendement = 98 %).
$^1$H RMN (DMSO$d_6$, 250 MHz) δ = 1,34-1,73 (m, 4H), 2,36 (t, 2H), 2,53 (t, 2H), 3,59 (s, 3H), 7,66 (dd, 1H), 7,79 (d, 1H), 8,09 (d, 1H).

**PREPARATION 12**

**Acide 5-(2-aminophényl)-4-pentynoïque, méthyl ester.**

En opérant de façon analogue à la préparation 2, au départ du composé obtenu selon la préparation 9, on obtient le produit attendu sous forme d'une huile incolore (rendement = 53 %).
$^1$H RMN (DMSO$d_6$, 250 MHz) δ = 2,67 (m, 4H), 3,63 (s, 3H), 5,25 (s, 2H), 6,46 (m, 1H), 6,65 (dd, 1H), 7,02 (m, 2H).

**PREPARATION 13**

**Acide 6-(2-amino-5-chlorophényl)-5-hexynoïque, méthyl ester.**

En opérant de façon analogue à la préparation 2, au départ du composé obtenu selon la préparation 10, on obtient le produit attendu sous forme d'un solide jaune (rendement = 41 %).
$^1$H RMN (DMSO$d_6$, 250 MHz) δ = 1,83 (m, 2H), 2,48 (m, 4H), 3,59 (s, 3H), 5,40 (s, 2H), 6,67 (d, 1H), 7,03 (dd, 1H), 7,08 (d, 1H).

**PREPARATION 14**

**Acide 7-(2-amino-5-chlorophényl)-6-heptynoïque, méthyl ester.**

En opérant de façon analogue à la préparation 2, au départ du composé obtenu selon la préparation 11, on obtient le produit attendu sous forme d'un solide jaune (rendement = 68 %).
F = 66°C.

**PREPARATION 15**

**Acide 5-(2-amino-5-fluorophényl)-4-pentynoïque, méthyl ester.**

En opérant de façon analogue à la préparation 5, au départ de la 4-fluoro-2-iodo-aniline, on obtient le produit attendu sous forme d'un solide marron (rendement = 45 %).
$^1$H RMN (DMSO$d_6$, 300 MHz) δ = 2,6-2,75 (m, 4H), 3,63 (s, 3H), 5,16 (s, 2H), 6,6-6,7 (m, 1H), 6,8-6,85 (m, 2H).

**PREPARATION 16**

**Acide 5-(2-amino-4,5-dichlorophényl)-4-pentynoïque, méthyl ester.**

En opérant de façon analogue à la préparation 5, au départ de la 4,5-dichloro-2-iodo-aniline, on obtient le produit attendu sous forme de cristaux beiges (rendement = 87 %).
$^1$H RMN (DMSO$d_6$, 300 MHz) δ = 2,65-2,75 (m, 4H), 3,63 (s, 3H), 5,70 (s, 2H), 6,87 (s, 1H), 7,24 (s, 1H).

**PREPARATION 17**

**Acide 5-(2-amino-5,6-dichlorophényl)-4-pentynoïque, méthyl ester.**

En opérant de façon analogue à la préparation 5, au départ de la 3,4-dichloro-2-iodo-aniline, on obtient le produit attendu sous forme d'une huile incolore (rendement = 49 %).
$^1$H RMN (DMSO$d_6$, 300 MHz) δ = 2,6-2,8 (m, 4H), 3,63 (s, 3H), 5,74 (s, 2H), 6,65 (d, 1H), 7,20 (d, 1H).

**PREPARATION 18**

**Acide 5-[2-amino-4-(trifluorométhyl)phényl]-4-pentynoïque, méthyl ester.**

En opérant de façon analogue à la préparation 5, au départ de la 5-(trifluorométhyl)-2-iodo-aniline, on obtient le produit attendu sous forme de cristaux orange (rendement = 71 %).
F = 42°C.

**PREPARATION 19**

**Acide 5-(2-amino-5-acétylphényl)-4-pentynoïque, méthyl ester.**

En opérant de façon analogue à la préparation 5, au départ de la 4-amino-3-iodoacétophénone, on obtient le produit attendu sous forme d'un solide jaune (rendement = 39 %).
$^1$H RMN (DMSO$d_6$, 300 MHz) δ = 2,39 (s, 3H), 2,65-2,75 (m, 4H), 3,64 (s, 3H), 6,15 (s, 2H), 6,69 (d, 1H), 7,64 (dd, 1H), 7,69 (d, 1H).

**PREPARATION 20**

**Acide 5-(2-amino-4-chloro-5-fluorophényl)-4-pentynoïque, méthyl ester.**

En opérant de façon analogue à la préparation 5, au départ de 5-chloro-4-fluoro-2-iodoaniline, on obtient le produit attendu sous forme de cristaux marron (rendement = 81 %).
F = 67-68 °C.

**PREPARATION 21**

**Acide 5-(2-amino-5-cyanophényl)-4-pentynoïque, méthyl ester.**

En opérant de façon analogue à la préparation 5, au départ de 4-amino-3-iodobenzonitrile, on obtient le produit attendu sous forme d'une huile incolore (rendement = 52 %).
$^1$H RMN (DMSO$d_6$, 300 MHz) δ = 2,65-2,75 (m, 4H), 3,63 (s, 3H), 6,27 (s, 2H), 6,73 (d, 1H), 7,38 (dd, 1H), 7,46 (d, 1 Hz, 1H).

**PREPARATION 22**

**Acide 5-(2-amino-5-benzoylphényl)-4-pentynoïque, méthyl ester.**

En opérant de façon analogue à la préparation 5, au départ de 4-amino-3-iodobenzophénone, on obtient le produit attendu sous forme d'une huile jaune (rendement = 54 %).
$^1$H RMN (DMSO$d_6$, 300 MHz) δ =2,63-2,73 (m, 4H), 3,62 (s, 3H), 6,28 (s, 2H), 6,75 (d, 1H), 7,47-7,61 (m, 7H).

**PREPARATION 23**

**Acide 5-(2-amino-3,5-dichlorophényl)-4-pentynoïque, méthyl ester.**

En opérant de façon analogue à la préparation 5, au départ de 2,4-dichloro-6-iodoaniline, on obtient le produit attendu sous forme d'une huile foncée (rendement = 87 %).
$^1$H RMN ($DMSOd_6$, 300 MHz) δ = 2,6-2,8 (m, 4H), 3,64 (s, 3H), 5,55 (s, 2H), 7,13 (d, 1H), 7,33 (d, 1H).

**PREPARATION 24**

**Acide 5-[2-[(phénylsulfonyl)amino]phényl]-4-pentynoïque, méthyl ester.**

En opérant de façon analogue à la préparation 3, au départ du composé obtenu selon la préparation 12, on obtient le produit attendu sous forme d'une huile incolore (rendement = 82%).
$^1$H RMN ($DMSOd_6$, 300 MHz) δ = 2,57 (s, 4H), 3,65 (s, 3H), 7,13 (m, 1H), 7,22-7,28 (m, 3H), 7,52-7,62 (m, 3H), 7,71 (dd, 2H), 9,49 (s, 1H).

**PREPARATION 25**

**Acide 5-[5-chloro-2-[[(4-méthylphényl)sulfonyl]amino]phényl]-4-pentynoïque, méthyl ester.**

En opérant de façon analogue à la préparation 3, au départ du chlorure de p-toluènesulfonyle, on obtient le produit attendu sous forme d'une huile jaune (rendement = 85 %).
$^1$H RMN ($DMSOd_6$, 250 MHz) δ = 2,35 (s, 3H), 2,56 (s, 4H), 3.65 (s, 3H), 7,31 (m, 5H), 7,59 (d, 2H), 9,57 (s, 1H).

**PREPARATION 26**

**Acide 5-[5-chloro-2-[[(2,3-dichlorophényl)sulfonyl]amino]phényl]-4-pentynoïque, méthyl ester.**

En opérant de façon analogue à la préparation 3, au départ du chlorure de 2,3-dichlorobenzènesulfonyle, on obtient le produit attendu sous forme d'un solide jaune (rendement = 85 %).
F = 64°C.

**PREPARATION 27**

**Acide 5-[5-chloro-2-[[(3-méthylphényl)sulfonyl]amino]phényl]-4-pentynoïque, méthyl ester.**

En opérant de façon analogue à la préparation 3, au départ du chlorure de m-toluènesulfonyle, on obtient le produit attendu sous forme d'un solide beige (rendement = 82 %).
F = 69°C.

**PREPARATION 28**

**Acide 5-[5-chloro-2-[[(2,4-dichlorophényl)sulfonyl]amino]phényl]-4-pentynoïque, méthyl ester.**

En opérant de façon analogue à la préparation 3, au départ du chlorure de 2,4-dichlorobenzènesulfonyle, on obtient le produit attendu sous forme d'une huile incolore (rendement = 96 %).
$^1$H RMN ($DMSOd_6$, 300 MHz) δ = 2,56 (s, 4H), 3,64 (s, 3H), 7,27 (dd, *J* = 8,4, 0,78 Hz, 1H), 7,36 (m, 2H), 7,58 (dd, 1H), 7,84 (d, 1H), 7,89 (s, 1H), 10,06 (s, 1H).

**PREPARATION 29**

**Acide 5-[5-chloro-2-[[[4-(trifluorométhyl)phényl]sulfonyl]amino]phényl]-4-pentynoïque, méthyl ester.**

En opérant de façon analogue à la préparation 3, au départ du chlorure de 4-(trifluorométhyl)benzènesulfonyle, on obtient le produit attendu sous forme d'un solide rose (rendement = 50 %).
F = 80°C.

**PREPARATION 30**

**Acide 5-[5-chloro-2-[[(4-méthoxyphényl)sulfonyl]amino]phényl]-4-pentynoïque, méthyl ester.**

En opérant de façon analogue à la préparation 3, au départ du chlorure de 4-méthoxybenzènesulfonyle, on obtient le produit attendu sous forme d'une huile jaune (rendement = 84 %).
$^1$H RMN (DMSO$d_6$, 300 MHz) δ = 2,58 (s, 4H), 3,65 (s, 3H), 3,80 (s, 3H), 7,05 (m, 2H), 7,29 (m, 2H), 7,36 (dd, 1H), 7,63 (m; 2H), 9,47 (s, 1H).

**PREPARATION 31**

**Acide 5-[5-chloro-2-[[(4-acétylphényl)sulfonyl]amino]phényl]-4-pentynoïque, méthyl ester.**

En opérant de façon analogue à la préparation 3, au départ du chlorure de 4-acétylbenzènesulfonyle, on obtient le produit attendu sous forme d'un solide beige (rendement = 59 %).
F = 88°C.

**PREPARATION 32**

**Acide 5-[2-[([1,1'-biphényl]-4-ylsulfonyl)amino]-5-chlorophényl]-4-pentynoïque, méthyl ester.**

En opérant de façon analogue à la préparation 3, au départ du chlorure de (1,1'-biphényl]-4-ylsulfonyle, on obtient le produit attendu sous forme d'un solide beige (rendement = 81 %).
F = 93°C.

**PREPARATION 33**

**Acide 5-[5-chloro-2-[[[2-(trifluorométhyl)phényl]sulfonyl]amino]phényl]-4-pentynoïque, méthyl ester.**

En opérant de façon analogue à la préparation 3, au départ du chlorure de 2-(trifluorométhyl)benzènesulfonyle, on obtient le produit attendu sous forme d'une huile orange (rendement = 73 %).
$^1$H RMN (DMSO$d_6$, 250 MHz) δ = 2,56 (s, 4H), 3,63 (s, 3H), 7,27-7,42 (m, 3H), 7,83 (m, 2H), 7,96 (m, 2H), 9,95 (s, 1H).

**PREPARATION 34**

**Acide 5-[5-chloro-2-[[(3-méthoxyphényl)sulfonyl]amino]phényl]-4-pentynoïque, méthyl ester.**

En opérant de façon analogue à la préparation 3, au départ du chlorure de 3-méthoxybenzènesulfonyle, on obtient le produit attendu sous forme d'une huile jaune (rendement = 86 %).
$^1$H RMN (DMSO$d_6$, 300 MHz) δ = 2,56 (s, 4H), 3,65 (s, 3H), 3,76 (s, 3H), 7,12-7,31 (m, 4H), 7,37 (dd, 1H), 7,44 (m, 1H), 7,69 (s, 1H), 9,69 (s, 1H).

**PREPARATION 35**

**Acide 5-[5-chloro-2-[[(2,5-diméthoxyphényl)sulfonyl]amino]phényl]-4-pentynoïque, méthyl ester.**

En opérant de façon analogue à la préparation 3, au départ du chlorure de 2,5-diméthoxybenzènesulfonyle, on obtient le produit attendu sous forme d'un solide jaune (rendement = 92 %).
LC/MS : m/z= 438 ; Tr = 5,97 mn.

**PREPARATION 36**

**Acide 5-[5-chloro-2-[[[4-(1,1-diméthyléthyl)phényl]sulfonyl]amino]phényl]-4-pentynoïque, méthyl ester.**

En opérant de façon analogue à la préparation 3, au départ du chlorure de 4-*tert*-butylbenzènesulfonyle, on obtient le produit attendu sous forme d'une huile incolore (rendement = 88 %).
LC/MS : m/z= 434 ; Tr = 6,89 mn.

**PREPARATION 37**

**Acide 5-[5-chloro-2-[[(4-éthylphényl)sulfonyl]amino]phényl]-4-pentynoïque, méthyl ester.**

En opérant de façon analogue à la préparation 3, au départ du chlorure de 4-éthylbenzènesulfonyle, on obtient le produit attendu sous forme d'une huile jaune (rendement = 37 %).
LC/MS : m/z= 406 ; Tr = 6,40 mn.

**PREPARATION 38**

**Acide 5-[5-chloro-2-[[[4-(1-méthyléthyl)phényl]sulfonyl]amino]phényl]-4-pentynoïque, méthyl ester.**

En opérant de façon analogue à la préparation 3, au départ du chlorure de 4-isopropyllbenzènesulfonyle, on obtient le produit attendu sous forme d'une huile orange (rendement = 92 %).
LC/MS : m/z= 420 ; Tr = 6,95 mn.

**PREPARATION 39**

**Acide 5-[5-chloro-2-[[(4-propylphényl)suifonyl]amino]phényl]-4-pentynoïque, méthyl ester.**

En opérant de façon analogue à la préparation 3, au départ du chlorure de 4-propylbenzènesulfonyle, on obtient le produit attendu sous forme d'une huile orange (rendement = 22 %).
LC/MS : m/z= 420 ; Tr = 6,92 mn.

**PREPARATION 40**

**Acide 5-[5-chloro-2-[[(4-pentylphényl)sulfonyl]amino]phényl]-4-pentynoïque, méthyl ester.**

En opérant de façon analogue à la préparation 3, au départ du chlorure de 4-pentylbenzènesulfonyle, on obtient le produit attendu sous forme d'une huile orange (rendement = 88 %).
LC/MS : m/z= 448 ; Tr = 7,61 mn.

**PREPARATION 41**

**Acide 5-[5-chloro-2-[[(3,5-diméthylphényl)sulfonyl]amino]phényl]-4-pentynoïque, méthyl ester.**

En opérant de façon analogue à la préparation 3, au départ du chlorure de 3,5-diméthylbenzènesulfonyle, on obtient le produit attendu sous forme d'une huile orange (rendement = 92 %).
LC/MS : m/z= 406 ; Tr = 6,70 mn.

**PREPARATION 42**

**Acide 5-[5-chloro-2-[[(2,4,6-triméthylphényl)sulfonyl]amino]phényl]-4-pentynoïque, méthyl ester.**

En opérant de façon analogue à la préparation 3, au départ du chlorure de 2,4,6-triméthylbenzènesulfonyle, on obtient le produit attendu sous forme d'une huile orange (rendement = 23 %).
LC/MS : m/z= 420 ; Tr = 6,66 mn.

**PREPARATION 43**

**Acide 5-[5-chloro-2-[[(4-chlorophényl)sulfonyl]amino]phényl]-4-pentynoïque, méthyl ester.**

En opérant de façon analogue à la préparation 3, au départ du chlorure de 4-chlorobenzènesulfonyle, on obtient le produit attendu sous forme d'une huile orange (rendement = 56 %).
LC/MS : m/z= 412 ; Tr = 6,27 mn.

**PREPARATION 44**

**Acide 5-[5-chloro-2-[[(4-fluorophényl)sulfonyl]amino]phényl]-4-pentynoïque, méthyl ester.**

En opérant de façon analogue à la préparation 3, au départ du chlorure de 4-fluorobenzènesulfonyle, on obtient le produit attendu sous forme d'une huile orange (rendement = 28 %).
LC/MS : m/z= 396 ; Tr = 5,99 mn.

**PREPARATION 45**

**Acide 5-[5-chloro-2-[[(4-chloro-3-méthylphényl)sulfonyl] amino]phényl]-4-pentynoïque, méthyl ester.**

En opérant de façon analogue à la préparation 3, au départ du chlorure de 4-chloro-3-méthylbenzènesulfonyle, on obtient le produit attendu sous forme d'une huile jaune (rendement = 59 %).
LC/MS : m/z= 426 ; Tr = 6,80 mn.

**PREPARATION 46**

**Acide 5-[5-chloro-2-[[[3-(trifluorométhyl)phényl]sulfonyl] amino]phényl]-4-pentynoïque, méthyl ester.**

En opérant de façon analogue à la préparation 3, au départ du chlorure de 3-(trifluorométhyl)benzènesulfonyle, on obtient le produit attendu sous forme d'une huile orange (rendement = 50 %).
LC/MS : m/z= 446 ; Tr = 6,58 mn.

**PREPARATION 47**

**Acide 5-[2-[[[4-(acétylamino)phényl]sulfonyl]amino]-5-chlorophényl]-4-pentynoïque, méthyl ester.**

En opérant de façon analogue à la préparation 3, au départ du chlorure de 4-(acétylamino)benzènesulfonyle, on obtient le produit attendu sous forme d'un solide orange (rendement = 74 %).
LC/MS : m/z= 435 ; Tr = 5,15 mn.

**PREPARATION 48**

**Acide 5-[5-chloro-2-[[(4-cyanophényl)sulfonyl]amino]phényl]-4-pentynoïque, méthyl ester.**

En opérant de façon analogue à la préparation 3, au départ du chlorure de 4-cyanobenzènesulfonyle, on obtient le produit attendu sous forme d'un solide jaune (rendement = 87 %).
LC/MS : m/z= 403 ; Tr = 5,71 mn.

**PREPARATION 49**

**Acide 5-[5-chloro-2-[[(4-phénoxyphényl)sulfonyl]amino]phényl]-4-pentynoïque, méthyl ester.**

En opérant de façon analogue à la préparation 3, au départ du chlorure de 4-phénoxybenzènesulfonyle, on obtient le produit attendu sous forme d'un solide jaune (rendement = 71 %).
LC/MS : m/z= 470 ; Tr = 6,67 mn.

**PREPARATION 50**

**Acide 5-[5-chloro-2-[(1-naphtalènylsulfonyl)amino]phényl]-4-pentynoïque, méthyl ester.**

En opérant de façon analogue à la préparation 3, au départ du chlorure de 1-naphtalènesulfonyle, on obtient le produit attendu sous forme d'une huile brune (rendement = 89 %).
$^1$H RMN (DMSO$d_6$, 300 MHz) δ = 2,15 (t, 2H), 2,44 (t, 2H), 3,63 (s, 3H), 7,18 (s, 1H), 7,34 (s, 2H), 7,62 (m, 3H), 8,01 (dd, 1H), 8,04 (d, 1H), 8,22 (d, 1H), 8,72 (d, 1H), 10,01 (s, 1H).

**PREPARATION 51**

**Acide 5-[5-chloro-2-[(2-naphtalènylsulfonyl)amino]phényl]-4-pentynoïque, méthyl ester.**

En opérant de façon analogue à la préparation 3, au départ du chlorure de 2-naphtalènesulfonyle, on obtient le produit attendu sous forme d'une huile incolore (rendement = 22%).
LC/MS : m/z= 428 ; Tr = 6,63 mn.

**PREPARATION 52**

**Acide 5-[5-chloro-2-[[(4-méthyl-1-naphtalènyl)sulfonyl]amino]phényl]-4-pentynoïque, méthyl ester.**

En opérant de façon analogue à la préparation 3, au départ du chlorure de 4-méthyl-1-naphtalènesulfonyle, on obtient le produit attendu sous forme d'une huile jaune (rendement = 77 %).
$^{1}$H RMN (DMSO$d_6$, 250 MHz) δ = 2,24 (t, 2H), 2,37 (t, 2H), 2,70 (s, 3H), 3,64 (s, 3H), 7,25 (s, 1H), 7,33 (s, 2H), 7,45 (dd, 1H), 7,68 (m, 2H), 7,93 (d, 1H), 8,15 (m, 1H), 8,75 (m, 1H), 9,91 (s, 1H).

**PREPARATION 53**

**Acide 5-[2-[[[5-(acétylamino)-1-naphtalènyl]sulfonyl]amino] 5-chloro phényl]-4-pentynoïque, méthyl ester.**

En opérant de façon analogue à la préparation 3, au départ du chlorure de 5-(acétylamino)-1-naphtalènesulfonyle, on obtient le produit attendu sous forme d'une huile incolore (rendement = 94 %).
$^{1}$H RMN (DMSO$d_6$, 300 MHz) δ = 2,18 (s, 3H), 2,23 (t, 2H), 2,41(t, 2H), 3,62 (s, 3H), 7,18 (s, 1H), 7,32 (s, 2H), 7,62 (m, 2H), 7,76 (d, 1H), 8,04 (dd, 1H), 8,35 (d, 1H), 8,58 (d, 1H), 10,03 (s, 1H).

**PREPARATION 54**

**Acide 5-[5-chloro-2-[(8-quinolinylsulfonyl)amino]phényl]-4-pentynoïque, méthyl ester.**

En opérant de façon analogue à la préparation 3, au départ du chlorure de 8-quinoleïnesulfonyle, on obtient le produit attendu sous forme d'une huile jaune (rendement = 76 %).
LC/MS : m/z= 429 ; Tr = 5,96 mn.

**PREPARATION 55**

**Acide 6-[5-chloro-2-[(phénylsulfonylamino)phényl]-5-hexynoïque, méthyl ester.**

En opérant de façon analogue à la préparation 3, au départ du composé obtenu selon la préparation 13, on obtient le produit attendu sous forme d'un solide orange (rendement = 66 %).
F = 90˚C.

**PREPARATION 56**

**Acide 6-[5-chloro-2-[[(2,3-dichlorophényl)sulfonyl]amino]phényl]-5-hexynoïque, méthyl ester.**

En opérant de façon analogue à la préparation 55, au départ du chlorure de 2,3-dichlorobenzènesulfonyle, on obtient le produit attendu sous forme d'une huile jaune (rendement = 87 %).
$^{1}$H RMN (DMSO$d_6$, 250 MHz) δ =1,72 (m, 2H), 2,35 (t, 2H), 2,43 (t, 2H), 3,60 (s, 3H), 7,25-7,36 (m, 2H), 7,39 (s; 1H), 7,48 (t, 1H), 7,79 (d, 1H), 7,90 (d, 1H), 10,28 (s, 1H).

**PREPARATION 57**

**Acide 6-[5-chloro-2-[[(4-méthoxyphényl)sulfonyl]amino]phényl]-5-hexynoïque, méthyl ester.**

En opérant de façon analogue à la préparation 55, au départ du chlorure de 4-méthoxybenzènesulfonyle, on obtient le produit attendu sous forme d'une huile jaune (rendement = 43 %).
$^{1}$H RMN (DMSO$d_6$, 250 MHz) δ = 1,73 (m, 2H), 2,35-2,48 (m, 4H), 3,62 (s, 3H), 3,81 (s, 3H), 7,04 (d, 2H), 7,25-7,35

(m, 3H), 7,60 (d, 2H), 9,57 (s, 1H).

**PREPARATION 58**

**Acide 6-[5-chloro-2-[(8-quinolinylsulfonyl)amino]phényl]-5-hexynoïque, méthyl ester.**

En opérant de façon analogue à la préparation 55, au départ du chlorure de 8-quinoleïnesulfonyle, on obtient le produit attendu sous forme d'une poudre blanche (rendement = 48 %).
$^1$H RMN ($DMSOd_6$, 300 MHz) $\delta$ = 1,77 (m, 2H), 2,44 (m, 4H), 3,61 (s, 3H), 7,29 (s, 2H), 7,43 (d, 1H), 7,75 (m, 2H), 8,32 (d, 1H), 8,39 (d, 1H), 8,55 (d, 1H), 9,01 (s, 1H), 9,08 (d, 1H).

**PREPARATION 59**

**Acide 6-[5-chloro-2-[[[4-(1-méthyléthyl)phényl]sulfonyl]amino]phényl]-5-hexynoïque, méthyl ester.**

En opérant de façon analogue à la préparation 55, au départ du chlorure de 4-isopropylbenzènesulfonyle, on obtient le produit attendu sous forme d'une huile orange (rendement = 86 %).
$^1$H RMN ($DMSOd_6$, 300 MHz) $\delta$ = 1,20 (d, 6H), 1,71 (m, 2H), 2,34 (t, 2H), 2,41 (t, 2H), 2,73 (m, 1H), 3,61 (s, 3H), 7,26-7,62 (m, 5H), 9,71 (s, 1H).

**PREPARATION 60**

**Acide 6-[5-chloro-2-[(2-naphtalènylsulfonyl)amino]phényl]-5-hexynoïque, méthyl ester.**

En opérant de façon analogue à la préparation 55, au départ du chlorure de 2-naphtalènesulfonyle, on obtient le produit attendu sous forme d'une huile jaune (rendement = 69 %).
$^1$H RMN ($DMSOd_6$, 300 MHz) $\delta$ = 1,59 (m, 2H), 2,20 (t, *J* = 7,1 Hz, 2H), 2,32, (t, *J* = 7,4 Hz, 2H), 3,61 (s, 3H), 7,08-7,34 (m, 3H), 7,63-7,74 (m, 3H), 7,99-8,33 (m, 3H), 9,91 (s, 1H).

**PREPARATION 61**

**Acide 6-[5-chloro-2-[[(3,5-diméthylphényl)sulfonyl]amino]phényl]-5-hexynoïque, méthyl ester.**

En opérant de façon analogue à la préparation 55, au départ du chlorure de 3,5-diméthylbenzènesulfonyle, on obtient le produit attendu sous forme d'une poudre blanche (rendement = 71 %).
F = 92-94 °C.

**PREPARATION 62**

**Acide 6-[5-chloro-2-[[(3-méthoxyphényl)sulfonyl]amino]phényl]-5-hexynoïque, méthyl ester.**

En opérant de façon analogue à la préparation 55, au départ du chlorure de 3-méthoxybenzènesulfonyle, on obtient le produit attendu sous forme d'une poudre jaune (rendement = 82 %).
F = 71-76°C.

**PREPARATION 63**

**Acide 6-[5-chloro-2-[[(2,5-diméthoxyphényl)sulfonyl]amino]phényl]-5-hexynoïque, méthyl ester.**

En opérant de façon analogue à la préparation 55, au départ du chlorure de 2,5-diméthoxybenzènesulfonyle, on obtient le produit attendu sous forme d'une poudre blanche (rendement = 80 %).
F = 115-117°C.

**PREPARATION 64**

**Acide 6-[5-chloro-2-[(1-naphtalènylsulfonyl)amino]phényl]-5-hexynoïque, méthyl ester.**

En opérant de façon analogue à la préparation 55, au départ du chlorure de 1-naphtalènesulfonyle, on obtient

le produit attendu sous forme d'une poudre jaune (rendement = 81 %).
F = 93-95 °C.

### PREPARATION 65

**Acide 5-fluoro-1*H*-indole-2-propanoïque, méthyl ester.**

En opérant de façon analogue à la préparation 6, au départ du composé obtenu selon la préparation 15, on obtient le produit attendu sous forme d'un solide beige (rendement = 71 %).
$^1$H RMN ($DMSOd_6$, 300 MHz) $\delta$ = 2,75 (t, 2H), 2,98 (t, 2H), 3,61 (s, 3H), 6,14 (dd, 1H), 6,82 (ddd, 1H), 7,15 (dd, 1H), 7,25 (dd, 4,68 Hz, 1H), 11,02 (s, 1H).

### PREPARATION 66

**Acide 5,6-dichloro-1*H*-indole-2-propanoïque, méthyl ester.**

En opérant de façon analogue à la préparation 6, au départ du composé obtenu selon la préparation 16, on obtient le produit attendu sous forme d'un solide marron (rendement = 100 %).
F = 142°C.

### PREPARATION 67

**Acide 4,5-dichloro-1*H*-indole-2-propanoïque, méthyl ester.**

En opérant de façon analogue à la préparation 6, au départ du composé obtenu selon la préparation 17, on obtient le produit attendu sous forme d'un solide marron (rendement = 90 %).
$^1$H RMN ($DMSOd_6$, 300 MHz) $\delta$ = 2,78 (t, 2H), 3,01 (t, 2H), 3,61 (s, 3H), 6,24 (s, 1H), 7,17 (d, 1H), 7,29 (d, 1H) 11,49 (s, 1H).

### PREPARATION 68

**Acide 6-(trifluorométhyl)-1*H*-indole-2-propanoïque, méthyl ester.**

En opérant de façon analogue à la préparation 6, au départ du composé obtenu selon la préparation 18, on obtient le produit attendu sous forme d'un solide beige (rendement = 91 %).
F = 108-110 °C.

### PREPARATION 69

**Acide 5-acétyl-1*H*-indole-2-propanoïque, méthyl ester.**

En opérant de façon analogue à la préparation 6, au départ du composé obtenu selon la préparation 19, on obtient le produit attendu sous forme d'un solide beige (rendement = 91 %).
$^1$H RMN ($DMSOd_6$, 250 MHz) $\delta$ = 2,57 (s, 3H), 2,77 (t, 2H), 3,01 (t, 2H), 3,61 (s, 3H), 6,32 (s, 1H), 7,34 (d, 1H), 7,66 (dd, 1H), 8,15 (d, 1H), 11,33 (s, 1H).

### PREPARATION 70

**Acide 6-chloro-5-fluoro-1*H*-indole-2-propanoïque, méthyl ester.**

En opérant de façon analogue à la préparation 6, au départ du composé obtenu selon la préparation 20, on obtient le produit attendu sous forme d'un solide gris (rendement = 92 %).
F = 138-139°C.

### PREPARATION 71

**Acide 5,7-dichloro-1*H*-indole-2-propanoïque, méthyl ester.**

En opérant de façon analogue à la préparation 6, au départ du composé obtenu selon la préparation 23, on

obtient le produit attendu sous forme d'un solide brun (rendement = 30 %).
$^1$H RMN (DMSOd$_6$, 250 MHz) δ = 2,77 (t, 2H), 3,02 (t, 2H), 3,61 (s, 3H), 6,26 (s, 1H), 7,15 (d, 1H), 7,47 (d, 1H), 11,47 (s, 1H).

**PREPARATION 72**

**Acide 5-cyano-1*H*-indole-2-propanoïque, méthyl ester.**

En opérant de façon analogue à la préparation 6, au départ du composé obtenu selon la préparation 21, on obtient le produit attendu sous forme d'un solide beige (rendement = 72 %).
$^1$H RMN (DMSOd$_6$, 250 MHz) δ = 2,77 (t, 2H), 3,02 (t, 2H) , 3,60 (s, 3H), 6,33 (s, 1H), 7,35 (dd, 1H), 7,35 (dd, 1,6 Hz, 1H), 7,4 (d, 1H), 7,93 (d, 1H), 11,56 (s, 1H).

**PREPARATION 73**

**Acide 5-benzoyl-1*H*-indole-2-propanoïque, méthyl ester.**

En opérant de façon analogue à la préparation 6, au départ du composé obtenu selon la préparation 22, on obtient le produit attendu sous forme d'un solide brun (rendement = 44 %).
$^1$H RMN (DMSOd$_6$, 250 MHz) δ = 2,77 (t, 2H), 3,02 (t, 2H), 3,61 (s, 3H), 6,33 (s, 1H), 7,42 (d, 1H), 7,45-7,75 (m, 7H), 7,86 (d, 1H), 11,42 (s, 1H).

**PREPARATION 74**

**Acide 7-[5-chloro-2-[(phénylsulfonyl)amino]phényl]-6-heptynoïque, méthyl ester.**

En opérant de façon analogue à la préparation 3, au départ du composé obtenu selon la préparation 14, on obtient le produit attendu sous forme d'une huile incolore (rendement = 54 %).
$^1$H RMN (DMSOd$_6$, 250 MHz) δ = 1,55 (m, 4H), 2,33 (m, 4H), 3,60 (s, 3H), 7,22 (m, 3H), 7,61 (m, 5H), 9,75 (s, 1H).

**PREPARATION 75**

**Acide 6-[5-chloro-2-[[(3,4-dihydro-4-méthyl-2*H*-1,4-benzoxazin-7-yl)sulfonyl] amino]phényl]-5-hexynoïque, méthyl ester.**

En opérant de façon analogue à la préparation 55, au départ de chlorure de 3,4-dihydro-4-méthyl-2*H*-1,4-benzoxazin-7-sulfonyle, on obtient le produit attendu sous forme d'une poudre blanche (rendement = 88 %).
F=131-133°C.

**PREPARATION 76**

**Acide 6-[5-chloro-2-[[[1,2,3,4-tétrahydro-2-(trifluoroacétyl)-7-isoquinolinyl]sulfonyl]amino]phényl]-5-hexynoique, méthyl ester**

En opérant de façon analogue à la préparation 55, au départ de chlorure de 1,2,3,4-tétrahydro-2-(trifluoroacétyl)-7-isoquinolinesulfonyle, on obtient le produit attendu sous forme d'une huile jaune (rendement = 89 %).
$^1$H RMN (DMSOd$_6$, 300 MHz) δ = 1,70-1,75 (m, 2H), 2,35-2,45 (m, 4H), 3,0 (t, 2H), 3,85 (t, 2H), 4,80 (s, 2H), 7,30-7,35 (m, 4H), 7,60 (dd, 1H), 7,65 (d, 1H), 9,80 (s, 1H).

**PREPARATION 77**

**Acide 6-[5-chloro-2-[[(2,3-dihydro-1,4-benzodioxin-6-yl)sulfonyl]amino] phényl]-5-hexynoique, méthyl ester**

En opérant de façon analogue à la préparation 55, au départ de chlorure de (2,3-dihydro-1,4-benzodioxin-6-sulfonyle, on obtient le produit attendu sous forme d'une huile jaune (rendement = 95 %).
$^1$H RMN (DMSOd$_6$, 250 MHz) d = 1,70-1,81 (m, 2H), 2,37-2,51 (m, 4H), 3,61 (s, 3H), 4,25-4,32 (m, 4H), 6,59-7,37 (m, 6H), 9,63 (s, 1H).

**PREPARATION 78**

**Acide 6-[5-chloro-2-[[(6-benzothiazolyl)sulfonyl]amino] phényl]-5-hexynoique, méthyl ester**

En opérant de façon analogue à la préparation 55, au départ de chlorure de 6-benzothiazolesulfonyle, on obtient le produit attendu sous forme d'une huile jaune (rendement = 63 %).
$^1$H RMN (DMSO$d_6$, 300 MHz) $\delta$ = 1,56-1,66 (m, 2H), 2,23 (t, 2H), 2,35 (t, 2H), 3,60 (s, 3H), 7,27-7,39 (m, 3H), 7,79 (d, 1H), 8,21 (d, 1H), 8,60 (s, 1H), 9,61 (s, 1H), 9,97 (s, 1H).

**PREPARATION 79**

**Acide 6-[5-chloro-2-[[[6-(4-morpholinyl)-3-pyridinyl]sulfonyl]amino] phényl]-5-hexynoique, méthyl ester**

En opérant de façon analogue à la préparation 55, au départ de chlorure de 6-(4-morpholinyl)-3-pyridinesulfonyle, on obtient le produit attendu sous forme d'une huile jaune (rendement = 66 %).
$^1$H RMN (DMSO$d_6$, 300 MHz) $\delta$ = 1,69-1,79 (m, 2H), 2,36-2,51 (m, 4H), 3,55-3,58 (m, 4H), 3,60 (s, 3H), 3,64-3,68 (m, 4H), 6,87 (d, 1H), 7,29-7,39 (m, 3H), 7,64 (dd, 1H), 8,30 (d, 1H), 9,58 (s, 1H).

**PREPARATION 80**

**Acide 6-[5-chloro-2-[[(3,5-diméthyl-4-isoxazolyl)sulfonyl]amino] phényl]-5-hexynoique, méthyl ester**

En opérant de façon analogue à la préparation 55, au départ de chlorure de 3,5-diméthyl-4-isoxazolesulfonyle, on obtient le produit attendu sous forme d'une poudre jaune (rendement = 62 %).
F=107-109°C.

**PREPARATION 81**

**Acide 6-[5-chloro-2-[[(1,3,5-triméthyl-1*H*-pyrazol-4-yl)sulfonyl]amino] phényl]-5-hexynoïque, méthyl ester**

En opérant de façon analogue à la préparation 55, au départ de chlorure de 1,3,5-triméthyl-1*H*-pyrazole-4-sulfonyle, on obtient le produit attendu sous forme d'une huile jaune (rendement = 86 %).
$^1$H RMN (DMSO$d_6$, 250 MHz) $\delta$ = 1,73-1,81 (m, 2H), 2,05 (s, 3H), 2,15 (s, 3H), 2,36 (d, 2H), 2,42 (d, 2H), 3,61 (s, 3H), 3,62 (s, 3H), 7,31-7,41 (m, 3H), 9,38 (s, 1H).

**PREPARATION 82**

**Acide 6-[5-chloro-2-[[(1-méthyl-1*H*-imidazol-4-yl)sulfonyl]amino] phényl]-5-hexynoïque, méthyl ester**

En opérant de façon analogue à la préparation 55, au départ de chlorure de 1-méthyl-1*H*-imidazole-4-sulfonyle, on obtient le produit attendu sous forme d'une poudre jaune (rendement = 89 %).
F = 76-79°C.

**PREPARATION 83**

**Acide 6-[2-[(2,1,3-benzothiadiazol-4-yl)sulfonyl]amino]-5-chloro phényl]-5-hexynoïque, méthyl ester**

En opérant de façon analogue à la préparation 55, au départ de chlorure de 2,1,3-benzothiadiazole-4-sulfonyle, on obtient le produit attendu sous forme d'une huile jaune (rendement = 87 %).
$^1$H RMN (DMSO$d_6$, 300 MHz) $\delta$ = 1,49-1,59 (m, 2H), 2,03 (t, 2H), 2,32 (t, 2H), 3,60 (s, 3H), 7,25-7,38 (m, 3H), 7,82(dd, 1H), 8,13 (dd, 1H), 8,37 (dd, 1H), 9,83 (s, 1H).

**PREPARATION 84**

**Acide 6-[2-[(2,1,3-benzothiadiazol-5-yl)sulfonyl]amino]-5-chloro phényl]-5-hexynoïque, méthyl ester**

En opérant de façon analogue à la préparation 55, au départ de chlorure de 2,1,3-benzothiadiazole-5-sulfonyle, on obtient le produit attendu sous forme d'une huile jaune (rendement = 22 %).

$^1H$ RMN ($DMSOd_6$, 300 MHz) δ = 1,52-1,62 (m, 2H), 2,20 (t, 2H), 2,33 (t, 2H), 3,59 (s, 3H), 7,31-7,40 (m, 3H), 7,95 (dd, 1H), 8,31 (dd, 1H), 8,36 (dd, 1H), 10,3 (s, 1H).

**PREPARATION 85**

**Acide 6-[5-chloro-2-[[(3,4-dihydro-2,2-diméthyl-2*H*-1-benzopyran-6-yl)sulfonyl]amino]phényl]-5-hexynoïque, méthyl ester**

En opérant de façon analogue à la préparation 55, au départ de chlorure de 3,4-dihydro-2,2-diméthyl-2*H*-1-benzopyrane-6-sulfonyle, on obtient le produit attendu sous forme d'une huile jaune (rendement = 88 %).
$^1H$ RMN ($DMSOd_6$, 300 MHz) δ = 1,28 (s, 6H), 1,70-1,79 (m, 4H), 2,35-2,51 (m, 4H), 2,72 (t, 2H), 3,60 (s, 3H), 6,80 (d, 1H), 7,25-7,65 (m, 5H), 9,48 (s, 1H).

**PREPARATION 86**

**Acide 6-[5-chloro-2-[[(5,6,7,8-tétrahydro-5,5,8,8-tétraméthyl-2-naphthalènyl)sulfonyl]amino]phényl]-5-hexynoïque, méthyl ester**

En opérant de façon analogue à la préparation 55, au départ de chlorure de 5,6,7,8-tétrahydro-5,5,8,8-tétraméthyl-2-naphthalènesulfonyle, on obtient le produit attendu sous forme d'une poudre jaune (rendement = 65 %).
F = 113-115°C.

**PREPARATION 87**

**Acide 6-[2-[[(1-acétyl-2,3-dihydro-1*H*-indol-5-yl)sulfonyl]amino]-5-chlorophényl]-5-hexynoïque, méthyl ester**

En opérant de façon analogue à la préparation 55, au départ de chlorure de 1-acétyl-2,3-dihydro-1*H*-indole-5-sulfonyle, on obtient le produit attendu sous forme d'un solide pâteux (rendement = 88 %).
$^1H$ RMN ($DMSOd_6$, 250 MHz) δ = 1,68-1,80 (m, 2H), 2,17 (s, 3H), 2,39 (t, 2H), 2,43 (t, 2H), 3,14 (t, 2H), 3,61 (s, 3H), 4,14 (t, 2H), 7,25 (dd, 1H), 7,32-7,37 (m, 2H ), 7,48 (d, 1H), 7,52 (d, 1H), 8,07 (d, 1H), 9,59 (s, 1H).

**PREPARATION 88**

**Acide 6-[5-chloro-2-[[(2-méthyl-6-benzothiazolyl)sulfonyl]amino] phényl]-5-hexynoïque, méthyl ester**

En opérant de façon analogue à la préparation 55, au départ de chlorure de 2-méthyl-6-benzothiazolesulfonyle, on obtient le produit attendu sous forme d'un solide jaune (rendement = 68 %).
F = 103-106°C.

**PREPARATION 89**

**Acide 6-[2-[[[2-(acétylamino)-6-benzothiazolyl]sulfonyl]amino]-5-chlorophényl]-5-hexynoïque, méthyl ester**

En opérant de façon analogue à la préparation 55, au départ de chlorure de 2-(acétylamino)-6-benzothiazolesulfonyle, on obtient le produit attendu sous forme d'une poudre blanche (rendement =80%).
F = 138-140°C.

**PREPARATION 90**

**Acide 6-[2-[[(2-amino-6-benzothiazolyl)sulfonyl]amino]-5-chlorophényl]-5-hexynoïque, méthyl ester**

En opérant de façon analogue à la préparation 55, au départ de chlorure de 2-amino-6-benzothiazolesulfonyle, on obtient le produit attendu sous forme d'un solide orange (rendement = 96 %).
F = 61-65°C.

**PREPARATION 91**

**Acide 6-[5-chloro-2-[[(2-méthyl-6-benzoxazolyl)sulfonyl]amino]phényl]-5-hexynoïque, méthyl ester**

En opérant de façon analogue à la préparation 55, au départ de chlorure de 2-méthyl-6-benzoxazolesulfonyle, on obtient le produit attendu sous forme d'une huile orange (rendement = 93 %).
$^1H$ RMN ($DMSOd_6$, 250 MHz) $\delta$ = 1,65 (m, 2H), 2,15 (t, 2H), 2,39 (t, 2H), 2,65 (s, 3H), 3,61 (s, 3H), 7,26-7,39 (m, 3H), 7,65 (dd, 1H), 7,81 (d, 1H), 7,95 (s, 1H), 9,93 (s, 1H).

**PREPARATION 92**

**Acide 6-[5-chloro-2-[[(2,3-dihydro-5-benzofuranyl)sulfonyl]amino]phényl]-5-hexynoïque, méthyl ester**

En opérant de façon analogue à la préparation 55, au départ de chlorure de 2,3-dihydro-5-benzofuranesulfonyle, on obtient le produit attendu sous forme d'une huile orange (rendement = 99 %).
$^1H$ RMN ($DMSOd_6$, 300 MHz) $\delta$ = 1,76 (m, 2H), 2,40-2,49 (m, 4H), 3.23 (t, 2H), 3,85 (s, 3H), 4,62 (t, 2H), 6,85 (d, 1H), 7,25-7,45 (m, 4H), 7,77 (s, 1H), 9,51 (s, 1H).

**PREPARATION 93**

**Acide 6-[5-chloro-2-[[(2-méthyl-5-benzothiazolyl)sulfonyl]amino] phényl]-5-hexynoïque, méthyl ester**

En opérant de façon analogue à la préparation 55, au départ de chlorure de 2-méthyl-5-benzothiazolesulfonyle, on obtient le produit attendu sous forme d'un solide jaune (rendement = 42 %).
F = 68-72°C.

**PREPARATION 94**

**Acide 6-[2-[[(2-amino-6-benzoxazolyl)sulfonyl]amino]-5-chlorophényl]-5-hexynoïque, méthyl ester**

En opérant de façon analogue à la préparation 55, au départ de chlorure de 2-amino-6-benzoxazolesulfonyle, on obtient le produit attendu sous forme d'un solide blanc (rendement = 9 %).
F = 135°C.

**PREPARATION 95**

**Acide 6-[2-[[[2-(acétylamino)-4-méthyl-5-thiazolyl]sulfonyl]amino]-5-chlorophényl]-5-hexynoïque, méthyl ester**

En opérant de façon analogue à la préparation 55, au départ de chlorure de 2-(acétylamino)-4-méthyl-5-thiazolesulfonyle, on obtient le produit attendu sous forme d'une poudre blanche (rendement = 62%).
F=147-149°C.

**PREPARATION 96**

**Acide 6-[5-chloro-2-[[(1,2,3,4-tétrahydro-2-oxo-6-quinolinyl)sulfonyl]amino] phényl]-5-hexynoïque, méthyl ester**

En opérant de façon analogue à la préparation 55, au départ de chlorure de 1,2,3,4-tétrahydro-2-oxo-6-quinolinesulfonyle, on obtient le produit attendu sous forme d'une poudre jaune (rendement = 27 %).
F=53-57°C.

**PREPARATION 97**

**Acide 5-[2-[[(6-benzothiazolyl)sulfonyl]amino]-5-chloro phényl]-4-pentynoique, méthyl ester**

En opérant de façon analogue à la préparation 3, au départ de chlorure de 6-benzothiazolesulfonyle, on obtient le produit attendu sous forme d'une huile jaune (rendement = 92 %).

$^{1}H$ RMN ($DMSOd_6$, 300 MHz) δ = 2,44 (s, 4H), 3,63 (s, 3H), 7,26-7,39 (m, 3H), 7,25-7,65 (m, 5H), 7,80 (dd, 1H), 8,21 (dd 1H), 8,62 d, 1H), 9,62 (s, 1H), 9,88 (s, 1H).

**PREPARATION 98**

**Acide 5-[2-[[[2-(acétylamino)-6-benzothiazolyl]sulfonyl]amino]-5-chlorophényl]-4-pentynoïque, méthyl ester**

En opérant de façon analogue à la préparation 3, au départ de chlorure de 2-(acétylamino)-6-benzothiazolesulfonyle, on obtient le produit attendu sous forme d'un solide beige (rendement = 99 %).
F = 85°C.

**PREPARATION 99**

**Acide 5-[2-[[(1-acétyl-2,3-dihydro-1*H*-indol-5-yl)sulfonyl]amino]-5-chlorophényl]-4-pentynoïque, méthyl ester**

En opérant de façon analogue à la préparation 3, au départ de chlorure de 1-acétyl-2,3-dihydro-1*H*-indole-5-sulfonyle, on obtient le produit attendu sous forme d'un solide beige (rendement = 84 %).
F = 154°C.

**PREPARATION 100**

**Acide 5-[2-[[(**1,3-benzodioxol-5-yl**)sulfonyl]amino]-5-chloro phényl]-4-pentynoique, méthyl ester**

En opérant de façon analogue à la préparation 3, au départ de chlorure de 1,3-benzodioxole-5-sulfonyle, on obtient le produit attendu sous forme d'une huile brune (rendement = 98 %).
$^{1}H$ RMN ($DMSOd_6$, 300 MHz) δ = 2,60 (s, 4H), 3,65 (s, 3H), 6,15 (s, 2H), 7,01 (d, J = 8,73 Hz, 1H), 7,20 (dd, 2H), 7,27 (s, H), 7,30-7,38 (m, 2H).

**PREPARATION 101**

**Acide 6-[2-[[(1,3-benzodioxol-5-yl)sulfonyl]amino]-5-chloro phényl]-5-hexynoique, méthyl ester**

En opérant de façon analogue à la préparation 55, au départ de chlorure de 1,3-benzodioxole-5-sulfonyle, on obtient le produit attendu sous forme d'une huile brune (rendement = 89 %).
$^{1}H$ RMN ($DMSOd_6$, 300 MHz) δ = 1,76 (m, 2H), 2,37-2,45 (m, 4H), 3,61 (s, 3H), 6,14 (s, 2H), 7,0 (d, 1H), 7,38-7,71 (m, 5H), 9,68 (s, 1H).

**PREPARATION 102**

**Acide 6-[5-chloro-2-[[[4-(4-morpholinylsulfonyl)phényl]sulfonyl]amino] phényl]-5-hexynoique, méthyl ester**

En opérant de façon analogue à la préparation 55, au départ de chlorure de 4-(4-morpholinylsulfonyl)benzènesulfonyle, on obtient le produit attendu sous forme d'un solide blanc (rendement = 66 %).
F = 135-139°C.

**PREPARATION 103**

**Acide 6-[2-amino-5-(trifluorométhyl)phényl]-5-hexynoïque, méthyl ester**

En opérant de façon analogue à la préparation 5, au départ d'ester méthylique de l'acide 5-hexynoïque, on obtient le produit attendu sous forme d'une huile brune (rendement = 84 %).
$^{1}H$ RMN ($DMSOd_6$, 250 MHz) δ = 1,86 (q, 2H), 2,45-2,54 (m, 4H), 3.6 (s,3H); 5,96 (s, NH2), 6,78 (d, 1H), 7,30 (dd, 1H), 7,36 (d, 1H).

**PREPARATION 104**

**Acide 5-[2-[[(6-benzothiazolyl)sulfonyl]amino]-5-(trifluorométhyl) phényl]-4-pentynoique, méthyl ester**

En opérant de façon analogue à la préparation 97, au départ de l'ester méthylique de l'acide 5-[2-amino-5-(trifluorométhyl)phényl]-4-pentynoique (Préparation 5), on obtient le produit attendu sous forme d'une huile orange (rendement = 83 %).
$^1$H RMN (DMSO$d_6$, 500 MHz) δ = 2,53 (d, 2H), 2,55 (d, 2H), 3,65 (s, 3H), 6,80 (d, 1H), 7,54-7,66 (m, 3H), 7,90 (dd, J1H), 8,22 (d, 1H), 8,73 (d, 1H), 9,63 (s, 1H), 10,13 (s, 1H).

**PREPARATION 105**

**Acide 6-[2-[[(2-méthyl-6-benzoxazolyl)sulfonyl]amino]-5-(trifluorométhyl) phényl]-5-hexynoïque, méthyl ester**

En opérant de façon analogue à la préparation 91, au départ de l'ester méthylique de l'acide 6-[2-amino-5-(trifluorométhyl)phényl]-5-hexynoïque (préparation 103) et du chlorure de 2-méthyl-6-benzoxazolesulfonyle, on obtient le produit attendu sous forme d'une huile orange (rendement = 54 %).
$^1$H RMN (DMSO$d_6$, 300 MHz) δ = 1,65-1,75 (m, 2H), 2,33-2,43 (m, 4H), 2,65 (s, 3H), 3,60 (s, 3H), 7,49 (d, 1H), 7,62 (s, 1H), 7,63 (d, 1H), 7,74 (dd, 1H), 7,82 (d, 1H), 8,07 (s, 1H), 10,16 (s, 1H).

**PREPARATION 106**

**Acide 6-[2-[[(1-acétyl-2,3-dihydro-1*H*-indol-5-yl)sulfonyl]amino]-5-(trifluorométhyl)phényl]-5-hexynoïque, méthyl ester**

En opérant de façon analogue à la préparation 105, au départ de chlorure de 1-acétyl-2,3-dihydro-1*H*-indole-5-sulfonyle, on obtient le produit attendu sous forme d'un solide pâteux (rendement = 56 %).
$^1$H RMN (DMSO$d_6$, 300 MHz) δ= 1,74-1,83 (m, 2H), 2,16 (s, 3H), 2,43-2,48 (m, 4H), 3,15 (t, 2H), 3,61 (s, 3H), 4,13 (t, 2H), 7,44-7,47 (m, 1H), 7,58-7,61 (m, 4H), 8,07-8,10 (m, 1H), 9,86 (s, 1H).

**PREPARATION 107**

**Acide 6-[2-[[(2,3-dihydro-5-benzofuranyl)sulfonyl]amino]-5-(trifluorométhyl) phényl]-5-hexynoïque, méthyl ester**

En opérant de façon analogue à la préparation 105, au départ de chlorure de 2,3-dihydro-5-benzofuranesulfonyle, on obtient le produit attendu sous forme d'une pâte jaune (rendement = 19 %).
$^1$H RMN (DMSO$d_6$, 250 MHz) δ = 1,80 (t, 2H), 2,43-2,51 (m, 4H), 3,21 (t, 2H), 3,61 (s, 3H), 4,62 (t, 2H), 6,87 (d, 1H), 7,48 (d, 1H), 7,55 (dd, 1H), 7,60-7,67 (m, 3H), 9,77 (s, 1H).

**PREPARATION 108**

**Acide 6-[2-[[(2-méthyl-5-benzothiazolyl)sulfonyl]amino]-5-(trifluorométhyl) phényl]-5-hexynoïque, méthyl ester**

En opérant de façon analogue à la préparation 105, au départ de chlorure de 2-méthyl-5-benzothiazolesulfonyle, on obtient le produit attendu sous forme d'une pâte jaune (rendement = 17 %).
$^1$H RMN (DMSO$d_6$, 250 MHz) δ = 1,61-1,73 (m, 2H), 2,32 (t, 2H), 2,38 (t, 2H), 2,83 (s, 3H), 3,60 (s, 3H), 7,52 (d, 1H), 7,60-7,66 (m, 3H), 7,72 (dd, 1H), 8,23 (d, 1H), 8,25 (s, 1H), 10,19 (s, 1H).

**PREPARATION 109**

**Acide 5-[2-[[(2-amino-6-benzothiazolyl)sulfonyl]amino]-5-(trifluorométhyl)-phényl]-4-pentynoïque, méthyl ester**

En opérant de façon analogue à la préparation 104, au départ de chlorure de 2-amino-6-benzothiazolesulfonyle, on obtient le produit attendu sous forme d'une huile jaune (rendement = 69 %).
$^1$H RMN (DMSO$d_6$, 250 MHz) δ = 2,65 (m, 4H), 3,68 (s, 3H), 7,40 (d, 1H), 7,54-7,66 (m, 4H), 7,99 (s, 2H), 8,19 (d, 1H),

9,71 (s, 1H).

**PREPARATION 110**

**Acide 5-[2-[[(2-méthyl-6-benzothiazolyl)sulfonyl]amino]-5-(trifluorométhyl) phényl]-4-pentynoïque, méthyl ester**

En opérant de façon analogue à la préparation 104, au départ de chlorure de 2-méthyl-6-benzothiazolesulfonyle, on obtient le produit attendu sous forme d'une huile jaune (rendement = 24 %).
$^1$H RMN (DMSOd$_6$, 300 MHz) δ = 2,54 (m, 4H), 2,84 (s, 3H), 3,65 (s, 3H), 7,53 (m, 2H), 7,56 (m, 1H), 7,65 (dd, 1H), 7,83 (dd, 1H), 8,05 (dd, 1H), 8,57 (d, 1H), 10,05 (s, 1H).

**PREPARATION 111**

**Acide 5-[2-[[(2-méthyl-5-benzothiazolyl)sulfonyl]amino]-5-(trifluorométhyl) phényl]-4-pentynoïque, méthyl ester**

En opérant de façon analogue à la préparation 104, au départ de chlorure de 2-méthyl-5-benzothiazolesulfonyle, on obtient le produit attendu sous forme d'une huile jaune (rendement = 46 %).
$^1$H RMN (DMSOd$_6$, 250 MHz) δ = 2.55 (m, 4H), 2,83 (s, 3H), 3,65 (s, 3H), 7,55 (m, 2H), 7,66 (dd, 1H), 7,72 (dd, 1H), 8.24 (d, 1H), 8,30 (d, 1H), 10,09 (s, 1H).

**PREPARATION 112**

**Acide 5-[2-[[(2-méthyl-6-benzoxazolyl)sulfonyl]amino]-5-(trifluorométhyl) phényl]-4-pentynoïque, méthyl ester**

En opérant de façon analogue à la préparation 104, au départ de chlorure de 2-méthyl-6-benzoxazolesulfonyle, on obtient le produit attendu sous forme d'une poudre blanche (rendement = 41 %).
$^1$H RMN (DMSOd$_6$, 250 MHz) δ = 2,56 (m, 4H), 2,66 (s, 3H), 3,65 (s, 3H), 7,55 (m, 2H), 7,62 (d, 1H), 7,75 (dd, 1H), 7,83 (dd, 1H), 8,12 (dd, 1H), 10,04 (s, 1H).

**PREPARATION 113**

**Acide 5-[2-[[(2-méthyl-7-benzothiazolyl)sulfonyl]amino]-5-(trifluorométhyl) phényl]-4-pentynoïque, méthyl ester**

En opérant de façon analogue à la préparation 104, au départ de chlorure de 2-méthyl-7-benzothiazolesulfonyle, on obtient le produit attendu sous forme d'une huile orange (rendement = 32 %).
$^1$H RMN (DMSOd$_6$, 250 MHz) δ = 2,40 (m, 4H), 2,78 (s, 3H), 3,64 (s, 3H), 7,68 (m, 3H), 8,17 (dd, 1H), 10,40 (s, 1H).

**PREPARATION 114**

**Acide 5-[2-[[(1-acétyl-2,3-dihydro-1*H*-indol-5-yl)sulfonyl]amino]-5-(trifluorométhyl)phényl]-4-pentynoïque, méthyl ester**

En opérant de façon analogue à la préparation 104, au départ de chlorure de 1-acétyl-2,3-dihydro-1*H*-indole-5-sulfonyle, on obtient le produit attendu sous forme d'un solide jaune (rendement = 32 %).
$^1$H RMN (DMSOd$_6$, 300 MHz) δ = 2,63 (m, 4H), 3,16 (t, 2H), 3,66 (s, 3H), 4,13 (t, 2H), 7,50 (d, 1H), 7,62 (m, 4H), 8,10 (d, 1H), 9,74 (s, 1H).

**PREPARATION 115**

**Acide 5-[2-[[(2,3-dihydro-5-benzofuranyl)sulfonyl]amino]-5-(trifluorométhyl) phényl]-4-pentynoïque, méthyl ester**

En opérant de façon analogue à la préparation 104, au départ de chlorure de 2,3-dihydro-5-benzofuranesulfonyle, on obtient le produit attendu sous forme d'une huile jaune (rendement = 53 %).
$^1$H RMN (DMSOd$_6$, 250 MHz) δ = 2,66 (m, 4H), 3,20 (t, 2H), 3,66 (s, 3H), 4,63 (t, 2H), 6,88 (d, 1H), 7,59 (m, 5H), 9,65 (s, 1H).

**PREPARATION 116**

**Acide 5-[2-[[[4-(4-morpholinylsulfonyl)phényl]sulfonyl]amino]-5-(trifluoro méthyl)phényl]-4-pentynoique, méthyl ester**

En opérant de façon analogue à la préparation 104, au départ de chlorure de 4-(4-morpholinylsulfonyl)benzènesulfonyle, on obtient le produit attendu sous forme d'une huile jaune (rendement = 56 %).
$^1$H RMN ($DMSOd_6$, 250 MHz) δ = 2,60 (s, 4H), 2,88 (t, 4H), 3,60 (t, 4H), 3,65 (s, 3H), 7,53 (s, 1H), 7,58 (d, 1H), 7,92 (dd, 1H), 7,99 (dd, 2H), 10,37 (s, 1H).

**PREPARATION 117**

***N*-(4-chloro-2-iodophenyl)-2-pyridinesulfonamide**

On prépare une solution de 1 g (3,95 mM) de 4-chloro-2-iodoaniline et 0,65 ml de pyridine dans 10 ml de dichlorométhane et on ajoute, à 0˚C et sous agitation, 1,68 g (9,5 mM) de chlorure de 2-pyridinesulfonyle. Le mélange réactionnel est ensuite agité à température ambiante pendant 16 heures, puis concentré sous pression réduite. L'huile résiduelle est purifiée par chromatographie sur gel de silice en éluant à l'aide d'un mélange cyclohexane/acétate d'éthyle (8/2 ; v/v). On obtient ainsi 0,74 g du produit attendu sous forme d'un solide blanc (rendement = 48 %).
F = 112-124 ˚C.

**PREPARATION 118**

**Acide 6-[2-[[(6-benzothiazolyl)sulfonyl]amino]-5-(trifluorométhyl)-phényl]-5-hexynoique, méthyl ester**

En opérant de façon analogue à la préparation 105, au départ du chlorure de 6-benzothiazolesulfonyle, on obtient le produit attendu sous forme d'une pâte orange (rendement = 59 %).
$^1$H RMN ($DMSOd_6$, 250 MHz) δ = 1,62-1,73 (m, 2H), 2,32 (t, 2H), 2,39 (t, 2H), 3,60 (s, 3H), 7,51 (d, 1H), 7,61-7,65 (m, 2H), 7,89 (dd, 1H), 8,23 (d, 1H), 8,69 (s, 1H), 9,62 (s, 1H), 10,22 (s, 1H).

**PREPARATION 119**

**Acide 5-(trifluorométhyl)-1*H*-indole-2-butanoïque, méthyl ester**

En opérant de façon analogue à la préparation 6, au départ de l'ester obtenu selon la préparation 103, on obtient le produit attendu sous forme d'un solide beige (rendement = 36 %).
F = 115˚C.

**PREPARATION 120**

***N*-(4-chloro-2-iodophényl)benzènesulfonamide**

On prépare une solution de 2g (7,89 mM) de 4-chloro-2-iodoaniline dans 30 ml de pyridine et on ajoute, à 0˚C et sous agitation, 1,21 ml (9,5 mM) de chlorure de benzènesulfonyle. Le mélange réactionnel est ensuite agité à température ambiante pendant 16 heures, puis concentré sous pression réduite. L'huile résiduelle est reprise par 50 ml d'acétate d'éthyle et la solution obtenue est lavée à l'eau, puis séchée sur sulfate de magnésium et concentrée sous pression réduite. L'analyse du produit brut montre la présence d'environ 12 % de *N*-(4-chloro-2-iodophényl)-N-(phénylsulfonyl)-benzènesulfonamide. Le produit brut est donc repris en solution dans 60 ml de dioxane et traité par 19 ml d'une solution de potasse 3M, à doux reflux pendant 8 heures. Le solvant est chassé sous pression réduite et le résidu est repris par de l'eau et acidifié jusqu'à pH 2 à l'aide d'une solution diluée d'acide chlorhydrique. Le précipité formé est séparé par filtration, lavé à l'eau sur le filtre et séché. On obtient ainsi 2,79 g du produit attendu sous forme d'un solide blanc (rendement = 90 %).
F = 126-128 ˚C.

**PREPARATION 121**

***N*-[2-iodo-4-(trifluorométhyl)phényl]benzènesulfonamide**

En opérant de façon analogue à la préparation 120, au départ de 2-iodo-4-(trifluorométhyl)aniline on obtient le produit attendu sous forme d'un solide blanc (rendement = 74%). '
F = 84-86°C.

**PREPARATION 122**

**Acide 2-[[3-(2-amino-5-chlorophényl)-2-propynyl]oxy]-2-méthylpropanoïque, méthyl ester**

On prépare un mélange de 2g (7,89 mM) de 4-chloro-2-iodoaniline, 75 mg (0,395 mM) d'iodure cuivreux, 277 mg (0,39 mM) de bis(triphénylphosphine)-dichloropalladium, 221 mg (0,79 mM) de tri(cyclohexyl)phosphine, 3,08 g (19,7 mM) d'ester méthylique de l'acide 2-méthyl-2-(2-propynyloxy)propanoïque et 15 ml de *tert*-butylamine. Le mélange réactionnel est chauffé à doux reflux pendant 16 heures, puis refroidi, hydrolysé sur 60 ml d'eau et extrait par 3 fois 40 ml de dichlorométhane. Les phases organiques rassemblées sont lavées à l'eau, puis séchées sur sulfate de magnésium et concentrées sous pression réduite. L'huile résiduelle est purifiée par chromatographie sur gel de silice en éluant à l'aide d'un mélange dichlorométhane/acétate d'éthyle (98/2 ; v/v). On obtient ainsi 1,84 g du produit attendu sous forme d'une huile orange (rendement = 83%).
$^1H$ RMN ($DMSOd_6$, 250 MHz) $\delta$ = 1,41 (s, 6H), 3,66 (s, 3H), 4,40 (s, 2H), 5,22 (s large, 2H), 6,69 (d, 1H), 7,07 (dd, 1H), 7,13 (d, 1H).

**PREPARATION 123**

**Acide 2-[[3-[2-[[(6-benzothiazolyl)sulfonyl]amino]-5-chlorophényl]-2-propynyl]oxy]-2-méthylpropanoïque, méthyl ester**

En opérant de façon analogue à la préparation 117, au départ du composé obtenu selon la préparation 122 et de chlorure de 6-benzothiazolesulfonyle, on obtient le produit attendu sous forme d'une huile jaune (rendement = 53 %).
$^1H$ RMN ($DMSOd_6$, 300 MHz) $\delta$ = 1,63 (s, 6H), 3,67 (s, 3H), 4,14 (s, 2H), 7,30 (d, 1H), 7,38 (d, 1H), 7,41 (dd, 1H), 7,85 (dd, 1H), 8,23 (d, 1H), 8,62 (d, 1H); 9,61 (s, 1H), 10,05 (s, 1H).

**PREPARATION 124**

**Acide 2-[[3-[2-amino-5-(trifluorométhyl)phényl]-2-propynyl]oxy]-2-méthylpropanoïque, méthyl ester**

En opérant de façon analogue à la préparation 122, au départ de 2-iodo-5-(trifluorométhyl)aniline, on obtient le produit attendu sous forme d'une huile orange (rendement = 80 %).
$^1H$ RMN ($DMSOd_6$, 300 MHz) $\delta$ = 1,42 (s, 6H), 3,65 (s, 3H), 4,41 (s, 2H), 6,08 (s, 2H), 6,79 (d, 1H), 7,34 (d, 1H), 7,39 (s, 1H).

**PREPARATION 125**

**Acide 2-[[3-[2-[[(6-benzothiazolyl)sulfonyl]amino]-5-(trifluorométhyl) phényl]-2-propynyl]oxy]-2-méthylpropanoïque, méthyl ester**

En opérant de façon analogue à la préparation 123, au départ du composé obtenu selon la préparation 124 et de chlorure de 6-benzothiazolesulfonyle, on obtient le produit attendu sous forme d'une pâte jaune (rendement = 23 %).
$^1H$ RMN ($DMSOd_6$, 300 MHz) $\delta$ = 1,38 (s, 6H); 3,67 (s, 3H) ; 4,25 (s, 2H) ; 7,52 (d, 1H) ; 7,66 (d, 2H) ; 7,94 (dd, 1H) ; 8,24 (dd, 1H) ; 8,72 (d, 1H) ; 9,63 (s, 1H) ; 10,33 (s, 1H).

**PREPARATION 126**

**Acide 2-[[3-[2-[[(2-méthyl-6-benzothiazolyl)sulfonyl]amino]-5-chlorophényl]-2-propynyl]oxy]-2-méthylpropanoïque, méthyl ester**

En opérant de façon analogue à la préparation 117, au départ du composé obtenu selon la préparation 122 et de chlorure de 2-méthyl-6-benzothiazolesulfonyle, on obtient le produit attendu sous forme d'une pâte jaune (rende-

ment = 58%).
$^1$H RMN (DMSOd$_6$, 250 MHz) δ = 1,36 (s, 6H), 2,84 (s, 3H), 3,68 (s, 3H), 4,14 (s, 2H), 7,28 (d, 1H), 7,36 (d, 1H), 7,40 (dd, 1H), 7,78 (dd, 1H), 8,03 (d, 1H), 8,46 (d, 1H), 9,96 (s, 1H).

**PREPARATION 127**

**Acide 2-[[3-[2-[[(2-méthyl-5-benzothiazolyl)sulfonyl]amino]-5-chlorophényl]-2-propynyl]oxy]-2-méthylpropanoïque, méthyl ester**

En opérant de façon analogue à la préparation 117, au départ du composé obtenu selon la préparation 122 et de chlorure de 2-méthyl-5-benzothiazolesulfonyle, on obtient le produit attendu sous forme d'une huile jaune (rendement = 45%).
$^1$H RMN (DMSOd$_6$, 300 MHz) δ = 1,36 (s, 6H), 2,83 (s, 3H), 3,67 (s, 3H), 4,11 (s, 2H), 7,30 (d, 1H), 7,36 (d, 1H), 7,41 (dd, 1H), 7,66 (dd, 1H), 8,18 (d, 1H), 8,21 (d, 1H), 10,01 (s, 1H).

**PREPARATION 128**

**Acide 2-[[3-(2-amino-5-chlorophényl)-2-propynyl]oxy]propanoïque, éthyl ester**

En opérant de façon analogue à la préparation 122, au départ l'ester éthylique de l'acide 2-(2-propynyloxy) propanoïque, on obtient le produit attendu sous forme d'une huile orange (rendement = 69 %).
$^1$H RMN (DMSOd$_6$, 300 MHz) δ = 1,19 (t, 3H), 1,31 (d, 3H), 4,13 (q, 2H), 4,25 (q, 1H), 4,42 (d, 1H), 4,53 (d, 1H), 5,56 (s, 2H), 6,69 (d, 1H), 7,08 (dd, 1H), 7,14 (d, 1H).

**PREPARATION 129**

**Acide 2-[[3-[2-[[(6-benzothiazolyl)sulfonyl]amino]-5-chlorophényl]-2-propynyl]oxy]propanoïque, éthyl ester**

En opérant de façon analogue à la préparation 123, au départ du composé obtenu selon la préparation 128 et de chlorure de 6-benzothiazolesulfonyle, on obtient le produit attendu sous forme d'un solide blanc (rendement = 82 %). F = 166-168°C.

**PREPARATION 130**

**Acide 6-[2-[[(1,3-benzodioxol-5-yl)sulfonyl]amino]-5-(trifluorométhyl) phényl]-5-hexynoique, méthyl ester**

En opérant de façon analogue à la préparation 118, au départ de chlorure de 1,3-benzodioxole-5-sulfonyle, on obtient le produit attendu sous forme d'une huile brune, utilisée dans l'étape suivante sans autre purification (rendement = 73 %).

**PREPARATION 131**

**Acide 5-[2-[[[2-(acétylamino)-6-benzothiazolyl]sulfonyl]amino]-5-(trifluorométhyl)phényl]-4-pentynoïque, méthyl ester**

En opérant de façon analogue à la préparation 104, au départ de chlorure de 2-(acétylamino)-6-benzothiazolesulfonyle, on obtient le produit attendu sous forme d'une huile brune, utilisée dans l'étape suivante sans autre purification (rendement = 66 %).

**PREPARATION 132**

**Acide 2-[[3-[2-[[(2-méthyl-5-benzothiazolyl)sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 2-méthyl-5-benzothiazolesulfonyle, on obtient le produit attendu sous forme d'une huile orange (rendement = 16 %).
$^1$H RMN (DMSOd$_6$, 300 MHz) δ : 10,30 (s, 1H) ; 8,27 (m, 2H) ; 7,76 (dd, 1H) ; 7,69 (m, 2H) ; 7,53 (d, 1H) ; 4,22 (s, 2H) ;

3,67 (s, 3H) ; 2,83 (s, 3H) ; 1,39 (s, 6H).

**PREPARATION 133**

**Acide 2-[[3-[2-[(1,3-benzodioxol-5-ylsulfonyl)amino]-5-(trifluorométhyl)-phényl]-2-propynyl]oxy]-2-méthyl-propanoïque, méthyl ester**

En opérant de façon analogue à l'exemple 3, au départ du composé obtenu selon la préparation 124 et de chlorure de 1,3-benzodioxole-5-sulfonyle, on obtient le produit attendu sous forme d'une huile jaune (rendement = 21 %).
$^1$H RMN ($DMSOd_6$, 250 MHz) δ : 9,99 (s, 1H) ; 7,69 (m, 2H) ; 7,47 (d, 1H) ; 7,34 (m, 2H) ; 7,04 (d, 1H) ; 6,15 (s, 2H) ; 4,37 (s, 2H) ; 3,68 (s, 3H) ; 1,43 (s, 6H).

**PREPARATION 134**

**Acide 2-[[3-[2-[[[2-(acétylamino)-6-benzothiazolyl]sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 2-(acétylamino)-6-benzothiazolesulfonyle, on obtient le produit attendu sous forme d'un solide blanc (rendement = 63 %).
F = 104-106°C.

**PREPARATION 135**

**Acide 2-[[3-[2-[(2,3-dihydrobenzofuran-5-ylsulfonyl)amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 2,3-dihydro-5-benzofuranesulfonyle, on obtient le produit attendu sous forme d'une huile jaune (rendement = 87 %).
$^1$H RMN ($DMSOd_6$, 250 MHz) δ : 9,88 (s, 1H) ; 7,68 (m, 3H) ; 7,61 (m, 1H) ; 7,49 (dd, 1H) ; 6,88 (d, 1H) ; 4,63 (t, 2H) ; 4,38 (s, 2H) ; 3,68 (s, 3H) ; 3,22 (t, 2H) ; 1,43 (s, 6H).

**PREPARATION 136**

**Acide 2-[[3-[2-[[(2-méthyl-7-benzothiazolyl)sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 2-méthyl-7-benzothiazolesulfonyle, on obtient le produit attendu sous forme d'une huile jaune (rendement = 44 %).
$^1$H RMN ($DMSOd_6$, 250 MHz) δ : 10,57 (s, 1H) ; 8,15 (d, 1H) ; 7,75 (m, 1H) ; 7,64 (m, 3H) ; 7,46 (d, 1H) ; 4,06 (s, 2H) ; 3,68 (s, 3H) ; 2,79 (s, 3H) ; 1,37 (s, 6H).

**PREPARATION 137**

**Acide 2-[[3-[2-[[(2,3-dihydro-1,4-benzodioxin-6-yl)sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de (2,3-dihydro-1,4-benzodioxin-6-sulfonyle, on obtient le produit attendu sous forme d'une pâte beige (rendement = 79 %).
$^1$H RMN ($DMSOd_6$, 250 MHz) δ : 9,98 (s, 1H) ; 7,68 (m, 2H) ; 7,48 (d, 1H) ; 7,31 (m, 2H) ; 7,02 (d, 1H) ; 4,37 (s, 2H) ; 4,29 (m, 4H) ; 3,68 (s, 3H) ; 1,43 (s, 6H).

**PREPARATION 138**

**Acide 2-[[3-[2-[[(3,4-dihydro-4-méthyl-2*H*-1,4-benzoxazin-7-yl)sulfonyl]-amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 3,4-dihydro-4-méthyl-2*H*-1,4-benzoxazine-7-sulfonyle, on obtient le produit attendu sous forme d'un solide beige (rendement = 69 %).
F = 98-100°C.

**PREPARATION 139**

**Acide 2-[[3-[2-[(1-acétyl-2,3-dihydro-1*H*-indol-5-yl)sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 1-acétyl-2,3-dihydro-1*H*-indole-5-sulfonyle, on obtient le produit attendu sous forme d'un solide beige (rendement = 44 %).
F 134-136°C.

**PREPARATION 140**

**Acide 2-[[3-[2-[[(3,5-diméthylphényl)sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 3,5-diméthylbenzènesulfonyle, on obtient le produit attendu sous forme d'un solide beige (rendement = 70 %).
F = 128-130°C.

**PREPARATION 141**

**Acide 2-[[3-[2-[[(2,5-diméthoxyphényl)sulfonyl]amino]-5-(trifluorométhyl)-phényl]-2-propynyl]oxy]-2-méthylpropanoïque, méthyl ester**

En opérant de façon analogue à l'exemple 3, au départ du composé obtenu selon la préparation 124 et de chlorure de 2,5-diméthoxybenzènesulfonyle, on obtient le produit attendu sous forme d'une huile jaune (rendement = 76 %).
$^1$H RMN (DMSO$d_6$, 300 MHz) δ : 9,13 (s, 1H) ; 7,13 (s, 1H) ; 7,66 (d, 1H) ; 7,56 (d, 1H) ; 7,32 (d, 1H) ; 7,20 (dd, 1H) ; 7,15 (d, 1H) ; 4,42 (s, 2H) ; 3,76 (s, 3H) ; 3,74 (s, 3H) ; 3,68 (s, 3H) ; 1,42 (s, 6H).

**PREPARATION 142**

**Acide 2-[[3-[2-[[[4-(1-méthyléthyl)phényl]sulfonyl]amino]-5-(trifluorométhyl)phényl]-2-propynyl]oxy]-2-méthylpropanoïque, méthyl ester**

En opérant de façon analogue à l'exemple 3, au départ du composé obtenu selon la préparation 124 et de chlorure de 4-(1-méthyléthyl)benzènesulfonyle, on obtient le produit attendu sous forme d'une huile jaune (rendement = 77 %).
$^1$H RMN (DMSO$d_6$, 250 MHz) δ : 10,08 (s, 1H) ; 8,23 (d, 1H) ; 8,03 (s, 1H) ; 7,92 (dd, 1H) ; 7,76 (d, 2H) ; 7,67 (m, 2H) ; 7,46 (m, 4H) ; 4,33 (s, 2H) ; 3,67 (s, 3H) ; 2,96 (hep, 1H) ; 1,42 (s, 6H) ; 1,19 (d, 6H).

**PREPARATION 143**

**Acide 2-[[3-[2-[(1,3-benzodioxol-5-ylsulfonyl)amino]-5-chlorophényl]-2-propynyl]oxy]-2-méthylpropanoïque, méthyl ester**

En opérant de façon analogue à l'exemple 3, au départ du composé obtenu selon la préparation 122 et de chlorure de 1,3-benzodioxole-5-sulfonyle, on obtient le produit attendu sous forme d'une huile jaune (rendement = 55 %).

$^1$H RMN (DMSOd$_6$, 250 MHz) δ : 9,71 (s, 1H) ; 7,39 (m, 2H) ; 7,23 (m, 3H) ; 7,02 (d, 1H) ; 6,15 (s, 2H) ; 4,30 (s, 2H) ; 3,67 (s, 3H) ; 1,42 (s, 6H).

**PREPARATION 144**

**Acide 2-[[3-[2-[[[2-(acétylamino)-6-benzothiazolyl]sulfonyl]amino]-5-chlorophényl]-2-propynyl]oxy]-2-méthyl-propanoïque, méthyl ester**

En opérant de façon analogue à l'exemple 3, au départ du composé obtenu selon la préparation 122 et de chlorure de 2-(acétylamino)-6-benzothiazolesulfonyle, on obtient le produit attendu sous forme d'un solide jaune (rendement = 50 %).
F = 80°C.

**PREPARATION 145**

**Acide 2-[[3-[2-[(1-acétyl-2,3-dihydro-1*H*-indol-5-yl)sulfonyl]amino]-5-chlorophényl]-2-propynyl]oxy]-2-méthyl-propanoïque, méthyl ester**

En opérant de façon analogue à l'exemple 3, au départ du composé obtenu selon la préparation 122 et de chlorure de 1-acétyl-2,3-dihydro-1*H*-indole-5-sulfonyle, on obtient le produit attendu sous forme d'une pâte jaune (rendement = 44 %).
$^1$H RMN (DMSOd$_6$, 300 MHz) δ : 9,68 (s, 1H) ; 8,08 (d, 1H) ; 7,54 (m, 2H) ; 7,38 (m, 2H) ; 7,27 (dd, 1H) ; 4,30 (s, 2H) ; 4,13 (t, 2H) ; 3,67 (s, 3H) ; 2,17 (s, 3H) ; 1,41 (s, 6H).

**PREPARATION 146**

**Acide 2-[[3-[5-chloro-2-[(2,3-dihydrobenzofuran-5-ylsulfonyl)amino] phényl]-2-propynyl]oxy]-2-méthylpropanoïque, méthyl ester**

En opérant de façon analogue à l'exemple 3, au départ du composé obtenu selon la préparation 122 et de chlorure de 2,3-dihydro-5-benzofuranesulfonyle, on obtient le produit attendu sous forme d'une huile jaune (rendement = 49 %).
$^1$H RMN (DMSOd$_6$, 300 MHz) δ : 9,59 (s, 1H); 7,61 (d, 1H) ; 7,48 (dd, 1H) ; 7,39 (m, 2H) ; 7,27 (d, 1H) ; 6,85 (d, 1H) ; 4,62 (t, 2H) ; 4,28 (s, 2H) ; 3,64 (s, 3H) ; 3,21 (t, 2H) ; 1,42 (s, 6H).

**PREPARATION 147**

**Acide 2-[[3-[5-chloro-2-[[(2-méthyl-7-benzothiazolyl)sulfonyl]amino]phényl]-2-propynyl]oxy]-2-méthylpropanoïque, méthyl ester**

En opérant de façon analogue à l'exemple 3, au départ du composé obtenu selon la préparation 122 et de chlorure de 2-méthyl-7-benzothiazolesulfonyle, on obtient le produit attendu sous forme d'une huile jaune (rendement = 33 %).
$^1$H RMN (DMSOd$_6$, 300 MHz) δ : 10,30 (s, 1H) ; 8,14 (dd, 1H) ; 7,64 (m, 2H) ; 7,41 (dd, 1H) ; 7,32 (d, 1H) ; 7,26 (d, 1H) ; 3,91 (s, 2H) ; 3,67 (s, 3H) ; 2,79 (s, 3H) ; 1,34 (s, 6H).

**PREPARATION 148**

**Acide 2-[[3-[5-chloro-2-[[(2,3-dihydro-1,4-benzodioxin-6-yl)sulfonyl]amino] phényl]-2-propynyl]oxy]-2-méthyl-propanoïque, méthyl ester**

En opérant de façon analogue à l'exemple 3, au départ du composé obtenu selon la préparation 122 et de chlorure de (2,3-dihydro-1,4-benzodioxin-6-sulfonyle, on obtient le produit attendu sous forme d'une huile jaune (rendement = 94 %).
$^1$H RMN (DMSOd$_6$, 250 MHz) δ : 9,70 (s, 1H) ; 7,40 (m, 2H) ; 7,24 (m, 3H) ; 6,97 (d, 1H) ; 4,28 (m, 6H) ; 3,67 (s, 3H) ; 1,41 (s, 6H).

**PREPARATION 149**

**Acide 2-[[3-[5-chloro-2-[[(3,4-dihydro-4-méthyl-2*H*-1,4-benzoxazin-7-yl)-sulfonyl]amino]phényl]-2-propynyl]oxy]-2-méthylpropanoïque, méthyl ester**

En opérant de façon analogue à l'exemple 3, au départ du composé obtenu selon la préparation 122 et de chlorure de 3,4-dihydro-4-méthyl-2*H*-1,4-benzoxazin-7-sulfonyle, on obtient le produit attendu sous forme d'un solide beige (rendement = 69 %).
F = 98-100°C.

**PREPARATION 150**

**Acide 2-[[3-[5-chloro-2-[[[4-(1-méthyléthyl)phényl]sulfonyl]amino]phényl]-2-propynyl]oxy]-2-méthylpropanoïque, méthyl ester**

En opérant de façon analogue à l'exemple 3, au départ du composé obtenu selon la préparation 122 et de chlorure de 4-(1-méthyléthyl)benzènesuifonyle, on obtient le produit attendu sous forme d'un solide beige (rendement = 86 %).
F = 65-67°C.

**PREPARATION 151**

**Acide 2-[[3-[5-chloro-2-[[(3,5-diméthylphényl)sulfonyl]amino]phényl]-2-propynyl]oxy]-2-méthylpropanoïque, méthyl ester**

En opérant de façon analogue à l'exemple 3, au départ du composé obtenu selon la préparation 122 et de chlorure de 3,5-diméthylbenzènesùlfonyle, on obtient le produit attendu sous forme d'un solide blanc (rendement = 62 %).
F = 78-80°C.

**PREPARATION 152**

**Acide 2-[[3-[5-chloro-2-[[(2,5-diméthoxyphényl)sulfonyl]amino]phényl]-2-propynyl]oxy]-2 méthylpropanoïque, méthyl ester**

En opérant de façon analogue à l'exemple 3, au départ du composé obtenu selon la préparation 122 et de chlorure de 2,5-diméthoxybenzènesulfonyle, on obtient le produit attendu sous forme d'une huile jaune (rendement = 90 %).
$^1$H RMN (DMSO$d_6$, 250 MHz) δ : 8,93 (s, 1H) ; 7,43 (d, 1H) ; 7,35 (m, 2H) ; 7,25 (d, 1H) ; 7,17 (m, 2H) ; 4,36 (s, 2H) ; 3,77 (s, 3H) ; 3,73 (s, 3H) ; 3,68 (s, 3H) ; 1,41 (s, 6H).

**PREPARATION 153**

**Acide 2-[[3-[5-chloro-2-[[(4-méthoxyphényl)sulfonyl]amino]phényl]-2-propynyl]oxy]-2-méthylpropanoïque, méthyl ester**

En opérant de façon analogue à l'exemple 3, au départ du composé obtenu selon la préparation 122 et de chlorure de 4-méthoxybenzènesulfonyle, on obtient le produit attendu sous forme d'un solide beige (rendement = 83 %).
F = 98-100°C.

**PREPARATION 154**

**Acide 2-[[3-[5-chloro-2-[[(2-méthyl-6-benzothiazolyl)sulfonyl]amino]phényl]-2-propynyl]oxy]propanoïque, éthyl ester**

En opérant de façon analogue à l'exemple 3, au départ du composé obtenu selon la préparation 128 et de chlorure de 2-méthyl-6-benzothiazolesulfonyle, on obtient le produit attendu sous forme d'une pâte jaune (rendement = 49 %).
$^1$H RMN (DMSO$d_6$, 300 MHz) δ : 10,04 (s, 1H) ; 8,45 (d, 1H) ; 8,03 (d, 1H) ; 7,78 (dd, 1H) ; 7,41 (m, 2H) ; 7,27 (dd, 1H) ;

4,34 (d, 1H) ; 4,16 (m, 4H) ; 2,83 (s, 3H) ; 1,29 (d, 3H) ; 1,19 (t, 3H).

**PREPARATION 155**

**Acide 2-[[3-[5-chloro-2-[[(2,3-dihydro-1,4-benzodioxin-6-yl)sulfonyl]amino]-phényl]-2-propynyl]oxy]propanoïque, éthyl ester**

En opérant de façon analogue à l'exemple 3, au départ du composé obtenu selon la préparation 128 et de chlorure de 2,3-dihydro-1,4-benzodioxine-6-sulfonyle, on obtient le produit attendu sous forme d'une huile jaune (rendement = 65 %).
$^1$H RMN (DMSOd$_6$, 300 MHz) δ : 9,78 (s, 1H) ; 7,43 (dd, 2H) ; 7,25 (d, 1H) ; 7,19 (m, 2H) ; 6,97 (d, 1H) ; 4,45 (d, 1H) ; 4,29 (m, 6H) ; 4,14 (m, 2H) ; 1,33 (d, 3H) ; 1,21 (t, 3H).

**PREPARATION 156**

**Acide 2-[[3-[5-chloro-2-[[(3,4-dihydro-4-méthyl-2*H*-1,4-benzoxazin-7-yl)-sulfonyl]amino]phényl]-2-propynyl]oxy]propanoïque, éthyl ester**

En opérant de façon analogue à l'exemple 3, au départ du composé obtenu selon la préparation 128 et de chlorure de 3,4-dihydro-4-méthyl-2*H*-1,4-benzoxazine-7-sulfonyle, on obtient le produit attendu sous forme d'une huile jaune (rendement = 63 %).
$^1$H RMN (DMSOd$_6$, 300 MHz) δ : 9,57 (s, 1H) ; 7,41 (m, 2H) ; 7,26 (d, 1H) ; 6,93 (m, 2H) ; 6,74 (d, 1H) ; 4,45 (d, 1H) ; 4,26 (m, 4H) ; 4,15 (m, 2H) ; 3,26 (m, 2H) ; 2,79 (s, 3H) ; 1,32 (d, 3H) ; 1,21 (t, 3H).

**PREPARATION 157**

**Acide 2-[[3-[5-chloro-2-[[(3,5-diméthyl-4-isoxazolyl)sulfonyl]amino]phényl]-2-propynyl]oxy]propanoïque, éthyl ester**

En opérant de façon analogue à l'exemple 3, au départ du composé obtenu selon la préparation 128 et de chlorure de 3,5-diméthyl-4-isoxazolesulfonyle, on obtient le produit attendu sous forme d'une huile jaune (rendement = 30 %).
$^1$H RMN (DMSOd$_6$, 300 MHz) δ : 10,29 (s, 1H) ; 7,52 (m, 2H) ; 7,37 (d, 1H) ; 4,43 (d, 1H) ; 4,26 (m, 2H) ; 4,15 (m, 2H) ; 2,27 (s, 3H) ; 2,16 (s, 3H) ; 1,32 (d, 3H) ; 1,22 (t, 3H).

**PREPARATION 158**

**Acide 2-[[3-[5-chloro-2-[[(2,5-diméthoxyphényl)sulfonyl]amino]phényl]-2-propynyl]oxy]propanoïque, éthyl ester**

En opérant de façon analogue à l'exemple 3, au départ du composé obtenu selon la préparation 128 et de chlorure de 2,5-diméthoxybenzènesulfonyle, on obtient le produit attendu sous forme d'une huile jaune (rendement = 62%).
$^1$H RMN (DMSOd$_6$, 300 MHz) δ : 9,11 (s, 1H) ; 7,46 (d, 1H) ; 7,37 (d, 1H) ; 7,29 (m, 1H) ; 7,24 (m, 1H) ; 7,16 (m, 2H) ; 4,49 (d, 1H) ; 4,36 (d, 1H) ; 4,24 (q, 1H) ; 4,16 (m, 2H) ; 3,75 (s, 3H) ; 3,72 (s, 3H) ; 1,31 (d, 3H) ; 1,21 (t, 3H).

**PREPARATION 159**

**Acide 2-[[3-[2-amino-5-(trifluorométhyl)phényl]-2-propynyl]oxy]propanoïque, éthyl ester**

En opérant de façon analogue à la préparation 122, au départ du composé obtenu selon la préparation 4 et de l'ester éthylique de l'acide 2-(2-propynyloxy)-propanoïque, on obtient le produit attendu sous forme d'une huile orange (rendement = 82 %).
$^1$H RMN (DMSOd$_6$, 300 MHz) δ : 7,42 (d, 1H) ; 7,35 (dd, 1H) ; 6,80 (d, 1H) ; 6,11 (s, 2H) ; 4,54 (d, 1H) ; 4,43 (d, 1H) ; 4,27 (q, 1H) ; 4,11 (m, 2H) ; 1,32 (d, 3H) ; 1,20 (t, 3H).

**PREPARATION 160**

**Acide 2-[[3-[2-[[(2-méthyl-6-benzothiazolyl)sulfonyl]amino]-5-(trifluorométhyl)phényl]-2-propynyl]oxy]propanoïque, éthyl ester**

En opérant de façon analogue à l'exemple 3, au départ du composé obtenu selon la préparation 159 et de chlorure de 2-méthyl-6-benzothiazolesulfonyle, on obtient le produit attendu sous forme d'une huile jaune (rendement = 44 %).
$^{1}H$ RMN ($DMSOd_6$, 300 MHz) δ : 10,33 (s, 1H) ; 8,55 (d, 1H) ; 8,06 (d, 1H) ; 7,87 (dd, 1H) ; 6,68 (m, 2H) ; 7,50 (d, 1H) ; 4,43 (d, 1H) ; 4,26 (m, 2H) ; 4,15 (m, 2H) ; 2,84 (s, 3H) ; 1,31 (d, 3H) ; 1,20 (t, 3H).

**PREPARATION 161**

**Acide 2-[[3-[2-[[(6-benzothiazolyl)sulfonyl]amino]-5-(trifluorométhyl)-phényl]-2-propynyl]oxy]propanoïque, éthyl ester**

En opérant de façon analogue à l'exemple 3, au départ du composé obtenu selon la préparation 159 et de chlorure de 6-benzothiazolesulfonyle, on obtient le produit attendu sous forme d'un solide jaune pâle (rendement = 28 %).
F = 112-114°C.

**PREPARATION 162**

**Acide 2-[[3-[2-[[(2,3-dihydro-1,4-benzodioxin-6-yl)sulfonyl]amino]-5-(trifluorométhyl)phényl]-2-propynyl]oxy] propanoïque, éthyl ester**

En opérant de façon analogue à l'exemple 3, au départ du composé obtenu selon la préparation 159 et de chlorure de (2,3-dihydro-1,4-benzodioxin-6-sulforiyle, on obtient le produit attendu sous forme d'une huile jaune (rendement = 68 %).
$^{1}H$ KMN ($DMSOd_6$, 300 MHz) δ : 10,07 (s, 1H) ; 7,70 (m, 2H) ; 7,48 (d, 1H) ; 7,29 (m, 2H) ; 7,00 (d, 1H) ; 4,51 (d, 1H) ; 4,38 (d, 1H) ; 4,31 (m, 5H) ; 4,16 (m, 2H) ; 1,34 (d, 3H) ; 1,20 (t, 3H).

**PREPARATION 163**

**Acide 2-[[3-[2-[[(3,4-dihydro-4-méthyl-2*H*-1,4-benzoxazin-7-yl)sulfonyl]-amino]-5-(trifluorométhyl)phényl]-2-propynyl]oxy]propanoïque, éthyl ester**

En opérant de façon analogue à l'exemple 3, au départ du composé obtenu selon la préparation 159 et de chlorure de 3,4-dihydro-4-méthyl-2*H*-1,4-benzoxazine-7-sulfonyle, on obtient le produit attendu sous forme d'une huile jaune (rendement = 57%).
$^{1}H$ RMN ($DMSOd_6$, 300 MHz) δ : 9,88 (s, 1H) ; 7,69 (m, 2H) ; 7,49 (d, 1H) ; 7,02 (m, 2H) ; 7,78 (d, 1H) ; 4,51 (d, 1H) ; 4,37 (d, 1H) ; 4,28 (m, 3H) ; 4,15 (m, 2H) ; 3,27 (m, 2H) ; 2,80 (s, 3H) ; 1,33 (d, 3H) ; 1,20 (t, 3H).

**PREPARATION 164**

**Acide 2-[[3-[2-[[(2,5-diméthoxyphényl)sulfonyl]amino]-5-(trifluorométhyl)-phényl]-2-propynyl]oxy]propanoïque, éthyl ester**

En opérant de façon analogue à l'exemple 3, au départ du composé obtenu selon la préparation 159 et de chlorure de 2,5-diméthoxybenzènesulfonyle, on obtient le produit attendu sous forme d'une huile jaune (rendement = 65 %).
$^{1}H$ RMN ($DMSOd_6$, 250 MHz) δ : 9,29 (s, 1H) ; 7,74 (s, 1H) ; 7,67 (d, 1H) ; 7,56 (d, 1H) ; 7,31 (d, 1H) ; 7,18 (m, 2H) ; 4,55 (d, 1H) ; 4,41 (d, 1H) ; 4,25 (q, 1H) ; 4,13 (m, 2H) ; 3,74 (s, 6H) ; 1,32 (d, 3H) ; 1,20 (t, 3H).

**PREPARATION 165**

**Acide (2S)-2-[[3-[2-amino-5-chlorophényl]-2-propynyl]oxy]propanoïque, éthyl ester**

a)- Ester éthylique de l'acide (2S)-2-(2-propynyloxy)propanoïque : ce composé est obtenu, avec un rendement de 24%, par réaction du bromure de propargyle sur l'ester éthylique de l'acide (S)-(-)-lactique préalablement sodé par l'hydrure de sodium dans le tétrahydrofurane (Eb = 70-73°C sous 13 hPa).
b)- En opérant de façon analogue à la préparation 122, au départ de l'ester éthylique de l'acide (2S)-2-(2-propynyloxy) propanoïque, on obtient le produit attendu sous forme d'une huile jaune (rendement = 99 %).
$^1$H RMN (DMSO$d_6$, 300 MHz) δ : 7,14 (d, 1H) ; 7,08 (dd, 1H) ; 6,69 (d, 1H) ; 5,56 (s, 2H) ; 4,53 (d, 1H) ; 4,42 (d, 1H) ; 4,25 (q, 1H) ; 4,13 (m, 2H) ; 1,30 (d, 3H) ; 1,21 (t, 3H).

**PREPARATION 166**

**Acide (2S)-2-[[3-[5-chloro-2-[[(6-benzothiazolyl)sulfonyl]amino]phényl]-2-propynyl]oxy]propanoïque, éthyl ester**

En opérant de façon analogue à l'exemple 3, au départ du composé obtenu selon la préparation 165 et de chlorure de 6-benzothiazolesulfonyle, on obtient le produit attendu sous forme d'un solide blanc (rendement = 43 %).
$^1$H RMN (DMSO$d_6$, 300 MHz) δ : 10,11 (s, 1H) ; 9,61 (s, 1H) ; 8,60 (d, 1H) ; 8,22 (d, 1H) ; 7,84 (dd, 1H) ; 7,40 (m, 2H) ; 7,30 (d, 1H) ; 4,32 (d. 1H) ; 4,15 (m, 4H) ; 1,28 (d, 3H) ; 1,21 (t, 3H).

**PREPARATION 167**

**Acide (2R)-2-[[3-[2-amino-5-chlorophényl]-2-propynyl]oxy]propanoïque, méthyl ester**

a)- Ester méthylique de l'acide (2R)-2-(2-propynyloxy)propanoïque : ce composé est obtenu, avec un rendement de 9,5%, par réaction du bromure de propargyle sur l'ester méthylique de l'acide (R)-(+)-lactique préalablement sodé par l'hydrure de sodium dans le tétrahydrofurane (Eb = 81-88°C à pression atmosphérique)
b)- En opérant de façon analogue à la préparation 122, au départ de l'ester méthylique de l'acide (2R)-2-(2-propynyloxy)propanoïque, on obtient le produit attendu sous forme d'une huile jaune (Rendement = 89 %).
$^1$H RMN (DMSO$d_6$, 300 MHz) δ : 7,14 (d, 1H) ; 7,08 (dd, 1H) ; 6,69 (d, 1H) ; 5,57 (s, 2H) ; 4,53 (d, 1H) ; 4,41 (d, 1H) ; 4,25 (q, 1H) ; 3,66 (s, 3H) ; 1,31 (d, 3H).

**PREPARATION 168**

**Acide (2R)-2-[[3-[5-chloro-2-[[(6-benzothiazolyl)sulfonyl]amino]phényl]-2-propynyl]oxy]propanoïque, méthyl ester**

En opérant de façon analogue à l'exemple 3, au départ du composé obtenu selon la préparation 167 et de chlorure de 6-benzothiazolesulfonyle, on obtient le produit attendu sous forme d'un solide blanc (rendement = 65 %).
F = 136°C.

**PREPARATION 169**

**Acide 6-[5-chloro-2-[[(2-méthyl-7-benzothiazolyl)sulfonyl]amino] phényl]-5-hexynoïque, méthyl ester**

En opérant de façon analogue à la préparation 55, au départ de chlorure de 2-méthyl-7-benzothiazolesulfonyle, on obtient le produit attendu sous forme d'une huile brune (rendement = 97 %).
$^1$H RMN (DMSO$d_6$, 300 MHz) δ : 10,21 (s, 1H) ; 8,13 (m, 1H) ; 7,60 (m, 2H) ; 7,38 (dd, 1H) ; 7,28 (m, 2H) ; 3,61 (s, 3H) ; 2,78 (s, 3H) ; 2,33 (t, 2H) ; 2,04 (t, 2H) ; 1,55 (quin, 2H).

**PREPARATION 170**

**Acide 6-[2-[[(1-acétyl-1*H*-indol-5-yl)sulfonyl]amino]-5-chlorophényl]-5-hexynoïque, méthyl ester**

En opérant de façon analogue à la prépparation 55, au départ de chlorure de 1-acétyl-1*H*-indole-5-sulfonyle, on obtient le produit attendu sous forme d'une huile brune (rendement = 79 %).
$^1$H RMN (DMSO$d_b$, 300 MHz) δ : 9,72 (s, 1H) ; 8,42 (d, 1H) ; 8,00 (d, 1H) ; 7,99 (d, 1H) ; 7,62 (dd, 1H) ; 7,33 (m, 3H) ; 6,87 (d, 1H) ; 3,59 (s, 3H) ; 2,67 (s, 3H) ; 2,35 (t, 2H) ; 2,27 (y, 2H) ; 1,64 (quin, 2H).

**PREPARATION 171**

**Acide 5-[5-chloro-2-[[(2-méthyl-7-benzothiazolyl)sulfonyl]amino] phényl]-4-pentynoïque, méthyl ester**

En opérant de façon analogue à l'exemple 3, au départ du composé obtenu selon la préparation 2 et du chlorure de 2-méthyl-7-benzothiazolesulfonyle, on obtient le produit attendu sous forme d'un solide amorphe (rendement = 45 %).
$^1$H RMN (DMSO$d_6$, 300 MHz) δ : 10,15 (s, 1H) ; 8,15 (m, 1H) ; 7,60 (m, 2H) ; 7,40 (dd, 1H) ; 7,31 (d, 1H) ; 7,23 (d, 1H) ; 3,63 (s, 3H) ; 2,79 (s, 3H) ; 2,37 (m, 2H) ; 2,25 (m, 2H).

**PREPARATION 172**

**Acide 5-[2-[[(2-amino-6-benzoxazolyl)sulfonyl]amino]-5-chlorophényl]-4-pentynoïque, méthyl ester**

En opérant de façon analogue à l'exemple 3, au départ du composé obtenu selon la préparation 2 et du chlorure de 2-amino-6-benzoxazolesulfonyle, on obtient le produit attendu sous forme d'un solide blanc (rendement = 9 %).
F = 170-171˚C.

**PREPARATION 173**

**Acide 5-[5-chloro-2-[[(2,3-dihydro-5-benzofuranyl)sulfonyl]amino]phényl]-4-pentynoïque, méthyl ester**

En opérant de façon analogue à l'exemple 3, au départ du composé obtenu selon la préparation 2 et du chlorure de 2,3-dihydro-5-benzofuranesulfonyle, on obtient le produit attendu sous forme d'un solide amorphe (rendement = 98 %).
$^1$H RMN (DMSO$d_6$, 300 MHz) δ : 9,39 (s, 1H) ; 7,60 (d, 1H) ; 7,44 (dd, 1H) ; 7,31 (m, 3H) ; 6,85 (d, 1H) ; 4,62 (t, 2H) ; 3,65 (s, 3H) ; 3,21 (t, 2H) ; 2,60 (m, 4H).

**PREPARATION 174**

**Acide 5-[2-[[(2-amino-6-benzothiazolyl)sulfonyl]amino]-5-chlorophényl]-4-pentynoïque, méthyl ester**

En opérant de façon analogue à l'exemple 3, au départ du composé obtenu selon la préparation 2 et du chlorure de 2-amino-6-benzothiazolesulfonyle, on obtient le produit attendu sous forme d'un solide blanc (rendement = 93 %).
F = 175˚C.

**PREPARATION 175**

***N*-(2- bromo- 4- méthylphényl)- 6- benzothiazolesulfonamide** En opérant de façon analogue à l'exemple 3, au départ de la 2-bromo-4-méthylaniline et du chlorure de 6-benzothiazolesulfonyle, on obtient le produit attendu sous forme d'un solide orange (rendement = 76 %).
F = 171-174 ˚C.

**PREPARATION 176**

***N*-(2-bromo-4-méthylphényl)benzènesulfonamide**

En opérant de façon analogue à la préparation 120, au départ de la 2-bromo-4-méthylaniline, on obtient le produit attendu sous forme d'un solide beige (rendement = 95 %).
$^1$H RMN (DMSO$d_6$, 250 MHz) δ : 9,75 (s, 1H) ; 7,69 (m, 3H) ; 7,63 (m, 2H) ; 7,40 (s, 1H) ; 7,09 (d, 1H) ; 7,02 (d, 1H) ; 2,23 (s, 3H).

**PREPARATION 177**

***N*-(4-chloro-2-iodophényl)-6-benzothiazolesulfonamide**

En opérant de façon analogue à la préparation 120, au départ du chlorure de 6-benzothiazolesulfonyle, et en utilisant le fluorure de tétrabutylammonium en réaction dans le THF pour éliminer le composé di-condensé, on obtient le produit attendu sous forme d'un solide jaune (rendement = 91 %).
F = 162˚C.

**PREPARATION 178**

**Acide 6-[5-chloro-2-[[(4-fluoro-3-nitrophényl)sulfonyl]amino]phényl]-5-hexynoïque, méthyl ester**

En opérant de façon analogue à la préparation 55, au départ du chlorure de 4-fluoro-3-nitrobenzènesulfonyle, et en utilisant la triéthylamine comme agent basique, on obtient le produit attendu sous forme d'une huile orange (rendement = 98 %).
$^1$H RMN (DMSO$d_6$, 300 MHz) δ : 10,31 (s, 1H) ; 8,38 (dd, 1H) ; 8,00 (m, 1H) ; 7,81 (dd, 1H) ; 7,41 (s, 1H) ; 7,38 (m, 1H) ; 7,23 (m, 1H) ; 3,60 (s, 3H) ; 2,41 (t, 2H) ; 2,34 (t, 2H) ; 1,68 (quin, 2H).

**Exemple 9**

**Acide 1-(phénylsulfonyl)-1*H*-indole-2-propanoïque, méthyl ester**

En opérant de façon analogue à l'exemple 1, au départ du composé obtenu selon la préparation 24, on obtient le produit attendu sous forme d'un solide blanc (rendement = 75 %).
F = 95-99 ˚C.

**Exemple 10**

**Acide 1-(phénylsulfonyl)-1*H*-indole-2-propanoïque**

En opérant de façon analogue à l'exemple 2, au départ du composé obtenu selon l'exemple 9, on obtient le produit attendu sous forme d'un solide blanc (rendement = 99 %).
F = 180-185 ˚C.

**Exemple 11**

**Acide 5-chloro-1-[(4-méthylphényl)sulfonyl]-1*H*-indole-2-propanoïque, méthyl ester**

En opérant de façon analogue à l'exemple 1, au départ du composé obtenu selon la préparation 25, on obtient le produit attendu sous forme d'un solide beige (rendement = 89 %).
F = 100-103 ˚C.

**Exemple 12**

**Acide 5-chloro-1-[(4-méthylphényl)sulfonyl]-1*H*-indole-2-propanoïque**

En opérant de façon analogue à l'exemple 2, au départ du composé obtenu selon l'exemple 11, on obtient le produit attendu sous forme d'un solide beige (rendement = 93 %).
F = 165-168 ˚C.

**Exemple 13**

**Acide 5-chloro-1-[(2,3-dichlorophényl)sulfonyl]-1*H*-indole-2-propanoïque, méthyl ester**

En opérant de façon analogue à l'exemple 1, au départ du composé obtenu selon la préparation 26, on obtient le produit attendu sous forme d'une huile jaune (rendement = 96 %).
$^1$H RMN (DMSO$d_6$, 300 MHz) δ = 2,77 (t, 2H), 3,15 (t, 2H), 3,59 (s, 3H), 6,65 (s, 1H), 7,26 (dd, 1H), 7,67 (m, 3H), 7,84 (dd, 1H), 8,04 (dd, 1H).

**Exemple 14**

**Acide 5-chloro-1-[(2,3-dichlorophényl)sulfonyl]-1*H*-indole-2-propanoïque**

En opérant de façon analogue à l'exemple 2, au départ du composé obtenu selon l'exemple 13, on obtient le produit attendu sous forme d'un solide blanc (rendement = 76 %).
F = 163-166 ˚C.

**Exemple 15**

**Acide 5-chloro-1-[(3-méthylphényl)sulfonyl]-1*H*-indole-2-propanoïque, méthyl ester**

En opérant de façon analogue à l'exemple 1, au départ du composé obtenu selon la préparation 27, on obtient le produit attendu sous forme d'un solide beige (rendement = 68 %).
F = 105-108 ˚C.

**Exemple 16**

**Acide 5-chloro-1-[(3-méthylphényl)sulfonyl]-1*H*-indole-2-propanoïque**

En opérant de façon analogue à l'exemple 2, au départ du composé obtenu selon l'exemple 15, on obtient le produit attendu sous forme d'un solide blanc (rendement = 92 %).
F = 161-165 ˚C.

**Exemple 17**

**Acide 5-chloro-1-[(2,4-dichlorophényl)sulfonyl]-1*H*-indole-2-propanoïque, méthyl ester**

En opérant de façon analogue à l'exemple 1, au départ du composé obtenu selon la préparation 28, on obtient le produit attendu sous forme d'une huile jaune (rendement = 83 %.
$^1$H RMN (DMSO$d_6$, 300 MHz) $\delta$ = 2,76 (t, 2H), 3,15 (t, 2H), 3,59 (s, 3H), 6,63 (s, 1H), 7,26 (dd, 1H), 7,71 (m, 3H), 7,94 (m, 2H).

**Exemple 18**

**Acide 5-chloro-1-[(2,4-dichlorophényl)sulfonyl]-1*H*-indole-2-propanoïque**

En opérant de façon analogue à l'exemple 2, au départ du composé obtenu selon l'exemple 17, on obtient le produit attendu sous forme d'un solide blanc (rendement = 76 %).
F = 179-181 ˚C.

**Exemple 19**

**Acide 5-chloro-1-[[4-(trifluorométhyl)phényl]sulfonyl]-1*H*-indole-2-propanoïque, méthyl ester**

En opérant de façon analogue à l'exemple 1, au départ du composé obtenu selon la préparation 29, on obtient le produit attendu sous forme d'un solide jaune (rendement = 97 %).
F = 82-86 ˚C.

**Exemple 20**

**Acide 5-chloro-1-[[4-(trifluorométhyl)phényl]sulfonyl]-1*H*-indole-2-propanoïque**

En opérant de façon analogue à l'exemple 2, au départ du composé obtenu selon l'exemple 19, on obtient le produit attendu sous forme d'un solide beige (rendement = 78 %).
F = 179-182 ˚C.

**Exemple 21**

**Acide 5-chloro-1-[(4-méthoxyphényl)sulfonyl]-1*H*-indole-2-propanoïque, méthyl ester**

En opérant de façon analogue à l'exemple 1, au départ du composé obtenu selon la préparation 30, on obtient le produit attendu sous forme d'un solide jaune (rendement = 68 %).
F = 98-99 ˚C.

**Exemple 22**

**Acide 5-chloro-1-[(4-méthoxyphényl)sulfonyl]-1*H*-indole-2-propanoïque**

En opérant de façon analogue à l'exemple 2, au départ du composé obtenu selon l'exemple 21, on obtient le produit attendu sous forme d'un solide blanc (rendement = 86 %).
F = 95-98 °C.

**Exemple 23**

**Acide 1-[(4-acétylphényl)sulfonyl]-5-chloro-1*H*-indole-2-propanoïque, méthyl ester**

En opérant de façon analogue à l'exemple 1, au départ du composé obtenu selon la préparation 31, on obtient le produit attendu sous forme d'un solide jaune (rendement = 93 %).
F = 83-87 °C.

**Exemple 24**

**Acide 1-[(4-acétylphényl)sulfonyl]-5-chloro-1*H*-indole-2-propanoïque**

En opérant de façon analogue à l'exemple 2, au départ du composé obtenu selon l'exemple 23, on obtient le produit attendu sous forme d'un solide jaune (rendement = 70 %).
F = 159-161 °C.

**Exemple 25**

**Acide 5-chloro-1-[(4-phénylphényl)sulfonyl]-1*H*-indole-2-propanoïque, méthyl ester**

En opérant de façon analogue à l'exemple 1, au départ du composé obtenu selon la préparation 32, on obtient le produit attendu sous forme d'une huile jaune (rendement = 58 %).
$^1$H RMN (DMSO$d_6$, 250 MHz) $\delta$ = 2,84 (t, 2H), 3,37 (t, 2H), 3,62 (s, 3H), 6,62 (s, 1H), 7,34 (dd, 1H), 7,47 (m, 3H), 7,61 (s, 1H), 7,68 (dd, 2H), 7,88 (d, 4H), 8,07 (d, 1H).

**Exemple 26**

**Acide 5-chloro-1-[(4-phénylphényl)sulfonyl]-1*H*-indole-2-propanoïque**

En opérant de façon analogue à l'exemple 2, au départ du composé obtenu selon l'exemple 25, on obtient le produit attendu sous forme d'un solide blanc (rendement = 78 %).
F = 160-162 °C.

**Exemple 27**

**Acide 5-chloro-1-[(3-méthoxyphényl)sulfonyl]-1*H*-indole-2-propanoïque, méthyl ester**

En opérant de façon analogue à l'exemple 1, au départ du composé obtenu selon la préparation 34, on obtient le produit attendu sous forme d'un solide jaune (rendement = 63 %).
F = 106-109 °C.

**Exemple 28**

**Acide 5-chloro-1-[(3-méthoxyphényl)sulfonyl]-1*H*-indole-2-propanoïque**

En opérant de façon analogue à l'exemple 2, au départ du composé obtenu selon l'exemple 27, on obtient le produit attendu sous forme d'un solide blanc (rendement = 62 %).
F = 182-184 °C.

**Exemple 29**

**Acide 5-chloro-1-[(2,5-diméthoxyphényl)sulfonyl]-1*H*-indole-2-propanoïque, méthyl ester**

En opérant de façon analogue à l'exemple 1, au départ du composé obtenu selon la préparation 35, on obtient le produit attendu sous forme d'un solide jaune (rendement = 85 %).
F = 120-124°C.

**Exemple 30**

**Acide 5-chloro-1-[(2,5-diméthoxyphényl)sulfonyl]-1*H*-indole-2-propanoïque**

En opérant de façon analogue à l'exemple 2, au départ du composé obtenu selon l'exemple 29, on obtient le produit attendu sous forme d'un solide blanc (rendement = 88 %).
F = 185-189 °C.

**Exemple 31**

**Acide 5-chloro-1-[[4-(1,1-diméthyléthyl)phényl]sulfonyl]-1*H*-indole-2-propanoïque, méthyl ester**

En opérant de façon analogue à l'exemple 1, au départ du composé obtenu selon la préparation 36, on obtient le produit attendu sous forme d'un solide beige (rendement = 87. %).
F = 130-133 °C.

**Exemple 32**

**Acide 5-chloro-1-[[4-(1,1-diméthyléthyl)phényl]sulfonyl]-1*H*-indole-2-propanoïque**

En opérant de façon analogue à l'exemple 2, au départ du composé obtenu selon l'exemple 31, on obtient le produit attendu sous forme d'un solide blanc (rendement = 98 %).
F = 169-171 °C.

**Exemple 33**

**Acide 5-chloro-1-[(4-éthylphényl)sulfonyl]-1*H*-indole-2-propanoïque, méthyl** ester

En opérant de façon analogue à l'exemple 1, au départ du composé obtenu selon la préparation 37, on obtient le produit attendu sous forme d'une huile jaune (rendement = 86 %).
$^1$H RMN (DMSO$d_6$, 300 MHz) $\delta$ =1,12 (t, *J* = 7,56 Hz, 3H), 2,63 (q, 2H), 2,81 (t, *J* = 7,62 Hz, 2H), 3,28 (t, *J* = 4,08 Hz, 2H), 3,61 (s, 3H), 6,57 (s, 1H), 7,31 (dd, *J* = 8,9 Hz, 2,22 Hz, 1H), 7,45 (d, *J* = 12,8 Hz, 2H), 7,59 (s,1H), 7,74 (d, *J* = 12,78 Hz, 2H), 8,02 (d, *J* = 8,91, 1H).

**Exemple 34**

**Acide 5-chloro-1-[(4-éthylphényl)sulfonyl]-1*H*-indole-2-propanoïque**

En opérant de façon analogue à l'exemple 2, au départ du composé obtenu selon l'exemple 33, on obtient le produit attendu sous forme d'un solide blanc (rendement = 70 %).
F = 130-133 °C.

**Exemple 35**

**Acide 5-chloro-1-[[4-(1-méthyléthyl)phényl]sulfonyl]-1*H*-indole-2-propanoïque, méthyl ester**

En opérant de façon analogue à l'exemple 1, au départ du composé obtenu selon la préparation 38, on obtient le produit attendu sous forme d'un solide blanc (rendement = 58 %).
F = 90-94 °C.

**Exemple 36**

**Acide 5-chloro-1-[[4-(1-méthyléthyl)phényl]sulfonyl]-1*H*-indole-2-propanoïque**

En opérant de façon analogue à l'exemple 2, au départ du composé obtenu selon l'exemple 35, on obtient le produit attendu sous forme d'un solide blanc (rendement = 70 %).
F = 150-154 °C.

**Exemple 37**

**Acide 5-chloro-1-[(4-propylphényl)sulfonyl]-1*H*-indole-2-propanoïque, méthyl ester**

En opérant de façon analogue à l'exemple 1, au départ du composé obtenu selon la préparation 39, on obtient le produit attendu sous forme d'un solide blanc (rendement = 87 %).
F = 85-88 °C.

**Exemple 38**

**Acide 5-chloro-1-[(4-propylphényl)sulfonyl]-1*H*-indole-2-propanoïque**

En opérant de façon analogue à l'exemple 2, au départ du composé obtenu selon l'exemple 37, on obtient le produit attendu sous forme d'un solide jaune (rendement = 92 %).
F = 144-148 °C.

**Exemple 39**

**Acide 5-chloro-1-[(4-pentylphényl)sulfonyl]-1*H*-indole-2-propanoïque, méthyl ester**

En opérant de façon analogue à l'exemple 1, au départ du composé obtenu selon la préparation 40, on obtient le produit attendu sous forme d'une huile jaune (rendement = 76 %).
$^1$H RMN (DMSO$d_6$, 300 MHz) δ = 0,81 (t, 3H), 1,22 (m, 4H), 1,50 (m, 2H), 2,58 (t, 2H), 2,81 (t, 2H), 3,28 (t, 2H), 3,61 (s, 3H), 6,58 (s, 1H), 7,31 (dd, 1H), 7,39 (d, 2H), 7,59 (d, 1H), 7,72 (d, 2H), 8,02 (d, 1H).

**Exemple 40**

**Acide 5-chloro-1-[(4-pentylphényl)sulfonyl]-1*H*-indole-2-propanoïque**

En opérant de façon analogue à l'exemple 2, au départ du composé obtenu selon l'exemple 39, on obtient le produit attendu sous forme d'un solide blanc (rendement = 79 %).
F = 131-134 °C.

**Exemple 41**

**Acide 5-chloro-1-[(3,5-diméthylphényl)sulfonyl]-1*H*-indole-2-propanoïque, méthyl ester**

En opérant de façon analogue à l'exemple 1, au départ du composé obtenu selon la préparation 41, on obtient le produit attendu sous forme d'un solide blanc (rendement = 90 %).
F = 146-150 °C.

**Exemple 42**

**Acide 5-chloro-1-[(3,5-diméthylphényl)sulfonyl]-1*H*-indole-2-propanoïque**

En opérant de façon analogue à l'exemple 2, au départ du composé obtenu selon l'exemple 41, on obtient le produit attendu sous forme d'un solide blanc (rendement = 88 %).
F = 189-193 °C.

**Exemple 43**

**Acide 5-chloro-1-[(2,4,6-triméthylphényl)sulfonyl]-1*H*-indole-2-propanoïque, méthyl ester**

En opérant de façon analogue à l'exemple 1, au départ du composé obtenu selon la préparation 42, on obtient le produit attendu sous forme d'un solide blanc (rendement = 75 %).
F = 145-148 °C.

**Exemple 44**

**Acide 5-chloro-1-[(2,4,6-triméthylphényl)sulfonyl]-1*H*-indole-2-propanoïque**

En opérant de façon analogue à l'exemple 2, au départ du composé obtenu selon l'exemple 43, on obtient le produit attendu sous forme d'un solide blanc (rendement = 50 %).
F = 132-134 °C.

**Exemple 45**

**Acide 5-chloro-1-[(4-chlorophényl)sulfonyl]-1*H*-indole-2-propanoïque, méthyl ester**

En opérant de façon analogue à l'exemple 1, au départ du composé obtenu selon la préparation 43, on obtient le produit attendu sous forme d'un solide blanc (rendement = 98 %).
F = 89-92 °C.

**Exemple 46**

**Acide 5-chloro-1-[(4-chlorophényl)sulfonyl]-1*H*-indole-2-propanoïque**

En opérant de façon analogue à l'exemple 2, au départ du composé obtenu selon l'exemple 45, on obtient le produit attendu sous forme d'un solide blanc (rendement =81%).
F = 158-160 °C.

**Exemple 47**

**Acide 5-chloro-1-[(4-fluorophényl)sulfonyl]-1*H*-indole-2-propanoïque, méthyl ester**

En opérant de façon analogue à l'exemple 1, au départ du composé obtenu selon la préparation 44, on obtient le produit attendu sous forme d'un solide blanc (rendement = 79 %).
F = 129-131 °C.

**Exemple 48**

**Acide 5-chloro-1-[(4-fluorophényl)sulfonyl]-1*H*-indole-2-propanoïque**

En opérant de façon analogue à l'exemple 2, au départ du composé obtenu selon l'exemple 47, on obtient le produit attendu sous forme d'un solide blanc (rendement = 78 %).
F = 145-148 °C.

**Exemple 49**

**Acide 5-chloro-1-[(4-chloro-3-méthylphényl)sulfonyl]-1*H*-indole-2-propanoïque, méthyl ester**

En opérant de façon analogue à l'exemple 1, au départ du composé obtenu selon la préparation 45, on obtient le produit attendu sous forme d'une huile jaune (rendement = 59 %).
$^1$H RMN ($DMSOd_6$, 300 MHz) $\delta$ = 2,35 (s, 3H), 2,82 (t, 2H), 3,27 (t, 2H), 3,60 (s, 3H), 6,61 (s, 1H), 7,31 (dd, 1H), 7,62 (m, 3H), 7,90 (s, 1H), 8,00 (d, *J* = 9,48, 1H).

**Exemple 50**

**Acide 5-chloro-1-[(4-chloro-3-méthylphényl)sulfonyl]-1*H*-indole-2-propanoïque**

En opérant de façon analogue à l'exemple 2, au départ du composé obtenu selon l'exemple 49, on obtient le produit attendu sous forme d'un solide blanc (rendement = 81 %).
F = 160-164 °C.

**Exemple 51**

**Acide 5-chloro-1-[[3-(trifluorométhyl)phényl]sulfonyl]-1*H*-indole-2-propanoïque, méthyl ester**

En opérant de façon analogue à l'exemple 1, au départ du composé obtenu selon la préparation 46, on obtient le produit attendu sous forme d'un solide blanc (rendement = 39 %).
F = 98-100 °C.

**Exemple 52**

**Acide 5-chloro-1-[[3-(trifluorométhyl)phényl]sulfonyl]-1*H*-indole-2-propanoïque**

En opérant de façon analogue à l'exemple 2, au départ du composé obtenu selon l'exemple 51, on obtient le produit attendu sous forme d'un solide blanc (rendement = 81 %).
F = 203-206 °C.

**Exemple 53**

**Acide 1-[[4-(acétylamino)phényl]sulfonyl]-5-chloro-1*H*-indole-2-propanoïque, méthyl ester**

En opérant de façon analogue à l'exemple 1, au départ du composé obtenu selon la préparation 47, on obtient le produit attendu sous forme d'un solide beige (rendement = 71 %).
F = 154-157 °C.

**Exemple 54**

**Acide 5-chloro-1-[(4-cyanophényl)sulfonyl]-1*H*-indole-2-propanoïque, méthyl ester**

En opérant de façon analogue à l'exemple 1, au départ du composé obtenu selon la préparation 48, on obtient le produit attendu sous forme d'un solide beige (rendement = 72 %).
F = 155-159 °C.

**Exemple 55**

**Acide 5-chloro-1-[(4-phénoxyphényl)sulfonyl]-1*H*-indole-2-propanoïque, méthyl ester**

En opérant de façon analogue à l'exemple 1, au départ du composé obtenu selon la préparation 49, on obtient le produit attendu sous forme d'une huile jaune (rendement = 80 %).
$^1$H RMN (DMSO$d_6$, 300 MHz) $\delta$ = 2,81 (t, 2H), 3,27 (t, 2H), 3,61 (s, 3H), 6,59 (s, 1H), 7,02-7,13 (m, 4H), 7,30 (m, 2H), 7,45 (m, 2H), 7,60 (s, 1H), 7,83 (d, 2H), 8,01 (d, 1H).

**Exemple 56**

**Acide 5-chloro-1-[(4-phénoxyphényl)sulfonyl]-1*H*-indole-2-propanoïque**

En opérant de façon analogue à l'exemple 2, au départ du composé obtenu selon l'exemple 55, on obtient le produit attendu sous forme d'un solide blanc (rendement = 93 %).
F = 70-75 °C.

**Exemple 57**

**Acide 5-chloro-1-[(1-naphtalènyl)sulfonyl]-1*H*-indole-2-propanoïque, méthyl ester**

En opérant de façon analogue à l'exemple 1, au départ du composé obtenu selon la préparation 50, on obtient le produit attendu sous forme d'un solide jaune (rendement = 80 %).
F = 88-93 ˚C.

**Exemple 58**

**Acide 5-chloro-1-[(1-naphtalènyl)sulfonyl]-1*H*-indole-2-propanoïque**

En opérant de façon analogue à l'exemple 2, au départ du composé obtenu selon l'exemple 57, on obtient le produit attendu sous forme d'un solide blanc (rendement = 98 %).
F = 165-175 ˚C.

**Exemple 59**

**Acide 5-chloro-1-[(2-naphtalényl)sulfonyl]-1*H*-indole-2-propanoïque, méthyl ester**

En opérant de façon analogue à l'exemple 1, au départ du composé obtenu selon la préparation 51, on obtient le produit attendu sous forme d'une huile jaune (rendement = 90 %).
$^1$H RMN ($DMSOd_6$, 300 MHz) δ=2,84 (t, 2H), 3,35 (t, 2H), 3,60 (s, 3H), 6,59 (s, 1H), 7,31 (dd, 1H), 7,57 (s, 1H), 7,69 (m, 3H), 7,99-8,2(m, 4H), 8,72 (s, 1H).

**Exemple 60**

**Acide 5-chloro-1-[(2-naphtalènyl)sulfonyl]-1*H*-indole-2-propanoïque**

En opérant de façon analogue à l'exemple 2, au départ du composé obtenu selon l'exemple 59, on obtient le produit attendu sous forme d'un solide blanc (rendement = 48 %).
F = 160˚C.

**Exemple 61**

**Acide 5-chloro-1-[(4-méthyl-1-naphtalènyl)sulfonyl]-1*H*-indole-2-propanoïque, méthyl ester**

En opérant de façon analogue à l'exemple 1, au départ du composé obtenu selon la préparation 52, on obtient le produit attendu sous forme d'une huile jaune (rendement = 94 %).
$^1$H RMN ($DMSOd_6$, 300 MHz) δ = 2,72 (t, 5H), 3,14 (t, 2H), 3,57 (s, 3H), 6,64 (s, 1H), 7,30 (dd, 1H), 7,53 (m, 2H), 7,69 (m, 3H), 7,92 (d, 1H), 8,21 (m, 1H), 8,37 (m 1H).

**Exemple 62**

**Acide 5-chloro-1-[(4-méthyl-1-naphtalènyl)sulfonyl]-1*H*-indole-2-propanoïque**

En opérant de façon analogue à l'exemple 2, au départ du composé obtenu selon l'exemple 61, on obtient le produit attendu sous forme d'un solide blanc (rendement = 95 %).
F = 190-196 ˚C.

**Exemple 63**

**Acide 5-chloro-1-[[5-(aminoacétyl)-1-naphtalènyl]sulfonyl]-1*H*-indole-2-propanoïque, méthyl ester**

En opérant de façon analogue à l'exemple 1, au départ du composé obtenu selon la préparation 53, on obtient le produit attendu sous forme d'un solide jaune (rendement = 66 %).
F = 206-210 ˚C.

**Exemple 64**

**Acide 5-chloro-1-[[5-(aminoacétyl)-1-naphtalènyl]sulfonyl]-1*H*-indole-2-propanoïque**

En opérant de façon analogue à l'exemple 2, au départ du composé obtenu selon l'exemple 63, on obtient le produit attendu sous forme d'un solide blanc (rendement = 96 %).
F = 130-135 °C.

**Exemple 65**

**Acide 5-chloro-1-[(8-quinolinyl)sulfonyl]-1*H*-indole-2-propanoïque, méthyl ester**

En opérant de façon analogue à l'exemple 1, au départ du composé obtenu selon la préparation 54, on obtient le produit attendu sous forme d'un solide brun (rendement = 78 %).
F = 157-161 °C.

**Exemple 66**

**Acide 5-chloro-1-[(8-quinolinyl)sulfonyl]-1*H*-indole-2-propanoïque**

En opérant de façon analogue à l'exemple 2, au départ du composé obtenu selon l'exemple 65, on obtient le produit attendu sous forme d'un solide brun (rendement = 80 %).
F = 215-222 °C.

**Exemple 67**

**Acide 5-chloro-1-(phénylsulfonyl)-1*H*-indole-2-butanoïque, méthyl ester**

En opérant de façon analogue à l'exemple 1, au départ du composé obtenu selon la préparation 55, on obtient le produit attendu sous forme d'un solide blanc (rendement = 81 %).
F = 109-112 °C.

**Exemple 68**

**Acide 5-chloro-1-(phénylsulfonyl)-1*H*-indole-2-butanoïque**

En opérant de façon analogue à l'exemple 2, au départ du composé obtenu selon l'exemple 67, on obtient le produit attendu sous forme d'un solide rose pâle (rendement = 92 %).
F = 198-202 °C.

**Exemple 69**

**Acide 5-chloro-1-[(2,3-dichlorophényl)sulfonyl]-1*H*-indole-2-butanoïque, méthyl ester**

En opérant de façon analogue à l'exemple 1, au départ du composé obtenu selon la préparation 56, on obtient le produit attendu sous forme d'une poudre rose (rendement = 72 %).
F = 115-117 °C.

**Exemple 70**

**Acide 5-chloro-1-[(2,3-dichlorophényl)sulfonyl]-1*H*-indole-2-butanoïque**

En opérant de façon analogue à l'exemple 2, au départ du composé obtenu selon l'exemple 69, on obtient le produit attendu sous forme d'un poudre blanche (rendement = 93 %).
F = 195-197 °C.

**Exemple 71**

**Acide 5-chloro-1-[(4-méthoxyphényl)sulfonyl]-1*H*-indole-2-butanoïque, méthyl ester**

En opérant de façon analogue à l'exemple 1, au départ du composé obtenu selon 1a préparation 57, on obtient le produit attendu sous forme d'une poudre jaune (rendement = 98 %).
F = 97-98 °C.

**Exemple 72**

**Acide 5-chloro-1-[(4-méthoxyphényl)sulfonyl]-1*H*-indole-2-butanoïque**

En opérant de façon analogue à l'exemple 2, au départ du composé obtenu selon l'exemple 71, on obtient le produit attendu sous forme d'une poudre rose (rendement = 96 %).
F = 138-142 °C.

**Exemple 73**

**Acide 5-chloro-1-[(8-quinolinyl)sulfonyl]-1*H*-indole-2-butanoïque, méthyl ester**

En opérant de façon analogue à l'exemple 1, au départ du composé obtenu selon la préparation 58, on obtient le produit attendu sous forme d'une poudre rose (rendement = 93 %).
F = 120-124 °C.

**Exemple 74**

**Acide 5-chloro-1-[(8-quinolinyl)sulfonyl]-1*H*-indole-2-butanoïque**

En opérant de façon analogue à l'exemple 2, au départ du composé obtenu selon l'exemple 73, on obtient le produit attendu sous forme d'une poudre blanche (rendement = 64 %).
F=217-219°C.

**Exemple 75**

**Acide 5-chloro-1-[[4-(1-méthyléthyl)phényl]sulfonyl]-1*H*-indole-2-butanoïque, méthyl ester**

En opérant de façon analogue à l'exemple 1, au départ du composé obtenu selon la préparation 59, on obtient le produit attendu sous forme d'une poudre rose (rendement = 81 %).
F = 95-97 °C.

**Exemple 76**

**Acide 5-chloro-1-[[4-(1-méthyléthyl)phényl]sulfonyl]-1*H*-indole-2-butanoïque**

En opérant de façon analogue à l'exemple 2, au départ du composé obtenu selon l'exemple 75, on obtient le produit attendu sous forme d'une poudre blanche (rendement = 95 %).
F = 148 °C.

**Exemple 77**

**Acide 5-chloro-1-[(2-naphtalényl)sulfonyl]-1*H*-indole-2-butanoïque, méthyl ester**

En opérant de façon analogue à l'exemple 1, au départ du composé obtenu selon la préparation 60, on obtient le produit attendu sous forme d'une poudre blanche (rendement =71%).
F = 116-118 °C.

**Exemple 78**

**Acide 5-chloro-1-[(2-naphtalènyl)sulfonyl]-1*H*-indole-2-butanoïque**

En opérant de façon analogue à l'exemple 2, au départ du composé obtenu selon l'exemple 77, on obtient le produit attendu sous forme d'une poudre blanche (rendement = 90 %).
F = 166 °C.

**Exemple 79**

**Acide 5-chloro-1-[(3,5-diméthylphényl)sulfonyl]-1*H*-indole-2-butanoïque, méthyl ester**

En opérant de façon analogue à l'exemple 1, au départ du composé obtenu selon la préparation 61, on obtient le produit attendu sous forme d'une poudre blanche (rendement = 81 %).
F = 140-143 °C.

**Exemple 80**

**Acide 5-chloro-1-[(3,5-diméthylphényl)sulfonyl]-1*H*-indole-2-butanoïque**

En opérant de façon analogue à l'exemple 2, au départ du composé obtenu selon l'exemple 79, on obtient le produit attendu sous forme d'une poudre blanche (rendement = 91 %).
F = 204-206 °C.

**Exemple 81**

**Acide 5-chloro-1-[(3-méthoxylphényl)sulfonyl]-1*H*-indole-2-butanoïque, méthyl ester**

En opérant de façon analogue à l'exemple 1, au départ du composé obtenu selon la préparation 62, on obtient le produit attendu sous forme d'une poudre blanche (rendement = 87 %).
F = 107-109 °C.

**Exemple 82**

**Acide 5-chloro-1-[(3-méthoxylphényl)sulfonyl]-1*H*-indole-2-butanoïque**

En opérant de façon analogue à l'exemple 2, au départ du composé obtenu selon l'exemple 81, on obtient le produit attendu sous forme d'une poudre blanche (rendement = 79 %).
F = 170-172 °C.

**Exemple 83**

**Acide 5-chloro-1-[(2,5-diméthoxyphényl)sulfonyl]-1*H*-indole-2-butanoïque, méthyl ester**

En opérant de façon analogue à l'exemple 1, au départ du composé obtenu selon la préparation 63, on obtient le produit attendu sous forme d'une poudre blanche (rendement = 92 %).
F = 152-154°C.

**Exemple 84**

**Acide 5-chloro-1-[(2,5-diméthoxyphényl)sulfonyl]-1*H*-indole-2-butanoïque**

En opérant de façon analogue à l'exemple 2, au départ du composé obtenu selon l'exemple 83, on obtient le produit attendu sous forme d'une poudre blanche (rendement = 92 %).
F = 201-209 °C.

**Exemple 85**

**Acide 5-chloro-1-[(1-naphtalènyl)sulfonyl]-1*H*-indole-2-butanoïque, méthyl ester**

En opérant de façon analogue à l'exemple 1, au départ du composé obtenu selon la préparation 64, on obtient le produit attendu sous forme d'une poudre crème (rendement = 44 %).
F = 94-97 °C.

**Exemple 86**

**Acide 5-chloro-1-[(1-naphtalènyl)sulfonyl]-1*H*-indole-2-butanoïque**

En opérant de façon analogue à l'exemple 2, au départ du composé obtenu selon l'exemple 85, on obtient le produit attendu sous forme d'une poudre blanche (rendement = 89 %).
F = 206-210 °C.

**Exemple 87**

**Acide 5-fluoro-1-(phénylsulfonyl)-1*H*-indole-2-propanoïque, méthyl ester**

En opérant de façon analogue à l'exemple 3, au départ du composé obtenu selon la préparation 65, on obtient le produit attendu sous forme de cristaux beiges (rendement = 58 %).
F = 79-80 °C.

**Exemple 88**

**Acide 5,6-dichloro-1-(phénylsulfonyl)-1*H*-indole-2-propanoïque, méthyl ester**

En opérant de façon analogue à l'exemple 3, au départ du composé obtenu selon la préparation 66, on obtient le produit attendu sous forme d'un solide blanc (rendement = 23 %).
F = 280 °C.

**Exemple 89**

**Acide 5,6-dichloro-1-(phénylsulfonyl)-1*H*-indole-2-propanoïque**

En opérant de façon analogue à l'exemple 2, au départ du composé obtenu selon l'exemple 88, on obtient le produit attendu sous forme d'une poudre fine rose (rendement = 61 %).
F = 192-198 °C.

**Exemple 90**

**Acide 4,5-dichloro-1-(phénylsulfonyl)-1*H*-indole-2-propanoïque, méthyl ester**

En opérant de façon analogue à l'exemple 3, au départ du composé obtenu selon la préparation 67, on obtient le produit attendu sous forme d'un solide jaune (rendement = 68 %).
F = 142 °C.

**Exemple 91**

**Acide 4,5-dichloro-1-(phénylsulfonyl)-1*H*-indole-2-propanoïque**

En opérant de façon analogue à l'exemple 2, au départ du composé obtenu selon l'exemple 90, on obtient le produit attendu sous forme d'une poudre blanche (rendement = 52 %).
F = 220 °C.

**Exemple 92**

**Acide 1-(phénylsulfonyl)-6-(trifluorométhyl)-1*H*-indole-2-propanoïque, méthyl ester**

En opérant de façon analogue à l'exemple 3, au départ du composé obtenu selon la préparation 68, on obtient le produit attendu sous forme de cristaux blancs (rendement = 38 %).
F = 112-114 °C.

**Exemple 93**

**Acide 1-(phénylsulfonyl)-6-(trifluorométhyl)-1*H*-indole-2-propanoïque**

En opérant de façon analogue à l'exemple 2, au départ du composé obtenu selon l'exemple 92, on obtient le produit attendu sous forme de cristaux blancs (rendement = 72 %).
F = 168-169 °C.

**Exemple 94**

**Acide 5-acétyl-1-(phénylsulfonyl)-1*H*-indole-2-propanoïque, méthyl ester**

En opérant de façon analogue à l'exemple 3, au départ du composé obtenu selon la préparation 69, on obtient le produit attendu sous forme d'un solide blanc (rendement = 41 %).
F = 122-127 °C.

**Exemple 95**

**Acide 5-acétyl-1-(phénylsulfonyl)-1*H*-indole-2-propanoïque**

En opérant de façon analogue à l'exemple 2, au départ du composé obtenu selon l'exemple 94, on obtient le produit attendu sous forme d'une poudre blanche (rendement = 83 %).
F = 175-181 °C.

**Exemple 96**

**Acide 6-chloro-5-fluoro-1-(phénylsulfonyl)-1*H*-indole-2-propanoïque, méthyl ester**

En opérant de façon analogue à l'exemple 3, au départ du composé obtenu selon la préparation 70, on obtient le produit attendu sous forme d'un solide beige (rendement = 51 %).
F = 127-130 °C.

**Exemple 97**

**Acide 6-chloro-5-fluoro-1-(phénylsulfonyl)-1*H*-indole-2-propanoïque**

En opérant de façon analogue à l'exemple 2, au départ du composé obtenu selon l'exemple 96, on obtient le produit attendu sous forme d'un solide marron (rendement = 94 %).
F = 199-204 °C.

**Exemple 98**

**Acide 5,7-dichloro-1-(phénylsulfonyl)-1*H*-indole-2-propanoïque, méthyl ester**

En opérant de façon analogue à l'exemple 3, au départ du composé obtenu selon la préparation 71, on obtient le produit attendu sous forme d'une huile jaune (rendement = 74 %).
$^1$H RMN (DMSO$d_6$, 250 MHz) $\delta$ = 2,77 (t, 2H), 3,24 (t, 2H), 3,61 (s, 3H), 6,73 (s, 1H), 7,40 (d, 1H), 7,55-7,65 (m, 3H), 7,65-7,8 (m, 3H).

**Exemple 99**

**Acide 5,7-dichloro-1-(phénylsulfonyl)-1*H*-indole-2-propanoïque**

En opérant de façon analogue à l'exemple 2, au départ du composé obtenu selon l'exemple 98, on obtient le produit attendu sous forme d'une huile jaune (rendement = 73 %).
$^1H$ RMN ($DMSOd_6$, 250 MHz) δ= 2,63 (t, 2H), 3,19 (t, 2H), 6,71 (s, 1H), 7,40 (d, 1H), 7,55-7,85 (m, 6H).

**Exemple 100**

**Acide 5-cyano-1-(phénylsulfonyl)-1*H*-indole-2-propanoïque, méthyl ester**

En opérant de façon analogue à l'exemple 3, au départ du composé obtenu selon la préparation 72, on obtient le produit attendu sous forme d'une huile jaune (rendement = 12 %).
$^1H$ RMN ($DMSOd_6$, 250 MHz) δ = 2,84 (t, 2H), 3,31 (t, 2H), 3,61 (s, 3H), 6,72 (s, 1H), 7,55-7,9 (m 6H), 8,07 (d, 1H), 8,20 (d, 1H).

**Exemple 101**

**Acide 5-cyano-1-(phénylsulfonyl)-1*H*-indole-2-propanoïque**

En opérant de façon analogue à l'exemple 2, au départ du composé obtenu selon l'exemple 100, on obtient le produit attendu sous forme d'un solide blanc (rendement = 67 %).
F = 187-190 °C.

**Exemple 102**

**Acide 5-benzoyl-1-(phénylsulfonyl)-1*H*-indole-2-propanoïque, méthyl ester**

En opérant de façon analogue à l'exemple 3, au départ du composé obtenu selon la préparation 73, on obtient le produit attendu sous forme d'un solide jaune (rendement = 43 %).
F = 37-51 °C.

**Exemple 103**

**Acide 5-benzoyl-1-(phénylsulfonyl)-1*H*-indole-2-propanoïque**

En opérant de façon analogue à l'exemple 2, au départ du composé obtenu selon l'exemple 102, on obtient le produit attendu sous forme d'un solide jaune (rendement = 83 %).
F = 138 °C.

**Exemple 104**

**Acide 5-chloro-1-(phénylsulfonyl)-1*H*-indole-2-pentanoïque, méthyl ester**

En opérant de façon analogue à l'exemple 1, au départ du composé obtenu selon la préparation 74, on obtient le produit attendu sous forme d'un solide beige (rendement = 75 %).
F = 95-98 °C.

**Exemple 105**

**Acide 5-chloro-1-(phénylsulfonyl)-1*H*-indole-2-pentanoïque**

En opérant de façon analogue à l'exemple 2, au départ du composé obtenu selon l'exemple 104, on obtient le produit attendu sous forme d'un solide blanc (rendement = 79 %).
F = 144-148 °C.

**Exemple 106**

**Acide 1-(phénylsulfonyl)-5-(trifluorométhoxy)-1*H*-indole-2-propanoïque, méthyl ester**

En opérant de façon analogue aux préparations 1, 2, 3 et à l'exemple 1, au départ de 2-iodo-4-(trifluorométhoxy)-1-nitrobenzène, on obtient le produit attendu sous forme d'une huile jaune (rendement de l'étape finale = 64%).
$^1$H RMN (DMSO$d_6$, 300 MHz) δ = 2,82 (t, 2H), 3,30 (t, 2H), 3,62 (s, 3H), 6,66 (s, 1H), 7,28 (ddd, 1H), 7,53-7,57 (m, 1H), 7,57-7,64 (m, 1H), 7,68-7,75 (m, 1H), 7,83-7,88 (m, 1H), 8,11 (d, *J* = 9,1 Hz, 1H).

**Exemple 107**

**Acide 1-(phénylsulfonyl)-5-(trifluorométhoxy)-1*H*-indole-2-propanoïque**

En opérant de façon analogue à l'exemple 2, au départ du composé obtenu selon l'exemple 106, on obtient le produit attendu sous forme d'un solide beige (rendement = 98 %).
F = 138-146 °C.

**Exemple 108**

**Acide 5-chloro-1-(phénylsulfonyl)-1*H*-indole-2-propanoïque, 1-méthyléthyl ester**

On porte à reflux pendant 40 heures un mélange de 130 mg (0,34 mM) d'ester méthylique obtenu selon l'exemple 1, 3 ml d'isopropanol (1-méthyléthanol) et 8,6 mg (0,34 mM) d'oxyde de dibutylétain. Le milieu réactionnel est ensuite concentré sous pression réduite et l'huile résiduelle est reprise dans 10 ml d'acétate d'éthyle. La phase organique obtenue est lavée par une solution de bicarbonate de sodium, puis à l'eau et enfin séchée sur sulfate de magnésium et concentrée sous pression réduite. Le produit obtenu est purifié par chromatographie sur gel de silice en éluant à l'aide d'un mélange toluène/acétate d'éthyle (9/1 ; v/v). On obtient ainsi 96 mg de l'ester attendu sous forme d'une huile jaune (rendement = 69 %).
$^1$H RMN (DMSO$d_6$, 300 MHz) δ =1,14 (d, 6H), 2,75 (t, 2H), 3,26 (t, 2H), 4,89 (m, 1H), 6,57 (s, 1H), 7,31 (dd, 1H), 7,58 (m, 3H), 7,69 (d, 1H), 7,82 (d, 2H), 8,02 (d, 1H).

**Exemple 109**

**Acide 5-chloro-1-[(3,4-dihydro-4-méthyl-2*H*-1,4-benzoxazin-7-yl)sulfonyl]-1*H*-indole-2-butanoïque, méthyl ester**

En opérant de façon analogue à la préparation 6, au départ du composé obtenu selon la préparation 75, on obtient le produit attendu sous forme d'une poudre blanche (rendement = 90 %).
F = 139-140 °C.

**Exemple 110**

**Acide 5-chloro-1-[(3,4-dihydro-4-méthyl-2*H*-1,4-benzoxazin-7-yl)sulfonyl]-1*H*-indole-2-butanoïque**

En opérant de façon analogue à l'exemple 2, au départ du composé obtenu selon l'exemple 109, on obtient le produit attendu sous forme d'une poudre blanche (rendement = 94 %).
F = 164-166 °C.

**Exemple 111**

**Acide 5-chloro-1-[[1,2,3,4-tétrahydro-2-(trifluoroacétyl)-7-isoquinolinyl] sulfonyl]-1*H*-indole-2-butanoïque, méthyl ester**

En opérant de façon analogue à la préparation 6, au départ du composé obtenu selon la préparation 76, on obtient le produit attendu sous forme d'une poudre rose (rendement = 89 %).
F = 111-114 °C.

**Exemple 112**

**Acide 5-chloro-1-[[1,2,3,4-tétrahydro-7-isoquinolinyl]sulfonyl]-1*H*-indole-2-butanoïque, méthyl ester**

En opérant de façon analogue à l'exemple 2, au départ du composé obtenu selon l'exemple 111, on obtient le produit attendu sous forme d'une poudre blanche (rendement = 74 %).
F = 176-182°C.

**Exemple 113**

**Acide 5-chloro-1-[[1,2,3,4-tétrahydro-7-isoquinolinyl]sulfonyl]-1*H*-indole-2-butanoïque**

En opérant de façon analogue à l'exemple 2, en utilisant 1,6 équivalent d'hydroxyde de lithium, au départ du composé obtenu selon l'exemple 112, on obtient le produit attendu sous forme d'une poudre blanche (rendement = 63 %).
F > 250 °C.

**Exemple 114**

**Acide 5-chloro-1-[(2,3-dihydro-1,4-benzodioxin-6-yl)sulfonyl]-1*H*-indole-2-butanoïque, méthyl ester**

En opérant de façon analogue à la préparation 6, au départ du composé obtenu selon la préparation 77, on obtient le produit attendu sous forme d'un solide rose (rendement = 87 %).
F = 101-104 °C.

**Exemple 115**

**Acide 5-chloro-1-[(2,3-dihydro-1,4-benzodioxin-6-yl)sulfonyl]-1*H*-indole-2-butanoïque**

En opérant de façon analogue à l'exemple 2, au départ du composé obtenu selon l'exemple 114, on obtient le produit attendu sous forme d'une poudre blanche (rendement = 95 %).
F = 131-134 °C.

**Exemple 116**

**Acide 5-chloro-1-[(6-benzothiazolyl)sulfonyl]-1*H*-indole-2-butanoïque, méthyl ester**

En opérant de façon analogue à la préparation 6, au départ du composé obtenu selon la préparation 78, on obtient le produit attendu sous forme d'une poudre jaune (rendement = 61 %).
F = 121-123 °C.

**Exemple 117**

**Acide 5-chloro-1-[(6-benzothiazolyl)sulfonyl]-1*H*-indole-2-butanoïque**

En opérant de façon analogue à l'exemple 2, au départ du composé obtenu selon l'exemple 116, on obtient le produit attendu sous forme d'une poudre jaune pâle (rendement = 83 %).
F = 74-80 °C.

**Exemple 118**

**Acide 5-chloro-1-[[6-(4-morpholinyl)-3-pyridinyl]sulfonyl]-1*H*-indole-2-butanoïque, méthyl ester**

En opérant de façon analogue à la préparation 6, au départ du composé obtenu selon la préparation 79, on obtient le produit attendu sous forme d'une poudre blanche (rendement = 89 %).
F = 130-132 °C.

**Exemple 119**

**Acide 5-chloro-1-[[6-(4-morpholinyl)-3-pyridinyl]sulfonyl]-1*H*-indole-2-butanoïque**

En opérant de façon analogue à l'exemple 2, au départ du composé obtenu selon l'exemple 118, on obtient le produit attendu sous forme d'une poudre blanche (rendement = 99 %).
F = 78-82 °C.

**Exemple 120**

**Acide 5-chloro-1-[(3,5-diméthyl-4-isoxazolyl)sulfonyl]-1*H*-indole-2-butanoïque, méthyl ester**

En opérant de façon analogue à la préparation 6, au départ du composé obtenu selon la préparation 80, on obtient le produit attendu sous forme d'une poudre jaune (rendement = 91 %).
F = 96-98 °C.

**Exemple 121**

**Acide 5-chloro-1-[(3,5-diméthyl-4-isoxazolyl)sulfonyl]-1*H*-indole-2-butanoïque**

En opérant de façon analogue à l'exemple 2, au départ du composé obtenu selon l'exemple 120, on obtient le produit attendu sous forme d'une poudre blanche (rendement = 98 %).
F = 150-154 °C.

**Exemple 122**

**Acide 5-chloro-1-[(1,3,5-triméthyl-1*H*-pyrazol-4-yl)sulfonyl]-1*H*-indole-2-butanoïque, méthyl ester**

En opérant de façon analogue à la préparation 6, au départ du composé obtenu selon la préparation 81, on obtient le produit attendu sous forme d'une poudre blanche (rendement = 73 %).
F = 125-127 °C.

**Exemple 123**

**Acide 5-chloro-1-[(1,3,5-triméthyl-1*H*-pyrazol-4-yl)sulfonyl]-1*H*-indole-2-butanoïque**

En opérant de façon analogue à l'exemple 2, au départ du composé obtenu selon l'exemple 122, on obtient le produit attendu sous forme d'une poudre légèrement rose (rendement = 98 %).
F = 142-145 °C.

**Exemple 124**

**Acide 5-chloro-1-[(1-méthyl-1*H*-imidazol-4-yl)sulfonyl]-1*H*-indole-2-butanoïque, méthyl ester**

En opérant de façon analogue à la préparation 6, au départ du composé obtenu selon la préparation 82, on obtient le produit attendu sous forme d'une poudre beige (rendement = 42 %).
F = 163-165 °C.

**Exemple 125**

**Acide 5-chloro-1-[(1-méthyl-1*H*-imidazol-4-yl)sulfonyl]-1*H*-indole-2-butanoïque**

En opérant de façon analogue à l'exemple 2, au départ du composé obtenu selon l'exemple 124, on obtient le produit attendu sous forme d'une poudre beige (rendement = 87 %).
F = 222-225 °C.

**Exemple 126**

**Acide 5-Chloro-1-[(2,1,3-benzothiadiazol-4-yl)sulfonyl]-1*H*-indole-2-butanoïque, méthyl ester**

En opérant de façon analogue à la préparation 6, au départ du composé obtenu selon la préparation 83, on obtient le produit attendu sous forme d'une poudre jaune (rendement = 89 %).
F = 123-126 °C.

**Exemple 127**

**Acide 5-chloro-1-[(2,1,3-benzothiadiazol-4-yl)sulfonyl]-1*H*-indole-2-butanoïque**

En opérant de façon analogue à l'exemple 2, au départ du composé obtenu selon l'exemple 126, on obtient le produit attendu sous forme d'une poudre jaune (rendement = 89 %).

**Exemple 128**

**Acide 5-chloro-1-[(2,1,3-benzothiadiazol-5-yl)sulfonyl]-1*H*-indole-2-butanoïque, méthyl ester**

En opérant de façon analogue à la préparation 6, au départ du composé obtenu selon la préparation 84, on obtient le produit attendu sous forme d'une poudre jaune (rendement = 83 %).
F = 103-106 °C.

**Exemple 129**

**Acide 5-chloro-1-[(2,1,3-benzothiadiazol-5-yl)sulfonyl]-1*H*-indole-2-butanoïque**

En opérant de façon analogue à l'exemple 2, au départ du composé obtenu selon l'exemple 128, on obtient le produit attendu sous forme d'une poudre marron (rendement = 92 %).
F = 172-175°C.

**Exemple 130**

**Acide 5-chloro-1-[(3,4-dihydro-2,2-diméthyl-2*H*-1-benzopyran-6-yl)sulfonyl]-1*H*-indole-2-butanoïque, méthyl ester**

En opérant de façon analogue à la préparation 6, au départ du composé obtenu selon la préparation 85, on obtient le produit attendu sous forme d'une poudre rose (rendement = 94 %).
F = 126-129 °C.

**Exemple 131**

**Acide 5-chloro-1-[(3,4-dihydro-2,2-diméthyl-2*H*-1-benzopyran-6-yl)sulfonyl]-1*H*-indole-2-butanoïque**

En opérant de façon analogue à l'exemple 2, au départ du composé obtenu selon l'exemple 130, on obtient le produit attendu sous forme d'une poudre blanche (rendement = 88 %).
F = 166-169°C.

**Exemple 132**

**Acide 5-chloro-1-[(5,6,7,8-tétrahydro-5,5,8,8-tétraméthyl-2-naphthalényl) sulfonyl]-1*H*-indole-2-butanoïque, méthyl ester**

En opérant de façon analogue à la préparation 6, au départ du composé obtenu selon la préparation 86, on obtient le produit attendu sous forme d'une huile (rendement = 89 %).
$^1$H RMN (DMSO$d_6$, 300 MHz) δ = 1,12 (s, 6H), 1,17(s, 6H), 1,58 (s, 4H), 1,90-2,00 (m, 2H), 2,41 (t, 2H), 3,00 (t, 2H), 3,58 (s, 3H), 6,61 (s, 1H), 7,33 (dd, 1H), 7,46-7,62 (m, 4H), 8,09(d, 1H).

**Exemple 133**

**Acide 5-chloro-1-[(5,6,7,8,-tétrahydro-5,5,8,8-tétraméthyl-2-naphthalenyl) sulfonyl]-1*H*-indole-2-butanoïque**

En opérant de façon analogue à l'exemple 2, au départ du composé obtenu selon l'exemple 132, on obtient le produit attendu sous forme d'une poudre blanche (rendement = 95 %).
F = 64-66°C.

**Exemple 134**

**Acide 1-[(1-acétyl-2,3-dihydro-1*H*-indol-5-yl)sulfonyl]-5-chloro-1*H*-indole-2-butanoïque, méthyl ester**

En opérant de façon analogue à la préparation 6, au départ du composé obtenu selon la préparation 87, on obtient le produit attendu sous forme d'une poudre blanche (rendement = 82 %).
F = 162-165 °C.

**Exemple 135**

**Acide 1-[(1-acétyl-2,3-dihydro-1*H*-indol-5-yl)sulfonyl]-5-chloro-1*H*-indole-2-butanoïque**

En opérant de façon analogue à l'exemple 2, au départ du composé obtenu selon l'exemple 134, on obtient le produit attendu sous forme d'une poudre blanche (rendement = 94 %).
F = 115-117°C.

**Exemple 136**

**Acide 5-chloro-1-[(2-méthyl-6-benzothiazolyl)sulfonyl]-1*H*-indole-2-butanoïque, méthyl ester**

En opérant de façon analogue à la préparation 6, au départ du composé obtenu selon la préparation 88, on obtient le produit attendu sous forme d'une poudre blanche (rendement = 74 %).
F = 151-153 °C.

**Exemple 137**

**Acide 5-chloro-1-[(2-méthyl-6-benzothiazolyl)sulfonyl]-1*H*-indole-2-butanoïque**

En opérant de façon analogue à l'exemple 2, au départ du composé obtenu selon l'exemple 136, on obtient le produit attendu sous forme d'une poudre blanche (rendement = 85 %).
F = 163-165°C.

**Exemple 138**

**Acide 1-[[2-(acétylamino)-6-benzothiazolyl]sulfonyl]-5-chloro-1*H*-indole-2-butanoïque, méthyl ester**

En opérant de façon analogue à la préparation 6, au départ du composé obtenu selon la préparation 89, on obtient le produit attendu sous forme d'une poudre beige (rendement = 63 %).
F = 120 °C.

**Exemple 139**

**Acide 1-[[2-(acétylamino)-6-benzothiazolyl]sulfonyl]-5-chloro-1*H*-indole-2-butanoïque**

En opérant de façon analogue à l'exemple 2, au départ du composé obtenu selon l'exemple 138, on obtient le produit attendu sous forme d'un solide blanc (rendement = 70 %).
F > 250 °C.

**Exemple 140**

**Acide 5-chloro-1-[(2-méthyl-6-benzoxazolyl)sulfonyl]-1*H*-indole-2-butanoïque, méthyl ester**

En opérant de façon analogue à la préparation 6, au départ du composé obtenu selon la préparation 91, on obtient le produit attendu sous forme d'un solide blanc (rendement = 83 %).
F = 100-110 °C.

**Exemple 141**

**Acide 5-chloro-1-[(2,3-dihydro-5-benzofuranyl)sulfonyl]-1*H*-indole-2-butanoïque, méthyl ester**

En opérant de façon analogue à la préparation 6, au départ du composé obtenu selon la préparation 92, on obtient le produit attendu sous forme d'un solide blanc (rendement = 85 %).
F = 132-137 °C.

**Exemple 142**

**Acide 5-chloro-1-[(2,3-dihydro-5-benzofuranyl)sulfonyl]-1*H*-indole-2-butanoïque**

En opérant de façon analogue à l'exemple 2, au départ du composé obtenu selon l'exemple 141, on obtient le produit attendu sous forme d'un solide blanc (rendement = 98 %).
F = 174-179°C.

**Exemple 143**

**Acide 5-chloro-1-[(2-méthyl-5-benzothiazolyl)sulfonyl]-1*H*-indole-2-butanoïque, méthyl ester**

En opérant de façon analogue à la préparation 6, au départ du composé obtenu selon la préparation 93, on obtient le produit attendu sous forme d'une poudre jaune (rendement = 77 %).
F = 136-138°C.

**Exemple 144**

**Acide 5-Chloro-1-[(2-méthyl-5-benzothiazolyl)sulfonyl]-1*H*-indole-2-butanoïque**

En opérant de façon analogue à l'exemple 2, au départ du composé obtenu selon l'exemple 143, on obtient le produit attendu sous forme d'une poudre blanche (rendement = 98 %).
F = 164°C.

**Exemple 145**

**Acide 1-[(2-amino-6-benzoxazolyl)sulfonyl]-5-chloro-1*H*-indole-2-butanoïque, méthyl ester**

En opérant de façon analogue à la préparation 6, au départ du composé obtenu selon la préparation 94, on obtient le produit attendu sous forme d'une poudre jaune (rendement = 46 %).
F = 238 °C.

**Exemple 146**

**Acide 1-[(2-amino-6-benzoxazolyl)sulfonyl]-5-chloro-1*H*-indole-2-butanoïque**

En opérant de façon analogue à l'exemple 2, au départ du composé obtenu selon l'exemple 145, on obtient le produit attendu sous forme d'une poudre jaune (rendement = 76 %).
F = 220°C.

**Exemple 147**

**Acide 1-[[2-(acétylamino)-4-méthyl-5-thiazolyl]sulfonyl]-5-chloro-1*H*-indole-2-butanoïque, méthyl ester**

En opérant de façon analogue à la préparation 6, au départ du composé obtenu selon la préparation 95, on obtient le produit attendu sous forme d'une poudre jaune (rendement = 36 %).
F = 156-160 °C.

**Exemple 148**

**Acide 1-[[2-(acétylamino)-4-méthyl-5-thiazolyl]sulfonyl]-5-chloro-1*H*-indole-2-butanoïque**

En opérant de façon analogue à l'exemple 2, au départ du composé obtenu selon l'exemple 147, on obtient le produit attendu sous forme d'une poudre blanche (rendement = 74 %).
F = 231-233°C.

**Exemple 149**

**Acide 5-chloro-1-[(1,2,3,4-tétrahydro-2-oxo-6-quinolinyl)sulfonyl]-1*H*-indole-2-butanoïque, méthyl ester**

En opérant de façon analogue à la préparation 6, au départ du composé obtenu selon la préparation 96, on obtient le produit attendu sous forme d'une poudre blanche (rendement = 19 %).
F = 198-205°C.

**Exemple 150**

**Acide 1-[(2-acétyl-1,2,3,4-tétrahydro-7-isoquinolinyl)sulfonyl]-5-chloro-1*H*-indole-2-butanoïque, méthyl ester**

On prépare une solution de 1,25 g (2,8 mM) du composé obtenu selon l'exemple 112 dans 12 ml de dichlorométhane et on ajoute 0,860 ml (6,17 mM) de triéthylamine puis, goutte à goutte, 0,2 ml de chlorure d'acétyle. Le mélange réactionnel est agité à température ambiante pendant 2 heures, puis versé dans 15 ml d'eau glacée. Le mélange est décanté, la phase aqueuse extraite par 20 ml de dichlorométhane et les phases organiques rassemblées sont lavées à l'eau, puis séchées sur sulfate de magnésium et concentrées sous pression réduite. L'huile résiduelle est purifiée par chromatographie sur gel de silice en éluant à l'aide d'un mélange dichlorométhane/méthanol (99/1 ; v/v).

On obtient ainsi 0,93 g du composé attendu sous forme d'une poudre blanche (rendement = 67 %).
F = 50-52°C.

**Exemple 151**

**Acide 1-[(2-acétyl-1,2,3,4-tétrahydro-7-isoquinolinyl)sulfonyl]-5-chloro-1*H*-indole-2-butanoïque**

En opérant de façon analogue à l'exemple 2, au départ du composé obtenu selon l'exemple 150, on obtient le produit attendu sous forme d'une poudre blanche (rendement = 98 %).
F = 95-97°C.

**Exemple 152**

**Acide 5-chloro-1-[(2-pyridinyl)sulfonyl]-1*H*-indole-2-butanoïque, méthyl ester**

On prépare une solution de 350 mg (0,89 mM) de N-(4-chloro-2-iodophényl)-2-pyridinesulfonamide (préparation 117) dans 6 ml de diméthylformamide et on ajoute 10 ml de diéthylamine, 8 mg (0,042mM) d'iodure cuivreux, 16 mg (0,02 mM) de bis(triphénylphosphine)dichloropalladium et enfin, 134 mg (1,06 mM) d'ester méthylique de l'acide 5-hexynoïque. Le mélange est agité à température de reflux des solvants pendant 1 heure, puis à température ambiante pendant une nuit. Après hydrolyse sur 20 ml d'eau, le mélange est extrait par 40 ml d'acétate d'éthyle. La phase organique obtenue est lavée par une solution d'acide chlorhydrique N, puis par une solution de chlorure de sodium, séchée sur sulfate de magnésium et concentrée sous pression réduite. Le résidu huileux est purifié par chromatographie sur gel de silice en éluant à l'aide d'un mélange cyclohexane/acétate d'éthyle (80/20 ; v/v). On obtient ainsi 0,23 g du composé attendu sous forme d'un solide brun (rendement = 84 %).

F = 94˚C.

**Exemple 153**

**Acide 5-chloro-1-[(2-pyridinyl)sulfonyl]- 1*H*-indole-2-butanoïque**

En opérant de façon analogue à l'exemple 2, au départ du composé obtenu selon l'exemple 152, on obtient le produit attendu sous forme d'une poudre blanche (rendement = 97 %).
F = 192˚C.

**Exemple 154**

**Acide 1-[(6-benzothiazolyl)sulfonyl]-5-chloro-1*H*-indole-2-propanoïque, méthyl ester**

En opérant de façon analogue à la préparation 6, au départ du composé obtenu selon la préparation 97, on obtient le produit attendu sous forme d'une poudre jaune (rendement = 73 %).
F = 134-138 ˚C.

**Exemple 155**

**Acide 1-[(6-benzothiazolyl)sulfonyl]-5-chloro-1*H*-indole-2-propanoïque**

En opérant de façon analogue à l'exemple 2, au départ du composé obtenu selon l'exemple 154, on obtient le produit attendu sous forme d'une poudre blanche (rendement = 96 %).
F = 96-100 ˚C.

**Exemple 156**

**Acide 1-[[2-(acétylamino)-6-benzothiazolyl]sulfonyl]-5-chloro-1*H*-indole-2-propanoïque, méthyl ester**

En opérant de façon analogue à la préparation 6, au départ du composé obtenu selon la préparation 98, on obtient le produit attendu sous forme d'une poudre beige (rendement = 60 %).
F = 217-221 ˚C.

**Exemple 157**

**Acide 1-[[2-(acétylamino)-6-benzothiazolyl]sulfonyl]-5-chloro-1*H*-indole-2-propanoïque**

En opérant de façon analogue à l'exemple 2, au départ du composé obtenu selon l'exemple 156, on obtient le produit attendu sous forme d'une poudre blanche (rendement = 88 %).
F > 250 ˚C.

**Exemple 158**

**Acide 1-[(1-acétyl-2,3-dihydro-1*H*-indol-5-yl)sulfonyl]-5-chloro-1*H*-indole-2-propanoïque, méthyl ester**

En opérant de façon analogue à la préparation 6, au départ du composé obtenu selon la préparation 99, on obtient le produit attendu sous forme d'une poudre beige (rendement = 89 %).
F = 129 ˚C.

**Exemple 159**

**Acide 1-[(1-acétyl-2,3-dihydro-1*H*-indol-5-yl)sulfonyl]-5-chloro-1*H*-indole-2-propanoïque**

En opérant de façon analogue à l'exemple 2, au départ du composé obtenu selon l'exemple 158, on obtient le produit attendu sous forme d'une poudre blanche (rendement = 94 %).
F = 220-223˚C.

**Exemple 160**

**Acide 1-[(1,3-benzodioxol-5-yl)sulfonyl]-5-Chloro-1*H*-indole-2-propanoïque, méthyl ester**

En opérant de façon analogue à la préparation 6, au départ du composé obtenu selon la préparation 100, on obtient le produit attendu sous forme d'une poudre beige (rendement = 90 %).
F = 122-129 °C.

**Exemple 161**

**Acide 1-[(-1,3-benzodioxol-5-yl)sulfonyl]-5-chloro-1*H*-indole-2-propanoïque**

En opérant de façon analogue à l'exemple 2, au départ du composé obtenu selon l'exemple 160, on obtient le produit attendu sous forme d'une poudre blanche (rendement = 98 %).
F = 207 °C.

**Exemple 162**

**Acide 1-[(1,3-benzodioxol-5-yl)sulfonyl]-5-chloro-1*H*-indole-2-butanoïque, méthyl ester**

En opérant de façon analogue à la préparation 6, au départ du composé obtenu selon la préparation 101, on obtient le produit attendu sous forme d'une poudre beige (rendement = 97 %).
F = 98-103 °C.

**Exemple 163**

**Acide 1-[(1,3-benzodioxol-5-yl)sulfonyl]-5-chloro-1*H*-indole-2-butanoïque**

En opérant de façon analogue à l'exemple 2, au départ du composé obtenu selon l'exemple 162, on obtient le produit attendu sous forme d'une poudre blanche (rendement = 96 %).
F = 154-156 °C.

**Exemple 164**

**Acide 5-chloro-1-[[4-(4-morpholinylsulfonyl)phényl]sulfonyl]-1*H*-indole-2-butanoïque, méthyl ester**

En opérant de façon analogue à la préparation 6, au départ du composé obtenu selon la préparation 102, on obtient le produit attendu sous forme d'une poudre jaune (rendement = 94 %).
F = 54 °C.

**Exemple 165**

**Acide 5-chloro-1-[[4-(4-morpholinylsulfonyl)phényl]sulfonyl]-1*H*-indole-2-butanoïque**

En opérant de façon analogue à l'exemple 2, au départ du composé obtenu selon l'exemple 164, on obtient le produit attendu sous forme d'une poudre blanche (rendement = 93 %).
F = 181 °C.

**Exemple 166**

**Acide 5-chloro-1-[(2-pyridinyl)sulfonyl]-1*H*-indole-2-propanoïque, méthyl ester**

En opérant de façon analogue à l'exemple 152, au départ d'ester méthylique de l'acide 4-pentynoïque, on obtient le produit attendu sous forme d'un solide orange (rendement = 72 %).
F = 121 °C.

**Exemple 167**

**Acide 5-chloro-1-[(2-pyridinyl)sulfonyl]- 1*H*-indole-2-propanoïque**

En opérant de façon analogue à l'exemple 2, au départ du composé obtenu selon l'exemple 166, on obtient le produit attendu sous forme d'une poudre blanche (rendement = 90 %).
F = 189˚C.

**Exemple 168**

**Acide 1-[(6-benzothiazolyl)sulfonyl]-5-(trifluorométhyl)-1*H*-indole-2-butanoïque, méthyl ester**

En opérant de façon analogue à la préparation 6, au départ du composé obtenu selon la préparation 118, on obtient le produit attendu sous forme d'une poudre jaune (rendement = 49 %).
F = 117-121 ˚C.

**Exemple 169**

**Acide 1-[(6-benzothiazolyl)sulfonyl]-5-(trifluorométhyl)-1*H*-indole-2-butanoïque**

En opérant de façon analogue à l'exemple 2, au départ du composé obtenu selon l'exemple 168, on obtient le produit attendu sous forme d'une poudre beige (rendement = 97 %).
F = 175-181 ˚C.

**Exemple 170**

**Acide 1-[(6-benzothiazolyl)sulfonyl]-5-(trifluorométhyl)-1*H*-indole-2-propanoïque, méthyl ester**

En opérant de façon analogue à la préparation 6, au départ du composé obtenu selon la préparation 104, on obtient le produit attendu sous forme d'une poudre jaune (rendement = 64 %).
F = 130-132 ˚C.

**Exemple 171**

**Acide 1-[(6-benzothiazolyl)sulfonyl]-5-(trifluorométhyl)-1*H*-indole-2-propanoïque**

En opérant de façon analogue à l'exemple 2, au départ du composé obtenu selon l'exemple 170, on obtient le produit attendu sous forme d'une poudre jaune (rendement = 97 %).

**Exemple 172**

**Acide 1-[(2-méthyl-6-benzoxazolyl)sulfonyl]-5-(trifluorométhyl)-1*H*-indole-2-butanoïque, méthyl ester**

En opérant de façon analogue à la préparation 6, au départ du composé obtenu selon la préparation 105, on obtient le produit attendu sous forme d'une poudre beige (rendement = 90 %).
F = 78-82 ˚C.

**Exemple 173**

**Acide 1-[(2-méthyl-6-benzoxazolyl)sulfonyl]-5-(trifluorométhyl)-1*H*-indole-2-butanoïque**

En opérant de façon analogue à l'exemple 2, au départ du composé obtenu selon l'exemple 172, on obtient le produit attendu sous forme d'une poudre beige (Rendement = 31 %).
F = 214-220 ˚C.

**Exemple 174**

**Acide 1-[(1-acétyl-2,3-dihydro-1*H*-indol-5-yl)sulfonyl]-5-(trifluorométhyl)-1*H*-indole-2-butanoïque, méthyl ester**

En opérant de façon analogue à la préparation 6, au départ du composé obtenu selon la préparation 106, on obtient le produit attendu sous forme d'une poudre beige (rendement = 70 %).
F = 135-139 °C.

**Exemple 175**

**Acide 1-[(1-acétyl-2,3-dihydro-1*H*-indol-5-yl)sulfonyl]-5-(trifluorométhyl)-1*H*-indole-2-butanoïque**

En opérant de façon analogue à l'exemple 2, au départ du composé obtenu selon l'exemple 174, on obtient le produit attendu sous forme d'un solide blanc (rendement = 45 %).
F = 183 °C.

**Exemple 176**

**Acide 1-[(2,3-dihydro-5-benzofuranyl)sulfonyl]-5-(trifluorométhyl)-1*H*-indole-2-butanoïque, méthyl ester**

En opérant de façon analogue à la préparation 6, au départ du composé obtenu selon la préparation 107, on obtient le produit attendu sous forme d'une pâte incolore (rendement = 47 %).
$^1$H RMN (DMSO$d_6$, 300 MHz) $\delta$ =1,95-2,03 (m, 2H), 2,45 (t, 2H), 3,06 (t, 2H), 3,16 (t, 2H), 3,59 (s, 3H), 4,60 (t, 2H), 6,74 (s, 1H), 6,90 (d, 1H), 7,60 (dd, 1H), 7,66 (dd, 1H), 7,75 (s, 1H), 7,92 (s, 1H), 8,23 (d, 1H).

**Exemple 177**

**Acide 1-[(2,3-dihydro-5-benzofuranyl)sulfonyl]-5-(trifluorométhyl)-1*H*-indole-2-butanoïque**

En opérant de façon analogue à l'exemple 2, au départ du composé obtenu selon l'exemple 176, on obtient le produit attendu sous forme d'un solide blanc (rendement = 98 %).
F = 144-149 °C.

**Exemple 178**

**Acide 1-[(2-méthyl-5-benzothiazolyl)sulfonyl]-5-(trifluorométhyl)-1*H*-indole-2-butanoïque, méthyl ester**

En opérant de façon analogue à la préparation 6, au départ du composé obtenu selon la préparation 108, on obtient le produit attendu sous forme d'une pâte marron (rendement = 70 %).
$^1$H RMN (DMSO$d_6$, 300 MHz) $\delta$ = 1,99 (t, 2H), 2,46 (t, 2H), 2,81 (s, 3H), 3,11 (t, 2H), 3,58 (s, 3H), 6,77 (s, 1H), 7,63 (dd, 1H), 7,80 (dd, 1H), 7,92 (s, 1H), 8,27 (d, 1H), 8,31 (d, 1H), 8,34 (s, 1H).

**Exemple 179**

**Acide 1-[(2-méthyl-5-benzothiazolyl)sulfonyl]-5-(trifluorométhyl)-1*H*-indole-2-butanoïque**

En opérant de façon analogue à l'exemple 2, au départ du composé obtenu selon l'exemple 178, on obtient le produit attendu sous forme d'une poudre beige (rendement = 98 %).
F = 171-178°C.

**Exemple 180**

**Acide 1-[(2-amino-6-benzothiazolyl)sulfonyl]-5-(trifluorométhyl)-1*H*-indole-2-propanoïque, méthyl ester**

En opérant de façon analogue à la préparation 6, au départ du composé obtenu selon la préparation 109, on obtient le produit attendu sous forme d'une poudre jaune (rendement = 62 %).
F = 135-136 °C.

**Exemple 181**

**Acide 1-[(2-amino-6-benzothiazolyl)sulfonyl]-5-(trifluorométhyl)-1*H*-indole-2-propanoïque**

En opérant de façon analogue à l'exemple 2, au départ du composé obtenu selon l'exemple 180, on obtient le produit attendu sous forme d'une poudre blanche (rendement = 19 %).
F > 250˚C.

**Exemple 182**

**Acide 1-[(2-méthyl-6-benzothiazolyl)sulfonyl]-5-(trifluorométhyl)-1*H*-indole-2-propanoïque, méthyl ester**

En opérant de façon analogue à la préparation 6, au départ du composé obtenu selon la préparation 110, on obtient le produit attendu sous forme d'une poudre blanche (rendement = 44 %).
F = 214-215 ˚C.

**Exemple 183**

**Acide 1-[(2-méthyl-6-benzothiazolyl)sulfonyl]-5-(trifluorométhyl)-1*H*-indole-2-propanoïque**

En opérant de façon analogue à l'exemple 2, au départ du composé obtenu selon l'exemple 182, on obtient le produit attendu sous forme d'une poudre jaune pâle (rendement = 62 %).
F = 186-187 ˚C.

**Exemple 184**

**Acide 1-[(2-méthyl-5-benzothiazolyl)sulfonyl]-5-(trifluorométhyl)-1*H*-indole-2-propanoïque, méthyl ester**

En opérant de façon analogue à la préparation 6, au départ du composé obtenu selon la préparation 111, on obtient le produit attendu sous forme d'une poudre beige (rendement = 53 %).
$^1$H RMN ($DMSOd_6$, 300 MHz) $\delta$ =2,81 (s, 3H), 2,85 (d, 2H), 3,32 (d, 2H), 3,61 (s, 3H), 6,74 (s, 1H), 7,63 (dd, 1H), 7,81 (dd, 1H), 7,93 (s, 1H), 8,27-8,31 (m, 2H), 8,35 (d, 1H).

**Exemple 185**

**Acide 1-[(2-méthyl-5-benzothiazolyl)sulfonyl]-5-(trifluorométhyl)-1*H*-indole-2-propanoïque**

En opérant de façon analogue à l'exemple 2, au départ du composé obtenu selon l'exemple 184, on obtient le produit attendu sous forme d'une poudre blanche (rendement = 80 %).
F = 235-236 ˚C.

**Exemple 186**

**Acide 1-[(2-méthyl-6-benzoxazolyl)sulfonyl]-5-(trifluorométhyl)-1*H*-indole-2-propanoïque, méthyl ester**

En opérant de façon analogue à la préparation 6, au départ du composé obtenu selon la préparation 112, on obtient le produit attendu sous forme d'une huile jaune (rendement = 56 %).
$^1$H RMN ($DMSOd_6$, 300 MHz) $\delta$ = 2,63 (s, 3H), 2,84 (d, 2H), 3,33 (d, 2H), 353 (s, 3H), 6,73 (s, 1H), 7,61 (dd, 1H), 7,78-7,93 (m, 3H), 8,29 (d, 1H), 8,35 (s, 1H).

**Exemple 187**

**Acide 1-[(2-méthyl-7-benzothiazolyl)sulfonyl]-5-(trifluorométhyl)-1*H*-indole-2-propanoïque, méthyl ester**

En opérant de façon analogue à la préparation 6, au départ du composé obtenu selon la préparation 113, on obtient le produit attendu sous forme d'une poudre jaune (rendement = 83 %).
F = 106-108 ˚C.

**Exemple 188**

**Acide 1-[(1-acétyl-2,3-dihydro-1*H*-indol-5-yl)sulfonyl]-5-(trifluorométhyl)-1*H*-indole-2-propanoïque, méthyl ester**

En opérant de façon analogue à la préparation 6, au départ du composé obtenu selon la préparation 114, on obtient le produit attendu sous forme d'une huile (rendement = 66 %).
$^1$H RMN (DMSOd$_6$, 300 MHz) δ = 2,1 (s, 3H), 2,8 (t, 2H), 3,1 (t, 2H), 3,3 (t, 2H), 3.61 (s, 3H), 4,1 (t, 2H), 6,7 (s, 1H), 7,6 (dd, 1H), 7,7 (s, 1H), 7,7 (dd, 1H), 7,9 (d, 1H), 8,2 (d, 1H).

**Exemple 189**

**Acide 1-[(1-acétyl-2,3-dihydro-1*H*-indol-5-yl)sulfonyl]-5-(trifluorométhyl)-1*H*-indole-2-propanoïque**

En opérant de façon analogue à l'exemple 2, au départ du composé obtenu selon l'exemple 188, on obtient le produit attendu sous forme d'une poudre blanche (rendement = 40 %).
F = 205-207 °C.

**Exemple 190**

**Acide 1-[(2,3-dihydro-5-benzofuranyl)sulfonyl]-5-(trifluorométhyl)-1*H*-indole-2-propanoïque, méthyl ester**

En opérant de façon analogue à la préparation 6, au départ du composé obtenu selon la préparation 115, on obtient le produit attendu sous forme d'une pâte incolore (rendement = 79 %).
$^1$H RMN (DMSOd$_6$, 300 MHz) δ = 2,83 (t, 2H), 3,19 (t, 2H), 3,32 (t, 2H), 3,62 (s, 3H), 4,61 (t, 2H), 6,71 (d, 1H), 6,90 (d, 1H), 7,60 (dd, 1H), 7,68 (dd, 1H), 7,76 (d, 1H), 7,93 (d, 1H), 8,23 (dd, 1H).

**Exemple 191**

**Acide 1-[(2,3-dihydro-5-benzofuranyl)sulfonyl]-5-(trifluorométhyl)-1*H*-indole-2-propanoïque**

En opérant de façon analogue à l'exemple 2, au départ du composé obtenu selon l'exemple 190, on obtient le produit attendu sous forme d'une poudre beige (rendement = 34 %).
F = 161-164 °C.

**Exemple 192**

**Acide 1-[[4-(4-morpholinylsulfonyl)phényl]sulfonyl]-5-(trifluorométhyl)-1*H*-indole-2-propanoïque, méthyl ester**

En opérant de façon analogue à la préparation 6, au départ du composé obtenu selon la préparation 116, on obtient le produit attendu sous forme d'un solide blanc (rendement = 78 %).
F = 186-187 °C.

**Exemple 193**

**Acide 1-[[4-(4-morpholinylsulfonyl)phényl]sulfonyl]-5-(trifluorométhyl)-1*H*-indole-2-propanoïque**

En opérant de façon analogue à l'exemple 2, au départ du composé obtenu selon l'exemple 192, on obtient le produit attendu sous forme d'une poudre blanche (rendement = 36 %).
F = 238-239 °C.

**Exemple 194**

**Acide 1-[(2-amino-6-benzothiazolyl)sulfonyl]-5-chloro-1*H*-indole-2-butanoïque, méthyl ester**

En opérant de façon analogue à la préparation 6, au départ du composé obtenu selon la préparation 90, on obtient le produit attendu sous forme d'un solide jaune (rendement = 44 %).

F = 235-239 °C.

**Exemple 195**

**Acide 1-[(2-amino-6-benzothiazolyl)sulfonyl]-5-chloro-1*H*-indole-2-butanoïque**

En opérant de façon analogue à l'exemple 2, au départ du composé obtenu selon l'exemple 194, on obtient le produit attendu sous forme d'une poudre beige (Rendement = 49 %).
F = 155-162°C.

**Exemple 196**

**Acide 1-[(2,3-dihydro-1*H*-indol-5-yl)sulfonyl]-5-(trifluorométhyl)-1*H*-indole-2-butanoïque**

En opérant de façon analogue à l'exemple 2, mais en utilisant 4 équivalents de lithine, au départ du composé obtenu selon l'exemple 174, on obtient le produit attendu sous forme d'un solide beige (rendement = 36 %).
F = 175°C.

**Exemple 197**

**Acide 1-[[2-(acétylamino)-6-benzothiazolyl]sulfonyl]-5-(trifluorométhyl)-1*H*-indole-2-butanoïque, méthyl ester**

En opérant de façon analogue à l'exemple 3, au départ du composé obtenu selon la préparation 119 et de chlorure de 2-(acétylamino)-6-benzothiazolesulfonyle, on obtient le produit attendu sous forme d'un solide jaune (rendement = 14 %).
F = 215°C.

**Exemple 198**

**Acide 1-[[2-(acétylamino)-6-benzothiazolyl]sulfonyl]-5-(trifluorométhyl)-1*H*-indole-2-butanoïque**

En opérant de façon analogue à l'exemple 2, au départ du composé obtenu selon l'exemple 197, on obtient le produit attendu sous forme d'une poudre blanche (rendement = 57 %).
F > 250 °C.

**Exemple 199**

**Acide 1-[(2-méthyl-6-benzothiazolyl)sulfonyl]-5-(trifluorométhyl)-1*H*-indole-2-butanoïque, méthyl ester**

En opérant de façon analogue à l'exemple 197, au départ de chlorure de 2-méthyl-6-benzothiazolesulfonyle, on obtient le produit attendu sous forme d'un solide beige (rendement = 12 %).
F = 163-168°C.

**Exemple 200**

**Acide 1-[(2-méthyl-6-benzothiazolyl)sulfonyl]-5-(trifluorométhyl)-1*H*-indole-2-butanoïque**

En opérant de façon analogue à l'exemple 2, au départ du composé obtenu selon l'exemple 199, on obtient le produit attendu sous forme d'un solide beige (rendement = 87 %).
F = 184-187°C.

**Exemple 201**

**Acide 2-[[5-chloro-1-(phénylsulfonyl)-1*H*-indol-2-yl]méthoxy]propanoïque, éthyl ester**

Dans un tube pour réaction sous micro-ondes, on prépare un mélange de 600 mg (1,52 mM) de N-(4-chloro-2-iodophényl)-benzènesulfonamide (préparation 120) dans 0,5 ml de diméthylformamide et on ajoute 14 mg (0,076 mM) d'iodure cuivreux, 27 mg (0,038 mM) de bis(triphénylphosphine)dichloropalladium, 357 mg (2,3 mM) d'ester éthylique

de l'acide 2-(2-propynyloxy)propanoïque et enfin 0,5 ml de diéthylamine. Le mélange est chauffé sous micro-ondes à 130˚C pendant 15 mn puis refroidi et hydrolysé par 10 ml d'eau. Le mélange est extrait 3 fois par 15 ml d'acétate d'éthyle et les phases organiques rassemblées sont lavées à l'eau puis séchées sur sulfate de magnésium et concentrées sous pression réduite. Le résidu est purifié par chromatographie sur gel de silice en éluant à l'aide d'un mélange cyclohexane/acétate d'éthyle (95/5 ; v/v). On obtient ainsi 0,44 g du composé attendu sous forme d'une huile jaune (rendement = 69 %).
$^1$H RMN (DMSO$d_6$, 300 MHz) $\delta$ = 1,17 (t, 3H), 1,32 (d, 3H), 4,13 (q, 2H), 4,22 (q, 1H), 4,87 (d, 1H), 4,99 (d, 1H), 6,85 (s, 1H), 7,36 (dd, 1H), 7,58 (t, 2H), 7,68 (d, 1H), 7,70 (t, 1H), 7,96 (d, 2H), 7,99 (d, 1H).

**Exemple 202**

**Acide 2-[[5-chloro-1-(phénylsulfonyl)-1*H*-indol-2-yl]méthoxy]propanoïque**

En opérant de façon analogue à l'exemple 2, au départ du composé obtenu selon l'exemple 201, on obtient le produit attendu sous forme d'un solide pâteux blanc (rendement = 85 %).
$^1$H RMN (DMSO$d_6$, 300 MHz) $\delta$ = 1,31 (d, 3H), 4,14 (q, 1H), 4,84 (d, 1H), 5,02 (d, 1H), 6,85 (d, 1H), 7,35 (dd, 1H), 7,57 (t, 2H), 7,67 (d, 1H), 7,70 (tt, 1H), 7,96 (dt, 2H), 7,99 (d, 1H), 12,80 (m large, 1H).

**Exemple 203**

**Acide [[5-chloro-1-(phénylsulfonyl)-1*H*-indol-2-yl]méthoxy]acétique, méthyl ester**

En opérant de façon analogue à l'exemple 201, au départ d'ester méthylique de l'acide (2-propynyloxy)acétique, on obtient le composé attendu sous forme d'un solide jaune pâle (rendement = 71 %).
F = 98-100 ˚C.

**Exemple 204**

**Acide [[5-chloro-1-(phénylsulfonyl)-1*H*-indol-2-yl]méthoxy]acétique**

En opérant de façon analogue à l'exemple 2, au départ du composé obtenu selon l'exemple 203, on obtient le produit attendu sous forme d'un solide blanc (rendement = 98 %).
F = 140-142 ˚C.

**Exemple 205**

**Acide 2-[[5-chloro-1-(phénylsulfonyl)-1*H*-indol-2-yl]méthoxy]-2-méthyl-propanoïque, éthyl ester**

En opérant de façon analogue à l'exemple 201, au départ d'ester méthylique de l'acide 2-méthyl-2-(2-propynyloxy)propanoïque, on obtient le composé attendu sous forme d'une huile jaune (rendement = 59 %).
$^1$H RMN (DMSO$d_6$, 300 MHz) $\delta$ = 1,45 (s, 6H), 3,66 (s, 3H), 4,83 (s, 2H), 6,82 (s, 1H), 7,35 (dd, 1H), 7,61 (t, 2H), 7,68 (m, 2H), 7,96 (dt, 2H), 8,01 (d, 1H).

**Exemple 206**

**Acide 2-[[5-chloro-1-(phénylsulfonyl)-1*H*-indol-2-yl]méthoxy]-2-méthyl propanoïque**

En opérant de façon analogue à l'exemple 2, au départ du composé obtenu selon l'exemple 205, on obtient le produit attendu sous forme d'un solide pâteux blanc (rendement = 83 %).
$^1$H RMN (DMSO$d_6$, 300 MHz) $\delta$ = 1,43 (s, 6H), 4,86 (s, 2H), 6,81 (s, 1H), 7,33 (dd, 1H), 7,58 (t, 2H), 7,67 (m, 2H), 7,94 (dt, 2H), 7,99 (d, 1H), 12,80 (m large, 1H).

**Exemple 207**

**Acide [[1-(phénylsulfonyl)-5-(trifluorométhyl)-1*H*-indol-2-yl]méthoxy] acétique, méthyl ester**

En opérant de façon analogue à l'exemple 203, au départ de la sulfonamide obtenue selon la préparation 121, on obtient le composé attendu sous forme d'un solide jaune (rendement = 50 %).

F = 90-92 °C.

**Exemple 208**

**Acide [[1-(phénylsulfonyl)-5-(trifluorométhyl)-1*H*-indol-2-yl]méthoxy] acétique**

En opérant de façon analogue à l'exemple 2, au départ du composé obtenu selon l'exemple 207, on obtient le produit attendu sous forme d'un solide jaune (Rendement = 85 %).
F = 158-160 °C.

**Exemple 209**

**Acide 2-[[1-(phénylsulfonyl)-5-(trifluorométhyl)-1*H*-indol-2-yl]méthoxy] propanoïque, éthyl ester**

En opérant de façon analogue à l'exemple 201, au départ de la sulfonamide obtenue selon la préparation 121, on obtient le composé attendu sous forme d'une huile jaune (rendement = 74 %).
$^1$H RMN ($DMSOd_6$, 300 MHz) $\delta$ = 1,19 (t, 3H), 1,32 (d, 3H), 4,13 (q, 2H), 4,24 (d, 1H), 4,91 (d, 1H), 5,03 (d, 1H), 7,01 (s, 1H), 7,60 (t, 2H), 7,69 (dd, 1H), 7,72 (t, 1H), 8,01 (dt, 2H), 8,21 (d, 1H).

**Exemple 210**

**Acide 2-[[1-(phénylsulfonyl)-5-(trifluorométhyl)-1*H*-indol-2-yl]méthoxy] propanoïque**

En opérant de façon analogue à l'exemple 2, au départ du composé obtenu selon l'exemple 209, on obtient le produit attendu sous forme d'un solide blanc (rendement = 50 %).
F = 72-74 °C.

**Exemple 211**

**Acide 2-méthyl-2-[[1-(phénylsulfonyl)-5-(trifluorométhyl)-1*H*-indol-2-yl]méthoxy]propanoïque, éthyl ester**

En opérant de façon analogue à l'exemple 205, au départ de la sulfonamide obtenue selon la préparation 121, on obtient le composé attendu sous forme d'un solide beige (rendement = 46 %).
F = 62-64 °C.

**Exemple 212**

**Acide 2-méthyl-2-[[1-(phénylsulfonyl)-5-(trifluorométhyl)-1*H*-indol-2-yl]méthoxy]propanoïque**

En opérant de façon analogue à l'exemple 2, au départ du composé obtenu selon l'exemple 211, on obtient le produit attendu sous forme d'un solide blanc (rendement = 50 %).
F = 134-136 °C.

**Exemple 213**

**Acide [2-[5-chloro-1-(phénylsulfonyl)-1*H*-indol-2-yl]éthoxy]acétique, éthyl ester**

En opérant de façon analogue à l'exemple 201, au départ d'ester éthylique de l'acide (3-butynyloxy)acétique, on obtient le composé attendu sous forme d'un solide orange (rendement = 79 %).
F = 60-62 °C.

**Exemple 214**

**Acide [2-[5-chloro-1-(phénylsulfonyl)-1*H*-indol-2-yl]éthoxy]acétique**

En opérant de façon analogue à l'exemple 2, au départ du composé obtenu selon l'exemple 213, on obtient le produit attendu sous forme d'un solide blanc (rendement = 97 %).
F = 135-137 °C.

**Exemple 215**

**Acide 2-méthyl-2-[[1-[(6-benzothiazolyl)sulfonyl]-5-chloro-1*H*-indol-2-yl]méthoxy]propanoïque, méthyl ester**

Dans un tube-réacteur pour micro-ondes, on prépare un mélange de 126 mg (0,26 mM) d'ester obtenu selon la préparation 123 dans 1 ml de 1,2-dichloroéthane et on ajoute 48 mg (0,26 mM) d'acétate de cuivre (cuivrique). Le mélange est chauffé sous micro-ondes à 150°C pendant 15 minutes, puis refroidi, dilué avec 6 ml de dichlorométhane et filtré sur papier Whatman. Le filtrat est concentré sous pression réduite et le produit brut est purifié par chromatographie sur gel de silice en éluant avec un mélange dichlotométhane/acétate d'éthyle (97/3 ; v/v). On obtient 79 mg du composé attendu sous forme d'un solide pâteux jaune (rendement = 63 %).
$^1$H RMN (DMSO$d_6$, 300 MHz) $\delta$ = 1,46 (s, 6H), 3,65 (s, 3H), 4,88 (s, 2H), 6,83 (s, 1H), 7,35 (dd, 1H), 7,66 (d, 1H); 8,02 (dd, 1H), 8,07 (d, 1H), 8,23 (d, 1H), 9,07 (d, 1H), 9,66 (s, 1H).

**Exemple 216**

**Acide 2-méthyl-2-[[1-[(6-benzothiazolyl)sulfonyl]-5-chloro-1*H*-indol-2-yl]méthoxy]propanoïque**

En opérant de façon analogue à l'exemple 2, au départ du composé obtenu selon l'exemple 215, on obtient le produit attendu sous forme d'un solide blanc (rendement = 94 %).
F = 74 °C.

**Exemple 217**

**Acide 2-méthyl-2-[[1-[(6-benzothiazolyl)sulfonyl]-5-(trifluorométhyl)-1*H*-indol-2-yl]méthoxy]propanoïque, méthyl ester**

En opérant de façon analogue à l'exemple 215, au départ du composé obtenu selon la préparation 125, on obtient le produit attendu sous forme d'une huile jaune (rendement = 72 %).
$^1$H RMN (DMSO$d_6$, 250 MHz) $\delta$ = 1,46 (s, 6H), 3,65 (s, 3H), 4,92 (s, 2H), 6,98 (s, 1H), 7,65 (dd, 1H), 8,02 (s, 1H), 8,07 (dd, 1H), 8,26 (t, 2H), 9,12 (d, 1H), 9,66 (s, 1H).

**Exemple 218**

**Acide 2-méthyl-2-[[1-[(6-benzothiazolyl)sulfonyl]-5-(trifluorométhyl)-1*H*-indol-2-yl]méthoxy]propanoïque**

En opérant de façon analogue à l'exemple 2, au départ du composé obtenu selon l'exemple 217, on obtient le produit attendu sous forme d'un solide blanc (rendement = 98 %).
F = 98-100 °C.

**Exemple 219**

**Acide 2-méthyl-2-[[1-[(2-méthyl-6-benzothiazolyl)sulfonyl]-5-chloro-1*H*-indol-2-yl]méthoxy]propanoïque, méthyl ester**

En opérant de façon analogue à l'exemple 215, au départ du composé obtenu selon la préparation 126, on obtient le produit attendu sous forme d'une pâte blanche (rendement = 73 %).
$^1$H RMN (DMSO$d_6$, 250 MHz) $\delta$ = 1,46 (s, 6H), 2,83 (s, 3H), 3,66 (s, 3H), 4,87 (s, 2H), 6,82 (s, 1H), 7,35 (dd, 1H), 7,66 (d, 1H), 7,96 (dd, 1H), 8,03 (d, 1H), 8,06 (d, 1H), 8,92 (d, 1H).

**Exemple 220**

**Acide 2-méthyl-2-[[1-[(2-méthyl-6-benzothiazolyl)sulfonyl]-5-chloro-1*H*-indol-2-yl]méthoxy]propanoïque**

En opérant de façon analogue à l'exemple 2, au départ du composé obtenu selon l'exemple 219, on obtient le produit attendu sous forme d'un solide blanc (rendement = 86 %).
F = 172-174 °C.

**Exemple 221**

**Acide 2-méthyl-2-[[1-[(2-méthyl-5-benzothiazolyl)sulfonyl]-5-chloro-1*H*-indol-2-yl]méthoxy]propanoïque, méthyl ester**

En opérant de façon analogue à l'exemple 215, au départ du composé obtenu selon la préparation 127, on obtient le produit attendu sous forme d'un solide blanc (rendement = 71 %).
F = 132-134 °C.

**Exemple 222**

**Acide 2-méthyl-2-[[1-[(2-méthyl-5-benzothiazolyl)sulfonyl]-5-chloro-1*H*-indol-2-yl]méthoxy]propanoïque**

En opérant de façon analogue à l'exemple 2, au départ du composé obtenu selon l'exemple 221, on obtient le produit attendu sous forme d'un solide blanc (rendement = 86 %).
F = 134-136 °C.

**Exemple 223**

**Acide 2-[[1-[(6-benzothiazolyl)sulfonyl]-5-chloro-1*H*-indol-2-yl]méthoxy] propanoïque, éthyl ester**

En opérant de façon analogue à l'exemple 215, au départ du composé obtenu selon la préparation 129, on obtient le produit attendu sous forme d'une huile jaune (rendement = 89 %).
$^1$H RMN ($DMSOd_6$, 250 MHz) $\delta$ = 1,17 (t, 3H), 1,32 (d, 3H), 4,11 (q, 2H), 4,22 (q, 1H), 4,92 (d, 1H), 5,03 (d, 1H), 6,86 (s, 1H), 7,36 (dd, 1H), 7,67 (d, 1H), 8,04 (dd, 1H), 8,08 (d, 1H), 8,21 (d, 1H), 9,08 (d, 1H), 9,66 (s, 1H).

**Exemple 224**

**Acide 2-[[1-[(6-benzothiazolyl)sulfonyl]-5-chloro-1*H*-indol-2-yl]méthoxy] propanoïque**

En opérant de façon analogue à l'exemple 2, au départ du composé obtenu selon l'exemple 223, on obtient le produit attendu sous forme d'un solide blanc (rendement = 98 %).
F = 102-104 °C.

**Exemple 225**

**Acide 1-[(1,3-benzodioxol-5-yl)sulfonyl]-5-(trifluorométhyl)-1*H*-indole-2-butanoïque, méthyl ester**

En opérant de façon analogue à la préparation 6, au départ du composé obtenu selon la préparation 130, on obtient le produit attendu sous forme d'une pâte beige (rendement = 43 %).
$^1$H RMN ($DMSOd_6$, 250 MHz) $\delta$ = 1,95-2,03 (m, 2H), 2,4 (t, 2H), 3,06 (t, 2H), 3,59 (s, 3H), 6,14 (s, 2H), 6,75 (s, 1H), 7,05 (d, 1H), 7,33 (s, 1H), 7,48 (dd, 1H),7,59 (dd, 1H), 7,93 (s, 1H), 8,23 (d, 1H).

**Exemple 226**

**Acide 1-[(1,3-benzodioxol-5-yl)sulfonyl]-5-(trifluorométhyl)-1*H*-indole-2-butanoïque**

En opérant de façon analogue à l'exemple 2, au départ du composé obtenu selon l'exemple 225, on obtient le produit attendu sous forme d'une poudre rose (rendement = 59 %).
F = 171-175 °C.

**Exemple 227**

**Acide 1-[[2-(acétylamino)-6-benzothiazolyl]sulfonyl]-5-(trifluorométhyl)-1*H*-indole-2-propanoïque, méthyl ester**

En opérant de façon analogue à la préparation 6, au départ du composé obtenu selon la préparation 131, on obtient le produit attendu sous forme d'une poudre jaune (rendement = 20 %).

$^1H$ RMN ($DMSOd_6$, 500 MHz) $\delta$ = 2,24 (s, 3H), 2,87(t, 2H), 3,40 (t, 2H), 3,64 (s, 3H), 6,75 (s, 1H), 7,64 (d, 1H), 7,85 (s, 2H), 7,95 (s, 1H), 8,29 (d, 1H), 8,80 (s, 1H), 12,68 (s, 1H).

**Exemple 228**

**Acide 1-[[2-(acétylamino)-6-benzothiazolyl]sulfonyl]-5-(trifluorométhyl)-1*H*-indole-2-propanoïque**

En opérant de façon analogue à l'exemple 2, au départ du composé obtenu selon l'exemple 227, on obtient le produit attendu sous forme d'une poudre jaune (rendement = 73 %).
F > 290-292 °C.

**Exemple 229**

**Acide 2-méthyl-2-[[1-[(2-méthyl-5-benzothiazolyl)sulfonyl]-5-(trifluorométhyl)-1*H*-indol-2-yl]méthoxy]propanoïque, méthyl ester**

En opérant de façon analogue à l'exemple 215, au départ du composé obtenu selon la préparation 132, on obtient le produit attendu sous forme d'un solide blanc (Rendement = 57 %).
F = 164-166 °C.

**Exemple 230**

**Acide 2-méthyl-2-[[1-[(2-méthyl-5-benzothiazolyl)sulfonyl]-5-(trifluorométhyl)-1*H*-indol-2-yl]méthoxy]propanoïque**

En opérant de façon analogue à l'exemple 2, au départ du composé obtenu selon l'exemple 229, on obtient le produit attendu sous forme d'un solide blanc (rendement = 97 %).
F = 188-190 °C.

**Exemple 231**

**Acide 2-[[1-(1,3-benzodioxol-5-ylsulfonyl)-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 133, on obtient le produit attendu sous forme d'une huile incolore (rendement = 71 %).
$^1H$ RMN ($DMSOd_6$, 250 MHz) $\delta$ : 8,19 (d, 1H) ; 8,02 (d, 1H) ; 7,64 (m, 2H) ; 7,57 (d, 1H) ; 7,08 (d, 1H) ; 6,97 (d, 1H) ; 6,14 (s, 2H) ; 4,85 (s, 2H) ; 3,68 (s, 3H) ; 1,49 (s, 6H).

**Exemple 232**

**Acide 2-[[1-(1,3-benzodioxol-5-ylsulfonyl)-5-(trifluorométhyl)-1*H*-indol-2-yl]méthoxy]-2-méthylpropanoïque**

En opérant de façon analogue à l'exemple 2, au départ du composé obtenu selon l'exemple 231, on obtient le produit attendu sous forme d'un solide blanc (rendement = 94 %).
F = 130-132 °C.

**Exemple 233**

**Acide 2-[[1-[[2-(acétylamino)-6-benzothiazolyl]sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 134, on obtient le produit attendu sous forme d'un solide blanc (rendement = 52 %).
F = 212-214 °C.

**Exemple 234**

**Acide 2-[[1-[[2-(acétylamino)-6-benzothiazolyl]sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 233, on obtient le produit attendu sous forme d'un solide beige (rendement = 86 %).
F = 144-146 °C.

**Exemple 235**

**Acide 2-[[1-[(2,3-dihydro-5-benzofuranyl)sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 135, on obtient le produit attendu sous forme d'une huile incolore (rendement = 75 %).
$^1$H RMN ($DMSOd_6$, 250 MHz) $\delta$ : 8,19 (d, 1H) ; 8,02 (s, 1H) ; 7,92 (d, 1H) ; 7,83 (dd, 1H) ; 7,63 (dd, 1H) ; 6,94 (s, 1H) ; 6,91 (d, 1H) ; 4,87 (s, 2H) 4,61 (t, 2H) ; 3,68 (s, 3H) ; 3,19 (t, 2H) ; 1,48 (s, 6H).

**Exemple 236**

**Acide 2-[[1-[(2,3-dihydro-5-benzofuranyl)sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 235, on obtient le produit attendu sous forme d'un solide blanc (rendement = 99 %).
F = 92-94 °C.

**Exemple 237**

**Acide 2-méthyl-2-[[1-[(2-méthyl-7-benzothiazolyl)sulfonyl]-5-(trifluorométhyl)-1*H*-indol-2-yl]méthoxy]propanoïque, méthyl ester**

En opérant de façon analogue à l'exemple 215, au départ du composé obtenu selon la préparation 136, on obtient le produit attendu sous forme d'un solide beige (rendement = 75 %).
F = 118-120 °C.

**Exemple 238**

**Acide 2-méthyl-2-[[1-[(2-méthyl-7-benzothiazolyl)sulfonyl]-5-(trifluorométhyl)-1*H*-indol-2-yl]méthoxy]propanoïque**

En opérant de façon analogue à l'exemple 2, au départ du composé obtenu selon l'exemple 237, on obtient le produit attendu sous forme d'un solide blanc (rendement = 91 %).
F = 98-100 °C.

**Exemple 239**

**Acide 2-[[1-[(2,3-dihydro-1,4-benzodioxin-6-yl)sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 137, on obtient le produit attendu sous forme d'un solide blanc (rendement = 81 %).
F = 128-130 °C.

**Exemple 240**

**Acide 2-[[1-[(2,3-dihydro-1,4-benzodioxin-6-yl)sulfonyl]-5-(trifluorométhyl)-1*H*-indol-2-yl]méthoxy]-2-méthyl-propanoïque**

En opérant de façon analogue à l'exemple 2, au départ du composé obtenu selon l'exemple 239, on obtient le produit attendu sous forme d'un solide blanc (rendement = 95 %).
F = 75°C.

**Exemple 241**

**Acide 2-[[1-[(3,4-dihydro-4-méthyl-2*H*-1,4-benzoxazin-7-yl)sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 138, on obtient le produit attendu sous forme d'un solide blanc (Rendement = 85 %).
F = 96-98°C.

**Exemple 242**

**Acide 2-[[1-[(3,4-dihydro-4-méthyl-2*H*-1,4-benzoxazin-7-yl)sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 241, on obtient le produit attendu sous forme d'un solide blanc (rendement = 99 %).
F = 148-150°C.

**Exemple 243**

**Acide 2-[[1-[(1-acétyl-2,3-dihydro-1*H*-indol-5-yl)sulfonyl]-5-(trifluorométhyl)-1*H*-indol-2-yl]méthoxy]-2-méthyl-propanoïque, méthyl ester**

En opérant de façon analogue à l'exemple 215, au départ du composé obtenu selon la préparation 139, on obtient le produit attendu sous forme d'un solide blanc (rendement = 84 %).
F = 154-156°C.

**Exemple 244**

**Acide 2-[[1-[(1-acétyl-2,3-dihydro-1*H*-indol-5-yl)sulfonyl]-5-(trifluorométhyl)-1*H*-indol-2-yl]méthoxy]-2-méthyl-propanoïque**

En opérant de façon analogue à l'exemple 2, au départ du composé obtenu selon l'exemple 243, on obtient le produit attendu sous forme d'un solide blanc (rendement = 61 %).
F = 176-178°C.

**Exemple 245**

**Acide 2-[[1-[(3,5-diméthylphényl)sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 140, on obtient le produit attendu sous forme d'un solide blanc (rendement = 86 %).
F = 132-134°C.

**Exemple 246**

**Acide 2-[[1-[(3,5-diméthylphényl)sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 245, on obtient le produit attendu sous forme d'un solide blanc (rendement = 82 %).
F = 150-152°C.

**Exemple 247**

**Acide 2-[[1-[(2,5-diméthoxyphényl)sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 141, on obtient le produit attendu sous forme d'un solide blanc (rendement = 84 %).
F = 130-132°C.

**Exemple 248**

**Acide 2-[[1-[(2,5-diméthoxyphényl)sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 247, on obtient le produit attendu sous forme d'un solide blanc (rendement = 50 %).
F = 186-188°C.

**Exemple 249**

**Acide 2-[[1-[[4-(1-méthyléthyl)phényl]sulfonyl]-5-(trifluorométhyl)-1*H*-indol-2-yl]méthoxy]-2-méthylpropanoïque, méthyl ester**

En opérant de façon analogue à l'exemple 215, au départ du composé obtenu selon la préparation 142, on obtient le produit attendu sous forme d'une huile incolore (rendement = 86 %).
$^1$H RMN (DMSO$d_6$, 250 MHz) $\delta$ : 8,23 (d, 1H) ; 8,02 (d, 1H) ; 7,91 (dt, 2H) ; 7,65 (dd, 1H) ; 7,49 (dt, 2H) ; 6,96 (d, 1H) ; 4,86 (s, 2H) ; 3,68 (s, 3H) ; 2,92 (hep, 1H) ; 1,46 (s, 6H) ; 1,15 (d, 6H).

**Exemple 250**

**Acide 2-[[1-[[4-(1-méthyléthyl)phényl]sulfonyl]-5-(trifluorométhyl)-1*H*-indol-2-yl]méthoxy]-2-méthylpropanoïque**

En opérant de façon analogue à l'exemple 2, au départ du composé obtenu selon l'exemple 249, on obtient le produit attendu sous forme d'un solide blanc (rendement = 74 %).
F = 132-134°C.

**Exemple 251**

**Acide 2-[[1-(1,3-benzodioxol-5-ylsulfonyl)-5-chloro-1*H*-indol-2-yl]méthoxy]-2-méthylpropanoïque, méthyl ester**

En opérant de façon analogue à l'exemple 215, au départ du composé obtenu selon la préparation 143, on obtient le produit attendu sous forme d'un solide blanc (rendement = 83 %).
F = 90-92°C.

**Exemple 252**

**Acide 2-[[1-(1,3-benzodioxol-5-ylsulfonyl)-5-chloro-1*H*-indol-2-yl]méthoxy]-2-méthylpropanoïque**

En opérant de façon analogue à l'exemple 2, au départ du composé obtenu selon l'exemple 251, on obtient

le produit attendu sous forme d'un solide blanc (rendement = 99 %).
F = 174-176 °C.

**Exemple 253**

**Acide 2-[[1-[[2-(acétylamino)-6-benzothiazolyl]sulfonyl]-5-chloro-1*H*-indol-2-yl]méthoxy]-2-méthylpropanoïque, méthyl ester**

En opérant de façon analogue à l'exemple 215, au départ du composé obtenu selon la préparation 144, on obtient le produit attendu sous forme d'un solide jaune (rendement = 34%).
F = 112-114 °C.

**Exemple 254**

**Acide 2-[[1-[[2-(acétylamino)-6-benzothiazolyl]sulfonyl]-5-chloro-1*H*-indol-2-yl]méthoxy]-2-méthylpropanoïque**

En opérant de façon analogue à l'exemple 2, au départ du composé obtenu selon l'exemple 253, on obtient le produit attendu sous forme d'un solide blanc (rendement = 90 %).
F = 162-164 °C.

**Exemple 255**

**Acide 2-[[1-[(1-acétyl-2,3-dihydro-1*H*-indol-5-yl)sulfonyl]-5-chloro-1*H*-indol-2-yl]méthoxy]-2-méthylpropanoïque, méthyl ester**

En opérant de façon analogue à l'exemple 215, au départ du composé obtenu selon la préparation 145, on obtient le produit attendu sous forme d'un solide beige (rendement = 90 %).
F = 128-130°C.

**Exemple 256**

**Acide 2-[[1-[(1-acétyl-2,3-dihydro-1*H*-indol-5-yl)sulfonyl]-5-chloro-1*H*-indol-2-yl]méthoxy]-2-méthylpropanoïque**

En opérant de façon analogue à l'exemple 2, au départ du composé obtenu selon l'exemple 255, on obtient le produit attendu sous forme d'un solide blanc (rendement = 95 %).
F = 142-144°C.

**Exemple 257**

**Acide 2-[[5-chloro-1-[(2,3-dihydro-5-benzofuranyl)sulfonyl]-1*H*-indol-2-yl]méthoxy]-2-méthylpropanoïque, méthyl ester**

En opérant de façon analogue à l'exemple 215, au départ du composé obtenu selon la préparation 146, on obtient le produit attendu sous forme d'une huile incolore (rendement = 80 %).
$^1$H RMN (DMSO$d_6$, 300 MHz) δ : 8,10 (d, 1H) ; 7,86 (d, 1H) ; 7,77 (dd, 1H) ; 7,66 (d, 1H) ; 7,33 (dd, 1H) ; 7,89 (d, 1H) ; 6,79 (s, 1H) ; 4,83 (s, 2H) ; 4,60 (t, 2H) ; 3,67 (s, 3H) ; 3,18 (t, 2H) ; 1,47 (s, 6H).

**Exemple 258**

**Acide 2-[[5-chloro-1-[(2,3-dihydro-5-benzofuranyl)sulfonyl]-1*H*-indol-2-yl]méthoxy]-2-méthylpropanoïque**

En opérant de façon analogue à l'exemple 2, au départ du composé obtenu selon l'exemple 257, on obtient le produit attendu sous forme d'un solide blanc (rendement = 94 %).
F = 130-132 °C.

**Exemple 259**

**Acide 2-méthyl-2-[[5-chloro-1-[(2-méthyl-7-benzothiazolyl)sulfonyl]-1*H*-indol-2-yl]méthoxy]propanoïque, méthyl ester**

En opérant de façon analogue à l'exemple 215, au départ du composé obtenu selon la préparation 147, on obtient le produit attendu sous forme d'une huile jaune (rendement = 78 %).
$^1$H RMN (DMSO$d_6$, 250 MHz) δ : 8,24 (dd, 1H) ; 7,95 (d, 1H) ; 7,84 (dd, 1H) ; 7,69 (m, 2H) ; 7,33 (dd, 1H) ; 6,87 (d, 1H) ; 4,81 (s, 2H) ; 3,62 (s, 3H) ; 2,85 (s, 3H) ; 1,34 (s, 6H).

**Exemple 260**

**Acide 2-méthyl-2-[[5-chloro-1-[(2-méthyl-7-benzothiazolyl)sulfonyl]-1*H*-indol-2-yl]méthoxy]propanoïque**

En opérant de façon analogue à l'exemple 2, au départ du composé obtenu selon l'exemple 259, on obtient le produit attendu sous forme d'un solide blanc (rendement = 94 %).
F = 128-130 ˚C.

**Exemple 261**

**Acide 2-[[5-chloro-1-[(2,3-dihydro-1,4-benzodioxin-6-yl)sulfonyl]-1*H*-indol-2-yl]méthoxy]-2-méthylpropanoïque, méthyl ester**

En opérant de façon analogue à l'exemple 215, au départ du composé obtenu selon la préparation 148, on obtient le produit attendu sous forme d'une huile incolore (rendement = 87 %).
$^1$H RMN (DMSO$d_6$, 300 MHz) δ : 8,02 (d, 1H) ; 7,88 (d, 2H) ; 7,67 (d, 1H) ; 7,46 (d, 2H) ; 7,36 (dd, 1H) ; 6,81 (s, 1H) ; 4,82 (s, 2H) ; 3,66 (s, 3H) ; 2,97 (hep, 1H) ; 1,45 (s, 6H) ; 1,15 (d, 6H).

**Exemple 262**

**Acide 2-[[5-chloro-1-[(2,3-dihydro-1,4-benzodioxin-6-yl)sulfonyl]-1*H*-indol-2-yl]méthoxy]-2-méthylpropanoïque**

En opérant de façon analogue à l'exemple 2, au départ du composé obtenu selon l'exemple 261, on obtient le produit attendu sous forme d'un solide blanc (rendement = 88 %).
F = 156-158˚C.

**Exemple 263**

**Acide 2-[[5-chloro-1-[(3,4-dihydro-4-méthyl-2*H*-1,4-benzoxazin-7-yl)-sulfonyl]-1*H*-indol-2-yl]méthoxy]-2-méthylpropanoïque, méthyl ester**

En opérant de façon analogue à l'exemple 215, au départ du composé obtenu selon la préparation 149, on obtient le produit attendu sous forme d'un solide blanc (rendement = 85 %).
F = 96-98˚C.

**Exemple 264**

**Acide 2-[[5-chloro-1-[(3,4-dihydro-4-méthyl-2*H*-1,4-benzoxazin-7-yl)-sulfonyl]-1*H*-indol-2-yl]méthoxy]-2-méthylpropanoïque**

En opérant de façon analogue à l'exemple 2, au départ du composé obtenu selon l'exemple 263, on obtient le produit attendu sous forme d'un solide blanc (rendement = 98 %).
F = 148-150˚C.

**Exemple 265**

**Acide 2-[[5-chloro-1-[[4-(1-méthyléthyl)phényl]sulfonyl]-1*H*-indol-2-yl]-méthoxy]-2-méthylpropanoïque, méthyl ester**

En opérant de façon analogue à l'exemple 215, au départ du composé obtenu selon la préparation 150, on obtient le produit attendu sous forme d'une huile incolore (rendement = 94 %).
$^1$H RMN (DMSO$d_6$, 300 MHz) δ : 8,02 (d, 1H) ; 7,88 (d, 2H) ; 7,67 (d, 1H) ; 7,46 (d, 2H) ; 7,36 (dd, 1H) ; 6,81 (s, 1H) ; 4,82 (s, 2H) ; 3,66 (s, 3H) ; 2,97 (hep, 1H) ; 1,45 (s, 6H) ; 1,15 (d, 6H).

**Exemple 266**

**Acide 2-[[5-chloro-1-[[4-(1-méthyléthyl)phényl]sulfonyl]-1*H*-indol-2-yl]-méthoxy]-2-méthylpropanoïque**

En opérant de façon analogue à l'exemple 2, au départ du composé obtenu selon l'exemple 265, on obtient le produit attendu sous forme d'un solide jaune pâle (rendement = 95 %).
F = 60°C.

**Exemple 267**

**Acide 2-[[5-chloro-1-[(3,5-diméthylphényl)sulfonyl]-1*H*-indol-2-yl]méthoxy]-2-méthylpropanoïque, méthyl ester**

En opérant de façon analogue à l'exemple 215, au départ du composé obtenu selon la préparation 151, on obtient le produit attendu sous forme d'un solide blanc (rendement = 94 %).
F = 110-112°C.

**Exemple 268**

**Acide 2-[[5-chloro-1-[(3,5-diméthylphényl)sulfonyl]-1*H*-indol-2-yl]méthoxy]-2-méthylpropanoïque**

En opérant de façon analogue à l'exemple 2, au départ du composé obtenu selon l'exemple 267, on obtient le produit attendu sous forme d'un solide blanc (rendement = 97 %).
F = 162-164°C.

**Exemple 269**

**Acide 2-[[5-chloro-1-[(2,5-diméthoxyphényl)sulfonyl]-1*H*-indol-2-yl]méthoxy]-2-méthylpropanoïque, méthyl ester**

En opérant de façon analogue à l'exemple 215, au départ du composé obtenu selon la préparation 152, on obtient le produit attendu sous forme d'un solide beige (rendement = 70 %).
F = 132-134°C.

**Exemple 270**

**Acide 2-[[5-chloro-1-[(2,5-diméthoxyphényl)sulfonyl]-1*H*-indol-2-yl]-méthoxy]-2-méthylpropanoïque**

En opérant de façon analogue à l'exemple 2, au départ du composé obtenu selon l'exemple 269, on obtient le produit attendu sous forme d'un solide blanc (rendement = 96 %).
F = 156-158°C.

**Exemple 271**

**Acide 2-[[5-chloro-1-[(4-méthoxyphényl)sulfonyl]-1*H*-indol-2-yl]méthoxy]-2-méthylpropanoïque, méthyl ester**

En opérant de façon analogue à l'exemple 215, au départ du composé obtenu selon la préparation 153, on obtient le produit attendu sous forme d'un solide blanc (rendement = 92 %).

F = 96-98°C.

**Exemple 272**

**Acide 2-[[5-chloro-1-[(4-méthoxyphényl)sulfonyl]-1*H*-indol-2-yl]méthoxy]-2-méthylpropanoïque**

En opérant de façon analogue à l'exemple 2, au départ du composé obtenu selon l'exemple 271, on obtient le produit attendu sous forme d'un solide blanc (rendement = 99 %).
F = 150-152°C.

**Exemple 273**

**Acide 2-[[5-chloro-1-[(2-méthyl-6-benzothiazolyl)sulfonyl]-1*H*-indol-2-yl]méthoxy]propanoïque, éthyl ester**

En opérant de façon analogue à l'exemple 215, au départ du composé obtenu selon la préparation 154, on obtient le produit attendu sous forme d'une huile jaune (rendement = 78 %).
$^1$H RMN ($DMSOd_6$, 250 MHz) $\delta$ : 8,93 (s, 1H) ; 8,00 (m, 3H) ; 7,66 (d, 1H) ; 7,35 (dd, 1H) ; 6,85 (s, 1H) ; 5,03 (d, 1H) ; 4,92 (d, 1H) ; 4,24 (d, 1H) ; 4,13 (d, 2H) ; 2,83 (s, 3H) ; 1,32 (d, 3H) ; 1,19 (t, 3H).

**Exemple 274**

**Acide 2-[[5-chloro-1-[(2-méthyl-6-benzothiazolyl)sulfonyl]-1*H*-indol-2-yl]méthoxy]propanoïque**

En opérant de façon analogue à l'exemple 2, au départ du composé obtenu selon l'exemple 273, on obtient le produit attendu sous forme d'un solide blanc (rendement = 95 %).
F = 106-108°C.

**Exemple 275**

**Acide 2-[[5-chloro-1-[(2,3-dihydro-1,4-benzodioxin-6-yl)sulfonyl]-1*H*-indol-2-yl]méthoxy]propanoïque, éthyl ester**

En opérant de façon analogue à l'exemple 215, au départ du composé obtenu selon la préparation 155, on obtient le produit attendu sous forme d'une huile jaune (rendement = 86 %).
$^1$H RMN ($DMSOd_6$, 300 MHz) $\delta$ : 7,98 (d, 1H) ; 7,68 (d, 1H) ; 7,46 (m, 2H) ; 7,35 (dd, 1H) ; 7,01 (d, 1H) ; 6,83 (s, 1H) ; 4,97 (d, 1H) ; 4,84 (d, 1H) ; 4,27 (m, 5H) ; 4,20 (q, 2H) ; 1,35 (d, 3H) ; 1,19 (t, 3H).

**Exemple 276**

**Acide 2-[[5-chloro-1-[(2,3-dihydro-1,4-benzodioxin-6-yl)sulfonyl]-1*H*-indol-2-yl]méthoxy]propanoïque**

En opérant de façon analogue à l'exemple 2, au départ du composé obtenu selon l'exemple 275, on obtient le produit attendu sous forme d'un solide jaune (rendement = 97 %).
F = 70°C.

**Exemple 277**

**Acide 2-[[5-chloro-1-[(3,4-dihydro-4-méthyl-2*H*-1,4-benzoxazin-7-yl)-sulfonyl]-1*H*-indol-2-yl]méthoxy]propanoïque, éthyl ester**

En opérant de façon analogue à l'exemple 215, au départ du composé obtenu selon la préparation 156, on obtient le produit attendu sous forme d'une huile incolore (rendement = 85 %).
$^1$H RMN ($DMSOd_6$, 250 MHz) $\delta$: 8,01 (d, 1H) ; 7,66 (d, 1H) ; 7,34 (dd, 1H) ; 7,13 (dd, 1H) ; 6,99 (d, 1H) ; 6,78 (m, 2H) ; 4,98 (d, 1H) ; 4,85 (d, 1H) ; 4,23 (m, 3H) ; 4,18 (q, 2H) ; 3,24 (m, 2H) ; 2,80 (s, 3H) ; 1,34 (d, 3H) ; 1,19 (t, 3H).

**Exemple 278**

**Acide 2-[[5-chloro-1-[(3,4-dihydro-4-méthyl-2*H*-1,4-benzoxazin-7-yl)-sulfonyl]-1*H*-indol-2-yl]méthoxy]propanoïque**

En opérant de façon analogue à l'exemple 2, au départ du composé obtenu selon l'exemple 277, on obtient le produit attendu sous forme d'un solide blanc (rendement = 98 %).
F = 68°C.

**Exemple 279**

**Acide 2-[[5-chloro-1-[(3,5-diméthyl-4-isoxazolyl)sulfonyl]-1*H*-indol-2-yl]-méthoxy]propanoïque, éthyl ester**

En opérant de façon analogue à l'exemple 215, au départ du composé obtenu selon la préparation 157, on obtient le produit attendu sous forme d'une huile incolore (rendement = 91 %).
$^1$H RMN (DMSO$d_6$, 300 MHz) δ : 7,84 (d, 1H) ; 7,78 (d, 1H) ; 7,40 (dd, 1H) ; 6,92 (s, 1H) ; 4,91 (d, 1H) ; 4,77 (d, 1H) ; 4,12 (q, 1H) ; 4,08 (q, 2H) ; 2,63 (s, 3H) ; 2,03 (s, 3H) ; 1,18 (m, 6H).

**Exemple 280**

**Acide 2-[[5-chloro-1-[(3,5-diméthyl-4-isoxazolyl)sulfonyl]-1*H*-indol-2-yl]méthoxy]propanoïque**

En opérant de façon analogue à l'exemple 2, au départ du composé obtenu selon l'exemple 279, on obtient le produit attendu sous forme d'un solide blanc (rendement = 92 %).
F = 110-112°C.

**Exemple 281**

**Acide 2-[[5-chloro-1-[(2,5-diméthoxyphényl)sulfonyl]-1*H*-indol-2-yl]-méthoxy]propanoïque, éthyl ester**

En opérant de façon analogue à l'exemple 215, au départ du composé obtenu selon la préparation 158, on obtient le produit attendu sous forme d'un solide beige (rendement = 88 %).
F = 118-120°C.

**Exemple 282**

**Acide 2-[[5-chloro-1-[(2,5-diméthoxyphényl)sulfonyl]-1*H*-indol-2-yl]-méthoxy]propanoïque**

En opérant de façon analogue à l'exemple 2, au départ du composé obtenu selon l'exemple 281, on obtient le produit attendu sous forme d'un solide blanc (rendement = 99 %).
F = 196-198°C.

**Exemple 283**

**Acide 2-[[1-[(2-méthyl-6-benzothiazolyl)sulfonyl]-5-(trifluorométhyl)-1*H*-indol-2-yl]méthoxy]propanoïque, éthyl ester**

En opérant de façon analogue à l'exemple 215, au départ du composé obtenu selon la préparation 160, on obtient le produit attendu sous forme d'une huile jaune (rendement = 83 %).
$^1$H RMN (DMSO$d_6$, 250 MHz) δ : 8,98 (m, 1H) ; 8,26 (d, 1H) ; 8,04 (m, 3H) ; 7,65 (dd, 1H) ; 7,01 (s, 1H) ; 5,07 (d, 1H) ; 4,95 (d, 1H) ; 4,26 (q, 1H) ; 4,14 (q, 2H) ; 2,83 (s, 3H) ; 1,34 (d, 3H) ; 1,16 (t, 3H).

**Exemple 284**

**Acide 2-[[1-[(2-méthyl-6-benzothiazolyl)sulfonyl]-5-(trifluorométhyl)-1*H*-indol-2-yl]méthoxy]propanoïque**

En opérant de façon analogue à l'exemple 2, au départ du composé obtenu selon l'exemple 283, on obtient le produit attendu sous forme d'un solide blanc (rendement = 84 %).

F = 146-148 °C.

**Exemple 285**

**Acide 2-[[1-[(6-benzothiazolyl)sulfonyl]-5-(trifluorométhyl)-1*H*-indol-2-yl]méthoxy]propanoïque, éthyl ester**

En opérant de façon analogue à l'exemple 215, au départ du composé obtenu selon la préparation 161, on obtient le produit attendu sous forme d'une huile jaune (rendement = 76 %).
$^1H$ RMN ($DMSOd_6$, 250 MHz) δ : 9,67 (s, 1H) ; 9,13 (d, 1H) ; 8,21 (m, 2H) ; 8,14 (dd, 1H) ; 8,11 (m, 1H) ; 7,68 (dd, 1H) ; 7,01 (s, 1H) ; 5,08 (d, 1H) ; 4,96 (d, 1H) ; 4,26 (q, 1H) ; 4,11 (q, 2H) ; 1,31 (d, 3H) ; 1,16 (t, 3H).

**Exemple 286**

**Acide 2-[[1-[(6-benzothiazolyl)sulfonyl]-5-(trifluorométhyl)-1*H*-indol-2-yl]-méthoxy]propanoïque**

En opérant de façon analogue à l'exemple 2, au départ du composé obtenu selon l'exemple 285, on obtient le produit attendu sous forme d'un solide pâteux beige (rendement = 57 %).
$^1H$ RMN ($DMSOd_6$, 300 MHz) δ : 9,67 (s, 1H) ; 9,15 (d, 1H) ; 8,24 (m, 2H) ; 8,11 (dd, 1H) ; 8,03 (s, 1H) ; 7,66 (dd, 1H) ; 7,02 (s, 1H) ; 5,10 (d, 1H) ; 4,94 (d, 1H) ; 4,18 (q, 1H) ; 1,32 (d, 3H).

**Exemple 287**

**Acide 2-[[1-[(2,3-dihydro-1,4-benzodioxin-6-yl)sulfonyl]-5-(trifluorométhyl)-1*H*-indol-2-yl]méthoxy]propanoïque, éthyl ester**

En opérant de façon analogue à l'exemple 215, au départ du composé obtenu selon la préparation 162, on obtient le produit attendu sous forme d'une huile jaune (rendement = 84 %).
$^1H$ RMN ($DMSOd_6$, 250 MHz) δ : 8,19 (d, 1H) ; 8,03 (s, 1H) ; 7,66 (dd, 1H) ; 7,53 (m, 2H) ; 7,04 (dd, 1H) ; 6,98 (s, 1H) ; 5,01 (d, 1H) ; 4,88 (d, 1H) ; 4,27 (m, 5H) ; 4,21 (q, 2H) ; 1,36 (d, 3H) ; 1,19 (t, 3H).

**Exemple 288**

**Acide 2-[[1-[(2,3-dihydro-1,4-benzodioxin-6-yl)sulfonyl]-5-(trifluorométhyl)-1*H*-indol-2-yl]méthoxy]propanoïque**

En opérant de façon analogue à l'exemple 2, au départ du composé obtenu selon l'exemple 287, on obtient le produit attendu sous forme d'un solide jaune (rendement = 99 %).
F = 70°C.

**Exemple 289**

**Acide 2-[[1-[(3,4-dihydro-4-méthyl-2*H*-1,4-benzoxazin-7-yl)sulfonyl]-5-(trifluorométhyl)-1*H*-indol-2-yl]méthoxy]propanoïque, éthyl ester**

En opérant de façon analogue à l'exemple 215, au départ du composé obtenu selon la préparation 163, on obtient le produit attendu sous forme d'une huile jaune (rendement = 88 %).
$^1H$ RMN ($DMSOd_6$, 300 MHz) δ: 8,22 (d , 1H) ; 8,02 (s, 1H) ; 7,65 (dd, 1H) ; 7,19 (dd, 1H) ; 7,04 (d, 1H) ; 6,96 (s, 1H) ; 6,78 (d, 1H) ; 5,03 (d, 1H) ; 4,89 (d, 1H) ; 4,24 (m, 3H) ; 4,14 (q, 2H) ; 3,24 (m, 2H) ; 2,80 (s, 3H) ; 1,36 (d, 3H) ; 1,19 (t, 3H).

**Exemple 290**

**Acide 2-[[1-[(3,4-dihydro-4-méthyl-2*H*-1,4-benzoxazin-7-yl)sulfonyl]-5-(trifluorométhyl)-1*H*-indol-2-yl]méthoxy]propanoïque**

En opérant de façon analogue à l'exemple 2, au départ du composé obtenu selon l'exemple 289, on obtient le produit attendu sous forme d'un solide blanc (rendement = 87 %).
F = 118-120°C.

**Exemple 291**

**Acide 2-[[1-[(2,5-diméthoxyphényl)sulfonyl]-5-(trifluorométhyl)-1*H*-indol-2-yl]méthoxy]propanoïque, éthyl ester**

En opérant de façon analogue à l'exemple 215, au départ du composé obtenu selon la préparation 164, on obtient le produit attendu sous forme d'un solide blanc (rendement = 89 %).
F = 130-132°C.

**Exemple 292**

**Acide 2-[[1-[(2,5-diméthoxyphényl)sulfonyl]-5-(trifluorométhyl)-1*H*-indol-2-yl]méthoxy]propanoïque**

En opérant de façon analogue à l'exemple 2, au départ du composé obtenu selon l'exemple 291, on obtient le produit attendu sous forme d'un solide blanc (rendement = 97 %).
F = 212-214°C.

**Exemple 293**

**Acide (2S)-2-[[5-chloro-1-[(6-benzothiazolyl)sulfonyl]-1*H*-indol-2-yl]-méthoxy]propanoïque, éthyl ester**

En opérant de façon analogue à l'exemple 215, au départ du composé obtenu selon la préparation 166, on obtient le produit attendu sous forme d'une huile jaune (rendement = 77 %).
$^1$H RMN ($DMSOd_6$, 300 MHz) δ : 9,66 (s, 1H) ; 9,08 (d, 1H) ; 8,21 (d, 1H) ; 8,05 (m, 2H) ; 7,67 (d, 1H) ; 7,36 (dd, 1H) ; 6,86 (s, 1H) ; 5,03 (d, 1H) ; 4,93 (d, 1H) ; 4,23 (q, 1H) ; 4,10 (q, 2H) ; 1,31 (d, 3H) ; 1,17 (t, 3H).

**Exemple 294**

**Acide (2S)-2-[[5-chloro-1-[(6-benzothiazolyl)sulfonyl]-1*H*-indol-2-yl]-méthoxy]propanoïque**

En opérant de façon analogue à l'exemple 2, au départ du composé obtenu selon l'exemple 293, on obtient le produit attendu sous forme d'un solide blanc (rendement = 66 %).
F = 82°C.

$[\alpha]_D^{28}$ = - 41° (c = 0,39; MeOH).

**Exemple 295**

**Acide (2R)-2-[[5-chloro-1-[(6-benzothiazolyl)sulfonyl]-1*H*-indol-2-yl]-méthoxy]propanoïque, méthyl ester**

En opérant de façon analogue à l'exemple 215, au départ du composé obtenu selon la préparation 168, on obtient le produit attendu sous forme d'une huile jaune (rendement = 80 %).
$^1$H RMN ($DMSOd_6$, 300 MHz) δ : 9,66 (s, 1H) ; 9,08 (d, 1H) ; 8,22 (d, 1H) ; 8,03 (m, 2H) ; 7,67 (d, 1H) ; 7,36 (dd, 1H) ; 6,86 (s, 1H) ; 5,03 (d, 1H) ; 4,92 (d, 1H) ; 4,27 (q, 1H) ; 3,66 (s, 3H) ; 1,32 (d, 3H).

**Exemple 296**

**Acide 5-chloro-1-[(2-méthyl-7-benzothiazolyl)sulfonyl]-1*H*-indole-2-butanoïque, méthyl ester**

En opérant de façon analogue à l'exemple 215, au départ du composé obtenu selon la préparation 169, on obtient le produit attendu sous forme d'un solide jaune (rendement = 76 %).
F = 129 °C.

**Exemple 297**

**Acide 5-chloro-1-[(2-méthyl-7-benzothiazolyl)sulfonyl]-1*H*-indole-2-butanoïque**

En opérant de façon analogue à l'exemple 2, au départ du composé obtenu selon l'exemple 296, on obtient le produit attendu sous forme d'une poudre blanche (rendement = 93 %).
F = 177-181 °C.

**Exemple 298**

**Acide 1-[(1-acétyl-1*H*-indol-5-yl)sulfonyl]-5-chloro-1*H*-indole-2-butanoïque, méthyl ester**

En opérant de façon analogue à l'exemple 215, au départ du composé obtenu selon la préparation 170, on obtient le produit attendu sous forme d'une poudre blanche (rendement = 89 %).
F = 127-131 °C.

**Exemple 299**

**Acide 1-[(1*H*-indol-5-yl)sulfonyl]-5-chloro-1*H*-indole-2-butanoïque**

En opérant de façon analogue à l'exemple 2, au départ du composé obtenu selon l'exemple 298, on obtient le produit attendu sous forme d'un solide blanc (rendement = 52 %).
F = 213°C.

**Exemple 300**

**Acide 5-chloro-1-[(2-méthyl-7-benzothiazolyl)sulfonyl]-1*H*-indole-2-propanoïque, méthyl ester**

En opérant de façon analogue à l'exemple 215, au départ du composé obtenu selon la préparation 171, on obtient le produit attendu sous forme d'un solide amorphe (rendement = 70 %).
$^1H$ RMN ($DMSOd_6$, 300 MHz) $\delta$ : 8,25 (dd, 1H) ; 7,93 (d, 1H) ; 7,76 (d, 1H) ; 7,68 (d, 1H) ; 7,63 (m, 1H) ; 7,30 (dd, 1H) ; 6,64 (s, 1H) ; 3,59 (s, 3H) ; 3,24 (t, 2H) ; 2,83 (s, 3H) ; 2,77 (t, 2H).

**Exemple 301**

**Acide 5-chloro-1-[(2-méthyl-7-benzothiazolyl)sulfonyl]-1*H*-indole-2-propanoïque**

En opérant de façon analogue à l'exemple 2, au départ du composé obtenu selon l'exemple 300, on obtient le produit attendu sous forme d'une poudre blanche (rendement = 86 %).
F = 188-189°C.

**Exemple 302**

**Acide 1-[(2-amino-6-benzoxazolyl)sulfonyl]-5-chloro-1*H*-indole-2-propanoïque, méthyl ester**

En opérant de façon analogue à l'exemple 215, au départ du composé obtenu selon la préparation 172, on obtient le produit attendu sous forme d'un solide beige (rendement = 50 %).
F = 190-195 °C.

**Exemple 303**

**Acide 1-[(2-amino-6-benzoxazolyl)sulfonyl]-5-chloro-1*H*-indole-2-propanoïque**

En opérant de façon analogue à l'exemple 2, au départ du composé obtenu selon l'exemple 302, on obtient le produit attendu sous forme d'un solide blanc (rendement = 53 %).
F = 242-249°C.

**Exemple 304**

**Acide 5-chloro-1-[(2,3-dihydro-5-benzofuranyl)sulfonyl]-1*H*-indole-2-propanoïque, méthyl ester**

En opérant de façon analogue l'exemple 215, au départ du composé obtenu selon la préparation 173, on obtient lé produit attendu sous forme d'un solide amorphe (rendement = 88 %).
$^1$H RMN ($DMSOd_6$, 300 MHz) δ : 8,02 (d, 1H) ; 7,70 (m, 1H) ; 7,63 (dd, 1H) ; 7,58 (d, 1H) ; 7,30 (dd, 1H) ; 6,89 (d, 1H) ; 6,56 (s, 1H) ; 4,60 (t, 2H) ; 3,62 (s, 3H) ; 3,28 (t, 2H) ; 3,18 (t, 2H); 2,81 (t, 2H).

**Exemple 305**

**Acide 5-chloro-1-[(2,3-dihydro-5-benzofuranyl)sulfonyl]-1*H*-indole-2-propanoïque**

En opérant de façon analogue à l'exemple 2, au départ du composé obtenu selon l'exemple 304, on obtient le produit attendu sous forme d'un solide blanc (rendement = 91 %).
F = 170-171˚C.

**Exemple 306**

**Acide 1-[(2-amino-6-benzothiazolyl)sulfonyl]-5-chloro-1*H*-indole-2-propanoïque, méthyl ester**

En opérant de façon analogue à l'exemple 215, au départ du composé obtenu selon la préparation 174, on obtient le produit attendu sous forme d'un solide jaune (rendement = 47 %).
F = 217-222 ˚C.

**Exemple 307**

**Acide 1-[(2-amino-6-benzothiazolyl)sulfonyl]-5-chloro-1*H*-indole-2-propanoïque**

En opérant de façon analogue à l'exemple 2, au départ du composé obtenu selon l'exemple 306, on obtient le produit attendu sous forme d'une poudre blanche (rendement = 45 %).
F = 250-255 ˚C.

**Exemple 308**

**Acide 2-[[[5-chloro-1-(phénylsulfonyl)-1*H*-indol-2-yl]méthyl]thio]-2-méthyl-propanoïque**

En opérant de façon analogue à l'exemple 201, au départ du composé obtenu selon la préparation 120 et d'acide 2-méthyl-2-(2-propynylthio)propanoïque, on obtient le produit attendu sous forme d'un solide beige (rendement = 14 %).
F = 150-152 ˚C.

**Exemple 309**

**Acide 2-[[[5-chloro-1-(phénylsulfonyl)-1*H*-indol-2-yl]méthyl]thio] propanoïque**

En opérant de façon analogue à l'exemple 201, au départ du composé obtenu selon la préparation 120 et d'acide 2-(2-propynylthio)propanoïque, on obtient le produit attendu sous forme d'un solide blanc (rendement = 17 %).
F = 138 ˚C.

**Exemple 310**

**Acide 2-[[[1-(phénylsulfonyl)-5-(trifluorométhyl)-1*H*-indol-2-yl]méthyl]thio]-2-méthyl-propanoïque**

En opérant de façon analogue à l'exemple 201, au départ du composé obtenu selon la préparation 121 et d'acide 2-méthyl-2-(2-propynylthio)propanoïque, on obtient le produit attendu sous forme d'un solide beige (rendement = 8 %).
F = 90 ˚C.

**Exemple 311**

**Acide 2-[[[1-(phénylsulfonyl)-5-(trifluorométhyl)-1*H*-indol-2-yl]méthyl]thio]propanoïque**

En opérant de façon analogue à l'exemple 201, au départ du composé obtenu selon la préparation 121 et d'acide 2-(2-propynylthio)propanoïque, on obtient le produit attendu sous forme d'un solide beige (rendement = 15 %).
F = 120 °C.

**Exemple 312**

**Acide 1-[(6-benzothiazolyl)sulfonyl]-5-méthyl-1*H*-indole-2-butanoïque, méthyl ester**

En opérant de façon analogue à l'exemple 201, au départ du composé obtenu selon la préparation 175 et d'ester méthylique de l'acide 5-hexynoïque, on obtient le produit attendu sous forme d'une poudre jaune (rendement = 47 %).
F = 128-130 °C.

**Exemple 313**

**Acide 1-[(6-benzothiazolyl)sulfonyl]-5-méthyl-1*H*-indole-2-butanoïque**

En opérant de façon analogue à l'exemple 2, au départ du composé obtenu selon l'exemple 312, on obtient le produit attendu sous forme d'une poudre jaune (rendement = 70 %).
F=128°C

**Exemple 314**

**Acide 1-(phénylsulfonyl)-5-méthyl-1*H*-indole-2-propanoïque, méthyl ester**

En opérant de façon analogue à l'exemple 201, au départ du composé obtenu selon la préparation 176 et d'ester méthylique de l'acide 4-pentynoïque, on obtient le produit attendu sous forme d'un solide beige (rendement = 13 %).
F = 98-102 °C.

**Exemple 315**

**Acide 1-(phénylsulfonyl)-5-méthyl-1*H*-indole-2-propanoïque**

En opérant de façon analogue à l'exemple 2, au départ du composé obtenu selon l'exemple 314, on obtient le produit attendu sous forme d'une poudre blanche (rendement = 59 %).
F = 176-182 °C

**Exemple 316**

**Acide 1-[(6-benzothiazolyl)suifonyl]-5-chloro-α,α-diméthyl-1*H*-indole-2-butanoïque**

En opérant de façon analogue à l'exemple 201, au départ du composé obtenu selon la préparation 177 et de l'acide 2,2-diméthyl-5-hexynoïque, on obtient le produit attendu sous forme d'une poudre jaune (rendement = 46 %).
F = 151 °C.

**Exemple 317**

**Acide 5-chloro-α,α-diméthyl-1-(phénylsulfonyl)-1*H*-indole-2-butanoïque**

En opérant de façon analogue à l'exemple 201, au départ du composé obtenu selon la préparation 120 et de l'acide 2,2-diméthyl-5-hexynoïque, on obtient le produit attendu sous forme d'un solide brun (rendement = 32 %).
F = 242 °C

**Exemple 318**

**Acide 5-chloro-1-[(2,3-dihydro-1*H*-indol-5-yl)sulfonyl]-1*H*-indole-2-propanoïque**

En opérant de façon analogue à l'exemple 2, au départ du composé obtenu selon l'exemple 158, on obtient le produit attendu sous forme d'un solide brun (rendement = 37 %).
F = 157˚C.

**Exemple 319**

**Acide 5-chloro-1-[(1*H*-indol-5-yl)sulfonyl]-1*H*-indole-2-propanoïque**

On prépare une solution de 40 mg (0,1 mM) du composé obtenu selon l'exemple 318 dans 4 ml de toluène et on ajoute, à température de reflux du solvant, une solution de 22 mg (0,1 mM) de DDQ (2,3-dichloro-5,6-dicyano-1,4-benzoquinone) dans 4 ml de toluène. Le mélange réactionnel est agité pendant 12 heures à température de reflux du solvant puis refroidi , acidifié par addition d'une solution d'acide chlorhydrique M, et dilué avec de l'acétate d'éthyle. La phase organique est lavée par une solution de thiosulfate de sodium, puis à l'eau, séchée sur sulfate de magnésium et concentrée sous pression réduite. Le résidu d'évaporation est purifié par chromatographie HPLC en éluant à l'aide d'un mélange en gradient acétonitrilé-eau. On obtient ainsi le produit attendu sous forme d'un solide beige (rendement : 28 %).
F = 79 ˚C.

**Exemple 320**

**Acide 5-chloro-1-[[4-amino-3-(méthylthio)phényl]sulfonyl]-1*H*-indole-2-butanoïque**

On prépare une solution de 1g (2,27 mM) du composé obtenu selon l'exemple 117 dans 16 ml d'éthanol et 16 ml d'une solution aqueuse de potasse 3,5 M. Le mélange est agité à température ambiante pendant 5 heures, puis on ajoute 0,9 ml d'iodure de méthyle et on agite à nouveau le mélange réactionnel pendant 1 heure à température ambiante. Le milieu est ensuite dilué par 100 ml d'eau et acidifié doucement par une solution d'acide chlorhydrique N. Le précipité formé est extrait par du dichlorométhane et la phase organique obtenue est lavée à l'eau, séchée sur sulfate de magnésium et concentrée sous pression réduite. On obtient le produit attendu sous forme d'un solide cristallin blanc (rendement = 95 %).
F = 158 ˚C.

**Exemple 321**

**Acide 5-chloro-1-[(4-fluoro-3-nitrophényl)sulfonyl]-1*H*-indole-2-butanoïque, méthyl ester**

En opérant de façon analogue l'exemple 215, au départ du composé obtenu selon la préparation 178, on obtient le produit attendu sous forme d'une poudre jaune pâle (rendement = 96 %).
F = 93˚C.

**Exemple 322**

**Acide 1-[(4-amino-3-nitrophényl)sulfonyl]-5-chloro-1*H*-indole-2-butanoïque, méthyl ester**

On prépare une solution de 100 mg (0,22 mM) du composé obtenu selon l'exemple 321 dans 1 ml de dioxanne et on ajoute 0,77 ml d'ammoniaque à 32%. Le mélange est agité à température ambiante pendant 30 mn, puis dilué par 8 ml d'acétate d'éthyle, lavé à l'eau, séché sur sulfate de magnésium et concentré sous pression réduite. On obtient le produit attendu sous forme d'une poudre jaune pâle (rendement = 95 %).
F = 157 ˚C.

**Exemple 323**

**Acide 5-chloro-1-[(3,4-diaminophényl)sulfonyl]-1*H*-indole-2-butanoïque, méthyl ester**

On prépare une suspension de 604 mg (1,33 mM) du composé obtenu selon l'exemple 322 dans 8 ml d'acide acétique et on ajoute, sous agitation, 390 mg (7 mM) de poudre de fer. Le mélange est agité à 60˚C pendant 1 heure,

puis dilué par de l'eau et de l'acétate d'éthyle. La phase organique est séparée, filtrée, lavée à l'eau, séchée sur sulfate de magnésium et concentrée sous pression réduite. On obtient le produit attendu sous forme d'une poudre jaune pâle (rendement = 70 %).
F = 158-160 °C.

**Exemple 324**

**Acide 1-(1*H*-benzimidazol-5-ylsulfonyl)-5-chloro-1*H*-indole-2-butanoïque, méthyl ester**

On prépare une suspension de 498 mg (1,18 mM) du composé obtenu selon l'exemple 323 dans 1,5 ml d'acide formique. Le mélange est agité à 60°C pendant 2 heures, puis dilué par de l'eau, neutralisé par 10 ml de soude N et extrait par l'acétate d'éthyle. La phase organique est séparée, lavée à l'eau, séchée sur sulfate de magnésium et concentrée sous pression réduite. Le produit brut est purifié par chromatographie sur gel de silice en éluant à l'aide d'un mélange toluène/isopropanol/ammoniaque (85/15/1 ; v/v/v). On obtient le produit attendu sous forme d'une mousse beige (rendement = 83 %).
$^{1}H$ RMN ($DMSOd_6$, 300 MHz) $\delta$ : 12,95 (s, 1H) ; 8,46 (s, 1H) ; 8,10 (m, 2H) ; 7,72 (d, 1H) ; 7,56 (m, 2H) ; 7,32 (dd, 1H) ; 7,19 (m, 2H) ; 6,58 (s, 1H) ; 3,58 (s, 3H) ; 3,05 (t, 2H) ; 2,45 (t, 2H) ; 1,99 (quin, 2H).

**Exemple 325**

**Acide 1-(1*H*-benzimidazol-5-ylsulfonyl)-5-chloro-1*H*-indole-2-butanoïque**

En opérant de façon analogue à l'exemple 2, au départ du composé obtenu selon l'exemple 324, on obtient le produit attendu sous forme d'une poudre blanche (rendement = 66 %).
F = 212°C.

**Exemple 326**

**Acide 5-chloro-1-[(2,3-dihydro-1*H*-indol-5-yl)sulfonyl]-1*H*-indole-2-butanoïque**

En opérant de façon analogue à l'exemple 2, au départ du composé obtenu selon l'exemple 134, on obtient le produit attendu sous forme d'un solide beige (rendement = 19 %).
F = 203°C.

Les composés selon l'invention décrits ci-dessus ont été reportés dans les tableaux suivants:

Le tableau I regroupe des composés selon l'invention dans lesquels X est une liaison simple et $R_3$ et $R_4$ sont chacun un atome d'hydrogène.
Le tableau II regroupe des exemples de composés de formule I selon l'invention dans lesquels X représente un atome d'oxygène.
Le tableau III regroupe des exemples de composés de formule I selon l'invention dans lesquels X représente un atome de soufre.
Le tableau IV rassemble les composés dans lesquels X est une liaison simple et R3 ou R4 sont différents d'un atome d'hydrogène.

Dans ces tableaux, Ac représente le groupe acétyle.

TABLEAU I

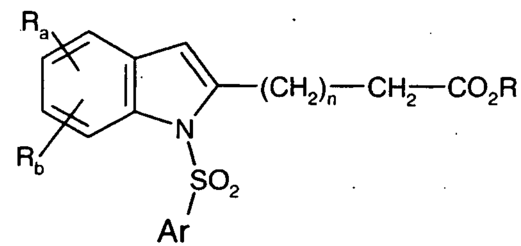

| Ex | Ra | Rb | Ar | n | R |
|---|---|---|---|---|---|
| 1 | 5-Cl | H | 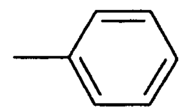 | 1 | $CH_3$ |
| 2 | 5-Cl | H | 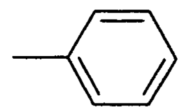 | 1 | H |
| 2a | 5-Cl | H | 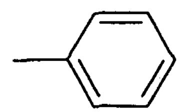 | 1 | Na |
| 3 | $5-CF_3$ | H | 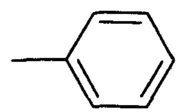 | 1 | $CH_3$ |
| 4 | $5-CF_3$ | H | 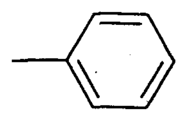 | 1 | H |

(suite)

| Ex | Ra | Rb | Ar | n | R |
|---|---|---|---|---|---|
| 5 | 5-Br | H | 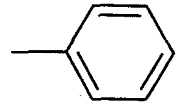 | 1 | $CH_3$ |
| 6 | 5-Br | H | 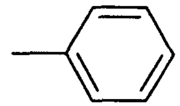 | 1 | H |
| 7 | 5-<br>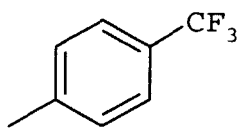 | H | 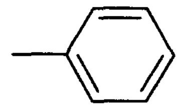 | 1 | $CH_3$ |
| 8 | 5-<br>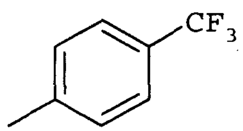 | H | 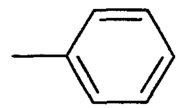 | 1 | H |
| 9 | H | H | 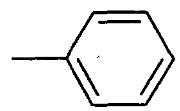 | 1 | $CH_3$ |
| 10 | H | H | 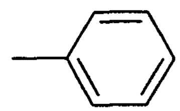 | 1 | H |
| 11 | 5-Cl | H | 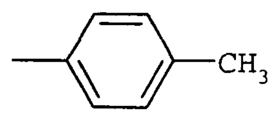 | 1 | $CH_3$ |

(suite)

| Ex | Ra | Rb | Ar | n | R |
|---|---|---|---|---|---|
| 12 | 5-Cl | H | 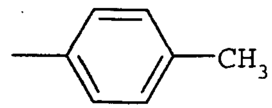 | 1 | H |
| 13 | 5-Cl | H | 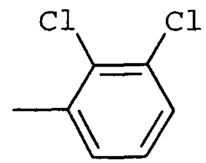 | 1 | $CH_3$ |
| 14 | 5-Cl | H | 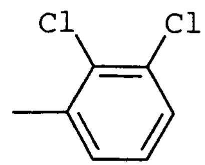 | 1 | H |
| 15 | 5-Cl | H | 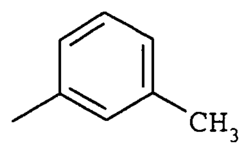 | 1 | $CH_3$ |
| 16 | 5-Cl | H | 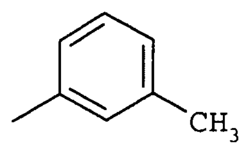 | 1 | H |
| 17 | 5-Cl | H | 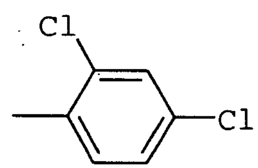 | 1 | $CH_3$ |

(suite)

| Ex | Ra | Rb | Ar | n | R |
|---|---|---|---|---|---|
| 18 | 5-Cl | H | 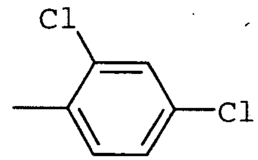 | 1 | H |
| 19 | 5-Cl | H | 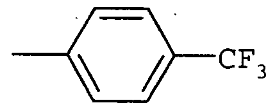 | 1 | $CH_3$ |
| 20 | 5-Cl | H | 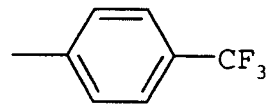 | 1 | H |
| 21 | 5-Cl | H | 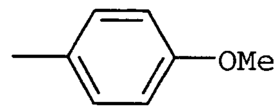 | 1 | $CH_3$ |
| 22 | 5-Cl | H | 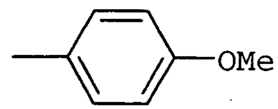 | 1 | H |
| 23 | 5-Cl | H | 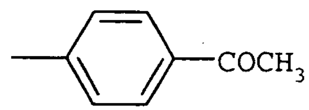 | 1 | $CH_3$ |
| 24 | 5-Cl | H | 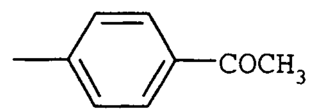 | 1 | H |

(suite)

| Ex | Ra | Rb | Ar | n | R |
|---|---|---|---|---|---|
| 25 | 5-Cl | H | 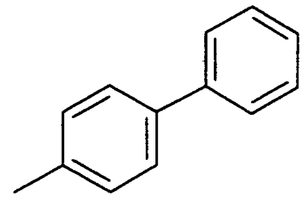 | 1 | $CH_3$ |
| 26 | 5-Cl | H | 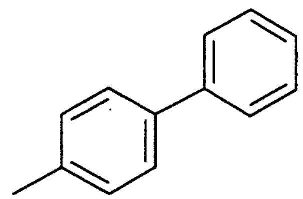 | 1 | H |
| 27 | 5-Cl | H | 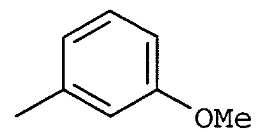 | 1 | $CH_3$ |
| 28 | 5-Cl | H | 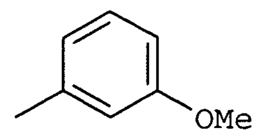 | 1 | H |
| 29 | 5-Cl | H | 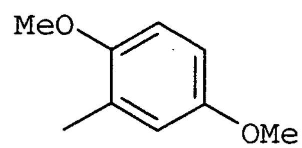 | 1 | $CH_3$ |
| 30 | 5-Cl | H | 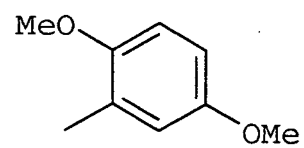 | 1 | H |

(suite)

| Ex | Ra | Rb | Ar | n | R |
|---|---|---|---|---|---|
| 31 | 5-Cl | H | 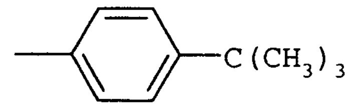 | 1 | $CH_3$ |
| 32 | 5-Cl | H | 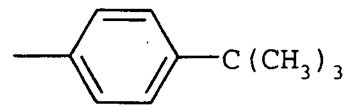 | 1 | H |
| 33 | 5-Cl | H | 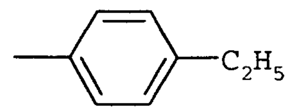 | 1 | $CH_3$ |
| 34 | 5-Cl | H | 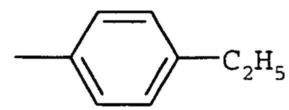 | 1 | H |
| 35 | 5-Cl | H | 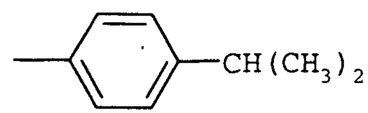 | 1 | $CH_3$ |
| 36 | 5-Cl | H | 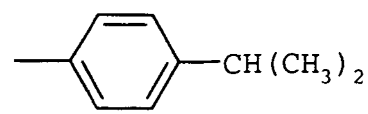 | 1 | H |
| 37 | 5-Cl | H | 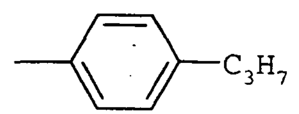 | 1 | $CH_3$ |
| 38 | 5-Cl | H | 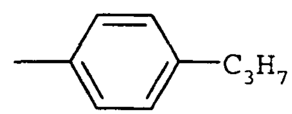 | 1 | H |

(suite)

| Ex | Ra | Rb | Ar | n | R |
|---|---|---|---|---|---|
| 39 | 5-Cl | H | 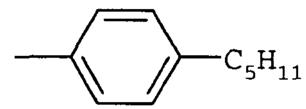 | 1 | $CH_3$ |
| 40 | 5-Cl | H | 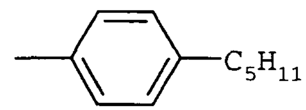 | 1 | H |
| 41 | 5-Cl | H | 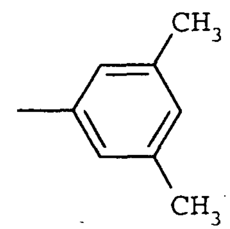 | 1 | $CH_3$ |
| 42 | 5-Cl | H | 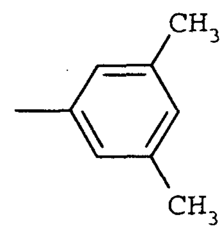 | 1 | H |
| 43 | 5-Cl | H | 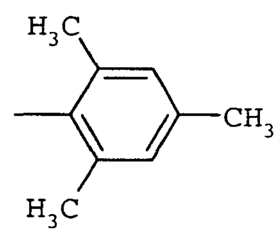 | 1 | $CH_3$ |

(suite)

| Ex | Ra | Rb | Ar | n | R |
|---|---|---|---|---|---|
| 44 | 5-Cl | H | 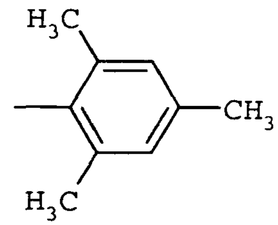 | 1 | H |
| 45 | 5-Cl | H | 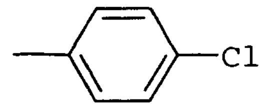 | 1 | $CH_3$ |
| 46 | 5-Cl | H | 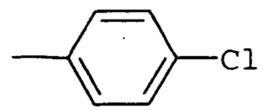 | 1 | H |
| 47 | 5-Cl | H | 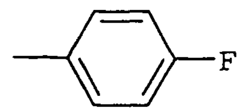 | 1 | $CH_3$ |
| 48 | 5-Cl | H | 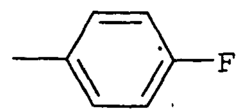 | 1 | H |
| 49 | 5-Cl | H | 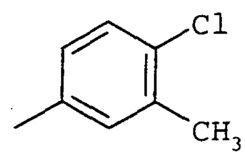 | 1 | $CH_3$ |
| 50 | 5-Cl | H | 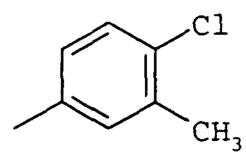 | 1 | H |

(suite)

| Ex | Ra | Rb | Ar | n | R |
|---|---|---|---|---|---|
| 51 | 5-Cl | H | 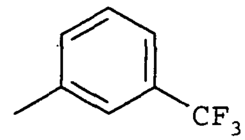 | 1 | $CH_3$ |
| 52 | 5-Cl | H | 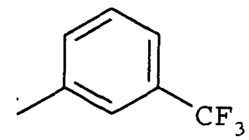 | 1 | H |
| 53 | 5-Cl | H | 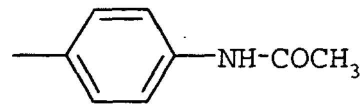 | 1 | $CH_3$ |
| 54 | 5-Cl | H | 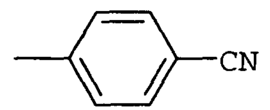 | 1 | $CH_3$ |
| 55 | 5-Cl | H | 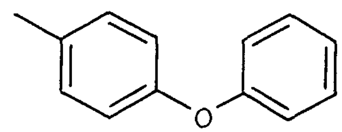 | 1 | $CH_3$ |
| 56 | 5-Cl | H | 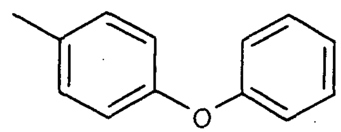 | 1 | H |
| 57 | 5-Cl | H | 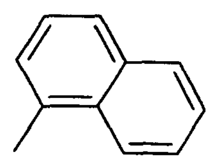 | 1 | $CH_3$ |

(suite)

| Ex | Ra | Rb | Ar | n | R |
|---|---|---|---|---|---|
| 58 | 5-Cl | H | 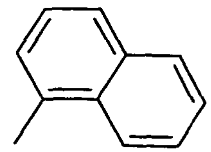 | 1 | H |
| 59 | 5-Cl | H | 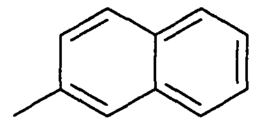 | 1 | $CH_3$ |
| 60 | 5-Cl | H | 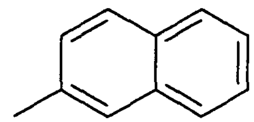 | 1 | H |
| 61 | 5-Cl | H | 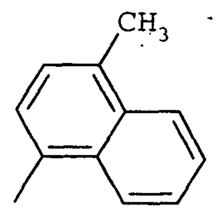 | 1 | $CH_3$ |
| 62 | 5-Cl | H | 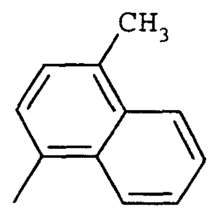 | 1 | H |
| 63 | 5-Cl | H | 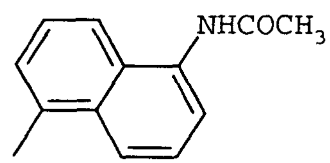 | 1 | $CH_3$ |

(suite)

| Ex | Ra | Rb | Ar | n | R |
|---|---|---|---|---|---|
| 64 | 5-Cl | H | 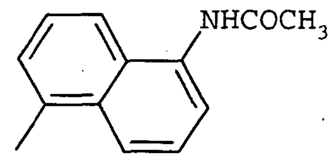 | 1 | H |
| 65 | 5-Cl | H | 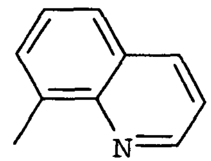 | 1 | $CH_3$ |
| 66 | 5-Cl | H | 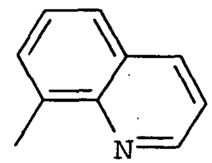 | 1 | H |
| 67 | 5-Cl | H | 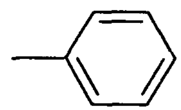 | 2 | $CH_3$ |
| 68 | 5-Cl | H | 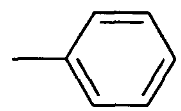 | 2 | H |
| 69 | 5-Cl | H | 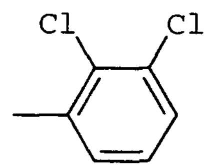 | 2 | $CH_3$ |

(suite)

| Ex | Ra | Rb | Ar | n | R |
|---|---|---|---|---|---|
| 70 | 5-Cl | H | 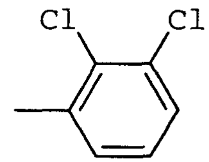 | 2 | H |
| 71 | 5-Cl | H | 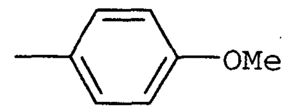 | 2 | $CH_3$ |
| 72 | 5-Cl | H | 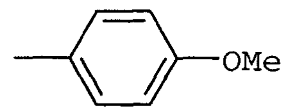 | 2 | H |
| 73 | 5-Cl | H | 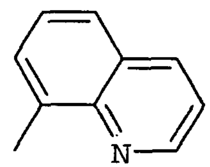 | 2 | $CH_3$ |
| 74 | 5-Cl | H | 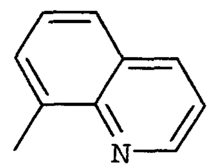 | 2 | H |
| 75 | 5-Cl | H | 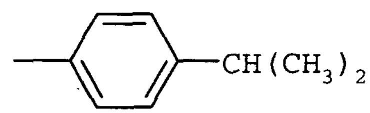 | 2 | $CH_3$ |
| 76 | 5-Cl | H | 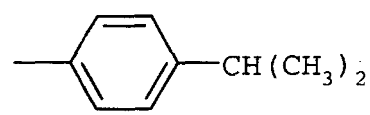 | 2 | H |

(suite)

| Ex | Ra | Rb | Ar | n | R |
|---|---|---|---|---|---|
| 77 | 5-Cl | H | 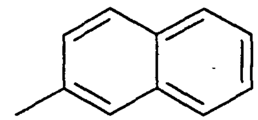 | 2 | $CH_3$ |
| 78 | 5-Cl | H | 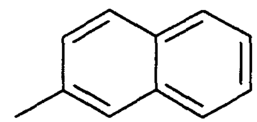 | 2 | H |
| 79 | 5-Cl | H | 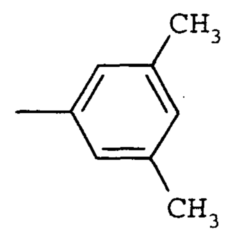 | 2 | $CH_3$ |
| 80 | 5-Cl | H | 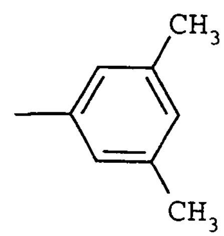 | 2 | H |
| 81 | 5-Cl | H | 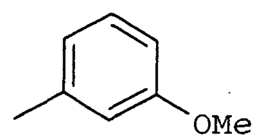 | 2 | $CH_3$ |
| 82 | 5-Cl | H | 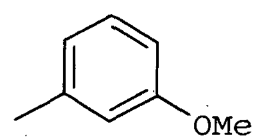 | 2 | H |

(suite)

| Ex | Ra | Rb | Ar | n | R |
|---|---|---|---|---|---|
| 83 | 5-Cl | H | 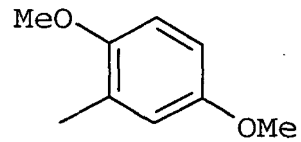 | 2 | $CH_3$ |
| 84 | 5-Cl | H | 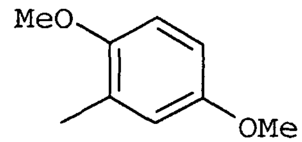 | 2 | H |
| 85 | 5-Cl | H | 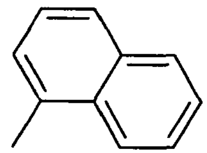 | 2 | $CH_3$ |
| 86 | 5-Cl | H | 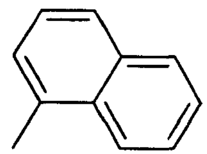 | 2 | H |
| 87 | 5-F | H | 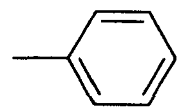 | 1 | $CH_3$ |
| 88 | 5-Cl | 6-Cl | 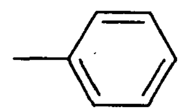 | 1 | $CH_3$ |
| 89 | 5-Cl | 6-Cl | 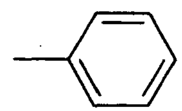 | 1 | H |

(suite)

| Ex | Ra | Rb | Ar | n | R |
|---|---|---|---|---|---|
| 90 | 4-Cl | 5-Cl | 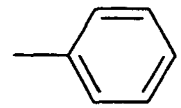 | 1 | $CH_3$ |
| 91 | 4-Cl | 5-Cl | 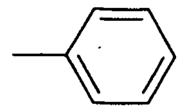 | 1 | H |
| 92 | $6-CF_3$ | H | 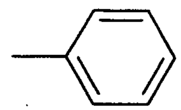 | 1 | $CH_3$ |
| 93 | $6-CF_3$ | H | 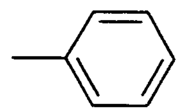 | 1 | H |
| 94 | $5-COCH_3$ | H | 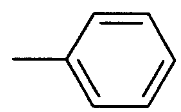 | 1 | $CH_3$ |
| 95 | $5-COCH_3$ | H | 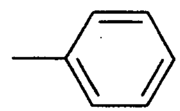 | 1 | H |
| 96 | 5-F | 6-Cl | 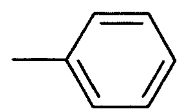 | 1 | $CH_3$ |
| 97 | 5-F | 6-Cl | 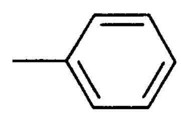 | 1 | H |

(suite)

| Ex | Ra | Rb | Ar | n | R |
|---|---|---|---|---|---|
| 98 | 5-Cl | 7-Cl | 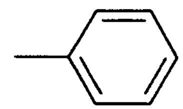 | 1 | $CH_3$ |
| 99 | 5-Cl | 7-Cl | 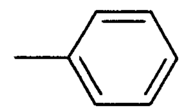 | 1 | H |
| 100 | 5-CN | H | 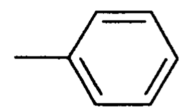 | 1 | $CH_3$ |
| 101 | 5-CN | H | 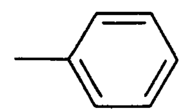 | 1 | H |
| 102 | 5-benzoyl | H | 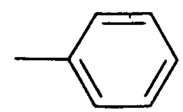 | 1 | $CH_3$ |
| 103 | 5-benzoyl | H | 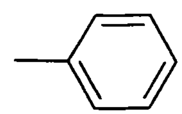 | 1 | H |
| 104 | 5-Cl | H | 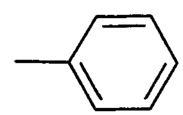 | 3 | $CH_3$ |
| 105 | 5-Cl | H | 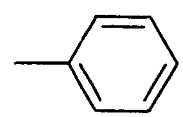 | 3 | H |

(suite)

| Ex | Ra | Rb | Ar | n | R |
|---|---|---|---|---|---|
| 106 | 5-$OCF_3$ | H | 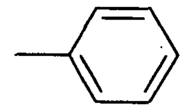 | 1 | $CH_3$ |
| 107 | 5-$OCF_3$ | H | 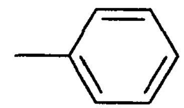 | 1 | H |
| 108 | 5-Cl | H | 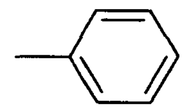 | 1 | $CH(CH_3)_2$ |
| 109 | 5-Cl | H | 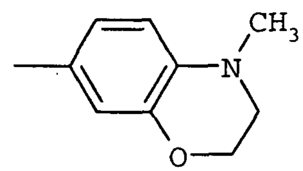 | 2 | $CH_3$ |
| 110 | 5-Cl | H | 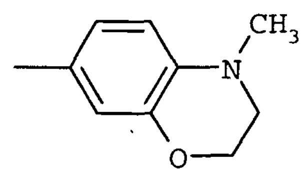 | 2 | H |
| 111 | 5-Cl | H | 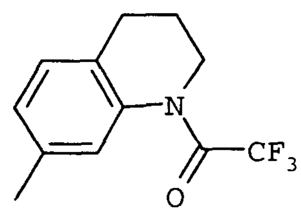 | 2 | $CH_3$ |

(suite)

| Ex | Ra | Rb | Ar | n | R |
|---|---|---|---|---|---|
| 112 | 5-Cl | H | 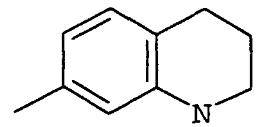 | 2 | $CH_3$ |
| 113 | 5-Cl | H | 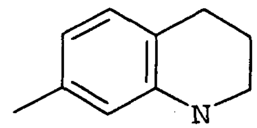 | 2 | H |
| 114 | 5-Cl | H | 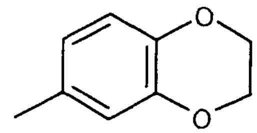 | 2 | $CH_3$ |
| 115 | 5-Cl | H | 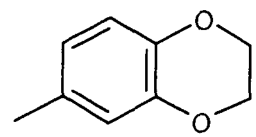 | 2 | H |
| 116 | 5-Cl | H | 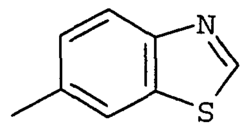 | 2 | $CH_3$ |
| 117 | 5-Cl | H | 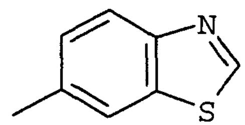 | 2 | H |
| 118 | 5-Cl | H | 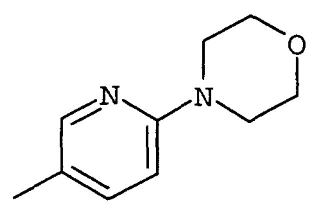 | 2 | $CH_3$ |

(suite)

| Ex | Ra | Rb | Ar | n | R |
|---|---|---|---|---|---|
| 119 | 5-Cl | H | 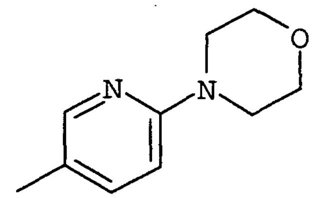 | 2 | H |
| 120 | 5-Cl | H | 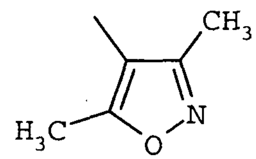 | 2 | $CH_3$ |
| 121 | 5-Cl | H | 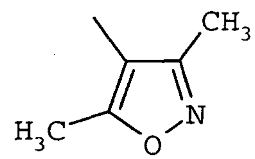 | 2 | H |
| 122 | 5-Cl | H | 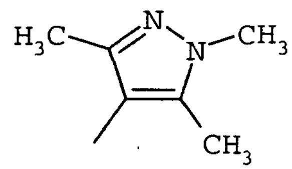 | 2 | $CH_3$ |
| 123 | 5-Cl | H | 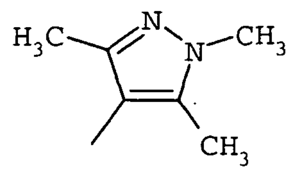 | 2 | H |
| 124 | 5-Cl | H | 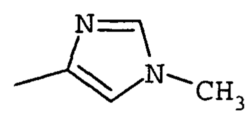 | 2 | $CH_3$ |

(suite)

| Ex | Ra | Rb | Ar | n | R |
|---|---|---|---|---|---|
| 125 | 5-Cl | H | 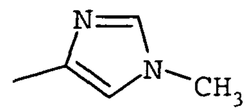 | 2 | H |
| 126 | 5-Cl | H | 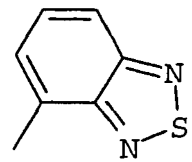 | 2 | $CH_3$ |
| 127 | 5-Cl | H | 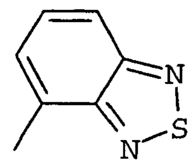 | 2 | H |
| 128 | 5-Cl | H | 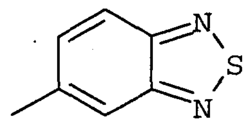 | 2 | $CH_3$ |
| 129 | 5-Cl | H | 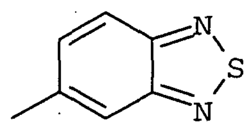 | 2 | H |
| 130 | 5-Cl | H | 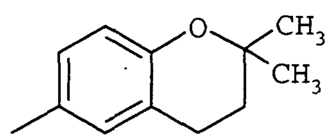 | 2 | $CH_3$ |
| 131 | 5-Cl | H | 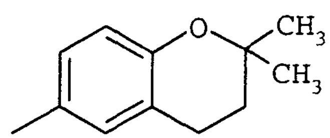 | 2 | H |

(suite)

| Ex | Ra | Rb | Ar | n | R |
|---|---|---|---|---|---|
| 132 | 5-Cl | H | 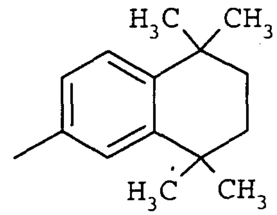 | 2 | $CH_3$ |
| 133 | 5-Cl | H | 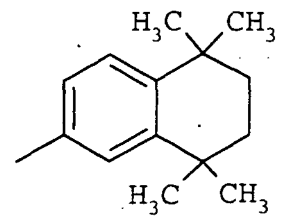 | 2 | H |
| 134 | 5-Cl | H | 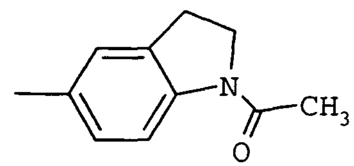 | 2 | $CH_3$ |
| 135 | 5-Cl | H | 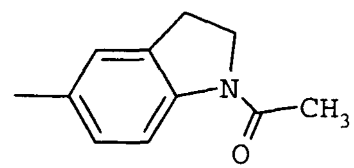 | 2 | H |
| 136 | 5-Cl | H | 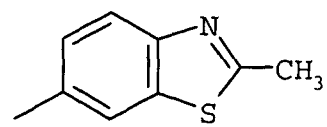 | 2 | $CH_3$ |

(suite)

| Ex | Ra | Rb | Ar | n | R |
|---|---|---|---|---|---|
| 137 | 5-Cl | H | 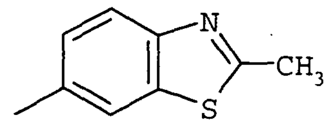 | 2 | H |
| 138 | 5-Cl | H | 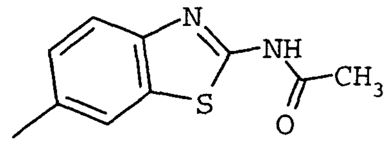 | 2 | $CH_3$ |
| 139 | 5-Cl | H | 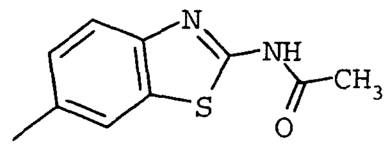 | 2 | H |
| 140 | 5-Cl | H | 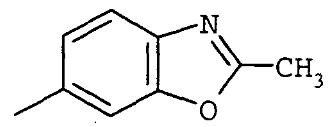 | 2 | $CH_3$ |
| 141 | 5-Cl | H | 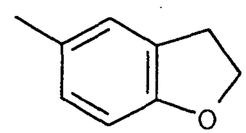 | 2 | $CH_3$ |
| 142 | 5-Cl | H | 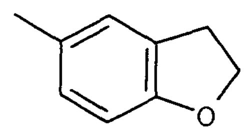 | 2 | H |
| 143 | 5-Cl | H | 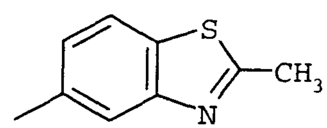 | 2 | $CH_3$ |

(suite)

| Ex | Ra | Rb | Ar | n | R |
|---|---|---|---|---|---|
| 144 | 5-Cl | H | 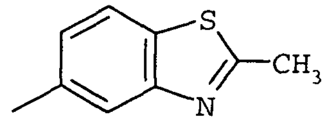 | 2 | H |
| 145 | 5-Cl | H | 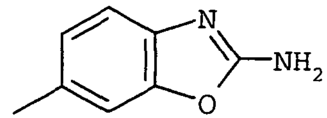 | 2 | $CH_3$ |
| 146 | 5-Cl | H | 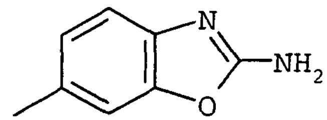 | 2 | H |
| 147 | 5-Cl | H | 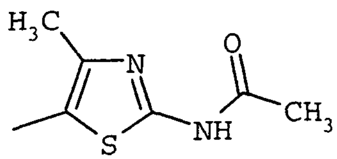 | 2 | $CH_3$ |
| 148 | 5-Cl | H | 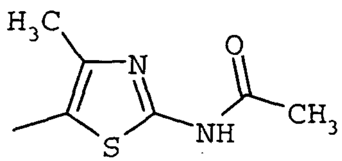 | 2 | H |
| 149 | 5-Cl | H | 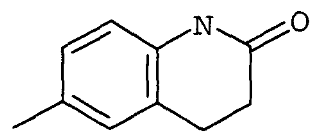 | 2 | $CH_3$ |

(suite)

| Ex | Ra | Rb | Ar | n | R |
|---|---|---|---|---|---|
| 150 | 5-Cl | H | 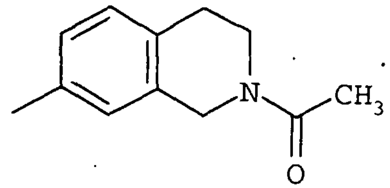 | 2 | $CH_3$ |
| 151 | 5-Cl | H | 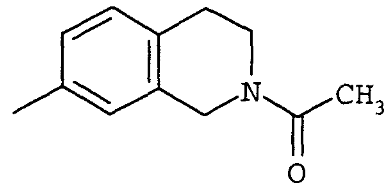 | 2 | H |
| 152 | 5-Cl | H | 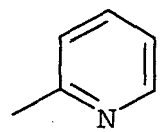 | 2 | $CH_3$ |
| 153 | 5-Cl | H | 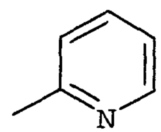 | 2 | H |
| 154 | 5-Cl | H | 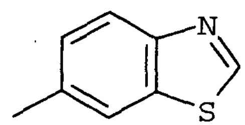 | 1 | $CH_3$ |
| 155 | 5-Cl | H | 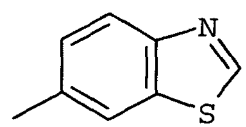 | 1 | H |

(suite)

| Ex | Ra | Rb | Ar | n | R |
|---|---|---|---|---|---|
| 156 | 5-Cl | H | 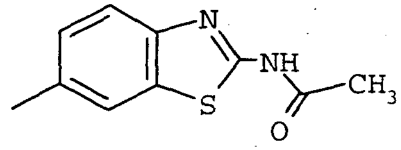 | 1 | $CH_3$ |
| 157 | 5-Cl | H | 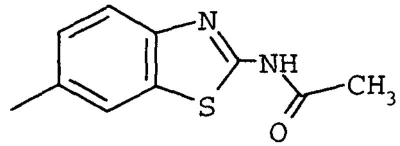 | 1 | H |
| 158 | 5-Cl | H | 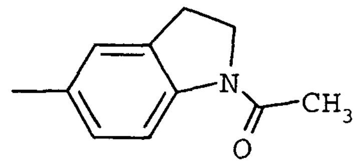 | 1 | $CH_3$ |
| 159 | 5-Cl | H | 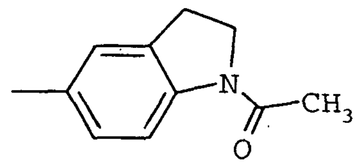 | 1 | H |
| 160 | 5-Cl | H | 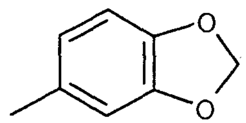 | 1 | $CH_3$ |
| 161 | 5-Cl | H | 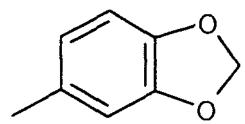 | 1 | H |

(suite)

| Ex | Ra | Rb | Ar | n | R |
|---|---|---|---|---|---|
| 162 | 5-Cl | H | 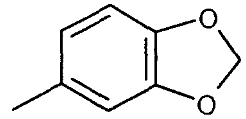 | 2 | $CH_3$ |
| 163 | 5-Cl | H | 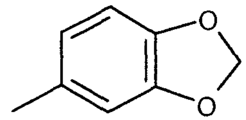 | 2 | H |
| 164 | 5-Cl | H | 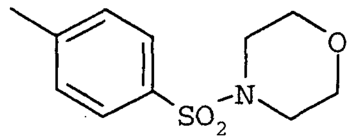 | 2 | $CH_3$ |
| 165 | 5-Cl | H | 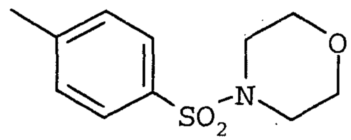 | 2 | H |
| 166 | 5-Cl | H | 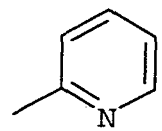 | 1 | $CH_3$ |
| 167 | 5-Cl | H | 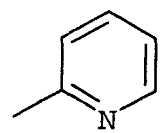 | 1 | H |
| 168 | $5-CF_3$ | H | 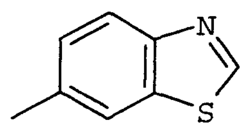 | 2 | $CH_3$ |

(suite)

| Ex | Ra | Rb | Ar | n | R |
|---|---|---|---|---|---|
| 169 | 5-$CF_3$ | H | 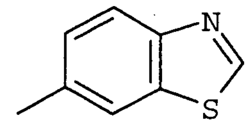 | 2 | H |
| 170 | 5-$CF_3$ | H | 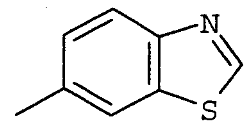 | 1 | $CH_3$ |
| 171 | 5-$CF_3$ | H | 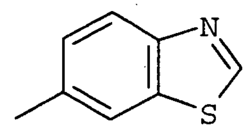 | 1 | H |
| 172 | 5-$CF_3$ | H | 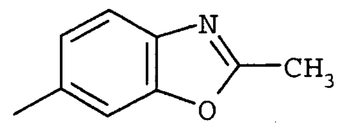 | 2 | $CH_3$ |
| 173 | 5-$CF_3$ | H | 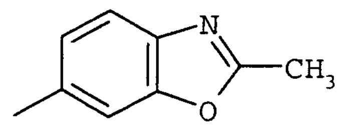 | 2 | H |
| 174 | 5-$CF_3$ | H | 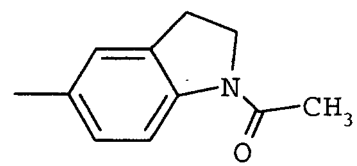 | 2 | $CH_3$ |
| 174 | 5-$CF_3$ | H | 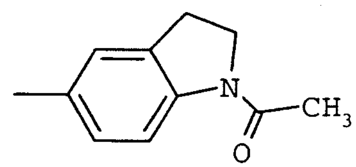 | 2 | H |

(suite)

| Ex | Ra | Rb | Ar | n | R |
|---|---|---|---|---|---|
| 176 | 5-$CF_3$ | H | 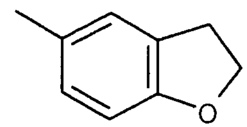 | 2 | $CH_3$ |
| 177 | 5-$CF_3$ | H | 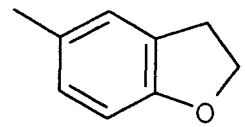 | 2 | H |
| 178 | 5-$CF_3$ | H | 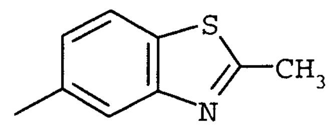 | 2 | $CH_3$ |
| 179 | 5-$CF_3$ | H | 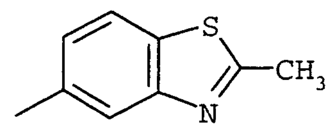 | 2 | H |
| 180 | 5-$CF_3$ | H | 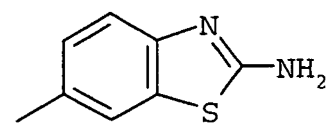 | 1 | $CH_3$ |
| 181 | 5-$CF_3$ | H | 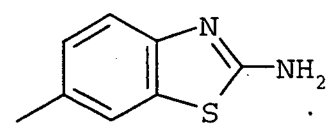 | 1 | H |
| 182 | 5-$CF_3$ | H | 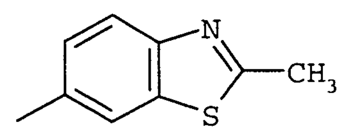 | 1 | $CH_3$ |

(suite)

| Ex | Ra | Rb | Ar | n | R |
|---|---|---|---|---|---|
| 183 | 5-$CF_3$ | H | 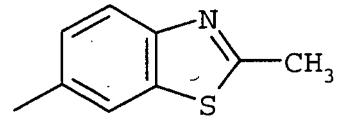 | 1 | H |
| 184 | 5-$CF_3$ | H | 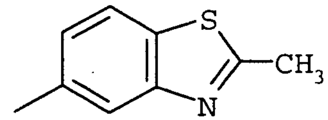 | 1 | $CH_3$ |
| 185 | 5-$CF_3$ | H | 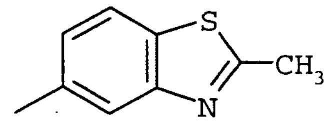 | 1 | H |
| 186 | 5-$CF_3$ | H | 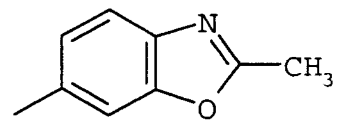 | 1 | $CH_3$ |
| 187 | 5-$CF_3$ | H | 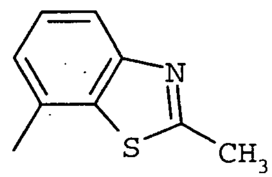 | 1 | $CH_3$ |
| 188 | 5-$CF_3$ | H | 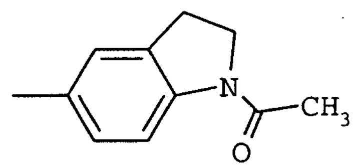 | 1 | $CH_3$ |

(suite)

| Ex | Ra | Rb | Ar | n | R |
|---|---|---|---|---|---|
| 189 | 5-$CF_3$ | H | 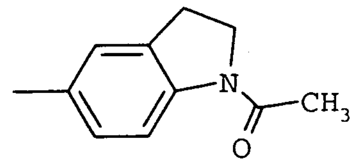 | 1 | H |
| 190 | 5-$CF_3$ | H | 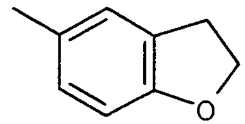 | 1 | $CH_3$ |
| 191 | 5-$CF_3$ | H | 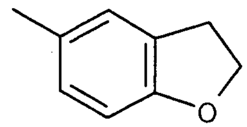 | 1 | H |
| 192 | 5-$CF_3$ | H | 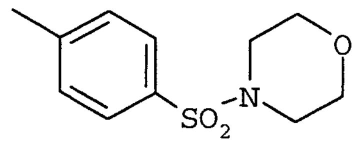 | 1 | $CH_3$ |
| 193 | 5-$CF_3$ | H | 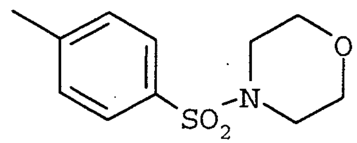 | 1 | H |
| 194 | 5-Cl | H | 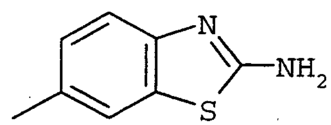 | 2 | $CH_3$ |
| 195 | 5-Cl | H | 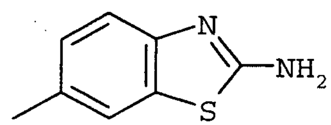 | 2 | H |

(suite)

| Ex | Ra | Rb | Ar | n | R |
|---|---|---|---|---|---|
| 196 | 5-$CF_3$ | H | 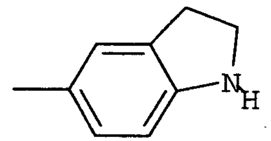 | 2 | H |
| 197 | 5-$CF_3$ | H | 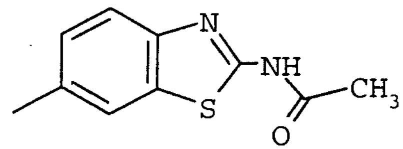 | 2 | $CH_3$ |
| 198 | 5-$CF_3$ | H | 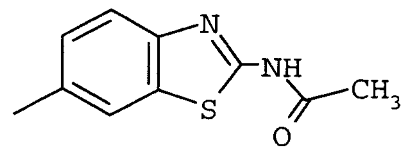 | 2 | H |
| 199 | 5-$CF_3$ | H | 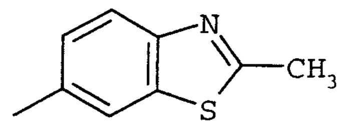 | 2 | $CH_3$ |
| 200 | 5-$CF_3$ | H | 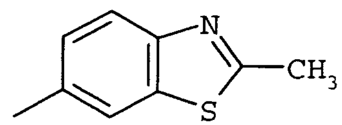 | 2 | H |
| 225 | 5-$CF_3$ | H | 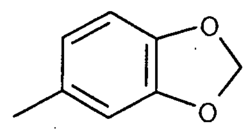 | 2 | $CH_3$ |
| 226 | 5-$CF_3$ | H | 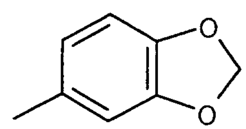 | 2 | H |

(suite)

| Ex | Ra | Rb | Ar | n | R |
|---|---|---|---|---|---|
| 227 | 5-$CF_3$ | H | 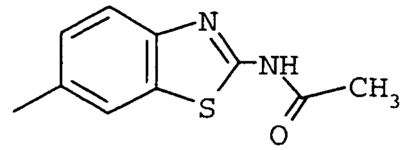 | 1 | $CH_3$ |
| 228 | 5-$CF_3$ | H | 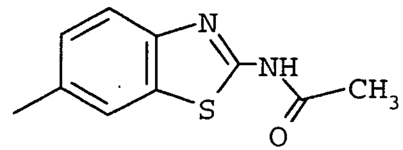 | 1 | H |
| 296 | 5-Cl | H | 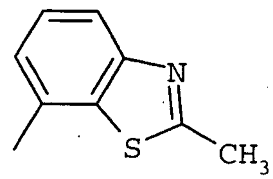 | 2 | $CH_3$ |
| 297 | 5-Cl | H | 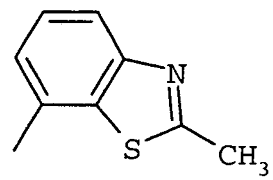 | 2 | H |
| 298 | 5-Cl | H | 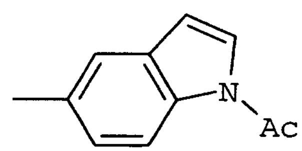 | 2 | $CH_3$ |
| 299 | 5-Cl | H | 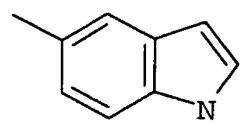 | 2 | H |

(suite)

| Ex | Ra | Rb | Ar | n | R |
|---|---|---|---|---|---|
| 300 | 5-Cl | H | 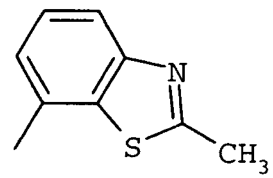 | 1 | $CH_3$ |
| 301 | 5-Cl | H | 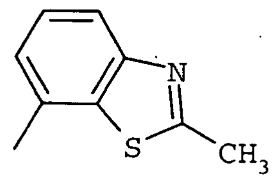 | 1 | H |
| 302 | 5-Cl | H | 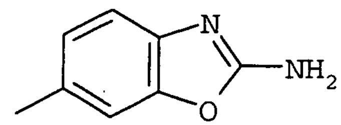 | 1 | $CH_3$ |
| 303 | 5-Cl | H | 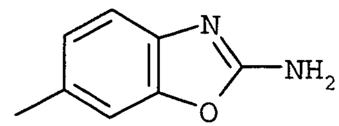 | 1 | H |
| 304 | 5-Cl | H | 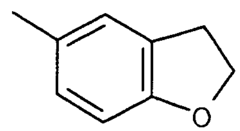 | 1 | $CH_3$ |
| 305 | 5-Cl | H | 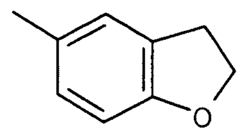 | 1 | H |

(suite)

| Ex | Ra | Rb | Ar | n | R |
|---|---|---|---|---|---|
| 306 | 5-Cl | H | 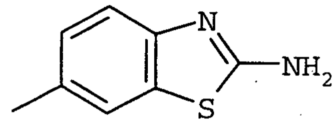 | 1 | $CH_3$ |
| 307 | 5-Cl | H | 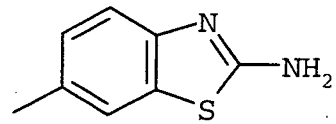 | 1 | H |
| 312 | 5-$CH_3$ | H | 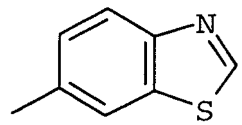 | 1 | $CH_3$ |
| 313 | 5-$CH_3$ | H | 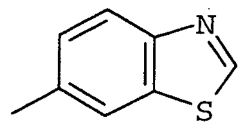 | 1 | H |
| 314 | 5-$CH_3$ | H | 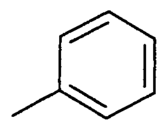 | 1 | $CH_3$ |
| 315 | 5-$CH_3$ | H | 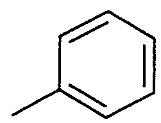 | 1 | H |
| 318 | 5-Cl | H | 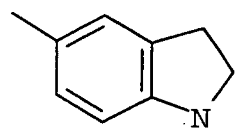 | 1 | H |

(suite)

| Ex | Ra | Rb | Ar | n | R |
|---|---|---|---|---|---|
| 319 | 5-Cl | H | 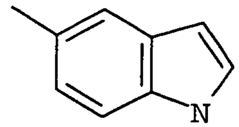 | 1 | H |
| 320 | 5-Cl | H | 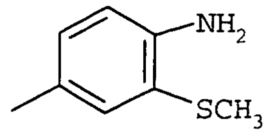 | 2 | H |
| 321 | 5-Cl | H | 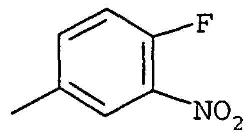 | 2 | $CH_3$ |
| 322 | 5-Cl | H | 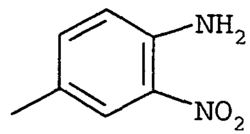 | 2 | $CH_3$ |
| 323 | 5-Cl | H | 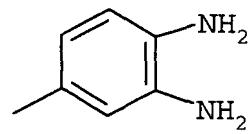 | 2 | $CH_3$ |
| 324 | 5-Cl | H | 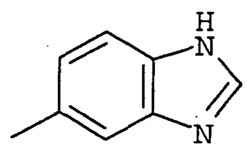 | 2 | $CH_3$ |
| 325 | 5-Cl | H | 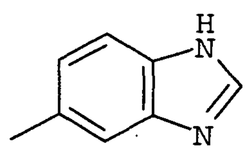 | 2 | H |

(suite)

| Ex | Ra | Rb | Ar | n | R |
|---|---|---|---|---|---|
| 326 | 5-Cl | H | 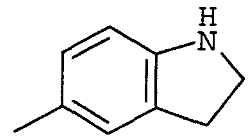 | 2 | H |

TABLEAU II

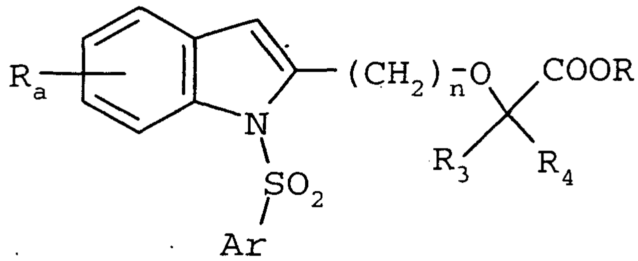

| Ex | Ra | n | $R_3$ | $R_4$ | Ar | R |
|---|---|---|---|---|---|---|
| 201 | 5-Cl | 1 | $CH_3$ | H | 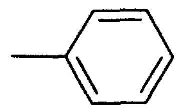 | $C_2H_5$ |
| 202 | 5-Cl | 1 | $CH_3$ | H | 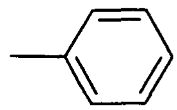 | H |
| 203 | 5-Cl | 1 | H | H | 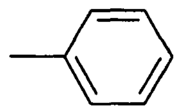 | $CH_3$ |
| 204 | 5-Cl | 1 | H | H | 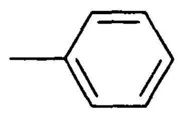 | H |
| 205 | 5-Cl | 1 | $CH_3$ | $CH_3$ | 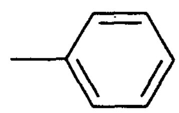 | $CH_3$ |
| 206 | 5-Cl | 1 | $CH_3$ | $CH_3$ | 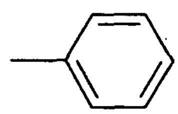 | H |
| 207 | 5-$CF_3$ | 1 | H | H | 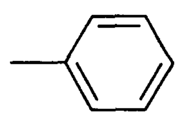 | $CH_3$ |
| 208 | 5-$CF_3$ | 1 | H | H | 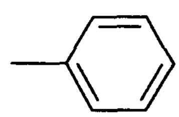 | H |
| 209 | 5-$CF_3$ | 1 | $CH_3$ | H | 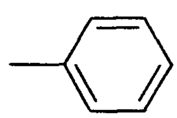 | $C_2H_5$ |
| 210 | 5-$CF_3$ | 1 | $CH_3$ | H | 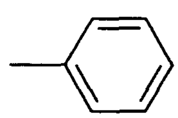 | H |

(suite)

| Ex | Ra | n | $R_3$ | $R_4$ | Ar | R |
|---|---|---|---|---|---|---|
| 211 | 5-$CF_3$ | 1 | $CH_3$ | $CH_3$ | 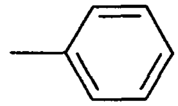 | $CH_3$ |
| 212 | 5-$CF_3$ | 1 | $CH_3$ | $CH_3$ | 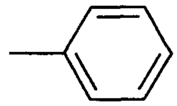 | H |
| 213 | 5-Cl | 2 | H | H | 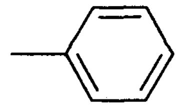 | $C_2H_5$ |
| 214 | 5-Cl | 2 | H | H | 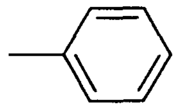 | H |
| 215 | 5-Cl | 1 | $CH_3$ | $CH_3$ | 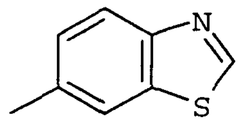 | $CH_3$ |
| 216 | 5-Cl | 1 | $CH_3$ | $CH_3$ | 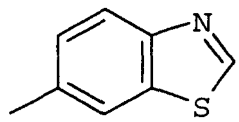 | H |
| 217 | 5-$CF_3$ | 1 | $CH_3$ | $CH_3$ | 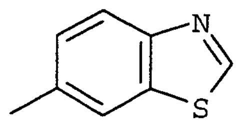 | $CH_3$ |
| 218 | 5-$CF_3$ | 1 | $CH_3$ | $CH_3$ | 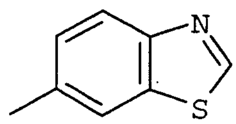 | H |
| 219 | 5-Cl | 1 | $CH_3$ | $CH_3$ | 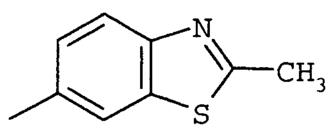 | $CH_3$ |
| 220 | 5-Cl | 1 | $CH_3$ | $CH_3$ | 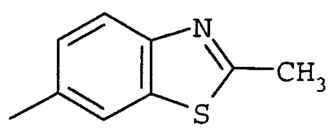 | H |
| 221 | 5-Cl | 1 | $CH_3$ | $CH_3$ | 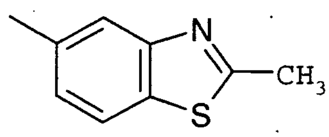 | $CH_3$ |
| 222 | 5-Cl | 1 | $CH_3$ | $CH_3$ | 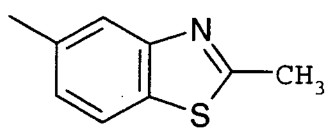 | H |

(suite)

| Ex | Ra | n | $R_3$ | $R_4$ | Ar | R |
|---|---|---|---|---|---|---|
| 223 | 5-Cl | 1 | $CH_3$ | H | 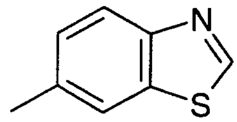 | $C_2H_5$ |
| 224 | 5-Cl | 1 | $CH_3$ | H | 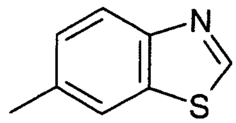 | H |
| 229 | 5-$CF_3$ | 1 | $CH_3$ | $CH_3$ | 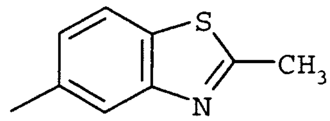 | $CH_3$ |
| 230 | 5-$CF_3$ | 1 | $CH_3$ | $CH_3$ | 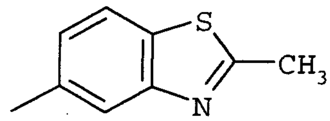 | H |
| 231 | 5-$CF_3$ | 1 | $CH_3$ | $CH_3$ | 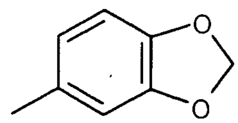 | $CH_3$ |
| 232 | 5-$CF_3$ | 1 | $CH_3$ | $CH_3$ | 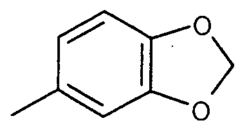 | H |
| 233 | 5-$CF_3$ | 1 | $CH_3$ | $CH_3$ | 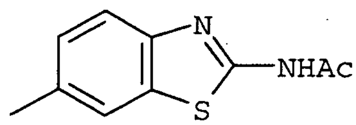 | $CH_3$ |
| 234 | 5-$CF_3$ | 1 | $CH_3$ | $CH_3$ | 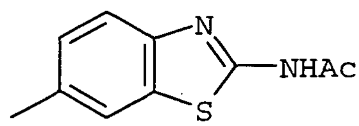 | H |
| 235 | 5-$CF_3$ | 1 | $CH_3$ | $CH_3$ | 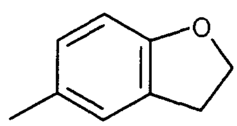 | $CH_3$ |
| 236 | 5-$CF_3$ | 1 | $CH_3$ | $CH_3$ | 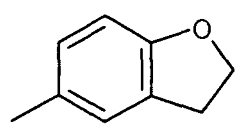 | H |
| 237 | 5-$CF_3$ | 1 | $CH_3$ | $CH_3$ | 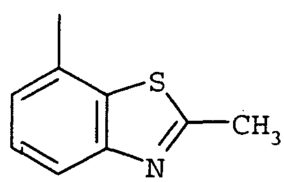 | $CH_3$ |

(suite)

| Ex | Ra | n | $R_3$ | $R_4$ | Ar | R |
|---|---|---|---|---|---|---|
| 238 | 5-$CF_3$ | 1 | $CH_3$ | $CH_3$ | 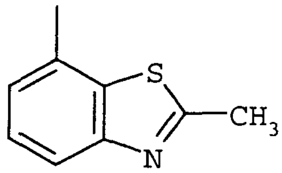 | H |
| 239 | 5-$CF_3$ | 1 | $CH_3$ | $CH_3$ | 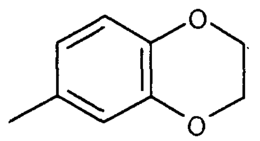 | $CH_3$ |
| 240 | 5-$CF_3$ | 1 | $CH_3$ | $CH_3$ | 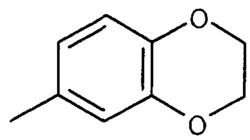 | H |
| 241 | 5-$CF_3$ | 1 | $CH_3$ | $CH_3$ | 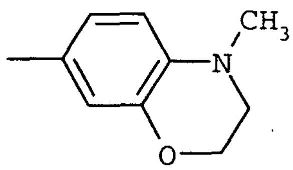 | $CH_3$ |
| 242 | 5-$CF_3$ | 1 | $CH_3$ | $CH_3$ | 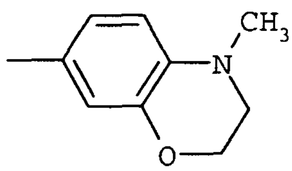 | H |
| 243 | 5-$CF_3$ | 1 | $CH_3$ | $CH_3$ | 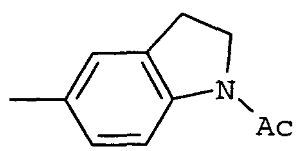 | $CH_3$ |
| 244 | 5-$CF_3$ | 1 | $CH_3$ | $CH_3$ | 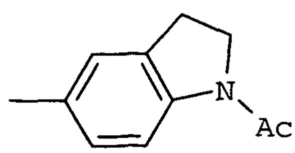 | H |
| 245 | 5-$CF_3$ | 1 | $CH_3$ | $CH_3$ | 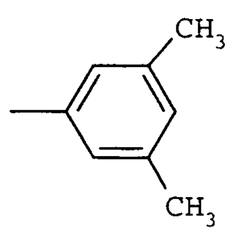 | $CH_3$ |

(suite)

| Ex | Ra | n | $R_3$ | $R_4$ | Ar | R |
|---|---|---|---|---|---|---|
| 246 | 5-$CF_3$ | 1 | $CH_3$ | $CH_3$ | 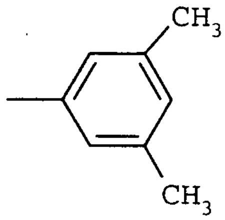 | H |
| 247 | 5-$CF_3$ | 1 | $CH_3$ | $CH_3$ | 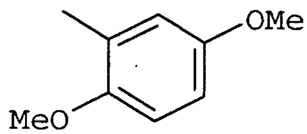 | $CH_3$ |
| 248 | 5-$CF_3$ | 1 | $CH_3$ | $CH_3$ | 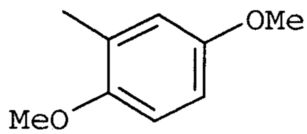 | H |
| 249 | 5-$CF_3$ | 1 | $CH_3$ | $CH_3$ | 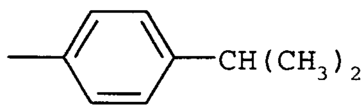 | $CH_3$ |
| 250 | 5-$CF_3$ | 1 | $CH_3$ | $CH_3$ | 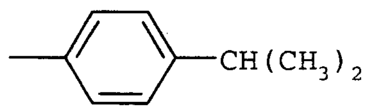 | H |
| 251 | 5-Cl | 1 | $CH_3$ | $CH_3$ | 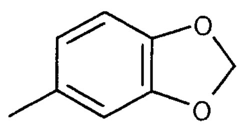 | $CH_3$ |
| 252 | 5-Cl | 1 | $CH_3$ | $CH_3$ | 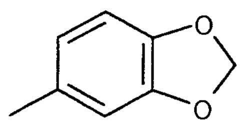 | H |
| 253 | 5-Cl | 1 | $CH_3$ | $CH_3$ | 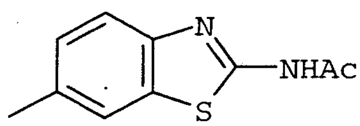 | $CH_3$ |
| 254 | 5-Cl | 1 | $CH_3$ | $CH_3$ | 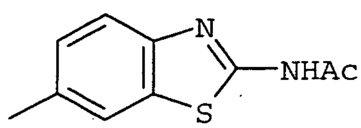 | H |
| 255 | 5-Cl | 1 | $CH_3$ | $CH_3$ | 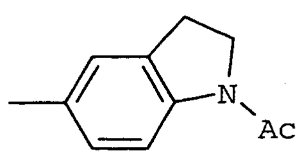 | $CH_3$ |

(suite)

| Ex | Ra | n | $R_3$ | $R_4$ | Ar | R |
|---|---|---|---|---|---|---|
| 256 | 5-Cl | 1 | $CH_3$ | $CH_3$ | 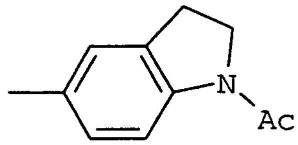 | H |
| 257 | 5-Cl | 1 | $CH_3$ | $CH_3$ | 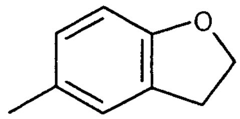 | $CH_3$ |
| 258 | 5-Cl | 1 | $CH_3$ | $CH_3$ | 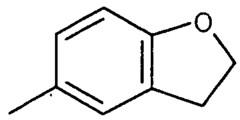 | H |
| 259 | 5-Cl | 1 | $CH_3$ | $CH_3$ | 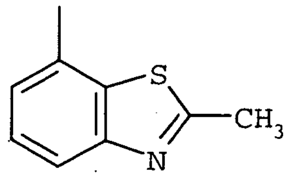 | $CH_3$ |
| 260 | 5-Cl | 1 | $CH_3$ | $CH_3$ | 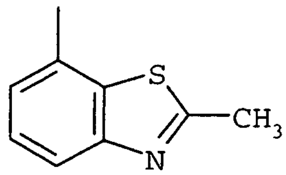 | H |
| 261 | 5-Cl | 1 | $CH_3$ | $CH_3$ | 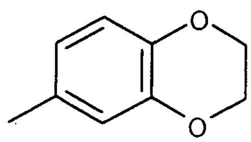 | $CH_3$ |
| 262 | 5-Cl | 1 | $CH_3$ | $CH_3$ | 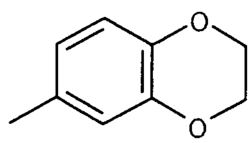 | H |
| 263 | 5-Cl | 1 | $CH_3$ | $CH_3$ | 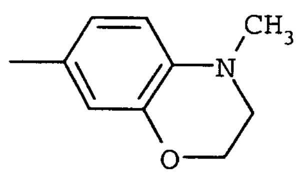 | $CH_3$ |
| 264 | 5-Cl | 1 | $CH_3$ | $CH_3$ | 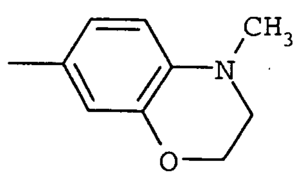 | H |
| 265 | 5-Cl | 1 | $CH_3$ | $CH_3$ | 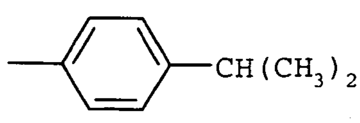 | $CH_3$ |

(suite)

| Ex | Ra | n | $R_3$ | $R_4$ | Ar | R |
|---|---|---|---|---|---|---|
| 266 | 5-Cl | 1 | $CH_3$ | $CH_3$ | 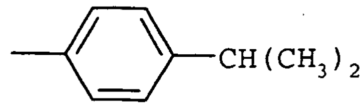 | H |
| 267 | 5-Cl | 1 | $CH_3$ | $CH_3$ | 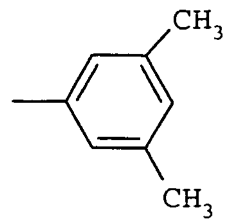 | $CH_3$ |
| 268 | 5-Cl | 1 | $CH_3$ | $CH_3$ | 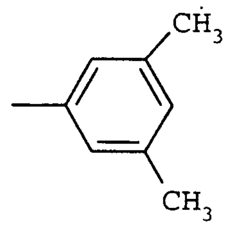 | H |
| 269 | 5-Cl | 1 | $CH_3$ | $CH_3$ | 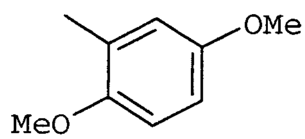 | $CH_3$ |
| 270 | 5-Cl | 1 | $CH_3$ | $CH_3$ | 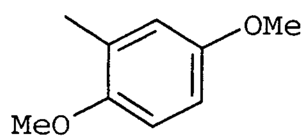 | H |
| 271 | 5-Cl | 1 | $CH_3$ | $CH_3$ | 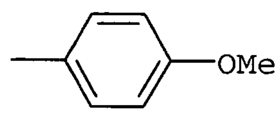 | $CH_3$ |
| 272 | 5-Cl | 1 | $CH_3$ | $CH_3$ | 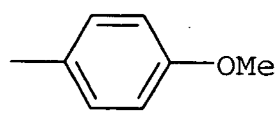 | H |
| 273 | 5-Cl | 1 | $CH_3$ | H | 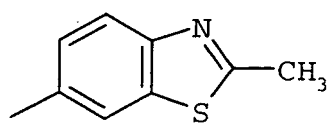 | $C_2H_5$ |
| 274 | 5-Cl | 1 | $CH_3$ | H | 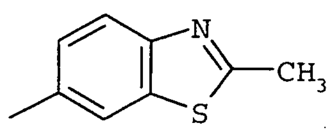 | H |
| 275 | 5-Cl | 1 | $CH_3$ | H | 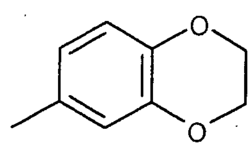 | $C_2H_5$ |

(suite)

| Ex | Ra | n | $R_3$ | $R_4$ | Ar | R |
|---|---|---|---|---|---|---|
| 276 | 5-Cl | 1 | $CH_3$ | H | 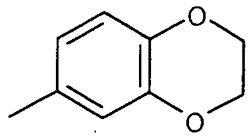 | H |
| 277 | 5-Cl | 1 | $CH_3$ | H | 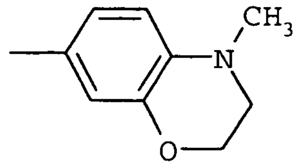 | $C_2H_5$ |
| 278 | 5-Cl | 1 | $CH_3$ | H | 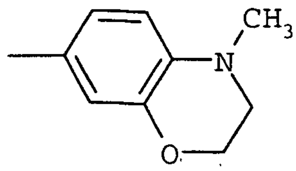 | H |
| 279 | 5-Cl | 1 | $CH_3$ | H | 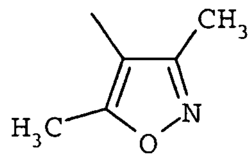 | $C_2H_5$ |
| 280 | 5-Cl | 1 | $CH_3$ | H | 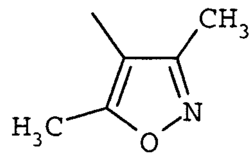 | H |
| 281 | 5-Cl | 1 | $CH_3$ | H | 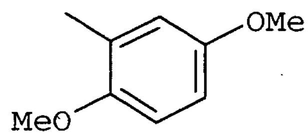 | $C_2H_5$ |
| 282 | 5-Cl | 1 | $CH_3$ | H | 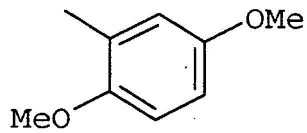 | H |
| 283 | 5-$CF_3$ | 1 | $CH_3$ | H | 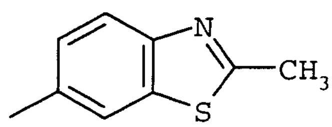 | $C_2H_5$ |
| 284 | 5-$CF_3$ | 1 | $CH_3$ | H | 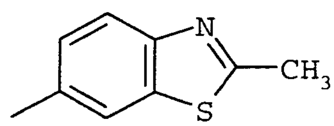 | H |
| 285 | 5-$CF_3$ | 1 | $CH_3$ | H | 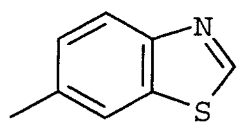 | $C_2H_5$ |

(suite)

| Ex | Ra | n | $R_3$ | $R_4$ | Ar | R |
|---|---|---|---|---|---|---|
| 286 | 5-$CF_3$ | 1 | $CH_3$ | H | 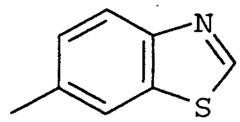 | H |
| 287 | 5-$CF_3$ | 1 | $CH_3$ | H | 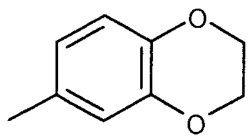 | $C_2H_5$ |
| 288 | 5-$CF_3$ | 1 | $CH_3$ | H | 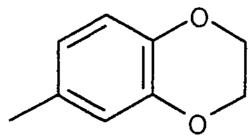 | H |
| 289 | 5-$CF_3$ | 1 | $CH_3$ | H | 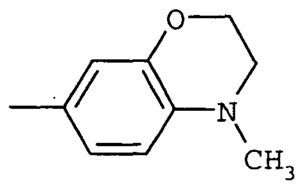 | $C_2H_5$ |
| 290 | 5-$CF_3$ | 1 | $CH_3$ | H | 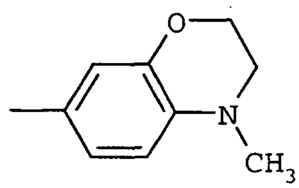 | H |
| 291 | 5-$CF_3$ 1 | 1 | $CH_3$ | H | 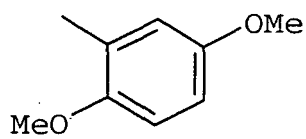 | $C_2H_5$ |
| 292 | 5-$CF_3$ | 1 | $CH_3$ | H | 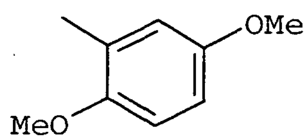 | H |
| 293 | 5-Cl | 1 | $CH_3$ (S) | H | 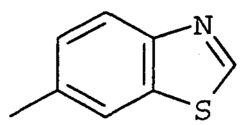 | $C_2H_5$ |
| 294 | 5-Cl | 1 | $CH_3$ (S) | H | 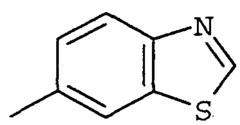 | H |
| 295 | 5-Cl | 1 | $CH_3$ (R) | H | 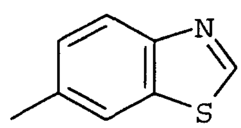 | $C_2H_5$ |

TABLEAU III

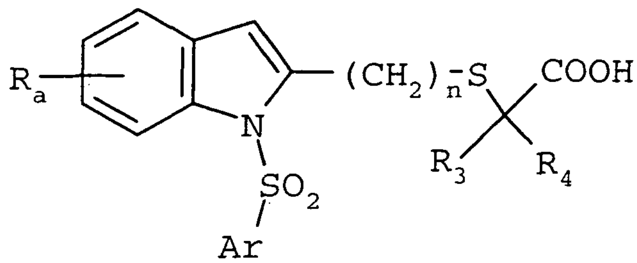

| Ex | Ra | n | $R_3$ | $R_4$ | Ar |
|---|---|---|---|---|---|
| 308 | 5-Cl | 1 | $CH_3$ | $CH_3$ | 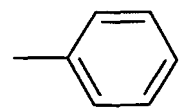 |
| 309 | 5-Cl | 1 | $CH_3$ | H | 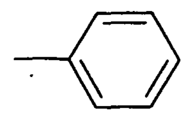 |
| 310 | 5-$CF_3$ | 1 | $CH_3$ | $CH_3$ | 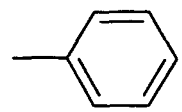 |
| 311 | 5-$CF_3$ | 1 | $CH_3$ | H | 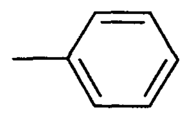 |

TABLEAU IV

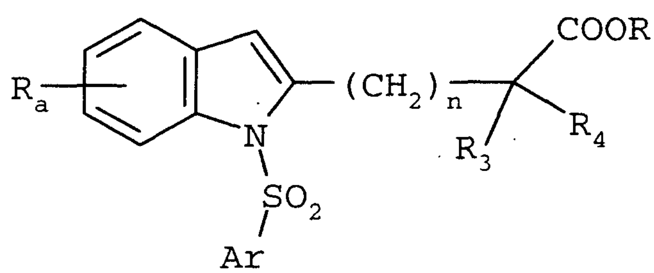

| Ex | Ra | n | $R_3$ | $R_4$ | Ar | R |
|---|---|---|---|---|---|---|
| 316 | 5-Cl | 2 | $CH_3$ | $CH_3$ | 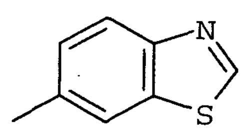 | H |
| 317 | 5-Cl | 2 | $CH_3$ | $CH_3$ | 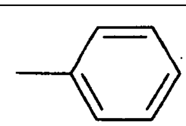 | H |

Activité pharmacologique

Les composés de l'invention ont été soumis à des tests biologiques de façon à évaluer leur potentiel à traiter ou prévenir certaines pathologies. Dans un premier temps, on a mesuré l'aptitude des composés à se comporter en activateur des récepteurs nucléaires PPAR.

Un test de transactivation est utilisé comme test de screening primaire. Des cellules Cos-7 sont transfectées avec un plasmide exprimant une chimère d'un récepteur murin ou humain PPAR-Gal4 (récepteur PPARa-Gal4 ou PPARδ-Gal4 ou PPARγ-Gal4) et d'un plasmide rapporteur 5Gal4pGL3 TK Luc. Les transfections sont réalisées à l'aide d'un agent chimique (Jet PEI).

Les cellules transfectées sont distribuées dans des plaques 384 puits et laissées au repos pendant 24 heures.

Au temps 24 heures le milieu de culture est changé. Les produits à tester sont ajoutés (concentration finale comprise entre $3.10^{-5}$ et $3.10^{-10}$ M) dans le milieu de culture. Après une nuit d'incubation, l'expression de luciférase est mesurée après addition de « SteadyGlo » selon les instructions du fabricant (Promega).

L'acide fénofibrique à $10^{-5}$ M (PPARα agoniste), le GW501516 à $10^{-8}$ M (PPARδ agoniste) et la rosiglitazone à $10^{-6}$ M (PPARγ agoniste) sont utilisés comme références.

Les résultats sont exprimés en taux d'induction (nombre de fois) comparativement au niveau basal en pourcentage d'activité de la référence adéquate (référence = 100 %). Les courbes effet-concentration et les $Eé_{50}$ sont calculées à l'aide du logiciel Assay Explorer (MDL).

A la concentration micromolaire, les composés selon l'invention présentent un taux d'induction allant jusqu'à 154 % (PPARα), 127 % (PPARδ) et 100 % (PPAR γ). Certains composés selon l'invention présentent une EC 50 inférieure à 50 nM, notamment sur le récepteur hPPARδ.

Une seconde série de tests a été pratiquée avec les composés selon l'invention, dans le but de confirmer l'activité déduite de leur affinité pour les récepteurs précédemment cités. Ce test consiste en une mesure de la β-oxydation sur cellules d'origine hépatique humaine HuH7 et cellules d'origine musculaire murine C2C12 après différenciation en myotubes.

Les cellules sont ensemencées dans des boîtes de Pétri comportant un puits central. Les produits sont ajoutés dans le milieu de culture et incubés pendant 48 heures à différentes concentrations. Après 22 heures d'incubation, de l'oléate radiomarqué au C14 (oléate 1-C14) est ajouté dans le milieu de culture. La réaction de β-oxydation est arrêtée 2 heures plus tard par addition d'acide perchlorique à 40%.

Le $CO_2$ dégagé au cours de l'oxydation de l'oléate est piégé par une solution de KOH puis compté.

Chaque essai est réalisé trois fois.

Les résultats sont exprimés en % de variation par rapport aux boîtes contrôles (boîtes sans composés).

Suivant cet essai, les composés selon l'invention augmentent la β-oxydation jusqu'à + 148% à une concentration de 10 μM sur cellules HuH7. La β-oxydation est également augmentée de 82% en présence, par exemple, du composé selon l'exemple 4 utilisé à une concentration de 100 μM lors d'un essai sur cellules C2C 12.

Certains composés selon l'invention ont été testés dans un modèle de souris db/db afin de confirmer leur potentiel de principe actif. Le protocole de l'essai est le suivant :

Des souris mâles C57BL/Ks-db homozygotes (souris db/db), âgées de 11-13 semaines à l'initiation des études, sont réparties par groupe de 9-10 animaux. Les produits sont administrés par voie orale, 1 fois par jour pendant 5 jours. Un groupe de souris reçoit le véhicule seul (solution de méthylcellulose à 0,5 ou 1%). Un prélèvement sanguin est réalisé au sinus rétro-orbitaire avant traitement et 4 heures après le dernier gavage.

Après centrifugation, le sérum est collecté et les taux de cholestérol, triglycérides et glucose sont mesurés à l'aide d'un analyseur multiparamétrique avec des kits commerciaux.

Les résultats sont exprimés en % de variation au jour final par rapport au groupe témoin.

A titre d'exemple parmi les composés selon l'invention, on obtient les résultats comparatifs suivants :

| Composé | Dose (mg/kg) | Glucose | Triglycérides | Cholestérol |
|---|---|---|---|---|
| Fénofibrate | 100 | -9 | -7 | +32 |
| Rosiglitazone | 3 | -41 | -52 | -30 |
| Ex 2 | 30 | 0 | -7 | +30 |
| Ex 4 | 30 | -30 | -12 | +41 |
| Ex 155 | 30 | -35 | -41 | +38 |
| Ex 163 | 30 | -38 | -35 | -15 |

(suite)

| Composé | Dose (mg/kg) | Glucose | Triglycérides | Cholestérol |
|---|---|---|---|---|
| Ex 202 | 30 | -52 | -48 | +25 |

Ces résultats, qui sont en accord avec les modifications attendues d'activateurs des récepteurs nucléaires PPARα et/ou PPARδ, confirment l'intérêt des composés selon l'invention pour leur utilisation en tant que principes actifs de médicaments destinés à la prévention ou au traitement des hypertriglycéridémies, des hypercholestérolémies et, d'une façon plus générale, au rétablissement de paramètres normaux lors d'une perturbation du métabolisme lipidique et glucidique. Les composés selon l'invention trouvent encore leur utilité dans le cas du traitement de la dysfonction endothéliale, de maladies inflammatoires ou de neurodégénérescences.
L'invention concerne également les compositions pharmaceutiques destinées à la prévention ou au traitement des maladies précédemment citées lorsqu'elles contiennent en tant que principe actif au moins l'un des composés de formule I selon l'invention.
Ces compositions pharmaceutiques peuvent être préparées de façon classique, à l'aide d'excipients pharmaceutiquement acceptables afin d'obtenir des formes administrables de préférence par voie orale, par exemple des comprimés ou des gélules.
De façon pratique, en cas d'administration du composé par voie orale, la posologie quotidienne chez l'homme sera de préférence comprise entre 5 et 500 mg.

**Revendications**

1. Dérivé de l'indole **caractérisé en ce qu'**il est choisi parmi :

i) les composés de formule :

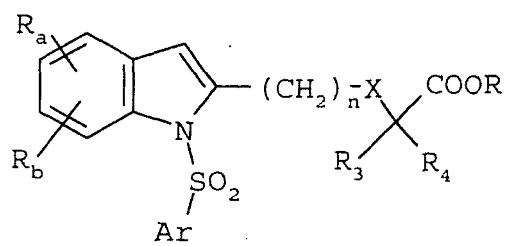

(I)

dans laquelle :

$R_a$ et $R_b$ représentent chacun indépendamment un atome d'hydrogène, un atome d'halogène, une chaîne hydrocarbonée ayant de 1 à 6 atomes de carbone linéaire, ramifiée ou cyclique, $CF_3$, CN, CO-$R_2$, $OR_2$ ou un groupe phényle éventuellement substitué par une chaîne hydrocarbonée ayant de 1 à 4 atomes de carbone linéaire, ramifiée ou cyclique ou $CF_3$ ;
$R_2$ représente une chaîne hydrocarbonée ayant de 1 à 4 atomes de carbone linéaire, ramifiée ou cyclique, $CF_3$ ou phényle éventuellement substitué par une chaîne hydrocarbonée ayant de 1 à 4 atomes de carbone linéaire, ramifiée ou cyclique ou $CF_3$ ;
$R_3$ et $R_4$ représentent chacun indépendamment un atome d'hydrogène ou une chaîne hydrocarbonée ayant de 1 à 4 atomes de carbone linéaire, ramifiée ou cyclique ;
R représente un atome d'hydrogène ou une chaîne hydrocarbonée ayant de 1 à 3 atomes de carbone linéaire, ramifiée ou cyclique;
n= 1, 2, ou 3 ;
X représente une liaison simple, un atome d'oxygène ou un atome de soufre;
Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle,

1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les chaînes hydrocarbonées ayant de 1 à 6 atomes de carbone linéaires, ramifiées ou cycliques, phényle, $CF_3$, CN, CO-$R_2$, $OR_2$, $SR_2$, NH-$COR_2$, morpholinyle, amino ou 4-morpholinosulfonyle ;
ainsi que
l'acide 5-chloro-1-[(4-fluoro-3-nitrophényl)sulfonyl]-1H-indole-2-butanoïque, méthylester
l'acide 1[(4-amino-3-nitrophényl)sulfonyl]-5-chloro-1H-indole-2-butanoïque, méthylester,

ii) leurs sels pharmaceutiquement acceptables.

2. Composé selon la revendication 1, **caractérisé en ce que** l'un au moins des substituants $R_a$ et $R_b$ n'est pas un atome d'hydrogène.

3. Composé selon l'une des revendications 1 ou 2, **caractérisé en ce que** Ar représente un groupe phényle ou hétéroaromatique azoté.

4. Composé selon l'une des revendications 1 à 3, **caractérisé en ce que** n est égal à 1 ou 2.

5. Composé selon l'une des revendications 1 à 4, pour son utilisation en tant que substance pharmacologiquement active.

6. Utilisation d'un composé selon l'une des revendications 1 à 4, pour la fabrication d'un médicament destiné à traiter les hypertriglycéridémies, les hyperlipidémies, les hypercholestérolémies, l'obésité et le diabète.

7. Utilisation d'un composé selon l'une des revendications 1 à 4, pour la fabrication d'un médicament destiné à traiter la dysfonction endothéliale.

8. Utilisation d'un composé selon l'une des revendications 1 à 4, pour la fabrication d'un médicament destiné au traitement des maladies cardiovasculaires, des maladies inflammatoires et des neurodégénérescences comme notamment la maladie d'Alzheimer ou la maladie de Parkinson.

9. Composition pharmaceutique **caractérisée en ce qu'**elle contient au moins un composé selon l'une des revendications 1 à 4 en tant que substance active.

10. Procédé de préparation d'un composé selon la revendication 1, **caractérisé en ce qu'**il comprend les étapes consistant à :

a) faire réagir selon la réaction dite de SONOGASHIRA, un composé de formule

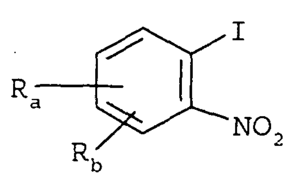

(II)

dans laquelle :

$R_a$, et $R_b$ représentent chacun indépendamment un atome d'hydrogène, de fluor, de chlore ou de brome, une chaîne hydrocarbonée ayant de 1 à 6 atomes de carbone linéaire, ramifiée ou cyclique, $CF_3$, CN, CO-$R_2$ ou $OR_2$ ;
$R_2$ représente une chaîne hydrocarbonée ayant de 1 à 4 atomes de carbone linéaire, ramifiée ou cyclique, $CF_3$ ou phényle éventuellement substitué par une chaîne hydrocarbonée ayant de 1 à 4 atomes de carbone linéaire, ramifiée ou cyclique ou $CF_3$ ;
avec un dérivé acétylénique de formule

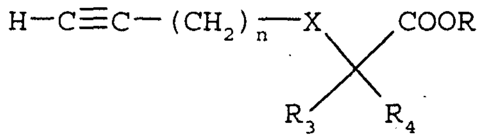

(III)

dans laquelle :

n= 1, 2, ou 3,

$R_3$ et $R_4$ représentent chacun indépendamment un atome d'hydrogène ou une chaîne hydrocarbonée ayant de 1 à 4 atomes de carbone linéaire, ramifiée ou cyclique,

R représente une chaîne hydrocarbonée ayant de 1 à 3 atomes de carbone linéaire, ramifiée ou cyclique,

X représente une liaison simple, un atome d'oxygène ou un atome de soufre,

en présence d'iodure cuivreux, d'un catalyseur à base de palladium tel que par exemple le tetrakis(triphénylphosphine) palladium et d'une base organique, dans un solvant à une température comprise entre 0 et 60 °C pendant 2 à 24 heures, pour obtenir le composé de formule

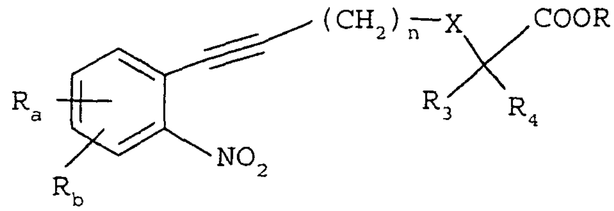

(IV)

dans laquelle :

$R_a$, $R_b$, n, X, $R_3$, $R_4$ et R conservent la même signification que dans les composés de départ ;

b) effectuer une réduction du groupe « nitro » porté par le composé de formule IV ci-dessus, par exemple par action de chlorure stanneux en présence d'éthanol et dans un solvant, à une température proche de la température ambiante et pendant 1 à 24 heures, pour obtenir l'aniline de formule

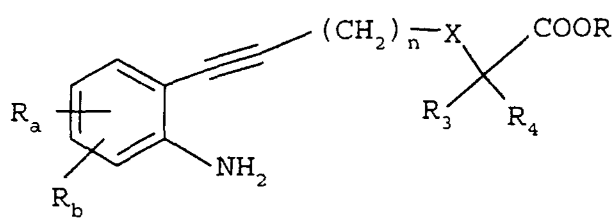

(V)

dans laquelle :

$R_a$, $R_b$, n, X, $R_3$, $R_4$ et R conservent la même signification que dans le composé de départ ;

c) faire réagir le composé de formule V avec un chlorure d'arylsulfonyle de formule

$$Ar-SO_2-Cl \quad (VI)$$

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les chaînes hydrocarbonées ayant de 1 à 6 atomes de carbone linéaires, ramifiées ou cycliques, phényle, $CF_3$, CN, CO-$R_2$, $OR_2$, $SR_2$, NH-$COR_2$, morpholinyle, amino ou 4-morpholinosulfonyle,

en présence de pyridine, à température ambiante, pendant 10 à 120 mn, pour obtenir le composé de formule

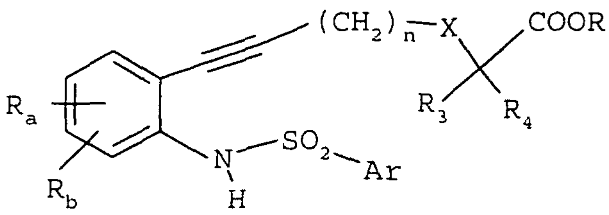

(VII)

dans laquelle :

$R_a$, $R_b$, n, X, $R_3$, $R_4$, R et Ar conservent la même signification que dans les composés de départ ;

d) effectuer une cyclisation du composé de formule VII, par exemple par action de l'acétate de cuivre II, dans un solvant à une température proche de la température de reflux du solvant, pendant 4 à 24 heures, pour obtenir le composé de formule

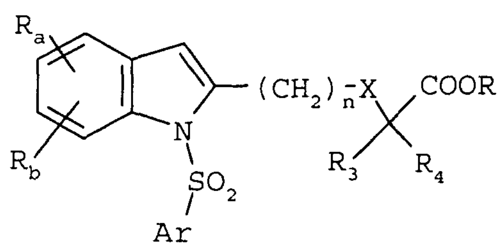

(Ia)

dans laquelle :

$R_a$, $R_b$, n, X, $R_3$, $R_4$, R et Ar conservent la même signification que dans les composés de départ ;

e) si nécessaire, hydrolyser la fonction ester du composé de formule Ia, pour obtenir, après traitement acide, le composé de formule I sous sa forme d'acide libre :

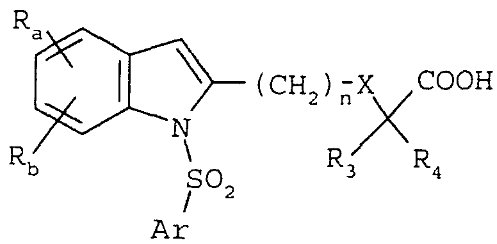

(Ib)

**11.** Procédé de préparation d'un composé selon la revendication 1, **caractérisé en ce qu'**il comprend les étapes consistant à :

a) effectuer une réaction d'halogénation, préférentiellement une iodation, d'une aniline de formule

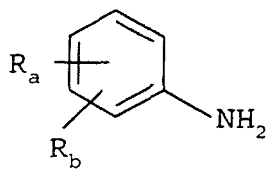

(VIII)

dans laquelle :

$R_a$ et $R_b$ représentent chacun indépendamment un atome d'hydrogène, un atome d'halogène, une chaîne hydrocarbonée ayant de 1 à 6 atomes de carbone linéaire, ramifiée ou cyclique, $CF_3$, CN, CO-$R_2$ ou $OR_2$ ;
$R_2$ représente une chaîne hydrocarbonée ayant de 1 à 4 atomes de carbone linéaire, ramifiée ou cyclique, $CF_3$ ou phényle éventuellement substitué par une chaîne hydrocarbonée ayant de 1 à 4 atomes de carbone linéaire, ramifiée ou cyclique ou $CF_3$ ;
à l'aide d'un agent halogénant tel que par exemple le dichloroiodate de benzyltriméthylammonium, dans un solvant tel que le dichlorométhane ou le méthanol, à température ambiante, pendant 5 à 24 heures pour obtenir le composé de formule

dans laquelle :

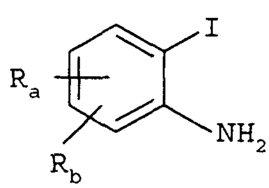

(IX)

$R_a$ et $R_b$ conservent la même signification que dans les composés de départ ;

b) faire réagir le composé de formule IX avec un dérivé acétylénique de formule

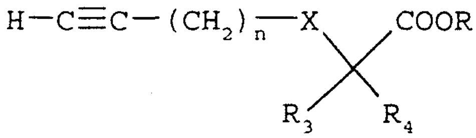

(III)

dans laquelle :

n= 1, 2, ou 3,
$R_3$ et $R_4$ représentent chacun indépendamment un atome d'hydrogène ou une chaîne hydrocarbonée ayant de 1 à 4 atomes de carbone linéaire, ramifiée ou cyclique,
R représente une chaîne hydrocarbonée ayant de 1 à 3 atomes de carbone linéaire, ramifiée ou cyclique,
X représente une liaison simple, un atome d'oxygène ou un atome de soufre,
dans des conditions analogues à celles décrites pour l'étape a) du procédé décrit à la revendication 10, pour obtenir le composé de formule

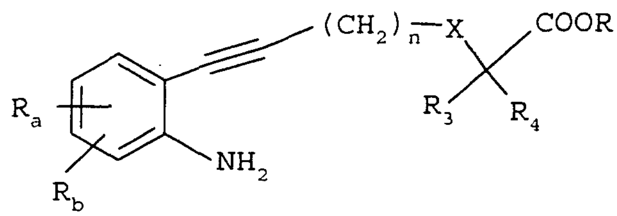

(V)

dans laquelle :

$R_a$, $R_b$, n, X, $R_3$, $R_4$ et R conservent la même signification que dans le composé de départ ;

c) cycliser le composé de formule V ci-dessus, dans des conditions analogues à celles décrites pour réaliser l'étape (d) du procédé décrit à la revendication 10, pour obtenir le composé indolique de formule

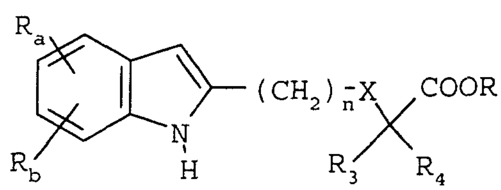

(X)

dans laquelle :

$R_a$, $R_b$, n, X, $R_3$, $R_4$ et R conservent la même signification que dans le composé de départ;

d) faire réagir le composé de formule (X) ci-dessus avec un chlorure de arylsulfonyle de formule

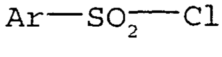

(VI)

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les chaînes hydrocarbonées ayant de 1 à 6 atomes de carbone linéaires, ramifiées ou cycliques, phényle, $CF_3$, CN, CO-$R_2$, O$R_2$, S$R_2$, NH-CO$R_2$, morpholinyle, amino ou 4-morpholinosulfonyle,

dans un solvant, à température ambiante et pendant 1 à 12 heures, généralement après activation des composés indolique de formule (X) par l'hydrure de sodium, pour obtenir le composé de formule (Ia)

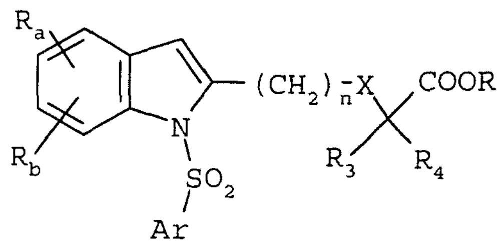
(Ia)

dans laquelle :

$R_a$, $R_b$, n, X, $R_3$, $R_4$, R et Ar conservent la même signification que dans les composés de départ ;

e) si nécessaire, hydrolyser la fonction ester du composé de formule Ia, par exemple par action d'une base minérale telle que la soude ou la lithine selon des modes opératoires bien connus de l'homme du métier, pour obtenir, après traitement acide, le composé de formule I sous sa forme d'acide libre :

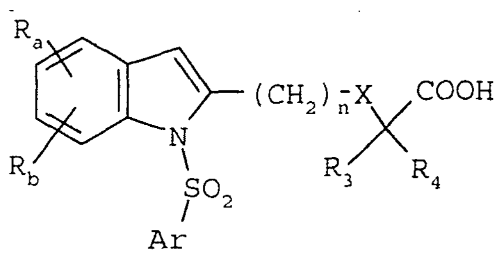
(Ib)

**12.** Procédé de préparation d'un composé selon la revendication 1, **caractérisé en ce qu'**il comprend les étapes consistant à :

a) faire réagir le composé de formule IX

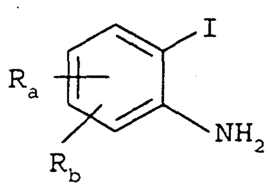
(IX)

dans laquelle :

$R_a$ et $R_b$ représentent chacun indépendamment un atome d'hydrogène, de fluor, de chlore ou de brome,

une chaîne hydrocarbonée ayant de 1 à 6 atomes de carbone linéaire, ramifiée ou cyclique, $CF_3$, CN, CO-$R_2$ ou $OR_2$ ;
$R_2$ représente une chaîne hydrocarbonée ayant de 1 à 4 atomes de carbone linéaire, ramifiée ou cyclique, $CF_3$ ou phényle éventuellement substitué par une chaîne hydrocarbonée ayant de 1 à 4 atomes de carbone linéaire, ramifiée ou cyclique ou $CF_3$ ;
avec un chlorure de arylsulfonyle de formule

$$Ar-SO_2-Cl \quad (VI)$$

dans laquelle :

Ar représente un noyau aromatique ou hétéroaromatique choisi parmi les groupes phényle, naphtyle, quinolinyle, isoquinolinyle, pyridinyle, pyrazolyle, imidazolyle, isoxazolyle, thiazolyle, benzimidazolyle, benzothiazolyle, 2,1,3-benzothiadiazolyle, 3,4-dihydro-1,4-benzoxazinyle, 5,6,7,8-tétrahydronaphtalényle, 1,2,3,4-tétrahydroquinolinyle, 1,2,3,4-tétrahydroisoquinolinyle, 1,2,3,4-tétrahydro-2-oxoquinolinyle, 3,4-dihydro-2H-benzopyranyle, indolyle, 2,3-dihydroindolyle, benzofuranyle, 2,3-dihydrobenzofuranyle, 1,3-benzodioxolyle, 1,4-benzodioxanyle ou benzoxazolyle, éventuellement substitué par un ou plusieurs des atomes ou groupe d'atomes choisis parmi les atomes d'halogène, les chaînes hydrocarbonées ayant de 1 à 6 atomes de carbone linéaires, ramifiées ou cycliques, phényle, $CF_3$, CN, CO-$R_2$, $OR_2$, $SR_2$, NH-$COR_2$, morpholinyle, amino ou 4-morpholinosulfonyle,
dans un solvant, à température ambiante et pendant 1 à 12 heures, pour obtenir le composé de formule

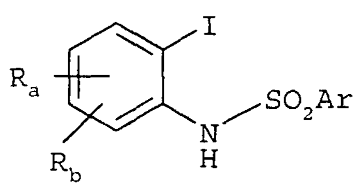

(XI)

dans laquelle :

$R_a$, $R_b$ et Ar conservent la même signification que dans les composés de départ,

b) faire réagir le composé de formule XI avec un dérivé acétylénique de

formule

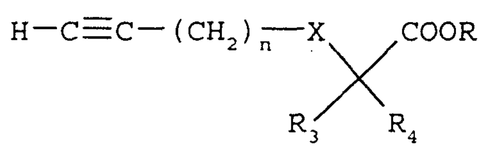

(III)

dans laquelle :

n= 1, 2, ou 3,
$R_3$ et $R_4$ représentent chacun indépendamment un atome d'hydrogène ou une chaîne hydrocarbonée ayant de 1 à 4 atomes de carbone linéaire, ramifiée ou cyclique,
R représente une chaîne hydrocarbonée ayant de 1 à 3 atomes de carbone linéaire, ramifiée ou cyclique,
X représente une liaison simple, un atome d'oxygène ou un atome de soufre,
dans des conditions analogues à celles décrites pour l'étape a) du procédé décrit à la revendication 10, pour obtenir le composé de formule

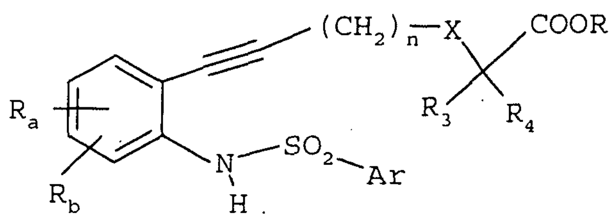

(VII)

dans laquelle :

$R_a$, $R_b$, n, X, $R_3$, $R_4$, R et Ar conservent la même signification que dans les composés de départ,

c) cycliser le composé de formule VII ci-dessus, dans des conditions analogues à celles décrites pour réaliser l'étape (d) du procédé décrit à la revendication 10, pour obtenir le composé indolique de formule

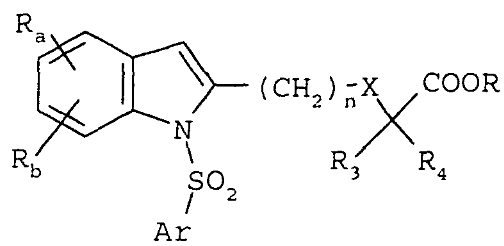

(Ia)

dans laquelle :

$R_a$, $R_b$, n, X, $R_3$, $R_4$, R et Ar conservent la même signification que dans les composés de départ,

d) si nécessaire, hydrolyser la fonction ester du composé de formule Ia, par exemple par action d'une base minérale telle que la soude ou la lithine selon des modes opératoires bien connus de l'homme du métier, pour obtenir, après traitement acide, le composé de formule I sous sa forme d'acide libre :

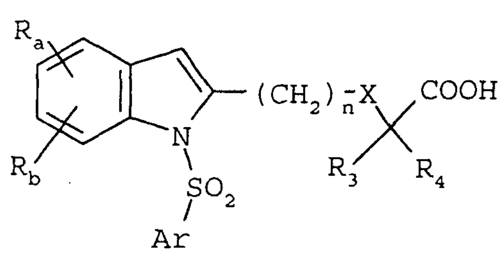

**13.** Procédé de préparation selon la revendication 12, d'un composé selon la revendication 1, **caractérisé en ce que**

les deux étapes b) et c) sont réalisées en une seule opération.

## Claims

1. Insole derivative, **characterized in that** it is selected from:

i) the compounds of the formula

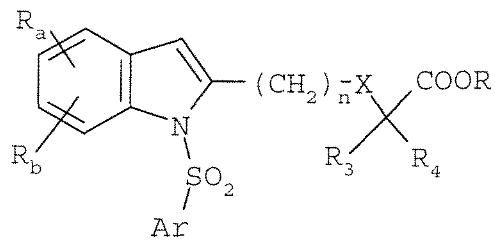

(I)

in which:

$R_a$ and $R_b$ independently are each a hydrogen atom, a halogen atom, a linear, branched or cyclic hydrocarbon chain having 1 to 6 carbon atoms, $CF_3$, CN, CO-$R_2$ or $OR_2$ group or a phenyl group optionally substituted by a linear, branched or cyclic hydrocarbon chain having 1 to 4 carbon atoms or $CF_3$ group;
$R_2$ is a linear, branched or cyclic hydrocarbon chain having 1 to 4 carbon atoms or $CF_3$ group or a phenyl group optionally substituted by a linear, branched or cyclic hydrocarbon chain having 1 to 4 carbon atoms or $CF_3$ group;
$R_3$ and $R_4$ independently are each a hydrogen atom or a linear, branched or cyclic hydrocarbon chain having 1 to 4 carbon atoms;
R is a hydrogen atom or a linear, branched or cyclic hydrocarbon chain having 1 to 3 carbon atoms;
n = 1, 2 or 3;
X is a single bond, an oxygen atom or a sulfur atom; and
Ar is an aromatic or heteroaromatic ring selected from phenyl, naphthyl, quinolinyl, isoquinolinyl, pyridinyl, pyrazolyl, imidazolyl, isoxazolyl, thiazolyl, benzimidazolyl, benzothiazolyl, 2,1,3-benzothiadiazolyl, 3,4-dihydro-1,4-benzoxazinyl, 5,6,7,8-tetrahydronaphthalenyl, 1,2,3,4-tetrahydroquinolinyl, 1,2,3,4-tetrahydroisoquinolinyl, 1,2,3,4-tetrahydro-2-oxoquinolinyl, 3,4-dihydro-2H-benzopyranyl, indolyl, 2,3-dihydroindolyl, benzofuranyl, 2,3-dihydrobenzofuranyl, 1,3-benzodioxolyl, 1,4-benzodioxanyl and benzoxazolyl groups optionally substituted by one or more atoms or groups of atoms selected from halogen atoms, linear, branched or cyclic hydrocarbon chains having 1 to 6 carbon atoms, phenyl, $CF_3$, CN, CO-$R_2$, $OR_2$, $SR_2$, NH-$COR_2$, morpholinyl, amino and 4-morpholinosulfonyl groups;
as well as
5-chloro-1-[(4-fluoro-3-nitrophenyl)sulfonyl]-1H-indole-2-butanoic acid, methyl ester
1[(4-amino-3-nitrophenyl)sulfonyl]-5-chloro-1H-indole-2-butanoic acid, methyl ester,

ii) their pharmaceutically acceptable salts.

2. Compound according to claim 1, **characterized in that** at least one of the substituents $R_a$ and $R_b$ is not a hydrogen atom.

3. Compound according to claim 1 or 2, **characterized in that** Ar is a phenyl or nitrogen-containing heteroaromatic group.

4. Compound according to one of claims 1 to 3, **characterized in that** n is equal to 1 or 2.

5. Compound according to one of claims 1 to 4 for use as a pharmacologically active substance.

6. Use of a compound according to one of claims 1 to 4 for the manufacture of a drug intended for the treatment of

hypertriglyceridemia, hyperlipidemia, hyper-cholesterolemia, obesity and diabetes.

**7.** Use of a compound according to one of claims 1 to 4 for the manufacture of a drug intended for the treatment of endothelial dysfunction.

**8.** Use of a compound according to one of claims 1 to 4 for the manufacture of a drug intended for the treatment of cardiovascular disease, inflammatory disease and neurodegeneration such as, in particular, Alzheimer's disease or Parkinson's disease.

**9.** Pharmaceutical composition, **characterized in that** it contains at least one compound according to one of claims 1 to 4 as an active substance.

**10.** Process for the preparation of a compound according to claim 1, **characterized in that** it comprises the steps consisting in

a) using the SONOGASHIRA reaction to react a compound of the formula

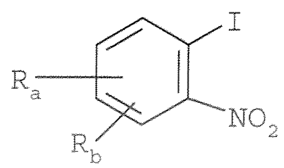

(II)

in which:

$R_a$ and $R_b$ independently are each a hydrogen, fluorine, chlorine or bromine atom or a linear, branched or cyclic hydrocarbon chain having 1 to 6 carbon atoms, $CF_3$, CN, CO-$R_2$ or $OR_2$ group; and
$R_2$ is a linear, branched or cyclic hydrocarbon chain having 1 to 4 carbon atoms or $CF_3$ group or a phenyl group optionally substituted by a linear, branched or cyclic hydrocarbon chain having I to 4 carbon atoms or $CF_3$ group,
with an acetylenic derivative of the formula

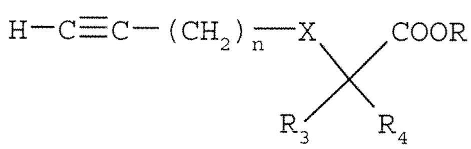

(III)

in which:

n = 1, 2 or 3;
$R_3$ and $R_4$ independently are each a hydrogen atom or a linear, branched or cyclic hydrocarbon chain having 1 to 4 carbon atoms;
R is a linear, branched or cyclic hydrocarbon chain having I to 3 carbon atoms; and
X is a single bond, an oxygen atom or a sulfur atom,
in the presence of cuprous iodide, a palladium-based catalyst, e.g. tetrakis-(triphenylphosphine)palladium, and an organic base, in a solvent, at a temperature between 0 and 60˚C, for 2 to 24 hours, to give the compound of the formula

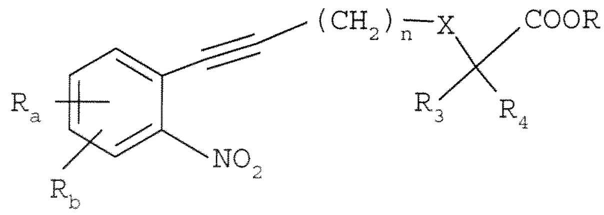

(IV)

in which:

$R_a$, $R_b$, n, X, $R_3$, $R_4$ and R are as defined in the starting compounds;

b) reducing the "nitro" group carried by the compound of formula IV above, e.g. by reaction with stannous chloride in the presence of ethanol, in a solvent, at a temperature close to room temperature, for 1 to 24 hours, to give the aniline of the formula

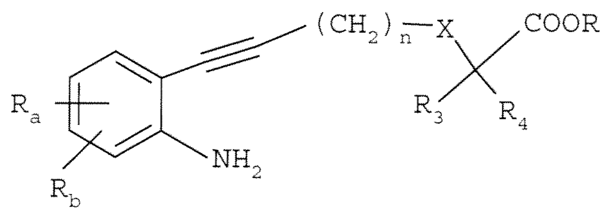

(V)

in which:

$R_a$, $R_b$, n, X, $R_3$, $R_4$ and R are as defined in the starting compound;

c) reacting the compound of formula V with an arylsulfonyl chloride of the formula

$Ar-SO_2-Cl$ (VI)

in which:

Ar is an aromatic or heteroaromatic ring selected from phenyl, naphthyl, quinolinyl, isoquinolinyl, pyridinyl, pyrazolyl, imidazolyl, isoxazolyl, thiazolyl, benzimidazolyl, benzothiazolyl, 2,1,3-benzothiadiazolyl, 3,4-dihydro-1,4-benzoxazinyl, 5,6,7,8-tetrahydronaphthalenyl, 1,2,3,4-tetrahydroquinolinyl, 1,2,3,4-tetrahydroisoquinolinyl, 1,2,3,4-tetrahydro-2-oxoquinolinyl, 3,4-dihydro-2H-benzopyranyl, indolyl, 2,3-dihydroindolyl, benzofuranyl, 2,3-dihydrobenzofuranyl, 1,3-benzodioxolyl, 1,4-benzodioxanyl and benzoxazolyl groups optionally substituted by one or more atoms or groups of atoms selected from halogen atoms, linear, branched or cyclic hydrocarbon chains having 1 to 6 carbon atoms, phenyl, $CF_3$, CN, CO-$R_2$, $OR_2$, $SR_2$, NH-$COR_2$, morpholinyl, amino and 4-morpholinosulfonyl groups,
in the presence of pyridine, at room temperature, for 10 to 120 min, to give the compound of the formula

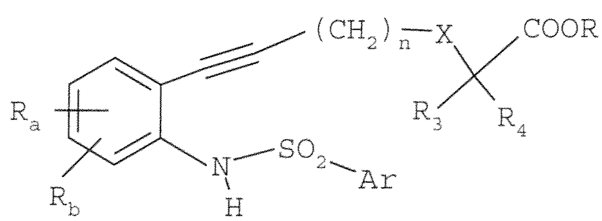

(VII)

in which:

$R_a$, $R_b$, n, X, $R_3$, $R_4$, R and Ar are as defined in the starting compounds;

d) cyclizing the compound of formula VII, e.g. by reaction with copper(II) acetate in a solvent at a temperature close to the reflux temperature of the solvent, for 4 to 24 hours, to give the compound of the formula

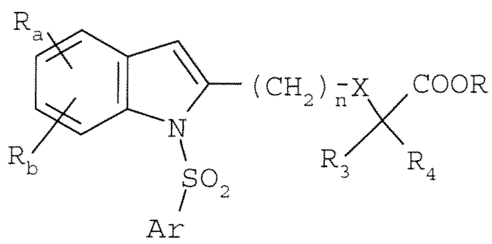

(Ia)

in which:

$R_a$, $R_b$, n, X, $R_3$, $R_4$, R and Ar are as defined in the starting compounds; and

e) if necessary, hydrolyzing the ester group of the compound of formula Ia, and then treating the product with acid to give the compound of formula I in its free acid form:

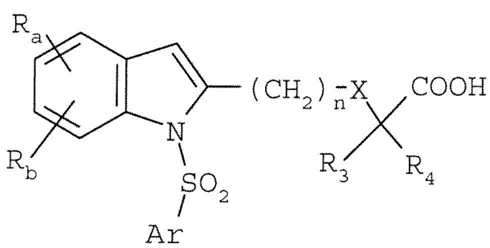

(Ib)

**11.** Process for the preparation of a compound according to claim 1, **characterized in that** it comprises the steps consisting in

a) carrying out a halogenation reaction, preferably an iodination, on an aniline of the formula

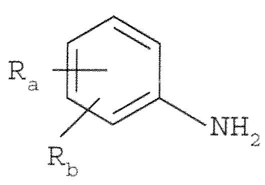

(VIII)

in which:

$R_a$ and $R_b$ independently are each a hydrogen atom, a halogen atom or a linear, branched or cyclic hydrocarbon chain having 1 to 6 carbon atoms, $CF_3$, CN, CO-$R_2$ or $OR_2$ group, and
$R_2$ is a linear, branched or cyclic hydrocarbon chain having I to 4 carbon atoms or $CF_3$ group or a phenyl group optionally substituted by a linear, branched or cyclic hydrocarbon chain having 1 to 4 carbon atoms or $CF_3$ group, with the aid of a halogenating agent, e.g. benzyltrimethylammonium dichloroiodate, in a

solvent such as dichloromethane or methanol, at room temperature, for 5 to 24 hours, to give the compound of the formula

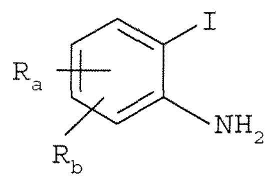

(IX)

in which:

$R_a$ and $R_b$ are as defined in the starting compounds;

b) reacting the compound of formula IX with an acetylenic derivative of the formula

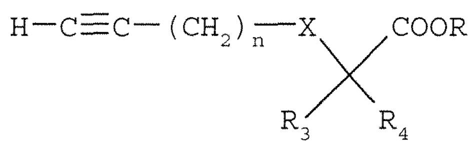

(III)

in which:

n = 1, 2 or 3;
$R_3$ and $R_4$ independently are each a hydrogen atom or a linear, branched or cyclic hydrocarbon chain having 1 to 4 carbon atoms;
R a linear, branched or cyclic hydrocarbon chain having 1 to 3 carbon atoms; and
X is a single bond, an oxygen atom or a sulfur atom,
under conditions analogous to those described for step a) of the process described in claim 10,
to give the compound of the formula

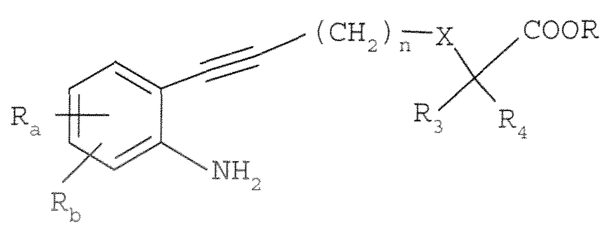

(V)

in which:

$R_a$, $R_b$, n, X, $R_3$, $R_4$ and R are as defined in the starting compound;

c) cyclizing the compound of formula V above, under conditions analogous to those described for carrying out step (d) of the process described in claim 10, to give the indole compound of the formula

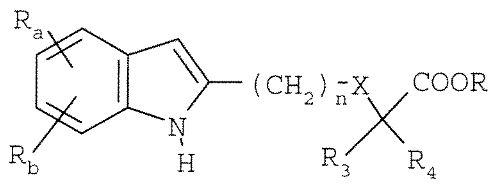

(X)

in which:

$R_a$, $R_b$, n, X, $R_3$, $R_4$ and R are as defined in the starting compound;

d) reacting the compound of formula (X) above with an arylsulfonyl chloride of the formula

$Ar\text{-}SO_2\text{-}Cl$ (VI)

in which:

Ar is an aromatic or heteroaromatic ring selected from phenyl, naphthyl, quinolinyl, isoquinolinyl, pyridinyl, pyrazolyl, imidazolyl, isoxazolyl, thiazolyl, benzimidazolyl, benzothiazolyl, 2,1,3-benzothiadiazolyl, 3,4-dihydro-1,4-benzoxazinyl, 5,6,7,8-tetrahydronaphthalenyl, 1,2,3,4-tetrahydroquinolinyl, 1,2,3,4-tetrahydroisoquinolinyl, 1,2,3,4-tetrahydro-2-oxoquinolinyl, 3,4-dihydro-2H-benzopyranyl, indolyl, 2,3-dihydroindolyl, benzofuranyl, 2,3-dihydrobenzofuranyl, 1,3-benzodioxolyl, 1,4-benzodioxanyl and benzoxazolyl groups optionally substituted by one or more atoms or groups of atoms selected from halogen atoms, linear, branched or cyclic hydrocarbon chains having 1 to 6 carbon atoms, phenyl, $CF_3$, CN, $CO\text{-}R_2$, $OR_2$, $SR_2$, $NH\text{-}COR_2$, morpholinyl, amino and 4-morpholinosulfonyl groups,
in a solvent, at room temperature, for 1 to 12 hours, generally after activation of the indole compounds of formula (X) with sodium hydride, to give the compound of formula (Ia):

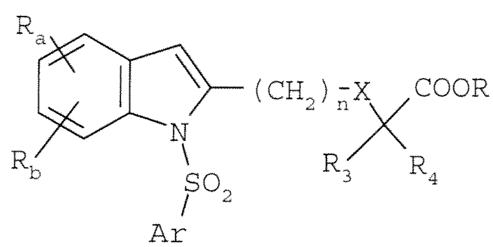

(Ia)

in which:

$R_a$, $R_b$, n, X, $R_3$, $R_4$, R and Ar are as defined in the starting compounds; and

e) if necessary, hydrolyzing the ester group of the compound of formula Ia, e.g. by reaction with a mineral base such as sodium hydroxide or lithium hydroxide, according to procedures well known to those skilled in the art, and then treating the product with acid to give the compound of formula I in its free acid form:

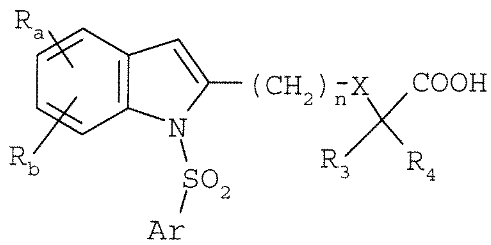

(Ib)

**12.** Process for the preparation of a compound according to claim 1, **characterized in that** it comprises the steps consisting in

a) reacting the compound of formula IX:

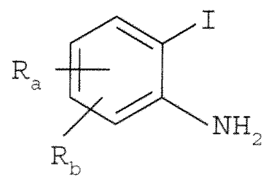

(IX)

in which:

$R_a$ and $R_b$ independently are each a hydrogen, fluorine, chlorine or bromine atom or a linear, branched or cyclic hydrocarbon chain having 1 to 6 carbon atoms, $CF_3$, CN, CO-$R_2$ or $OR_2$ group; and
$R_2$ is a linear, branched or cyclic hydrocarbon chain having 1 to 4 carbon atoms or $CF_3$ group or a phenyl group optionally substituted by a linear, branched or cyclic hydrocarbon chain having 1 to 4 carbon atoms or $CF_3$ group,
with an arylsulfonyl chloride of the formula

$Ar\text{-}SO_2\text{-}Cl$ (VI)

in which:

Ar is an aromatic or heteroaromatic ring selected from phenyl, naphthyl, quinolinyl, isoquinolinyl, pyridinyl, pyrazolyl, imidazolyl, isoxazolyl, thiazolyl, benzimidazolyl, benzothiazolyl, 2,1,3-benzothiadiazolyl, 3,4-dihydro-1,4-benzoxazinyl, 5,6,7,8-tetrahydronaphthalenyl, 1,2,3,4-tetrahydroquinolinyl, 1,2,3,4-tetrahydroisoquinolinyl, 1,2,3,4-tetrahydro-2-oxoquinolinyl, 3,4-dihydro-2H-benzopyranyl, indolyl, 2,3-dihydroindolyl, benzofuranyl, 2,3-dihydrobenzofuranyl, 1,3-benzodioxolyl, 1,4-benzodioxanyl and benzoxazolyl groups optionally substituted by one or more atoms or groups of atoms selected from halogen atoms, linear, branched or cyclic hydrocarbon chains having 1 to 6 carbon atoms, phenyl, $CF_3$, CN, CO-$R_2$, $OR_2$, $SR_2$, NH-$COR_2$, morpholinyl, amino and 4-morpholinosulfonyl groups, in a solvent, at room temperature, for 1 to 12 hours, to give the compound of the formula

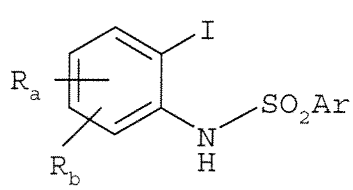

(XI)

in which:

$R_a$, $R_b$ and Ar are as defined in the starting compounds;

b) reacting the compound of formula XI with an acetylenic derivative of the formula

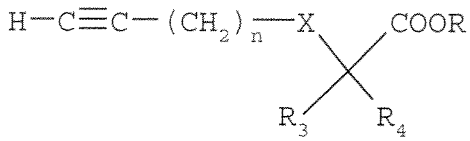

(III)

in which:

n = 1, 2 or 3;
$R_3$ and $R_4$ independently are each a hydrogen atom or a linear, branched or cyclic hydrocarbon chain having I to 4 carbon atoms;
R is a linear, branched or cyclic hydrocarbon chain having 1 to 3 carbon atoms; and
X is a single bond, an oxygen atom or a sulfur atom,
under conditions analogous to those described for step a) of the process described in claim 10, to give the compound of the formula

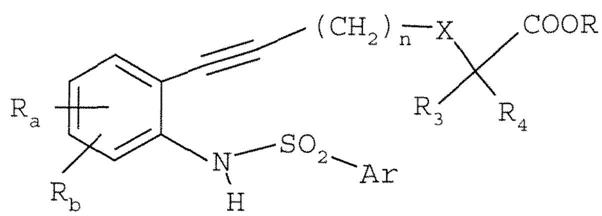

(VII)

in which:

$R_a$, $R_b$, n, X, $R_3$, $R_4$, R and Ar are as defined in the starting compounds;

c) cyclizing the compound of formula VII above, under conditions analogous to those described for carrying out step (d) of the process described in claim 10, to give the indole compound of the formula

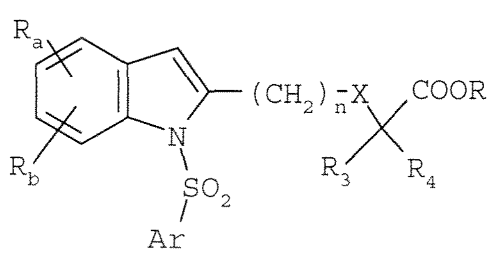

(Ia)

in which:

$R_a$, $R_b$, n, X, $R_3$, $R_4$, R and Ar are as defined in the starting compounds; and

d) if necessary, hydrolyzing the ester group of the compound of formula Ia, e.g. by reaction with a mineral base

such as sodium hydroxide or lithium hydroxide, by procedures well known to those skilled in the art, and then treating the product with acid to give the compound of formula I in its free acid form:

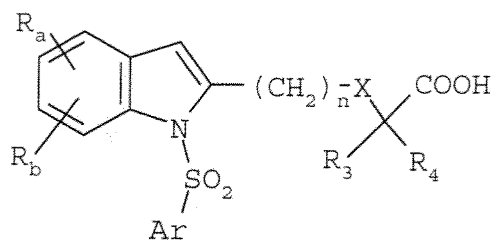

**13.** Process according to claim 12 for the preparation of a compound according to claim 1, **characterized in that** the two steps b) and c) are carried out in a single operation.

**Patentansprüche**

**1.** Indolderivat, **dadurch gekennzeichnet, daß** es aus folgendem ausgewählt ist:

i) den Verbindungen der Formel:

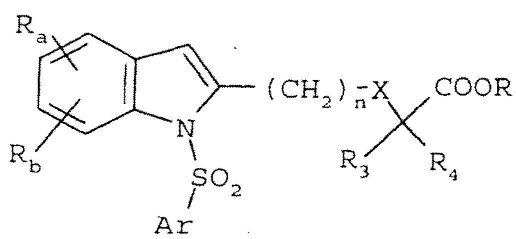

(I)

worin:

$R_a$ und $R_b$ jeweils unabhängig ein Wasserstoffatom, ein Halogenatom, eine lineare, verzweigte oder cyclische Kohlenwasserstoffkette mit 1 bis 6 Kohlenstoffatomen, $CF_3$, CN, CO-$R_2$, $OR_2$ oder eine Phenylgruppe, eventuell substituiert durch eine lineare, verzweigte oder cyclische Kohlenwasserstoffkette mit 1 bis 4 Kohlenstoffatomen oder $CF_3$ darstellen,
$R_2$ eine lineare, verzweigte oder cyclische Kohlenwasserstoffkette mit 1 bis 4 Kohlenstoffatomen, $CF_3$ oder Phenyl, eventuell substituiert durch eine lineare, verzweigte oder cyclische Kohlenwasserstoffkette mit 1 bis 4 Kohlenstoffatomen oder $CF_3$ darstellt,
$R_3$ und $R_4$ jeweils unabhängig ein Wasserstoffatom oder eine lineare, verzweigte oder cyclische Kohlenwasserstoffkette mit 1 bis 4 Kohlenstoffatomen darstellen,
R ein Wasserstoffatom oder eine lineare, verzweigte oder cyclische Kohlenwasserstoffkette mit 1 bis 3 Kohlenstoffatomen darstellt,
n = 1, 2 oder 3,
X eine einfache Bindung, ein Sauerstoffatom oder ein Schwefelatom darstellt,
Ar einen aromatischen oder heteroaromatischen Kern darstellt, ausgewählt aus den Gruppen Phenyl, Naphtyl, Chinolinyl, Isochinolinyl, Pyridinyl, Pyrazolyl, Imidazolyl, Isoxazolyl, Thiazolyl, Benzimidazolyl, Benzothiazolyl, 2,1,3-Benzothiadiazolyl, 3,4-Dihydro-1,4-benzoxazinyl, 5,6,7,8-Tetrahydronaphthalenyl, 1,2,3,4-Tetrahydrochinolinyl, 1,2,3,4-Tetrahydroisochinolinyl, 1,2,3,4-Tetrahydro-2-oxochinolinyl, 3,4-Dihydro-2H-benzopyranyl, Indolyl, 2,3-Dihydroindolyl, Benzofuranyl, 2,3-Dihydrobenzofuranyl, 1,3-Benzodioxolyl, 1,4-Benzodioxanyl oder Benzoxazolyl, eventuell substituiert durch ein(e) oder mehrere der Atome oder Atomgruppen, ausgewählt unter den Halogenatomen, linearen, verzweigten oder cyclischen Kohlenwasserstoffketten mit 1 bis 6 Kohlenstoffatomen, Phenyl, $CF_3$, CN, CO-$R_2$, $OR_2$, $SR_2$, NH-COR$_2$, Morpholinyl, Amino oder 4-Morpholinsulfonyl,

sowie
5-Chlor-1-[(4-fluor-3-nitrophenyl)sulfonyl]-1H-indol-2-Butansäure, Methylester,
1[(4-Amino-3-nitrophenyl)sulfonyl]-5-chlor-1H-indol-2-Butansäure, Methylester,

ii) ihren pharmazeutisch akzeptablen Salzen.

2. Verbindung nach Anspruch 1, **dadurch gekennzeichnet, daß** wenigstens einer der Substituenten $R_a$ und $R_b$ kein Wasserstoffatom ist.

3. Verbindung nach einem der Ansprüche 1 oder 2, **dadurch gekennzeichnet, daß** Ar eine Phenyl-Gruppe oder heteroaromatische Stickstoff-Gruppe darstellt.

4. Verbindung nach einem der Ansprüche 1 bis 3, **dadurch gekennzeichnet, daß** n gleich 1 oder 2 ist.

5. Verbindung nach einem der Ansprüche 1 bis 4, für ihre Verwendung als pharmakologisch aktive Substanz.

6. Verwendung einer Verbindung nach einem der Ansprüche 1 bis 4, für die Herstellung eines Medikaments, welches für die Behandlung von Hypertriglyzeridämien, Hyperlipidämien, Hypercholesterolämien, Fettleibigkeit und Diabetes bestimmt ist.

7. Verwendung einer Verbindung nach einem der Ansprüche 1 bis 4, für die Herstellung eines Medikaments, welches für die Behandlung von endothelialer Funktionsstörung bestimmt ist.

8. Verwendung einer Verbindung nach einem der Ansprüche 1 bis 4, für die Herstellung eines Medikaments, welches für die Behandlung von Herz-Kreislauf-Erkrankungen, Entzündungserkrankungen und neurodegenerativen Erkrankungen, wie insbesondere der Alzheimer-Krankheit oder der Parkinson-Krankheit bestimmt ist.

9. Pharmazeutische Zusammensetzung, **dadurch gekennzeichnet, daß** sie wenigstens eine Verbindung nach einem der Ansprüche 1 bis 4 als Wirkstoff enthält.

10. Verfahren zur Herstellung einer Verbindung nach Anspruch 1, **dadurch gekennzeichnet, daß** es die Schritte umfaßt, die darin bestehen:

a) eine Verbindung der Formel

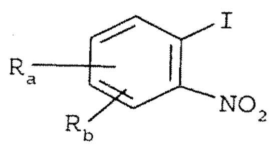

(II)

worin:

$R_a$ und $R_b$ jeweils unabhängig ein Wasserstoff-, Fluor-, Chlor- oder Bromatom, eine lineare, verzweigte oder cyclische Kohlenwasserstoffkette mit 1 bis 6 Kohlenstoffatomen, $CF_3$, CN, CO-$R_2$ oder $OR_2$ darstellen,
$R_2$ eine lineare, verzweigte oder cyclische Kohlenwasserstoffkette mit 1 bis 4 Kohlenstoffatomen, $CF_3$ oder Phenyl, eventuell substituiert durch eine lineare, verzweigte oder cyclische Kohlenwasserstoffkette mit 1 bis 4 Kohlenstoffatomen oder $CF_3$ darstellt,
nach der sogenannten SONOGASHIRA-Reaktion mit einem Acetylenderivat folgender Formel reagieren zu lassen

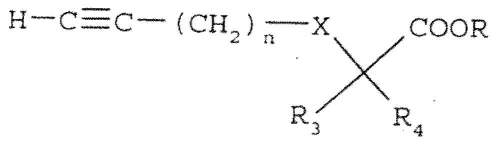

(III)

worin:

n = 1, 2 oder 3,
$R_3$ und $R_4$ jeweils unabhängig ein Wasserstoffatom oder eine lineare, verzweigte oder cyclische Kohlenwasserstoffkette mit 1 bis 4 Kohlenstoffatomen darstellen,
R eine lineare, verzweigte oder cyclische Kohlenwasserstoffkette mit 1 bis 3 Kohlenstoffatomen darstellt,
X eine einfache Bindung, ein Sauerstoffatom oder ein Schwefelatom darstellt,
und zwar in Anwesenheit von Kupfer(I)-iodid, einem Katalysator auf Palladiumbasis, wie zum Beispiel Tetrakis(triphenylphosphin)palladium, und einer organischen Base, in einem Lösungsmittel, bei einer Temperatur zwischen 0 und 60°C für 2 bis 24 Stunden, um die Verbindung folgender Formel zu erhalten

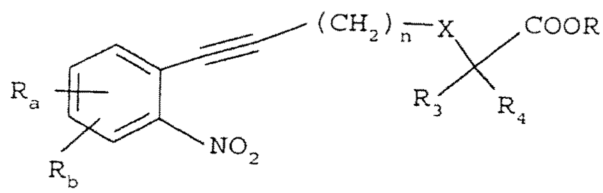

(IV)

worin:

$R_a$, $R_b$, n, X, $R_3$, $R_4$ und R die gleiche Bedeutung wie bei den Ausgangsverbindungen behalten,

b) eine Reduktion der von der Verbindung der obigen Formel IV getragenen "Nitro"-Gruppe zu vollziehen, beispielsweise durch Wirkung von Zinn(II)-chlorid in Anwesenheit von Ethanol und in einem Lösungsmittel, bei einer Temperatur nahe der Raumtemperatur und für 1 bis 24 Stunden, um das Anilin folgender Formel zu erhalten

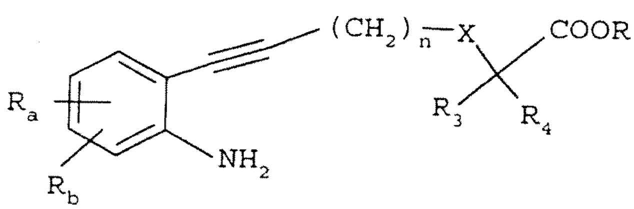

(V)

worin:

$R_a$, $R_b$, n, X, $R_3$, $R_4$ und R die gleiche Bedeutung wie bei der Ausgangsverbindung behalten,

c) die Verbindung der Formel V mit einem Arylsulfonylchlorid folgender Formel reagieren zu lassen

$$Ar-SO_2-Cl \quad (VI)$$

worin:

Ar einen aromatischen oder heteroaromatischen Kern darstellt, ausgewählt aus den Gruppen Phenyl, Naphtyl, Chinolinyl, Isochinolinyl, Pyridinyl, Pyrazolyl, Imidazolyl, Isoxazolyl, Thiazolyl, Benzimidazolyl, Benzothiazolyl, 2,1,3-Benzothiadiazolyl, 3,4-Dihydro-1,4-benzoxazinyl, 5,6,7,8-Tetrahydronaphthalenyl, 1,2,3,4-Tetrahydrochinolinyl, 1,2,3,4-Tetrahydroisochinolinyl, 1,2,3,4-Tetrahydro-2-oxochinolinyl, 3,4-Dihydro-2H-benzopyranyl, Indolyl, 2,3-Dihydroindolyl, Benzofuranyl, 2,3-Dihydrobenzofuranyl, 1,3-Benzodioxolyl, 1,4-Benzodioxanyl oder Benzoxazolyl, eventuell substituiert durch ein(e) oder mehrere der Atome oder Atomgruppen, ausgewählt unter den Halogenatomen, linearen, verzweigten oder cyclischen Kohlenwasserstoffketten mit 1 bis 6 Kohlenstoffatomen, Phenyl, $CF_3$, CN, CO-$R_2$, $OR_2$, $SR_2$, NH-$COR_2$, Morpholinyl, Amino oder 4-Morpholinsulfonyl,

und zwar in Anwesenheit von Pyridin, bei Raumtemperatur, für 10 bis 120 Min, um die Verbindung folgender Formel zu erhalten

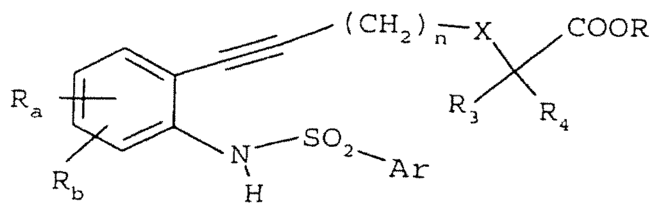

(VII)

worin:

$R_a$, $R_b$, n, X, $R_3$, $R_4$, R und Ar die gleiche Bedeutung wie bei den Ausgangsverbindungen behalten,

d) eine Cyclisierung der Verbindung der Formel VII zu vollziehen, beispielsweise durch Wirkung von Kupfer (II)-acetat, in einem Lösungsmittel, bei einer Temperatur nahe der Rückflußtemperatur des Lösungsmittels, für 4 bis 24 Stunden, um die Verbindung der folgenden Formel zu erhalten

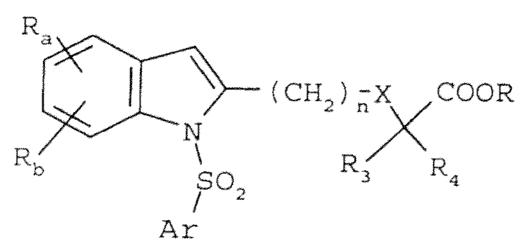

(Ia)

worin:

$R_a$, $R_b$, n, X, $R_3$, $R_4$, R und Ar die gleiche Bedeutung wie bei den Ausgangsverbindungen behalten,

e) falls erforderlich die Esterfunktion der Verbindung der Formel Ia zu hydrolysieren, um nach der Säurebehandlung die Verbindung der Formel I in ihrer Form einer freien Säure zu erhalten:

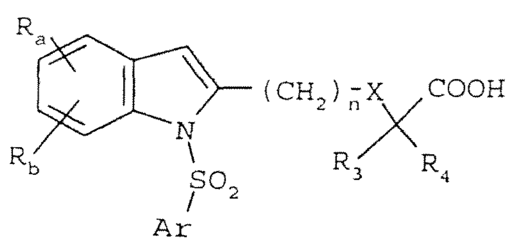

(Ib)

**11.** Verfahren zur Herstellung einer Verbindung nach Anspruch 1, **dadurch gekennzeichnet, daß** es die Schritte umfaßt, die darin bestehen:

a) eine Halogenierungs-Reaktion, vorzugsweise eine Jodierung, eines Anilins folgender Formel durchzuführen

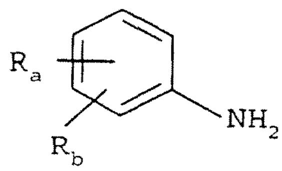

(VIII)

worin:

$R_a$ und $R_b$ jeweils unabhängig ein Wasserstoffatom, ein Halogenatom, eine lineare, verzweigte oder cyclische Kohlenwasserstoffkette mit 1 bis 6 Kohlenstoffatomen, $CF_3$,
CN, CO-$R_2$ oder $OR_2$ darstellen,
$R_2$ eine lineare, verzweigte oder cyclische Kohlenwasserstoffkette mit 1 bis 4 Kohlenstoffatomen, $CF_3$ oder Phenyl, eventuell substituiert durch eine lineare, verzweigte oder cyclische Kohlenwasserstoffkette mit 1 bis 4 Kohlenstoffatomen oder $CF_3$ darstellt,
und zwar mit Hilfe eines Halogenierungsmittels, wie zum Beispiel Benzyltrimethylammonium-dichloriodat, in einem Lösungsmittel wie Dichlormethan oder Methanol, bei Raumtemperatur, für 5 bis 24 Stunden, um die Verbindung folgender Formel zu erhalten

worin:

$R_a$ und $R_b$ die gleiche B

b) die Verbindung

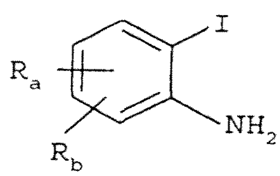

verbindungen behalten, (IX)derivat folgender Formel reagieren zu lassen

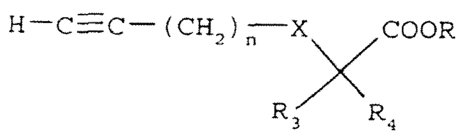

(III)

worin:

n = 1, 2 oder 3,
$R_3$ und $R_4$ jeweils unabhängig ein Wasserstoffatom oder eine lineare, verzweigte oder cyclische Kohlenwasserstoffkette mit 1 bis 4 Kohlenstoffatomen darstellen,
R eine lineare, verzweigte oder cyclische Kohlenwasserstoffkette mit 1 bis 3 Kohlenstoffatomen darstellt,
X eine einfache Bindung, ein Sauerstoffatom oder ein Schwefelatom darstellt,
und zwar unter ähnlichen Bedingungen wie sie für Schritt a) des in Anspruch 10 erläuterten Verfahrens

beschrieben sind,
um die Verbindung folgender Formel zu erhalten

worin:

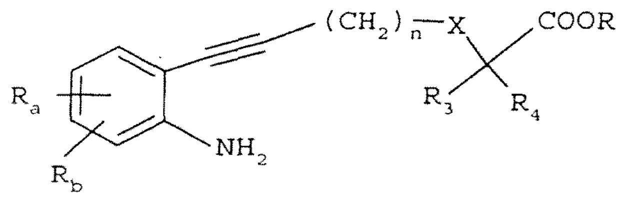

(V)

$R_a$, $R_b$, n, X, $R_3$, $R_4$ und R die gleiche Bedeutung wie bei der Ausgangsverbindung behalten,

c) die Verbindung der obigen Formel V unter ähnlichen Bedingungen zu cyclisieren wie sie für die Durchführung des Schrittes (d) des in Anspruch 10 erläuterten Verfahrens beschrieben sind, um die Indolverbindung folgender Formel zu erhalten

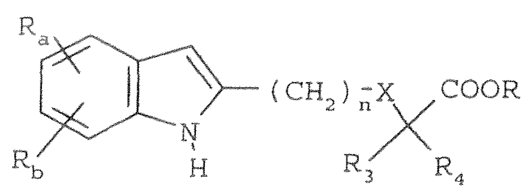

(X)

worin:

$R_a$, $R_b$, n, X, $R_3$, $R_4$ und R die gleiche Bedeutung wie bei der Ausgangsverbindung behalten,

d) die Verbindung der obigen Formel (X) mit einem Arylsulfonylchlorid folgender Formel reagieren zu lassen

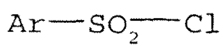

(VI)

worin:

Ar einen aromatischen oder heteroaromatischen Kern darstellt, ausgewählt aus den Gruppen Phenyl, Naphtyl, Chinolinyl, Isochinolinyl, Pyridinyl, Pyrazolyl, Imidazolyl, Isoxazolyl, Thiazolyl, Benzimidazolyl, Benzothiazolyl, 2,1,3-Benzothiadiazolyl, 3,4-Dihydro-1,4-benzoxazinyl, 5,6,7,8-Tetrahydronaphthalenyl, 1,2,3,4-Tetrahydrochinolinyl, 1,2,3,4-Tetrahydroisochinolinyl, 1,2,3,4-Tetrahydro-2-oxochinolinyl, 3,4-Dihydro-2H-benzopyranyl, Indolyl, 2,3-Dihydroindolyl, Benzofuranyl, 2,3-Dihydrobenzofuranyl, 1,3-Benzodioxolyl, 1,4-Benzodioxanyl oder Benzoxazolyl, eventuell substituiert durch ein(e) oder mehrere der Atome oder Atomgruppen, ausgewählt unter den Halogenatomen, linearen, verzweigten oder cyclischen Kohlenwasserstoffketten mit 1 bis 6 Kohlenstoffatomen, Phenyl, $CF_3$, CN, CO-$R_2$, $OR_2$, $SR_2$, NH-$COR_2$, Morpholinyl, Amino oder 4-Morpholinsulfonyl, und zwar in einem Lösungsmittel, bei Raumtemperatur, für 1 bis 12 Stunden, im allgemeinen nach Aktivierung der Indolverbindungen der Formel (X) durch Natriumhydrid, um die Verbindung der Formel (Ia) zu erhalten

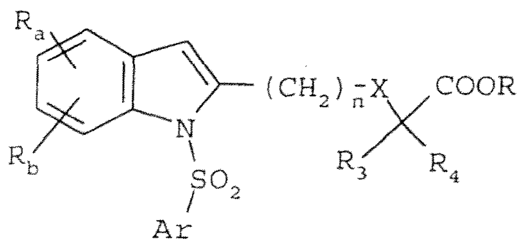

(Ia)

worin:

$R_a$, $R_b$, n, X, $R_3$, $R_4$, R und Ar die gleiche Bedeutung wie bei den Ausgangsverbindungen behalten,

c) falls erforderlich die Esterfunktion der Verbindung der Formel Ia zu hydrolysierten, beispielsweise durch Wirkung einer mineralischen Base, wie Natriumcarbonat oder Lithiumhydroxid, nach seitens des Fachmannes wohl bekannten Verfahrensweisen, um nach der Säurebehandlung die Verbindung der Formel I in ihrer Form einer freien Säure

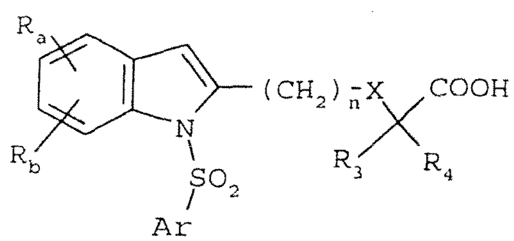

(Ib)

**12.** Verfahren zur Herstellung einer Verbindung nach Anspruch 1, **dadurch gekennzeichnet, daß** es die Schritte umfaßt, die darin bestehen:

a) die Verbindung der Formel IX

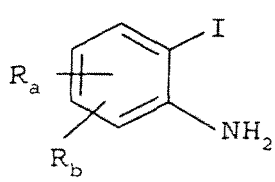

(IX)

worin:

$R_a$ und $R_b$ jeweils unabhängig ein Wasserstoff-, Fluor-, Chlor- oder Bromatom, eine lineare, verzweigte oder cyclische Kohlenwasserstoffkette mit 1 bis 6 Kohlenstoffatomen, $CF_3$, CN, CO-$R_2$ oder $OR_2$ darstellen, $R_2$ eine lineare, verzweigte oder cyclische Kohlenwasserstoffkette mit 1 bis 4 Kohlenstoffatomen, $CF_3$ oder Phenyl, eventuell substituiert durch eine lineare, verzweigte oder cyclische Kohlenwasserstoffkette mit 1 bis 4 Kohlenstoffatomen oder $CF_3$ darstellt,
mit einem Arylsulfonylchlorid folgender Formel reagieren zu lassen

$Ar-SO_2-Cl$ (VI)

worin:

Ar einen aromatischen oder heteroaromatischen Kern darstellt, ausgewählt aus den Gruppen Phenyl, Naphtyl, Chinolinyl, Isochinolinyl, Pyridinyl, Pyrazolyl, Imidazolyl, Isoxazolyl, Thiazolyl, Benzimidazolyl, Benzothiazolyl, 2,1,3-Benzothiadiazolyl, 3,4-Dihydro-1,4-benzoxazinyl, 5,6,7,8-Tetrahydronaphthalenyl, 1,2,3,4-Tetrahydrochinolinyl, 1,2,3,4-Tetrahydroisochinolinyl, 1,2,3,4-Tetrahydro-2-oxochinolinyl, 3,4-Dihydro-2H-benzopyranyl, Indolyl, 2,3-Dihydroindolyl, Benzofuranyl, 2,3-Dihydrobenzofuranyl, 1,3-Benzodioxolyl, 1,4-Benzodioxanyl oder Benzoxazolyl, eventuell substituiert durch ein(e) oder mehrere der Atome oder Atomgruppen, ausgewählt unter den Halogenatomen, linearen, verzweigten oder cyclischen Kohlenwasserstoffketten mit 1 bis 6 Kohlenstoffatomen, Phenyl, $CF_3$, CIV, CO-$R_2$, $OR_2$, $SR_2$, NH-$COR_2$, Morpholinyl, Amino oder 4-Morpholinsulfonyl, und zwar in einem Lösungsmittel, bei Raumtemperatur, für 1 bis 12 Stunden, um die Verbindung folgender Formel zu erhalten

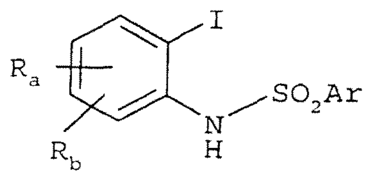

(XI)

worin:

$R_a$, $R_b$ und Ar die gleiche Bedeutung wie bei den Ausgangsverbindungen behalten,

b) die Verbindung der Formel XI mit einem Acetylenderivat folgender Formel reagieren zu lassen

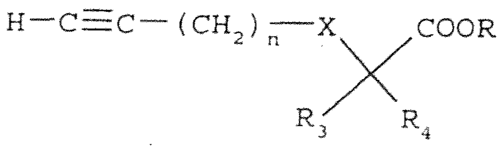

(III)

worin

n = 1, 2 oder 3,
$R_3$ und $R_4$ jeweils unabhängig ein Wasserstoffatom oder eine lineare, verzweigte oder cyclische Kohlenwasserstoffkette mit 1 bis 4 Kohlenstoffatomen darstellen,
R eine lineare, verzweigte oder cyclische Kohlenwasserstoffkette mit 1 bis 3 Kohlenstoffatomen darstellt,
X eine einfache Bindung, ein Sauerstoffatom oder ein Schwefelatom darstellt,
und zwar unter ähnlichen Bedingungen wie sie für Schritt a) des in Anspruch 10 erläuterten Verfahrens beschrieben sind, um die Verbindung folgender Formel zu erhalten

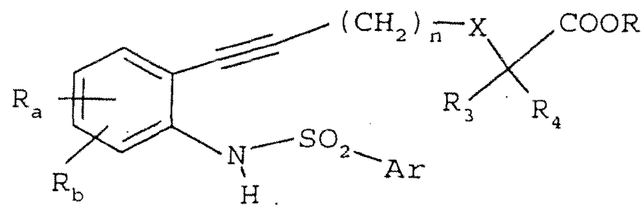

(VII)

worin:

$R_a$, $R_b$, n, X, $R_3$, $R_4$, R und Ar die gleiche Bedeutung wie bei den Ausgangsverbindungen behalten,

c) die Verbindung der obigen Formel VII unter ähnlichen Bedingungen zu cyclisieren wie sie für die Durchführung des Schrittes (d) des in Anspruch 10 erläuterten Verfahrens beschrieben sind, um die Indolverbindung folgender Formel zu erhalten

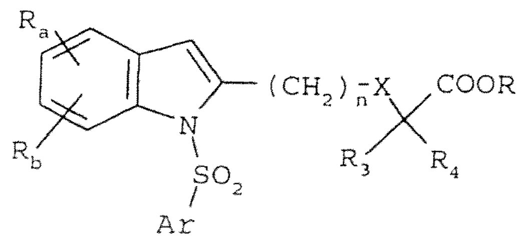

(Ia)

worin:

$R_a$, $R_b$, n, X, $R_3$, $R_4$, R und Ar die gleiche Bedeutung wie bei den Ausgangsverbindungen behalten,

d) falls erforderlich die Esterfunktion der Verbindung der Formel Ia zu hydrolysieren, beispielsweise durch Wirkung einer mineralischen Base, wie Natriumcarbonat oder Lithiumhydroxid, nach seitens des Fachmannes wohl bekannten Verfahrensweisen, um nach der Säurebehandlung die Verbindung der Formel I in ihrer Form einer freien Säure zu erhalten:

**13.** Verfahren nach Anspruch 12 zur Herstellung einer Verbindung nach Anspruch 1, **dadurch gekennzeichnet, daß** die beiden Schritte b) und c) in einem einzigen Arbeitsschritt durchgeführt werden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9728149 A
- WO 0160807 A
- WO 05009958 A
- WO 06060535 A
- WO 02071827 A
- WO 0046196 A
- WO 9907678 A
- WO 9841092 A


### Littérature non-brevet citée dans la description


- *FEBS letters,* 2000, vol. 473, 333-336
- *Proc. Nat. Ac. Sci. USA,* 2001, vol. 98, 5306-5311
- *Bioorg. Med. Chem. Lett.,* Juin 2004, vol. 14 (11), 2759-2763
- *Tet. Lett.,* 1975, 4467
- *J. Org. Chem.,* 2004, vol. 69 (4), 1126-1136